# EUROPEAN PATENT APPLICATION

(11) **EP 4 648 166 A1**
(43) Date of publication of application: **12.11.2025**
(21) Application number: 24753269.0
(22) Date of filing: 02.02.2024
(51) Int. Cl.: H01M 10/0567, C07D 251/34, H01M 10/052, H01M 10/054, H01M 10/0568, H01M 10/0569

(54) **NONAQUEOUS ELECTROLYTE, NONAQUEOUS ELECTROLYTE BATTERY, AND COMPOUND**

(30) Priority: 06.02.2023 JP 2023016464
(71) Applicant: Central Glass Company, Limited, Ube-shi, Yamaguchi 755-0001 (JP)
(72) Inventor: KAWABATA, Wataru, Tokyo 101-0054 (JP); TAKAHASHI, Mikihiro, Tokyo 101-0054 (JP); MORINAKA, Takayoshi, Tokyo 101-0054 (JP); NAKAHARA, Keita, Tokyo 101-0054 (JP); MIURA, Masahiro, Tokyo 101-0054 (JP)
(74) Representative: Manitz Finsterwald Patent- und Rechtsanwaltspartnerschaft mbB
(86) International application number: PCT/JP2024/003559
(87) International publication number: WO 2024/166826

(57) **Abstract**

A nonaqueous electrolyte solution capable of exhibiting an excellent initial input-output characteristic when used for a nonaqueous electrolyte solution battery, a nonaqueous electrolyte solution battery capable of exhibiting an excellent initial input-output characteristic, and a compound suitably used for the nonaqueous electrolyte solution are provided. A nonaqueous electrolyte solution containing (I) at least one compound selected from the group consisting of a compound represented by the general formula (1), a compound represented by the general formula (2), a compound represented by the general formula (3), and a compound represented by the general formula (4) described in the specification, (II) a solute, and (III) a nonaqueous organic solvent, a nonaqueous electrolyte solution battery containing the nonaqueous electrolyte solution, and the compound represented by any of the general formulae (1) to (4) described in the specification.

## Description

### TECHNICAL FIELD

The present disclosure relates to a nonaqueous electrolyte solution, a nonaqueous electrolyte solution battery, and a compound.

### BACKGROUND ART

In recent years, demand for batteries having a high capacity, high output, and a high energy density which can be installed as power storage systems for information-related devices and communication devices, namely, compact and high-energy-density applications such as personal computers, video cameras, digital cameras, mobile phones, and smartphones and as auxiliary power sources for electric vehicles, hybrid vehicles, and fuel cell vehicles has been rapidly increasing. In addition, demand for batteries that can be used for a long period of time even in power storage systems for large-scale power applications such as power storages is increasing. Nonaqueous electrolyte solution batteries such as lithium ion batteries, lithium batteries, and lithium ion capacitors have been actively developed as candidates for these power storage systems.

A lithium secondary battery is mainly composed of a positive electrode, a nonaqueous electrolyte solution, and a negative electrode. As the negative electrode constituting the lithium secondary battery, for example, metal lithium, a metal compound capable of occluding and releasing lithium (for example, simple metal element, an oxide, an alloy with lithium, or the like), a carbon material, and the like are known, and in particular, a lithium secondary battery using a carbon material such as coke, artificial graphite, and natural graphite capable of occluding and releasing lithium has been widely put into practical use. For example, it has been reported that, in a lithium secondary battery in which a highly crystallized carbon material such as natural graphite and artificial graphite is used as a negative electrode material, since the nonaqueous organic solvent in the nonaqueous electrolyte solution is reductively decomposed on the surface of the negative electrode during charging, the decomposition product or a gas generated thereby inhibits the original electrochemical reaction of the battery, and thus the cycle characteristic is deteriorated.

In addition, it is known that reductive decomposition of the nonaqueous organic solvent is caused more easily in a lithium secondary battery using lithium metal, an alloy thereof, a simple metal element of silicon, tin, or the like, an oxide thereof, or the like as a negative electrode material than in a negative electrode of a carbon material because pulverization of the negative electrode material proceeds during the cycles although the initial capacity is high, resulting in a decrease in the charge/discharge efficiency in the first cycle due to an increase in the initial irreversible capacity of the battery and a considerable decrease in the battery performance such as the battery capacity and the cycle characteristic associated therewith.

When lithium cations are inserted into the negative electrode during the first cycle charging, the negative electrode and the lithium cations or the negative electrode and the electrolyte solution solvent react with each other to form a film containing lithium oxide, lithium carbonate, or lithium alkyl carbonate as a main component on the surface of the negative electrode. The film on the surface of the electrode is called solid electrolyte interface (SEI), and the properties thereof greatly affect the battery performance, through suppression of reductive decomposition of the solvent, suppression of deterioration of the battery performance, or the like.

As described above, occlusion and release of lithium to and from the negative electrode cannot be smoothly performed due to accumulation of the decomposition product of the nonaqueous organic solvent, generation of gas, the adverse effect of the pulverization of the negative electrode material, and the like, and as a result, there is a problem of a significant decrease in the battery characteristics such as the cycle characteristic.

As the positive electrode, for example, LiCoO₂, LiMn₂O₄, LiNiO₂, LiFePO₄, and the like are known. It has been reported that, in a lithium secondary battery using these, when the temperature becomes high in the charged state, oxidative decomposition of the nonaqueous organic solvent occurs locally and partially at the interface between the positive electrode material and the nonaqueous electrolyte solution. The decomposition products or gases generated thereby inhibit the original electrochemical reaction of the battery, resulting in decreased battery performance, such as cycle characteristics. Similar to the negative electrode, it is known that a film made of an oxidative decomposition product is also formed on the surface of the positive electrode, and the film also plays an important role such as suppressing oxidative decomposition of the solvent and suppressing the amount of gas generated.

As described above, general lithium secondary batteries have factors which decrease the battery performance through inhibition of migration of lithium ions or swelling of the battery due to the decomposition products or the gases generated when the nonaqueous electrolyte solution is decomposed on the positive electrode or on the negative electrode.

In addition to overcoming these problems, in order to improve the battery performance including long-term durability and an output characteristic, it is important to form a SEI which has high ion conductivity and low electron conductivity and which is stable over a long period of time, and attempts have been widely made to actively form an excellent SEI by adding a small amount (usually 0.01% by mass or more and 10% by mass or less) of a compound referred to as an additive to the nonaqueous electrolyte solution.

Patent Literature 1 discloses that the storage characteristic of a battery is improved using a nonaqueous electrolyte solution containing a tricarboimide compound such as triallyl cyanurate and triallyl isocyanurate.

Patent Literature 2 discloses that the initial charge/discharge capacity, the input-output characteristic, and the impedance characteristic are improved using a nonaqueous electrolyte solution containing a fluorosulfonate.

Patent Literature 3 discloses that the battery characteristics such as the cycle characteristic and the high-temperature storage characteristic are improved using a nonaqueous electrolyte solution containing a compound having a cyanuric acid skeleton and at least one compound selected from the group consisting of a halogenated cyclic carbonate compound, a difluorophosphate, and a monofluorosulfonate.

Patent Literature 4 discloses a heterocyclic sulfonyl fluoride additive having a predetermined structure for an electrolyte composition for a lithium battery and discloses that an electrolyte composition containing the additive has excellent electrochemical characteristics, for example, a long cycle life, a high capacity retention rate, accumulation of low resistance during cycling, and excellent storage stability. In particular, in the Examples of Patent Literature 4, 3,3',3"-(2,4,6-trioxo-[1,3,5]-triazinane-1,3,5-triyl)-tris-ethanesulfonyl fluoride is used as the additive.

### CITATION LIST

### PATENT LITERATURE

Patent Literature 1: JPH07-192757A
Patent Literature 2: JP2013-152956A
Patent Literature 3: JP2014-063733A
Patent Literature 4: JP2020-527284A

### SUMMARY OF INVENTION

### TECHNICAL PROBLEM

However, as a result of studies by the present disclosers, it has been found that, when a nonaqueous electrolyte solution containing a tricarboimide compound such as triallyl cyanurate and triallyl isocyanurate is used as the nonaqueous electrolyte solution as in Patent Literature 1, the initial input-output characteristic is significantly decreased. In addition, it has been found that there is room for improvement in the initial input-output characteristic also when the lithium fluorosulfonate-containing nonaqueous electrolyte solution described in Patent Literature 2, a nonaqueous electrolyte solution containing a compound having a cyanuric acid skeleton and a compound such as a monofluorosulfonate as in Patent Literature 3, and a nonaqueous electrolyte solution containing the additive of Patent Literature 4 are used.

An object of the present disclosure is to provide a nonaqueous electrolyte solution capable of exhibiting an excellent initial input-output characteristic when used for a nonaqueous electrolyte solution battery, a nonaqueous electrolyte solution battery capable of exhibiting an excellent initial input-output characteristic, and a compound suitably used for the nonaqueous electrolyte solution.

### SOLUTION TO PROBLEM

In view of such problems, the present disclosers have intensively studied and found that a nonaqueous electrolyte solution battery capable of exhibiting an excellent initial input-output characteristic is obtained using a nonaqueous electrolyte solution containing (I) at least one compound selected from the group consisting of a compound represented by the general formula (1), a compound represented by the general formula (2), a compound represented by the general formula (3), and a compound represented by the general formula (4) (hereinafter sometimes referred to as "component (I)"), (II) a solute (hereinafter sometimes referred to as "component (II)"), and (III) a nonaqueous organic solvent (hereinafter sometimes referred to as "component (III)"). The problems have been thus solved.

That is, the present disclosers have found that the above problems can be solved by the following configurations.
[1] A nonaqueous electrolyte solution, containing:
   (I) at least one compound selected from the group consisting of a compound represented by the following general formula (1), a compound represented by the following general formula (2), a compound represented by the following general formula (3), and a compound represented by the following general formula (4);
   (II) a solute; and
   (III) a nonaqueous organic solvent. 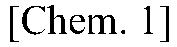 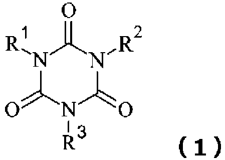
      [In the general formula (1), R¹ to R³ may be the same or different from each other and are a hydrogen atom or an organic group having 1 to 20 carbon atoms which may have a substituent. Here, at least one of R¹ to R³ has a group represented by the general formula (5).] 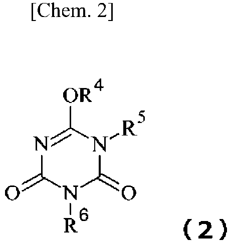
      [In the general formula (2), R⁴ to R⁶ may be the same or different from each other and are a hydrogen atom or an organic group having 1 to 20 carbon atoms which may have a substituent. Here, at least one of R⁴ to R⁶ has a group represented by the general formula (5).] 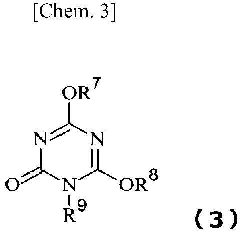
      [In the general formula (3), R⁷ to R⁹ may be the same or different from each other and are a hydrogen atom or an organic group having 1 to 20 carbon atoms which may have a substituent. Here, at least one of R⁷ to R⁹ has a group represented by the general formula (5).] 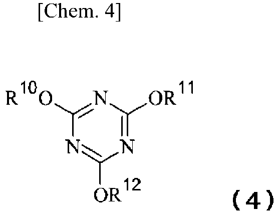
      [In the general formula (4), R¹⁰ to R¹² may be the same or different from each other and are a hydrogen atom or an organic group having 1 to 20 carbon atoms which may have a substituent. Here, at least one of R¹⁰ to R¹² has a group represented by the general formula (5).] 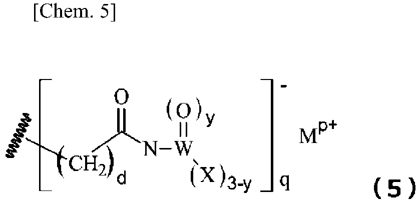
      [In the general formula (5), W represents a phosphorus atom or a sulfur atom, and y is l when W is a phosphorus atom and y is 2 when W is a sulfur atom. X is each independently a halogen atom, an alkyl group having 1 to 20 carbon atoms which may be substituted with a halogen atom, a cycloalkyl group having 5 to 20 carbon atoms which may be substituted with a halogen atom, an alkenyl group having 2 to 20 carbon atoms which may be substituted with a halogen atom, an alkynyl group having 2 to 20 carbon atoms which may be substituted with a halogen atom, an aryl group having 6 to 40 carbon atoms which may be substituted with a halogen atom, a heteroaryl group having 2 to 40 carbon atoms which may be substituted with a halogen atom, an alkoxy group having 1 to 20 carbon atoms which may be substituted with a halogen atom, a cycloalkoxy group having 5 to 20 carbon atoms which may be substituted with a halogen atom, an alkenyloxy group having 2 to 20 carbon atoms which may be substituted with a halogen atom, an alkynyloxy group having 2 to 20 carbon atoms which may be substituted with a halogen atom, an aryloxy group having 6 to 40 carbon atoms which may be substituted with a halogen atom, or a heteroaryloxy group having 2 to 40 carbon atoms which may be substituted with a halogen atom. d represents 0 to 5. M^{p+} represents a proton, a metal cation, or an onium cation, and p represents a valence of the cation. q represents a number satisfying p × q = 1.]
[2] The nonaqueous electrolyte solution according to [1], in which X in the general formula (5) is each independently a fluorine atom, a methyl group, an ethyl group, an n-propyl group, an isopropyl group, an n-butyl group, a tert-butyl group, an n-pentyl group, an n-hexyl group, a trifluoromethyl group, a trifluoroethyl group, an ethenyl group, a 2-propenyl group, a 2-propynyl group, a phenyl group, a naphthyl group, a pentafluorophenyl group, a methoxy group, an ethoxy group, an n-propoxy group, an isopropoxy group, an n-butoxy group, a tert-butoxy group, an n-pentyloxy group, an n-hexyloxy group, a trifluoromethoxy group, a trifluoroethoxy group, a hexafluoroisopropoxy group, an ethenyloxy group, a 2-propenyloxy group, a 2-propynyloxy group, a phenoxy group, a naphthyloxy group, a pentafluorophenoxy group, a pyrrolyl group, or a pyridinyl group.
[3] The nonaqueous electrolyte solution according to [1], in which X in the general formula (5) is each independently a fluorine atom, a methyl group, an ethyl group, an n-propyl group, an isopropyl group, an n-butyl group, a tert-butyl group, a trifluoromethyl group, a phenyl group, a naphthyl group, a pentafluorophenyl group, a pyrrolyl group, or a pyridinyl group.
[4] The nonaqueous electrolyte solution according to any one of [1] to [3], in which M^{p+} in the general formula (5) is a proton, a lithium cation, a sodium cation, a potassium cation, a tetraalkylammonium cation, or a tetraalkylphosphonium cation.
[5] The nonaqueous electrolyte solution according to any one of [1] to [4], in which the concentration of the (I) is 0.01 to 5.00% by mass with respect to the total amount of the nonaqueous electrolyte solution.
[6] The nonaqueous electrolyte solution according to any one of [1] to [5], in which the (II) is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.
[7] The nonaqueous electrolyte solution according to any one of [1] to [6], in which the (III) contains at least one selected from the group consisting of a cyclic ester, a chain ester, a cyclic ether, a chain ether, a sulfone compound, a sulfoxide compound, and an ionic liquid.
[8] The nonaqueous electrolyte solution according to [7], in which the cyclic ester contains a cyclic carbonate.
[9] The nonaqueous electrolyte solution according to [8], in which the cyclic carbonate contains at least one selected from the group consisting of ethylene carbonate, propylene carbonate, and fluoroethylene carbonate.
[10] The nonaqueous electrolyte solution according to [7], in which the chain ester contains a chain carbonate.
[11] The nonaqueous electrolyte solution according to [10], in which the chain carbonate contains at least one selected from the group consisting of ethyl methyl carbonate, dimethyl carbonate, diethyl carbonate, and methyl propyl carbonate.
[12] The nonaqueous electrolyte solution according to any one of [1] to [11], further containing at least one selected from the group consisting of vinylene carbonate, bis(oxalato)borate, difluorooxalatoborate, difluorobis(oxalato)phosphate, tetrafluorooxalato phosphate, (difluorophosphoryl)(fluorosulfonyl)imide salt, difluorophosphate, fluorosulfonate, nitrate, 1,3-propenesultone, 1,3-propanesultone, 1,6-diisocyanatohexane, dimethyl dicarbonate, ethynyl ethylene carbonate, trans-difluoroethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonic anhydride, methanesulfonyl fluoride, 1,4-dioxane-2,6-dione, tripropagyl phosphate, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, N,N'-carbonylbis(N-methylsulfamoylfluoride), tetravinylsilane, trivinylmethylsilane, t-butylbenzene, t-amylbenzene, fluorobenzene, and cyclohexylbenzene.
[13] The nonaqueous electrolyte solution according to any one of [1] to [12], further containing (IV) at least one selected from the group consisting of a compound represented by the following general formula [1b] and a compound represented by the following general formula [1b'], in which a content of the (IV) in the nonaqueous electrolyte solution is 10 to 25000 ppm by mass. 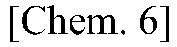 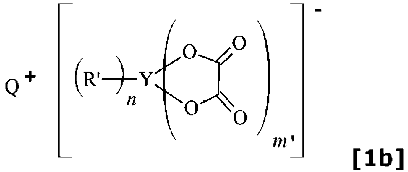
   (In the general formula [1b], Y represents a boron atom, and R' represents a fluorine atom. n is 0 to 4, and m' is 0 to 2. Q⁺ represents an alkali metal ion, a tetraalkylammonium cation, or a tetraalkylphosphonium cation.)

      Q⁺[Z]⁻ [1b']
   (In the general formula [1b'], the anion moiety represented by [Z]⁻ is the following structure [1b-1] or a chloride anion. Q⁺ represents an alkali metal ion, a tetraalkylammonium cation, or a tetraalkylphosphonium cation.) 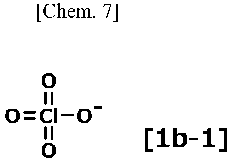
[14] The nonaqueous electrolyte solution according to [13], in which the (IV) is a salt compound containing a counter anion selected from a bis(oxalato)borate anion, a tetrafluoroborate anion, a chloride anion, or a perchlorate anion and a counter cation selected from a lithium cation, a sodium cation, a potassium cation, a tetraalkylammonium cation, or a tetraalkylphosphonium cation.
[15] The nonaqueous electrolyte solution according to [13] or [14], in which the content of the (IV) in the nonaqueous electrolyte solution is 10 to 8000 ppm by mass.
[16] The nonaqueous electrolyte solution according to [13] or [14], in which the content of the (TV) in the nonaqueous electrolyte solution is 50 to 5000 ppm by mass.
[17] A nonaqueous electrolyte solution battery, at least having: a positive electrode; a negative electrode; a separator; and the nonaqueous electrolyte solution according to any one of [1] to [16].
[18] A compound represented by the following general formula (1), (2), (3), or (4). 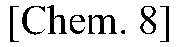 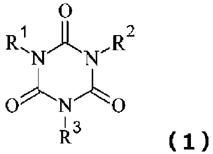
   [In the general formula (1), R¹ to R³ may be the same or different from each other and are a hydrogen atom or an organic group having 1 to 20 carbon atoms which may have a substituent. Here, at least two of R¹ to R³ have a group represented by the general formula (5).] 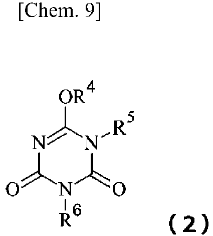
   [In the general formula (2), R⁴ to R⁶ may be the same or different from each other and are a hydrogen atom or an organic group having 1 to 20 carbon atoms which may have a substituent. Here, at least one of R⁴ to R⁶ has a group represented by the general formula (5).] 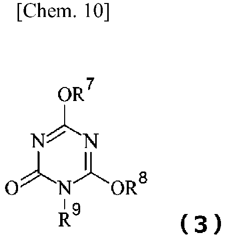
   [In the general formula (3), R⁷ to R⁹ may be the same or different from each other and are a hydrogen atom or an organic group having 1 to 20 carbon atoms which may have a substituent. Here, at least one of R⁷ to R⁹ has a group represented by the general formula (5).] 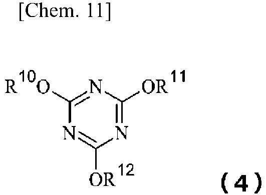
   [In the general formula (4), R¹⁰ to R¹² may be the same or different from each other and are a hydrogen atom or an organic group having 1 to 20 carbon atoms which may have a substituent. Here, at least one of R¹⁰ to R¹² has a group represented by the general formula (5).] 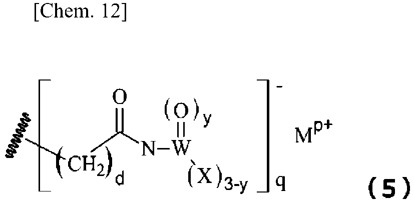
   [In the general formula (5), W represents a phosphorus atom or a sulfur atom, and y is l when W is a phosphorus atom and y is 2 when W is a sulfur atom. X is each independently a halogen atom, an alkyl group having 1 to 20 carbon atoms which may be substituted with a halogen atom, a cycloalkyl group having 5 to 20 carbon atoms which may be substituted with a halogen atom, an alkenyl group having 2 to 20 carbon atoms which may be substituted with a halogen atom, an alkynyl group having 2 to 20 carbon atoms which may be substituted with a halogen atom, an aryl group having 6 to 40 carbon atoms which may be substituted with a halogen atom, a heteroaryl group having 2 to 40 carbon atoms which may be substituted with a halogen atom, an alkoxy group having 1 to 20 carbon atoms which may be substituted with a halogen atom, a cycloalkoxy group having 5 to 20 carbon atoms which may be substituted with a halogen atom, an alkenyloxy group having 2 to 20 carbon atoms which may be substituted with a halogen atom, an alkynyloxy group having 2 to 20 carbon atoms which may be substituted with a halogen atom, an aryloxy group having 6 to 40 carbon atoms which may be substituted with a halogen atom, or a heteroaryloxy group having 2 to 40 carbon atoms which may be substituted with a halogen atom. d represents 0 to 5. M^{p+} represents a proton, a metal cation, or an onium cation, and p represents a valence of the cation. q represents a number satisfying p × q = 1.]

### ADVANTAGEOUS EFFECTS OF INVENTION

According to the present disclosure, a nonaqueous electrolyte solution capable of exhibiting an excellent initial input-output characteristic when used for a nonaqueous electrolyte solution battery, a nonaqueous electrolyte solution battery capable of exhibiting an excellent initial input-output characteristic, and a compound suitably used for the nonaqueous electrolyte solution can be provided.

### DESCRIPTION OF EMBODIMENTS

Configurations and combinations thereof in the following embodiments are examples, and additions, replacements, and other changes of the configurations may be made without departing from the scope of the present disclosure. In addition, the present disclosure is not limited by the embodiments but is only limited by the scope of claims.

The term "to" in the present specification is used with the meanings including the numerical values indicated before and after "to" as a lower limit value and an upper limit value.

In the present specification, an initial input-output characteristic refers to a resistance value of the nonaqueous electrolyte solution battery immediately after an initial charge and discharge operation for battery stabilization. Specifically, the initial input-output characteristic refers to a resistance value obtained by an initial measurement of a direct current internal resistance after four cycles of a charge and discharge operation for battery stabilization.

### [1. Nonaqueous Electrolyte Solution]

The nonaqueous electrolyte solution of the present disclosure is a nonaqueous electrolyte solution containing (I) at least one compound selected from the group consisting of a compound represented by the general formula (1), a compound represented by the general formula (2), a compound represented by the general formula (3), and a compound represented by the general formula (4), (II) a solute, and (III) a nonaqueous organic solvent.

### <Component (1)>

The nonaqueous electrolyte solution of the present disclosure contains at least one compound selected from the group consisting of a compound represented by the general formula (1), a compound represented by the general formula (2), a compound represented by the general formula (3), and a compound represented by the general formula (4), which is the component (I).

When the nonaqueous electrolyte solution containing the above component (1) is used in a nonaqueous electrolyte solution battery (for example, a lithium ion secondary battery or a sodium ion secondary battery), the component (I) decomposes on at least one of the positive electrode and the negative electrode and forms a film having excellent cation conductivity on the surface of at least one of the positive electrode and the negative electrode. It is considered that this film prevents direct contact between the nonaqueous organic solvent or the solute and the electrode active material and reduces the cation dissociation energy of the solute. The present disclosers presume that, as a result, an effect of reducing the initial resistance of the nonaqueous electrolyte solution battery is exhibited.

R¹ to R³ in the general formula (1) may be the same or different from each other and are a hydrogen atom or an organic group having 1 to 20 carbon atoms which may have a substituent. Here, at least one of R¹ to R³ has a group represented by the general formula (5).

R¹ to R³ may be an organic group having 1 to 15 carbon atoms or an organic group having 1 to 10 carbon atoms.

The organic group may be linear, branched, or cyclic.

The organic group is not particularly limited, but examples thereof include an alkyl group, a cycloalkyl group, an alkenyl group, an alkynyl group, an aryl group, a heteroaryl group, an alkoxy group, a cycloalkoxy group, an alkenyloxy group, an alkynyloxy group, an aryloxy group, a heteroaryloxy group, and the like. The organic group may be a group represented by the general formula (5).

The substituent which the organic group may have is not particularly limited, but examples thereof include a halogen atom, a hydroxyl group, a cyano group, an isocyanate group, an acryloyloxy group, a methacryloyloxy group, and the like.

At least one of R¹ to R³ has a group represented by the general formula (5). That is, at least one of R¹ to R³ is a group represented by the general formula (5) or has a group represented by the general formula (5) as a substituent.

At least two of R¹ to R³ may have a structure having a group represented by the general formula (5), or all of R¹ to R³ may have a structure having a group represented by the general formula (5).

When two or more of R¹ to R³ have a group represented by the general formula (5), the two or more groups represented by the general formula (5) may be the same or different.

The description and the specific examples of R⁴ to R⁶ in the general formula (2), R⁷ to R⁹ in the general formula (3), and R¹⁰ to R¹² in the general formula (4) are the same as those of R¹ to R³ described above.

W in the general formula (5) represents a phosphorus atom or a sulfur atom. W may be a sulfur atom.

X in the general formula (5) is each independently a halogen atom, an alkyl group having 1 to 20 carbon atoms which may be substituted with a halogen atom, a cycloalkyl group having 5 to 20 carbon atoms which may be substituted with a halogen atom, an alkenyl group having 2 to 20 carbon atoms which may be substituted with a halogen atom, an alkynyl group having 2 to 20 carbon atoms which may be substituted with a halogen atom, an aryl group having 6 to 40 carbon atoms which may be substituted with a halogen atom, a heteroaryl group having 2 to 40 carbon atoms which may be substituted with a halogen atom, an alkoxy group having 1 to 20 carbon atoms which may be substituted with a halogen atom, a cycloalkoxy group having 5 to 20 carbon atoms which may be substituted with a halogen atom, an alkenyloxy group having 2 to 20 carbon atoms which may be substituted with a halogen atom, an alkynyloxy group having 2 to 20 carbon atoms which may be substituted with a halogen atom, an aryloxy group having 6 to 40 carbon atoms which may be substituted with a halogen atom, or a heteroaryloxy group having 2 to 40 carbon atoms which may be substituted with a halogen atom.

The halogen atom may be a fluorine atom.

The alkyl group may be linear or branched and may be an alkyl group having 1 to 10 carbon atoms. An oxygen atom may be included in a carbon atom-carbon atom bond in the alkyl group. Specific examples of the alkyl group containing an oxygen atom in a carbon atom-carbon atom bond include a 2-methoxyethyl group, a 2-ethoxyethyl group, and the like, for example.

The cycloalkyl group may be monocyclic or polycyclic and may be a cycloalkyl group having 5 to 15 carbon atoms.

The alkenyl group may be linear or branched and may be an alkenyl group having 2 to 10 carbon atoms.

The alkynyl group may be linear or branched and may be an alkynyl group having 2 to 10 carbon atoms.

The aryl group may be monocyclic or polycyclic and may be an aryl group having 6 to 20 carbon atoms or an aryl group having 6 to 15 carbon atoms.

The heteroaryl group may contain at least one atom selected from the group consisting of an oxygen atom, a sulfur atom, and a nitrogen atom, may be monocyclic or polycyclic and may be a heteroaryl group having 2 to 20 carbon atoms or a heteroaryl group having 2 to 15 carbon atoms.

The alkoxy group may be linear or branched and may be an alkoxy group having 1 to 10 carbon atoms.

The cycloalkoxy group may be monocyclic or polycyclic and may be a cycloalkoxy group having 5 to 15 carbon atoms.

The alkenyloxy group may be linear or branched and may be an alkenyloxy group having 2 to 10 carbon atoms.

The alkynyloxy group may be linear or branched and may be an alkynyloxy group having 2 to 10 carbon atoms.

The aryloxy group may be monocyclic or polycyclic and may be an aryloxy group having 6 to 20 carbon atoms or an aryloxy group having 6 to 15 carbon atoms.

The heteroaryloxy group may contain at least one atom selected from the group consisting of an oxygen atom, a sulfur atom, and a nitrogen atom, may be monocyclic or polycyclic and may be a heteroaryloxy group having 2 to 20 carbon atoms or a heteroaryloxy group having 2 to 15 carbon atoms.

X in the general formula (5) may be each independently a fluorine atom, a methyl group, an ethyl group, an n-propyl group, an isopropyl group, an n-butyl group, a tert-butyl group, an n-pentyl group, an n-hexyl group, a trifluoromethyl group, a trifluoroethyl group, an ethenyl group, a 2-propenyl group, a 2-propynyl group, a phenyl group, a naphthyl group, a pentafluorophenyl group, a methoxy group, an ethoxy group, an n-propoxy group, an isopropoxy group, an n-butoxy group, a tert-butoxy group, an n-pentyloxy group, an n-hexyloxy group, a trifluoromethoxy group, a trifluoroethoxy group, a hexafluoroisopropoxy group, an ethenyloxy group, a 2-propenyloxy group, a 2-propynyloxy group, a phenoxy group, a naphthyloxy group, a pentafluorophenoxy group, a pyrrolyl group, or a pyridinyl group.

X in the general formula (5) may be each independently a fluorine atom, a methyl group, an ethyl group, an n-propyl group, an isopropyl group, an n-butyl group, a tert-butyl group, a trifluoromethyl group, a phenyl group, a naphthyl group, a pentafluorophenyl group, a pyrrolyl group, or a pyridinyl group.

M^{p+} in the general formula (5) represents a proton, a metal cation, or an onium cation and may be a proton, a lithium cation, a sodium cation, a potassium cation, a tetraalkylammonium cation, or a tetraalkylphosphonium cation.

p in the general formula (5) represents a valence of the cation. q represents a number satisfying p × q = l. p and q may be 1.

Specific examples of the compound represented by any of the general formulae (1) to (4) are shown below, but the compound is not limited thereto.

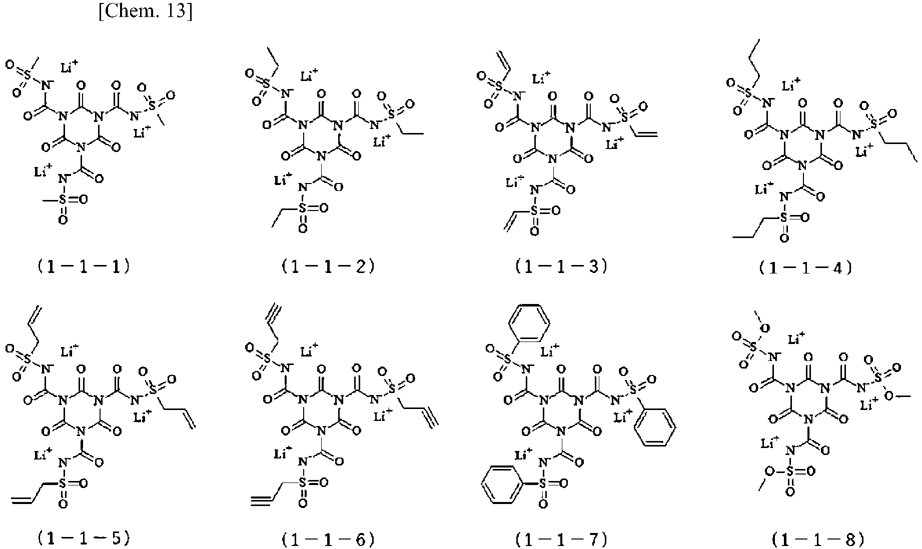

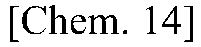

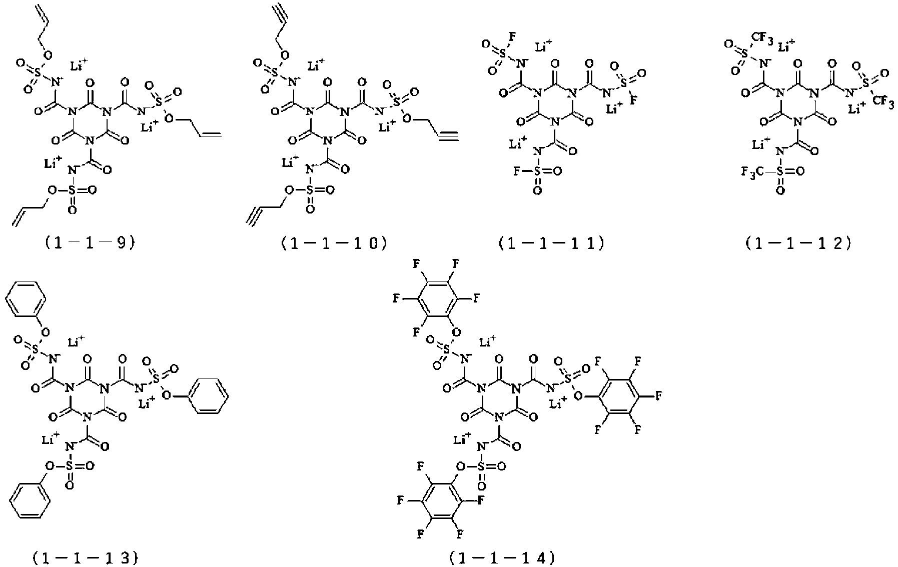

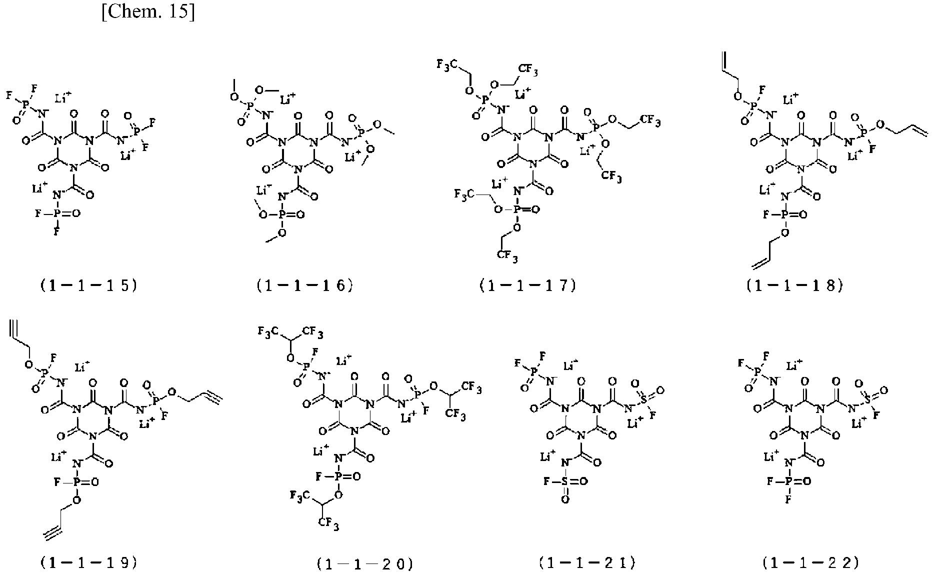

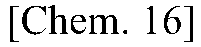

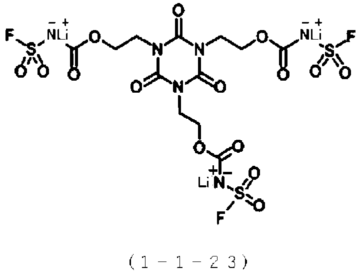

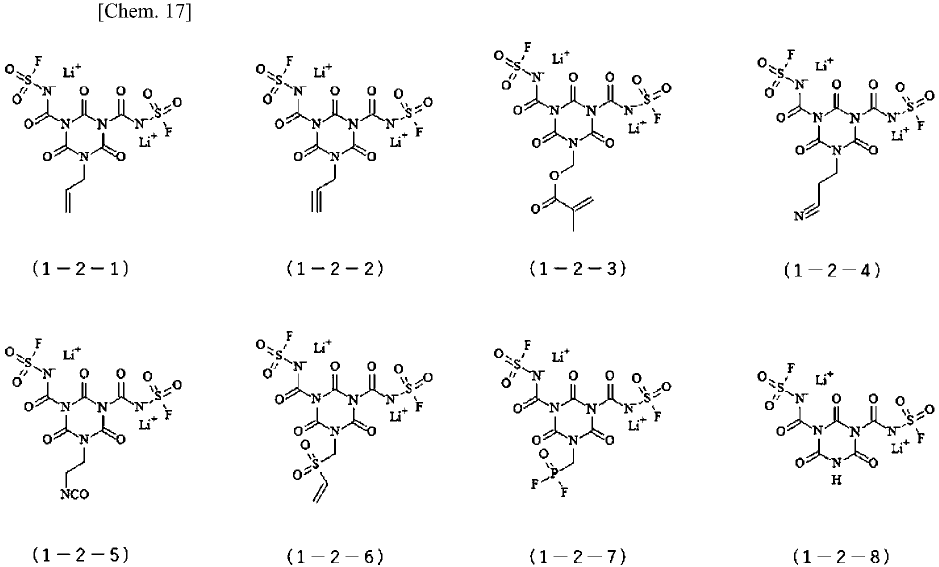

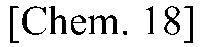

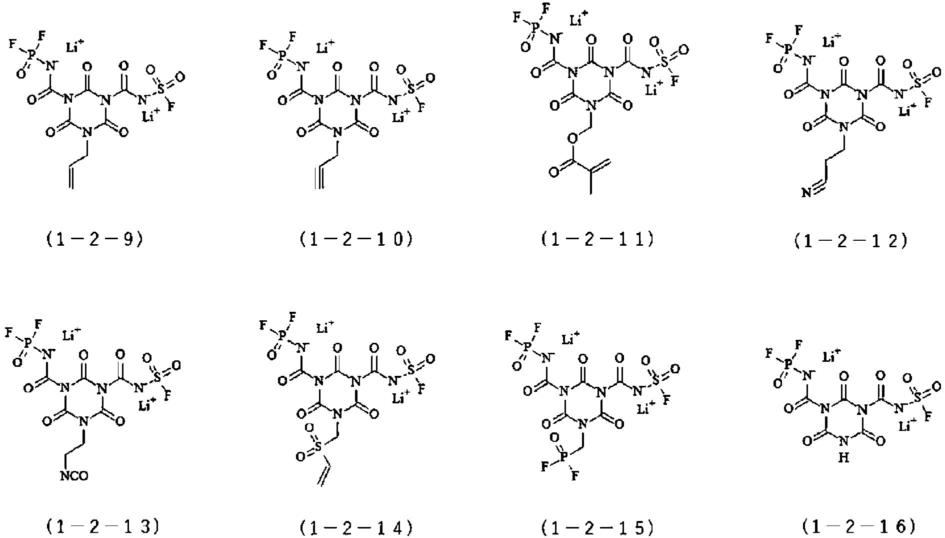

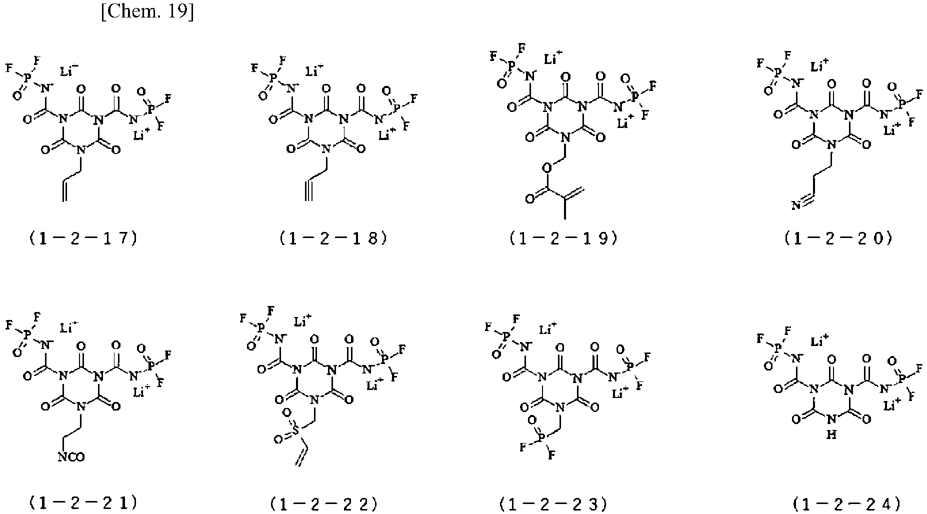

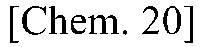

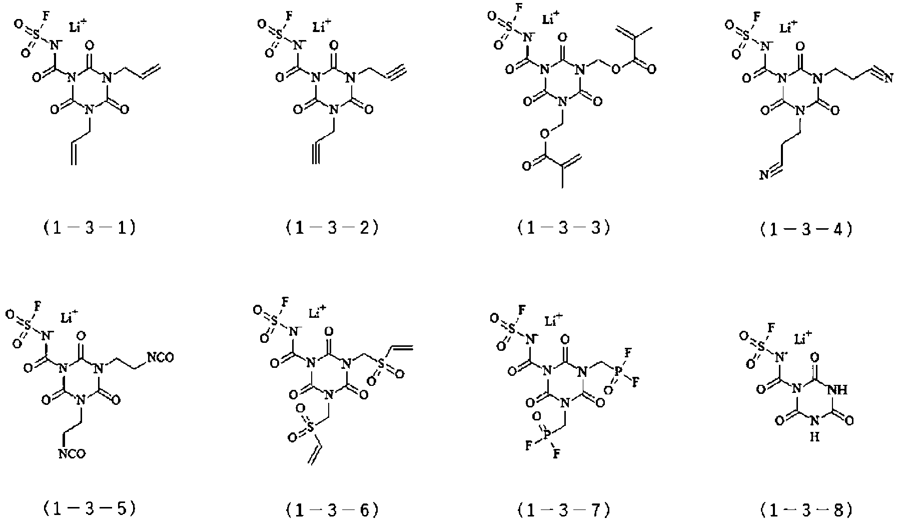

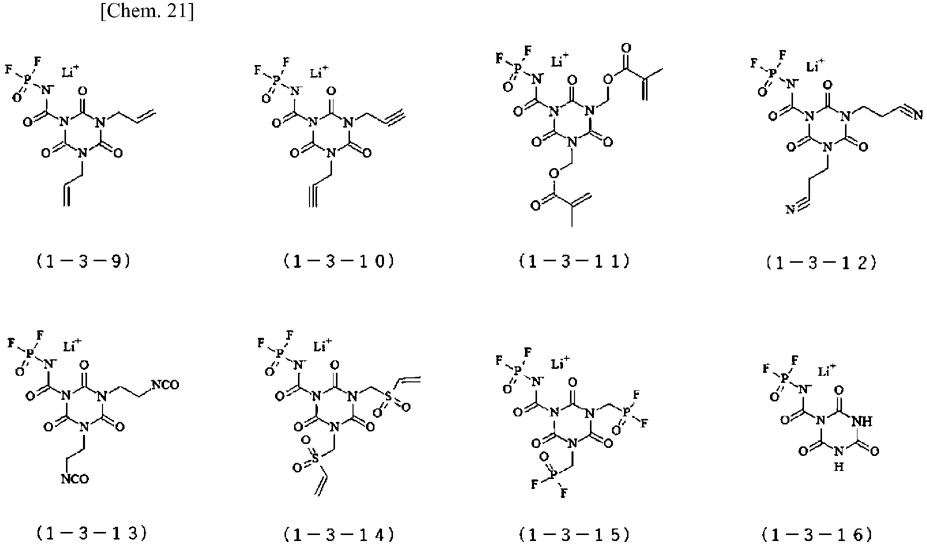

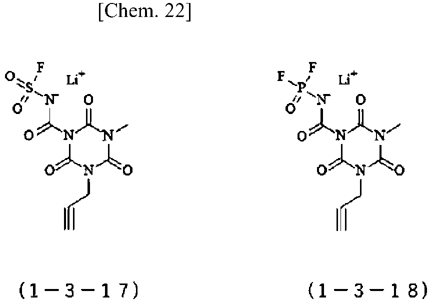

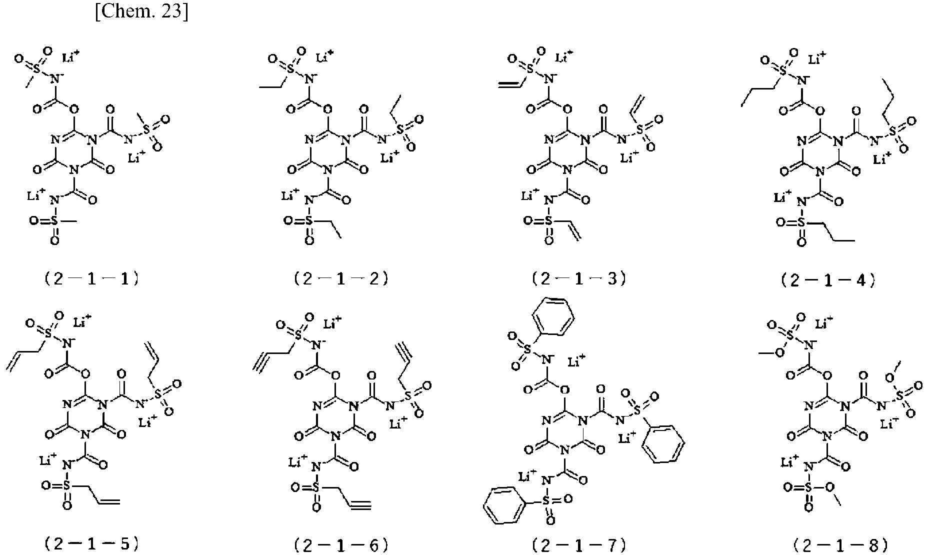

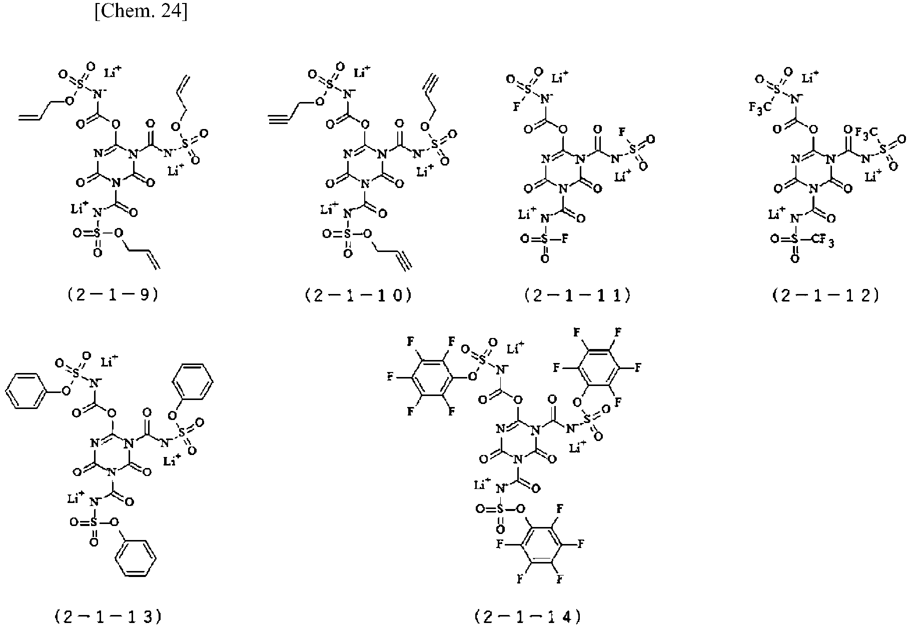

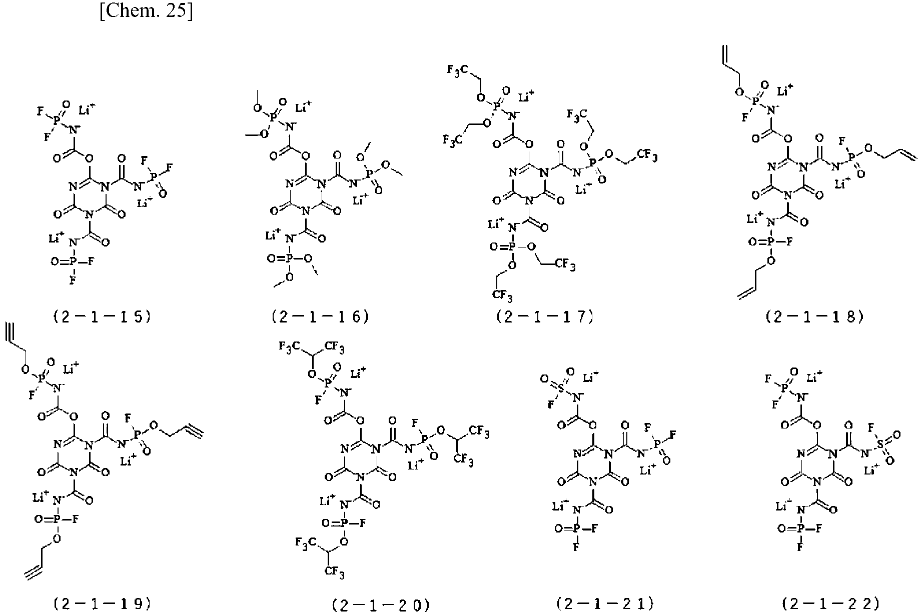

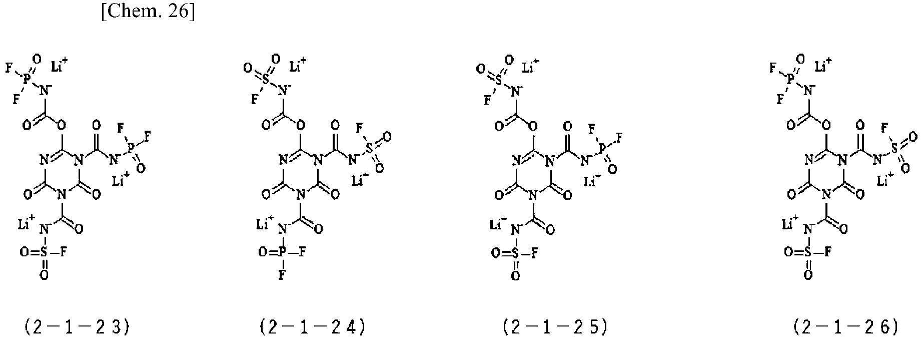

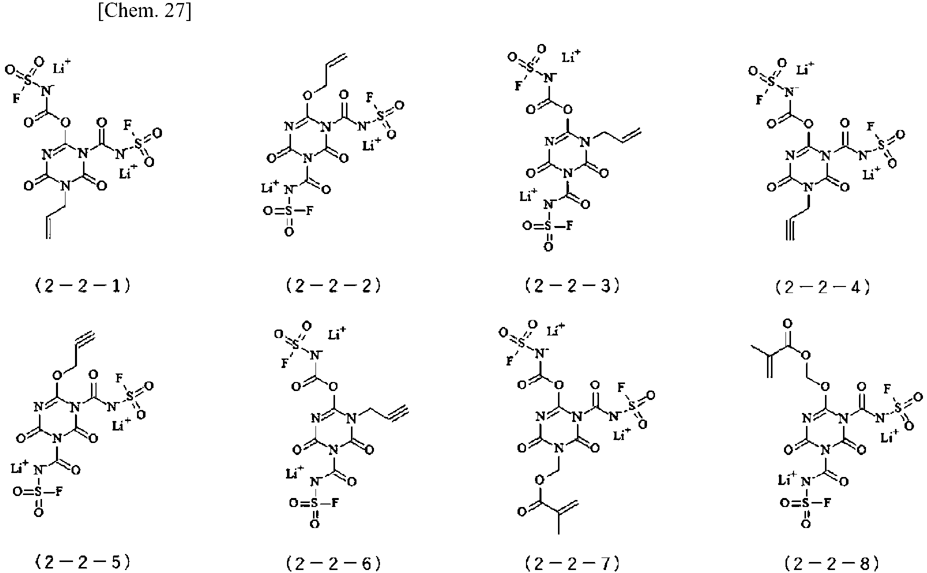

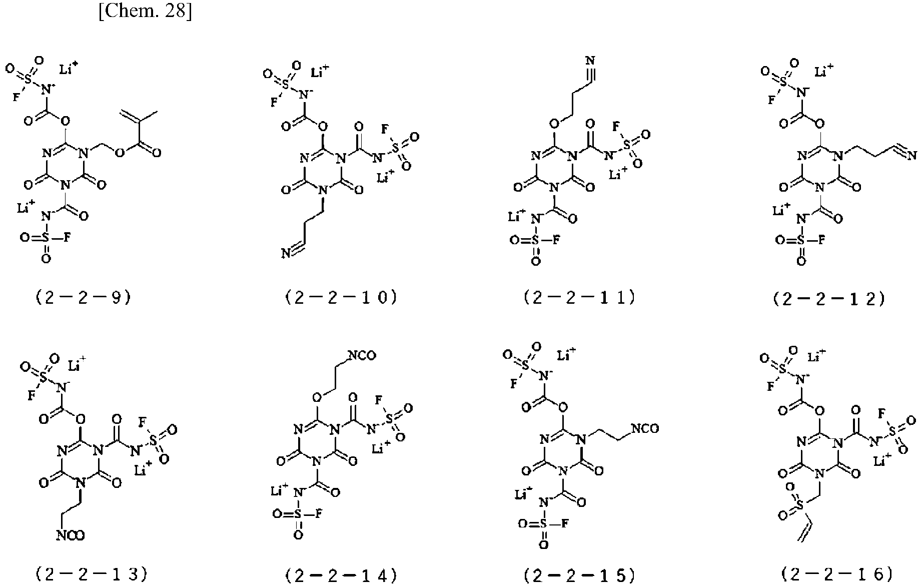

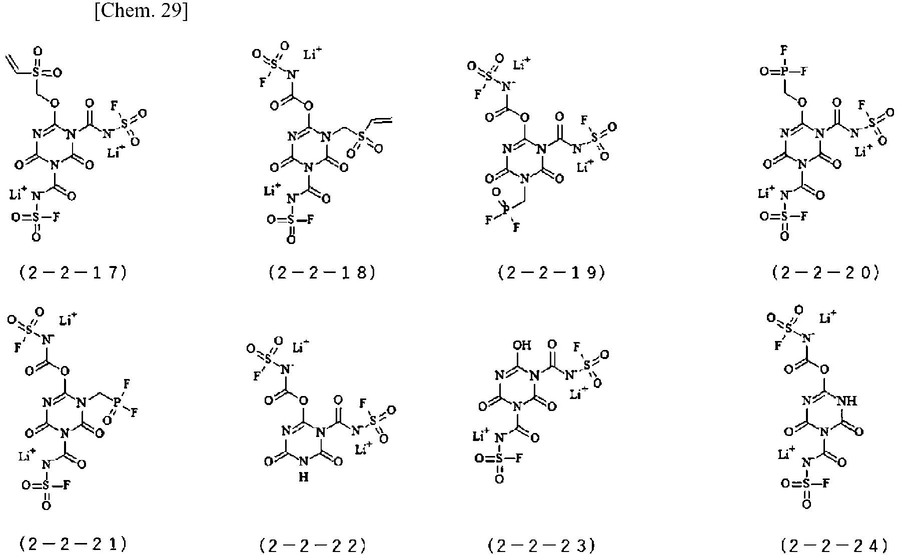

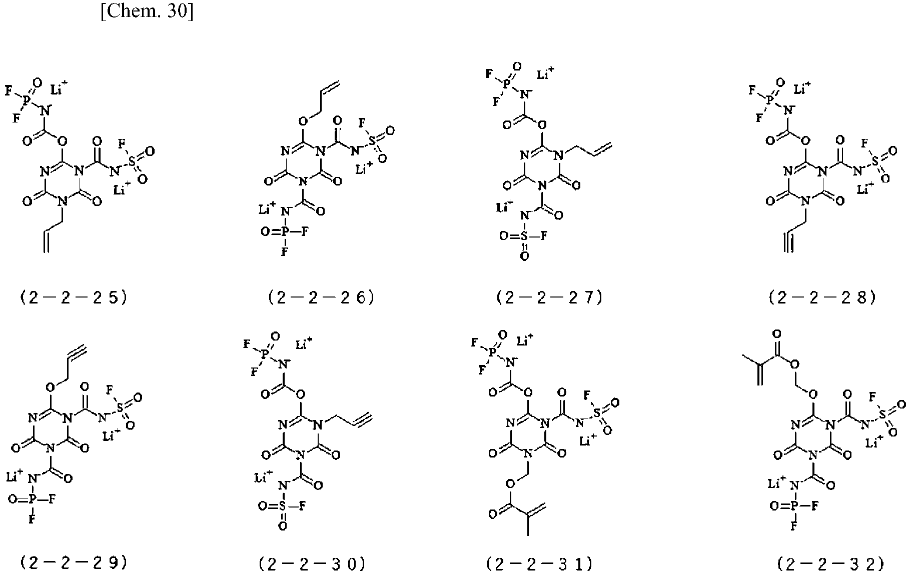

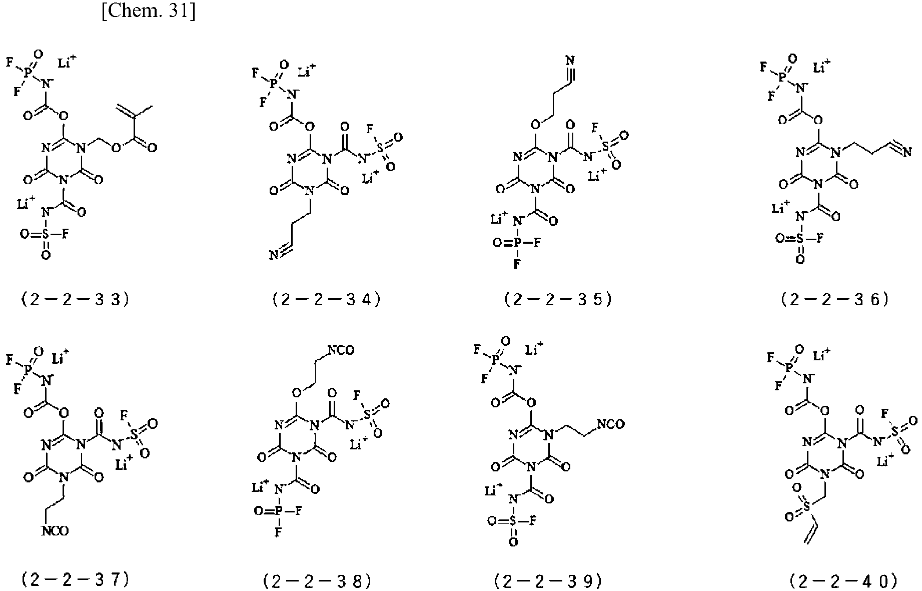

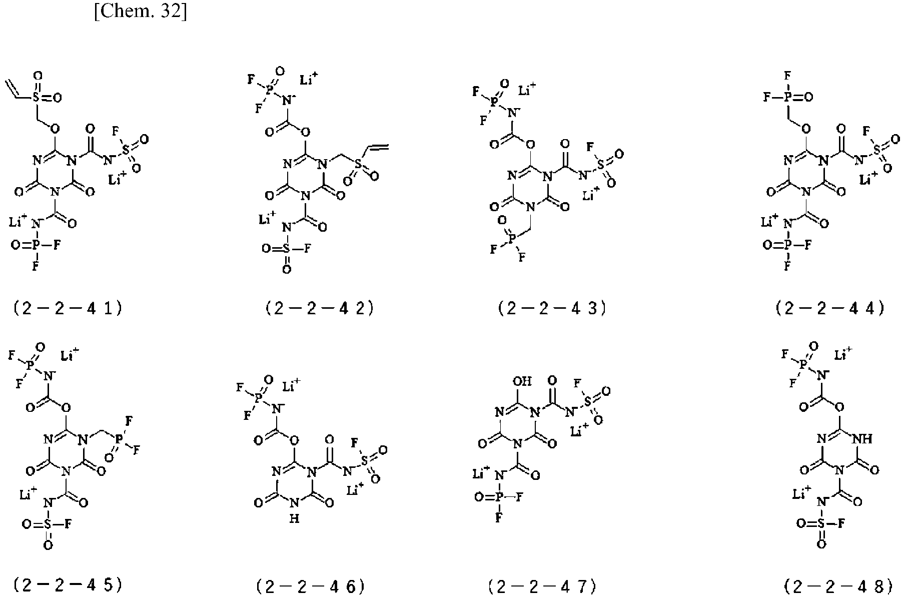

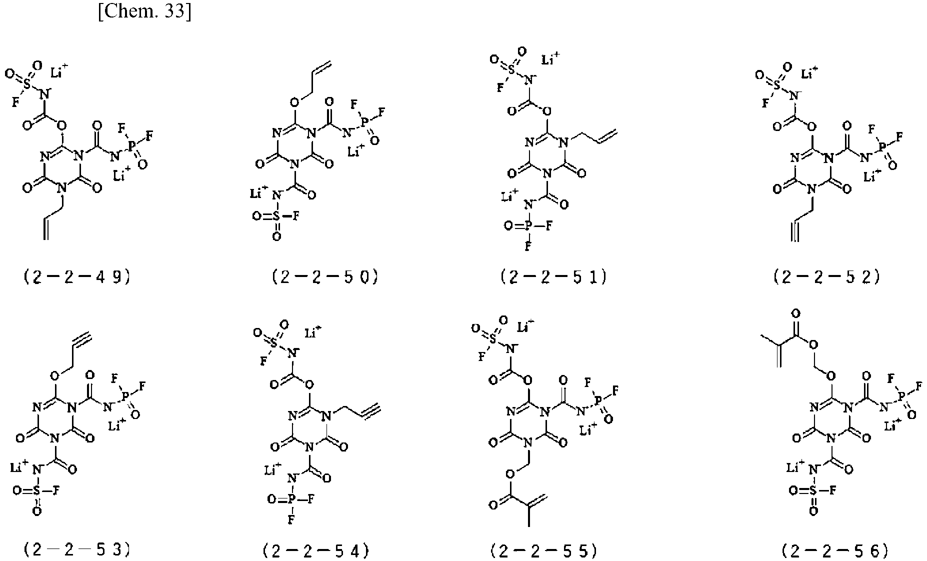

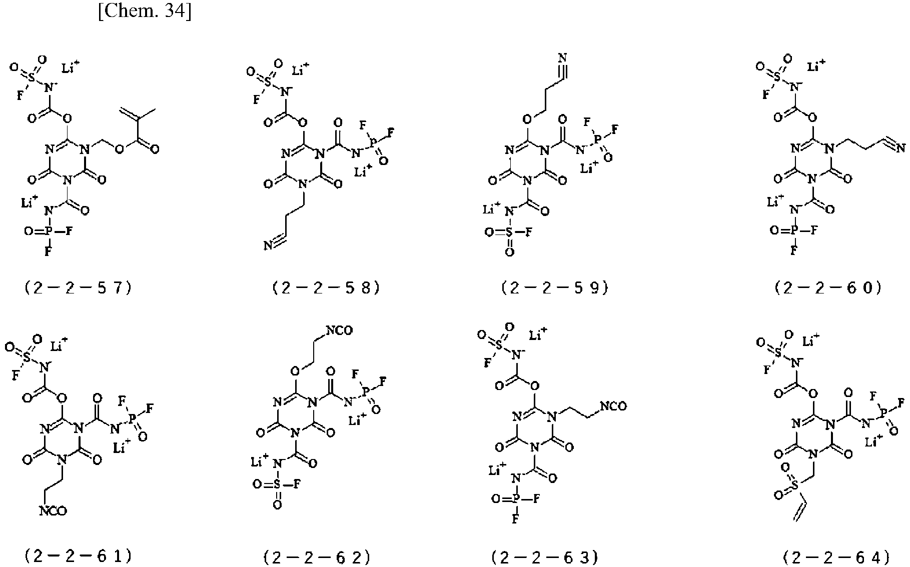

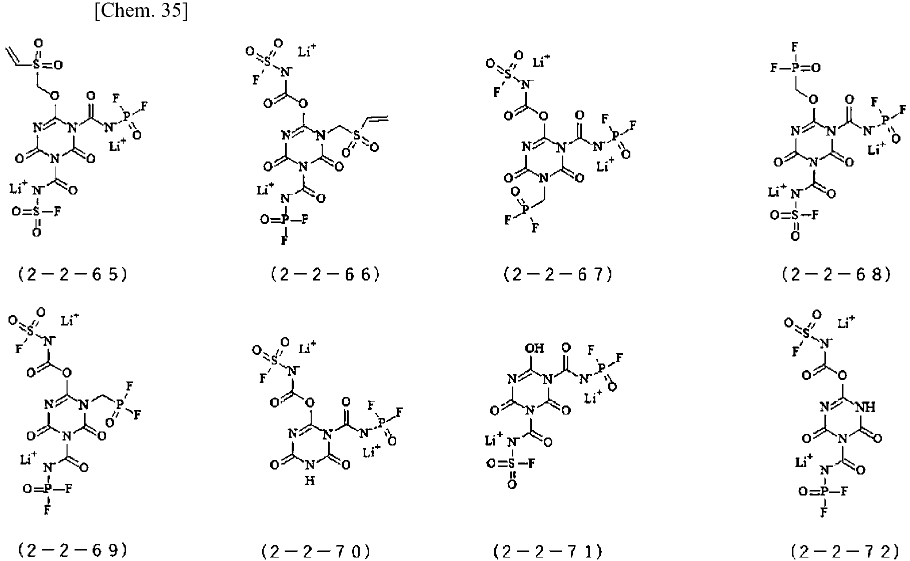

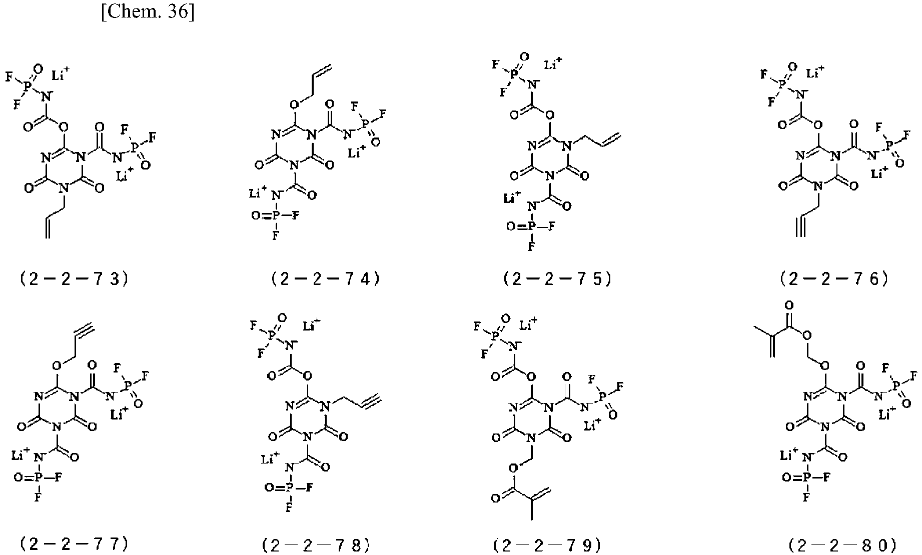

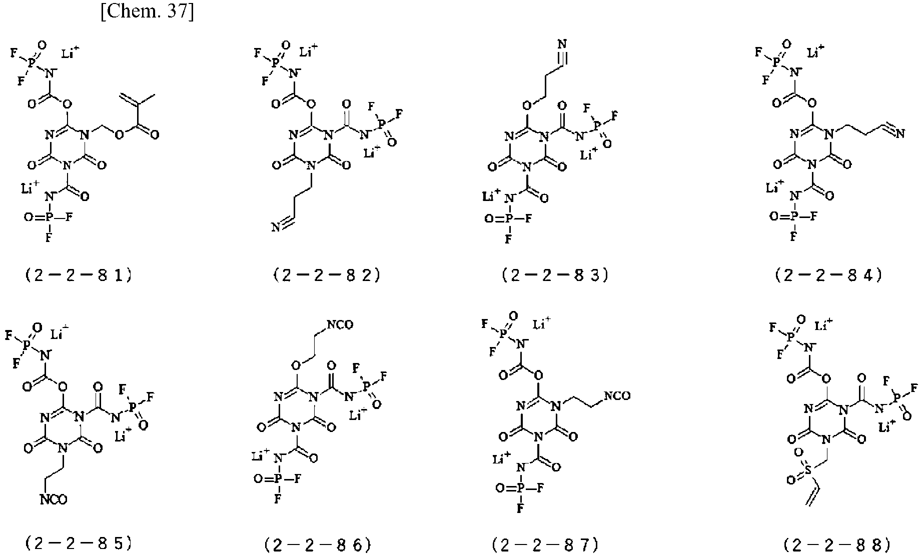

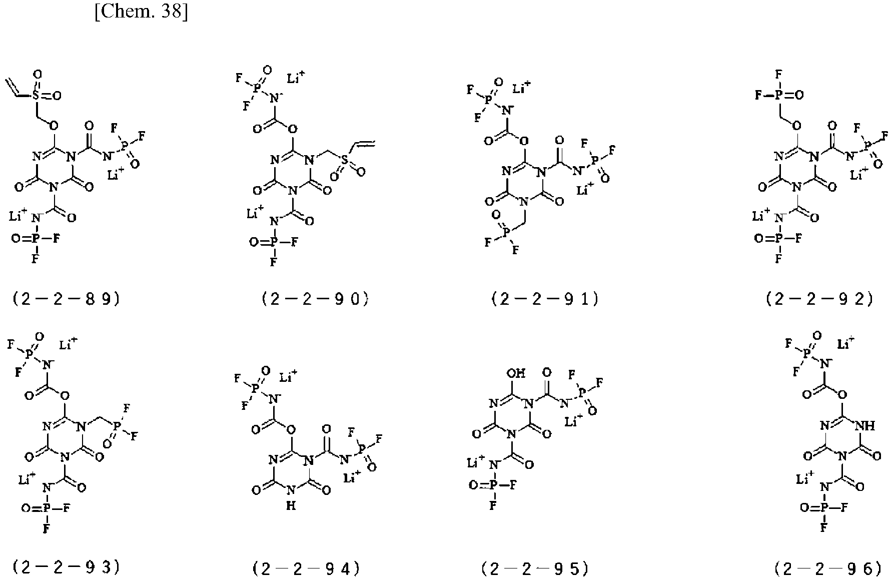

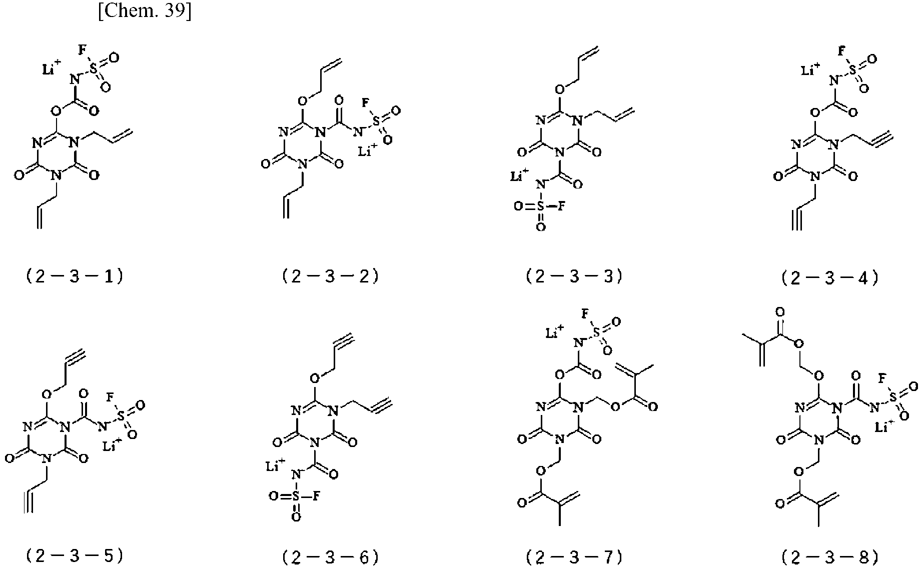

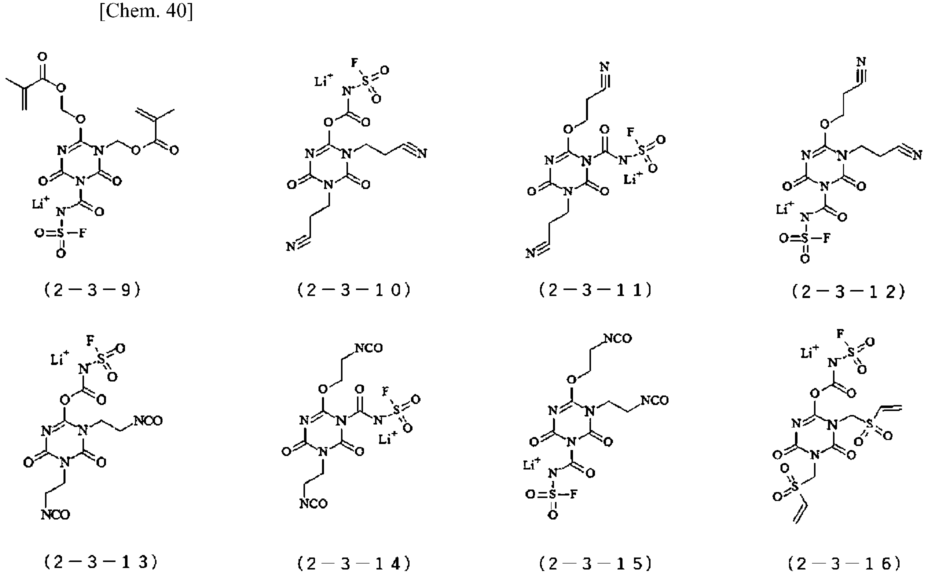

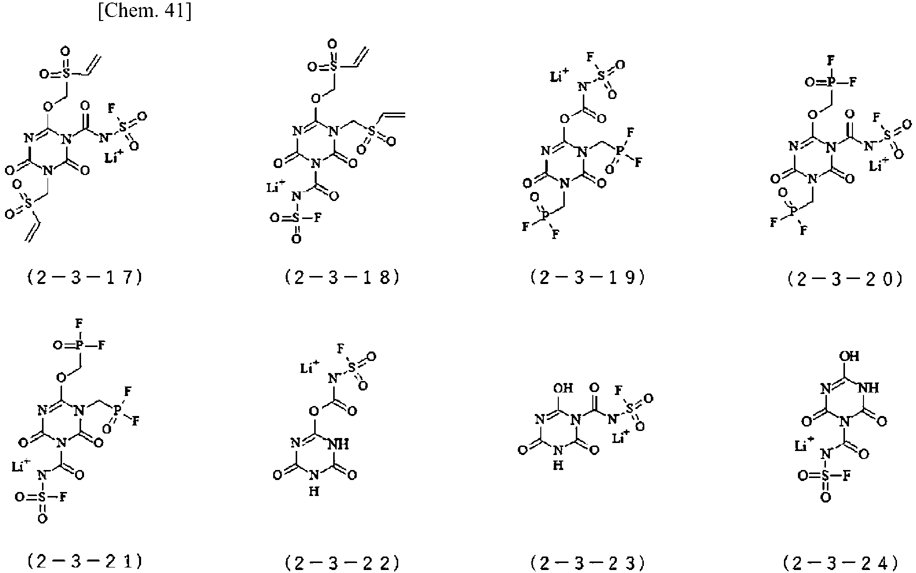

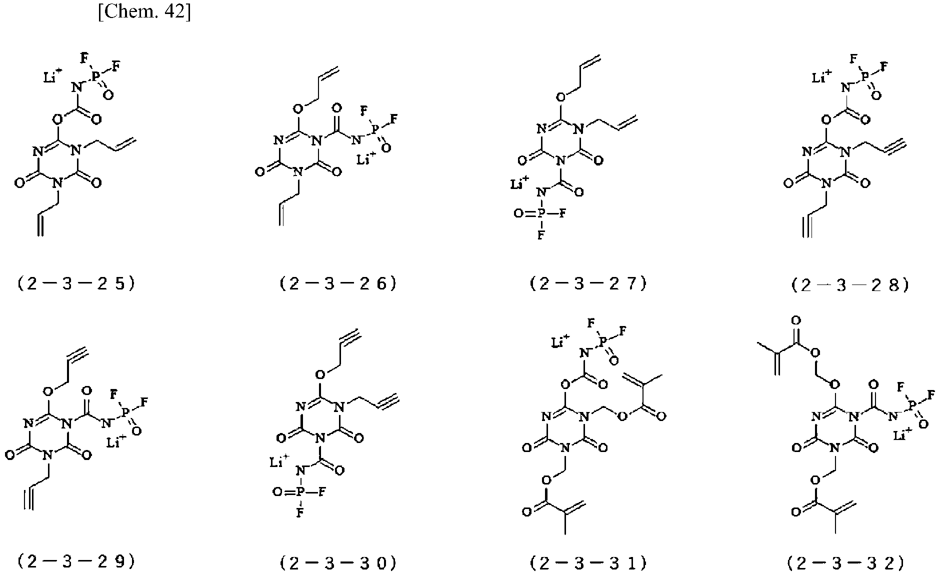

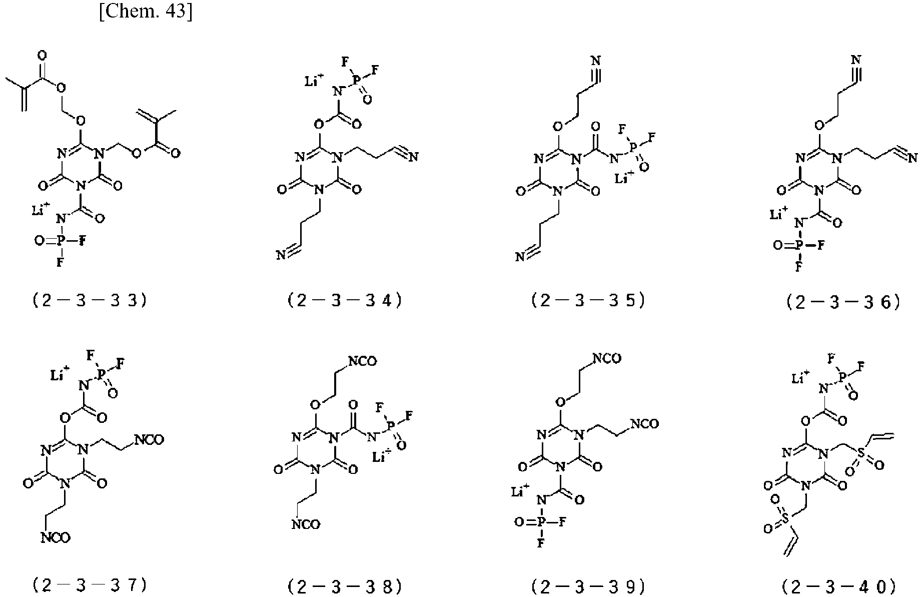

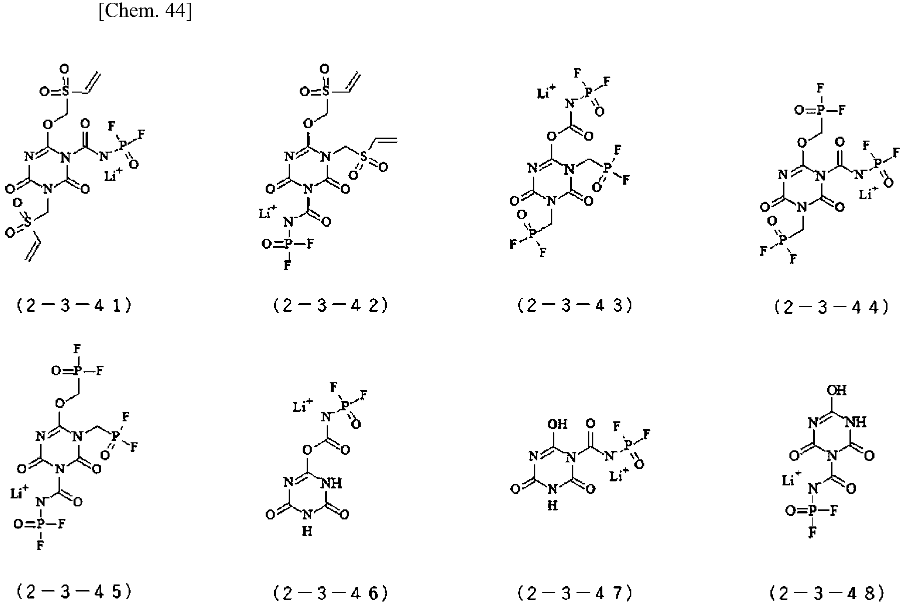

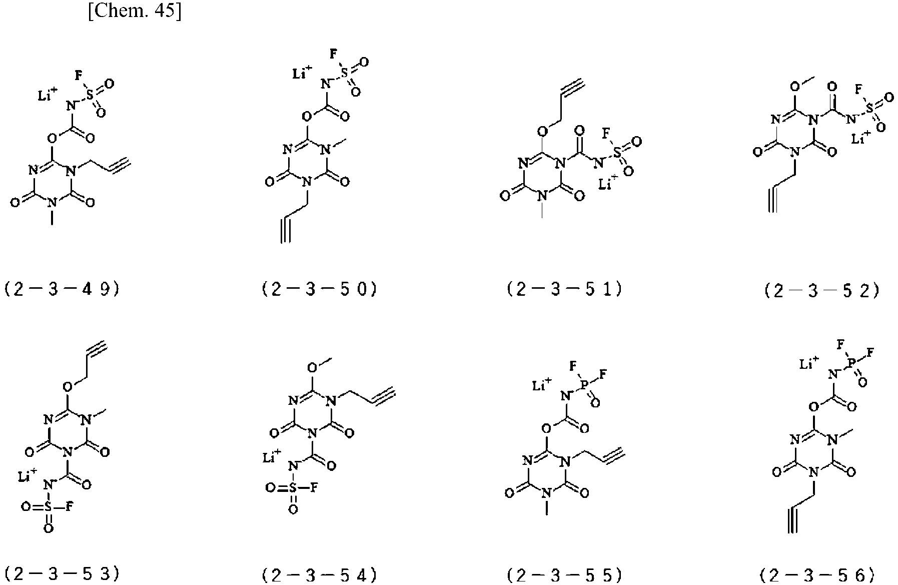

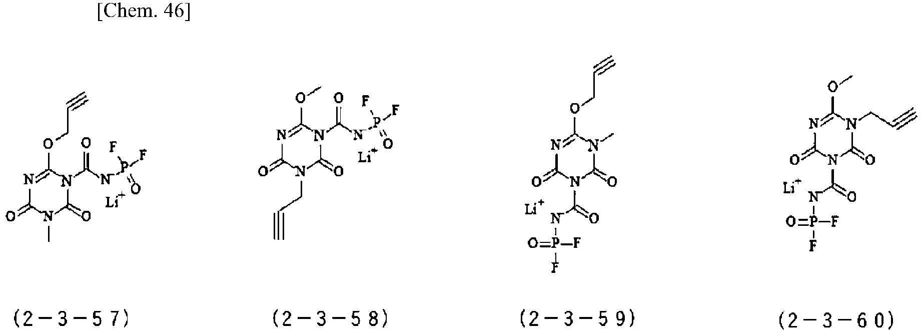

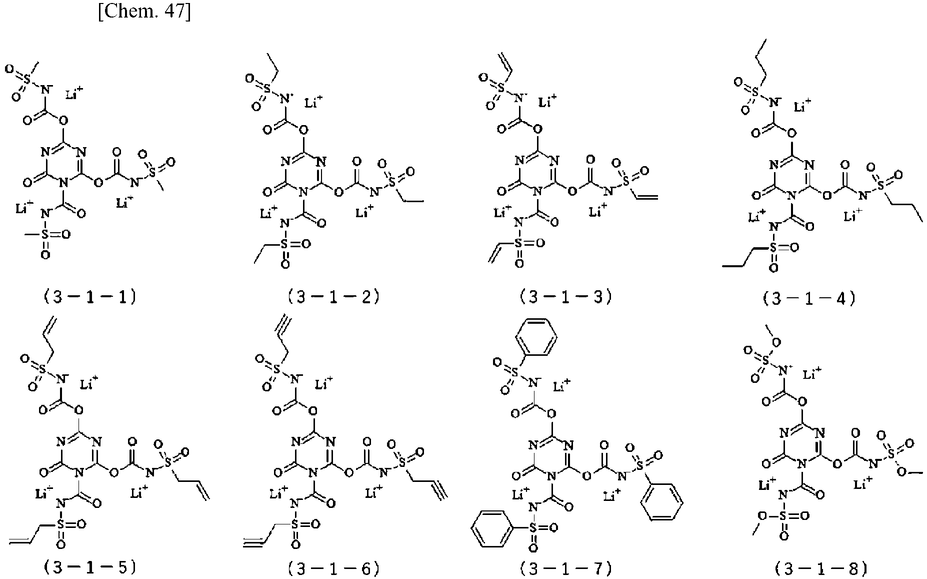

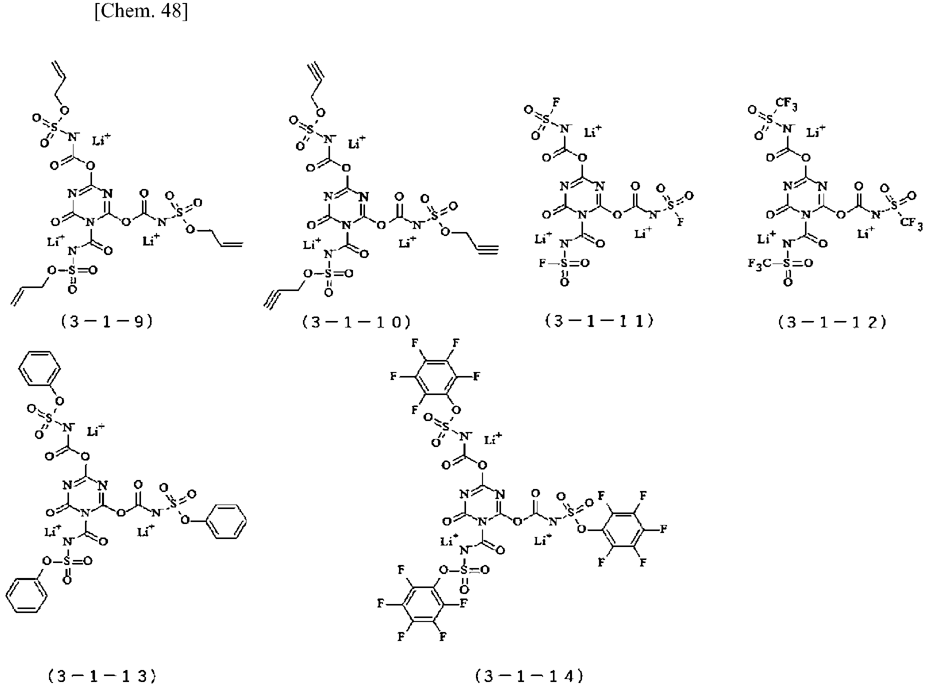

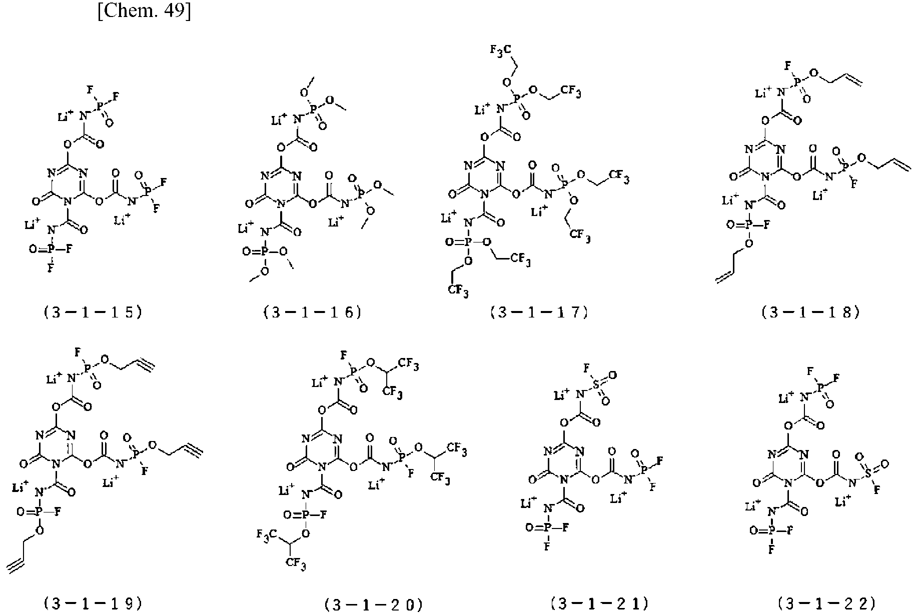

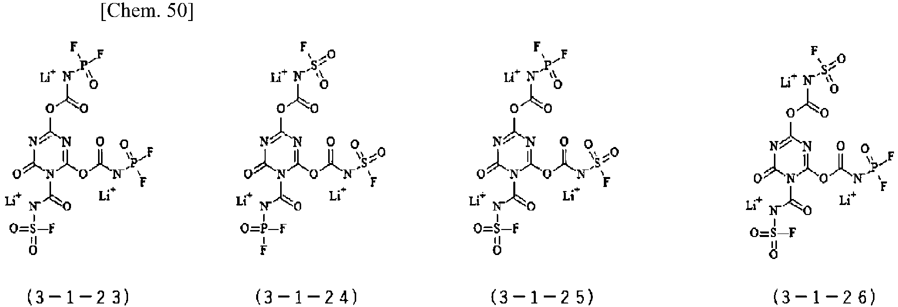

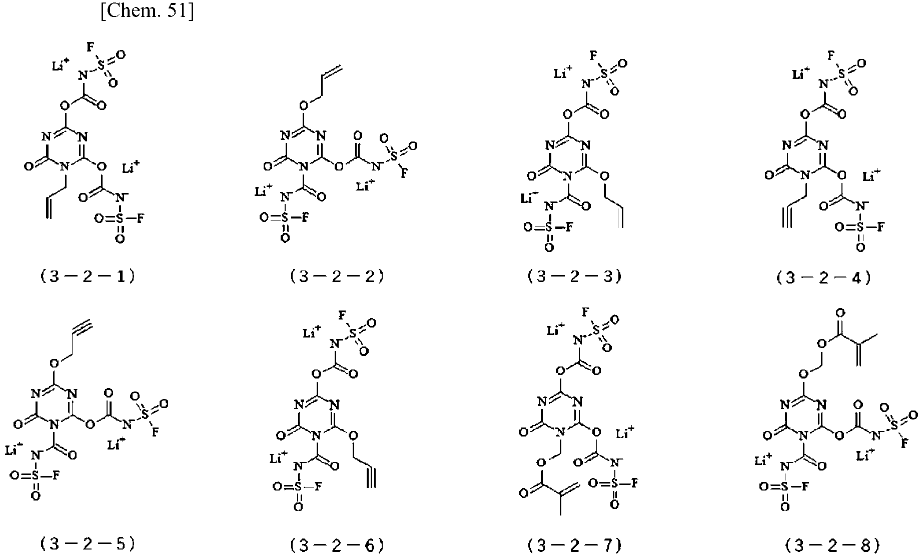

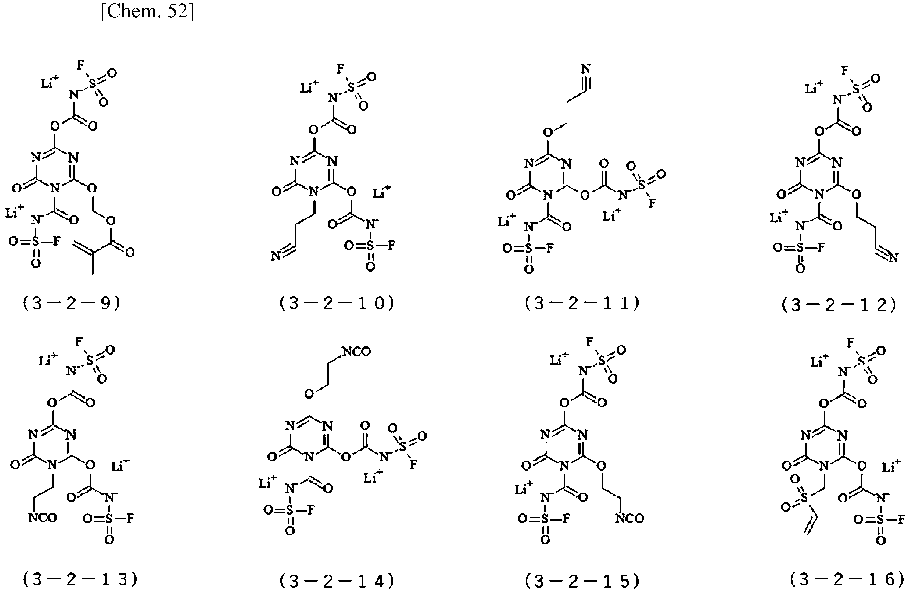

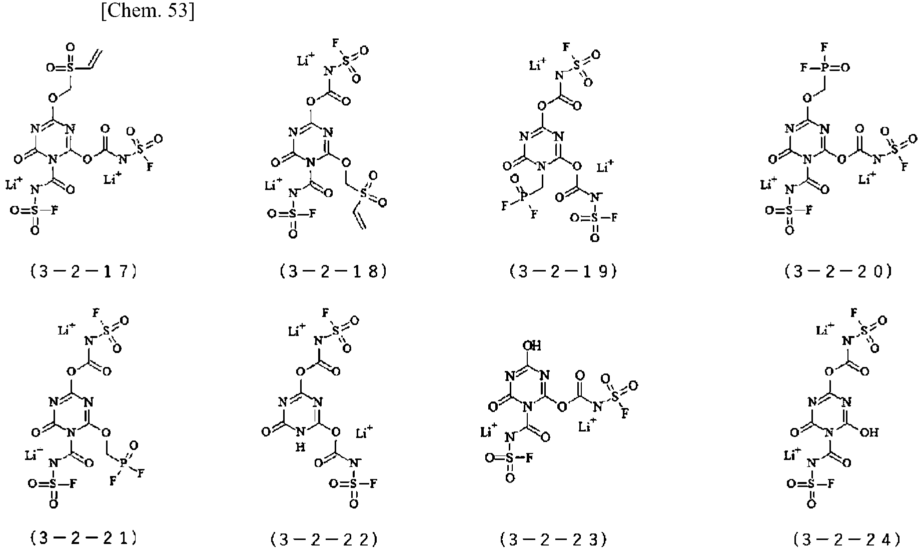

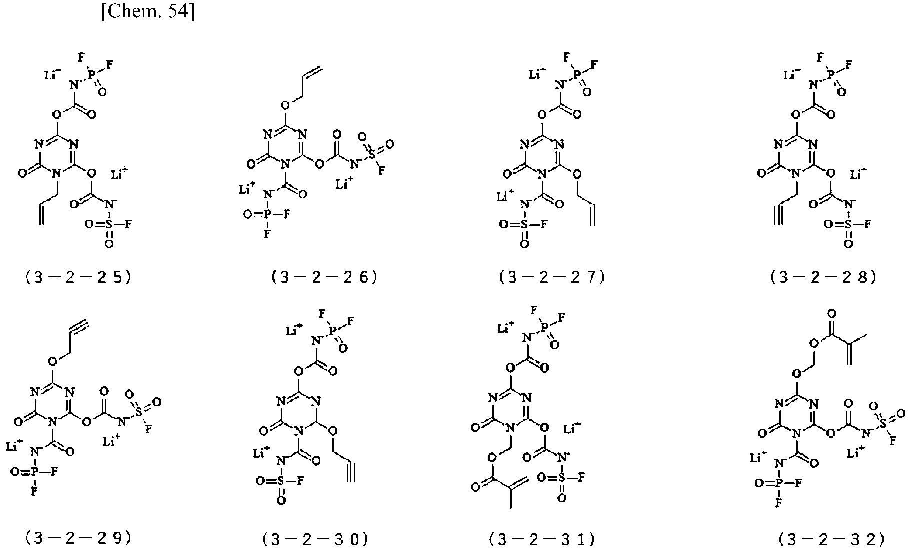

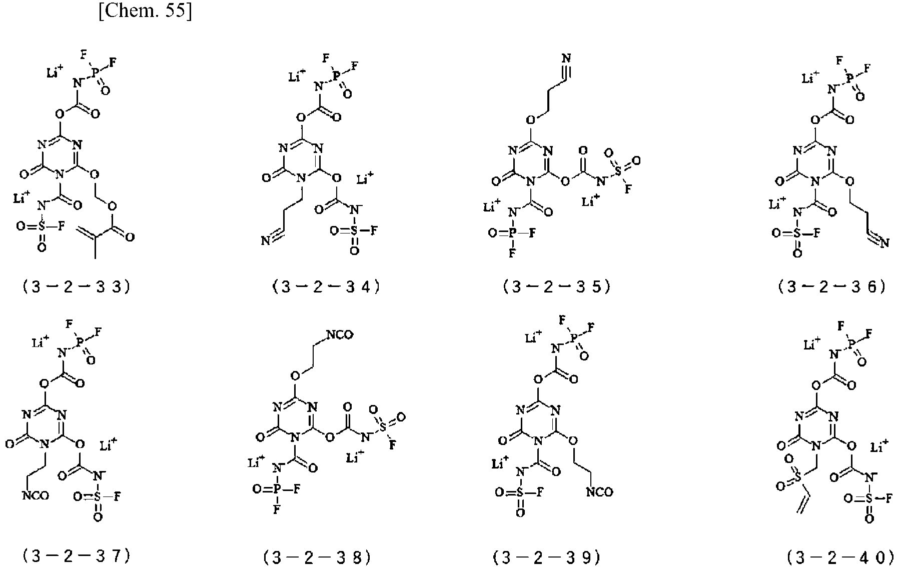

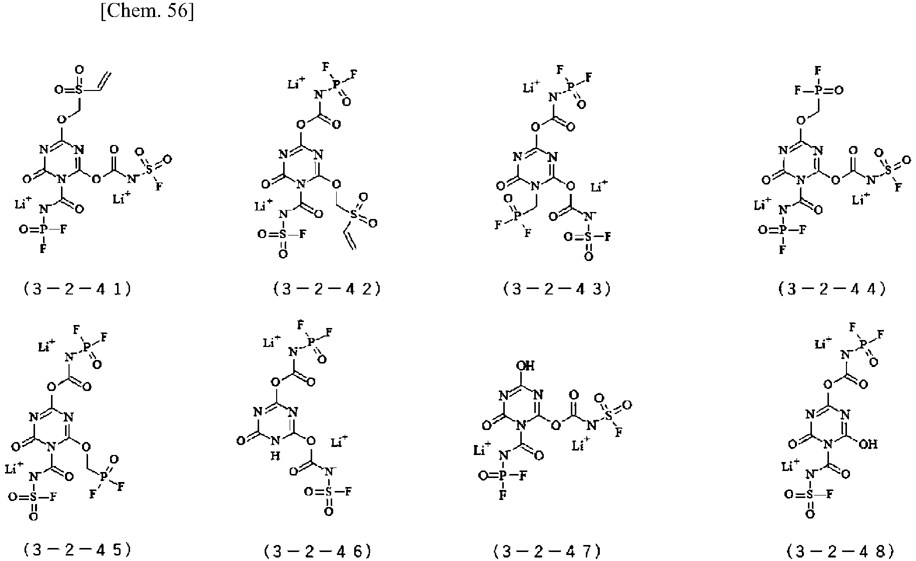

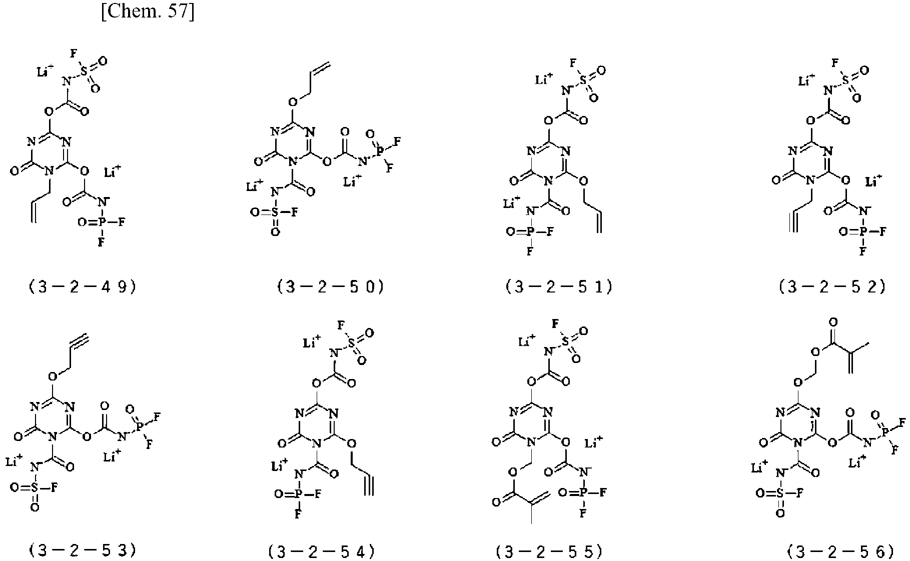

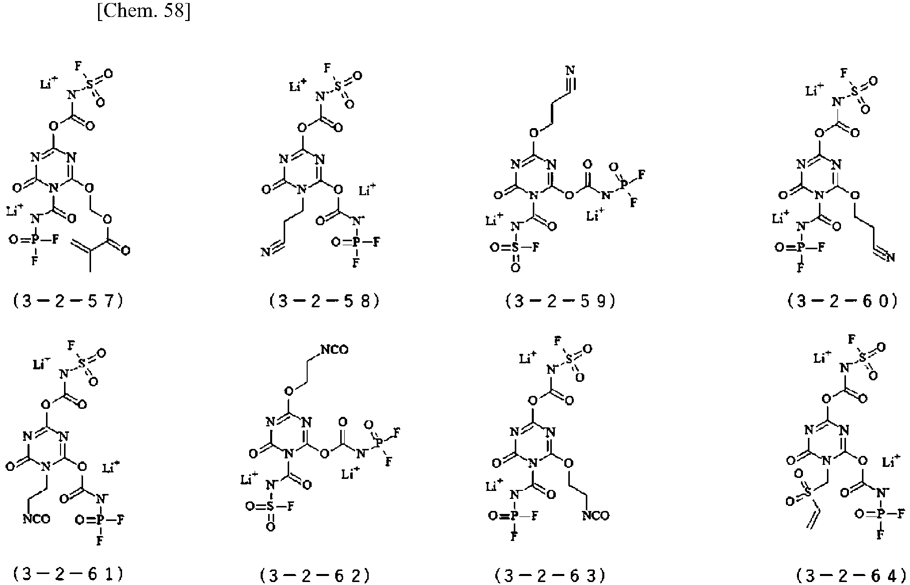

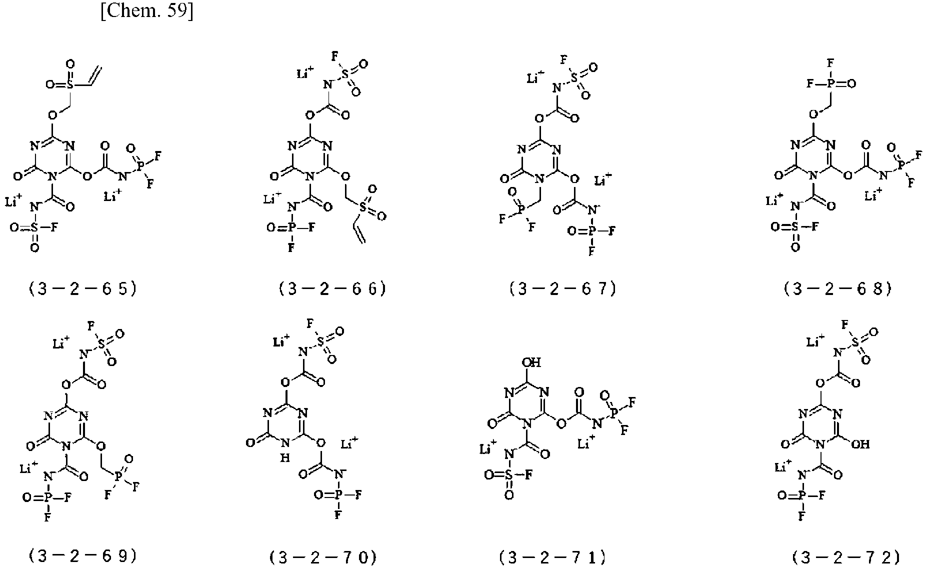

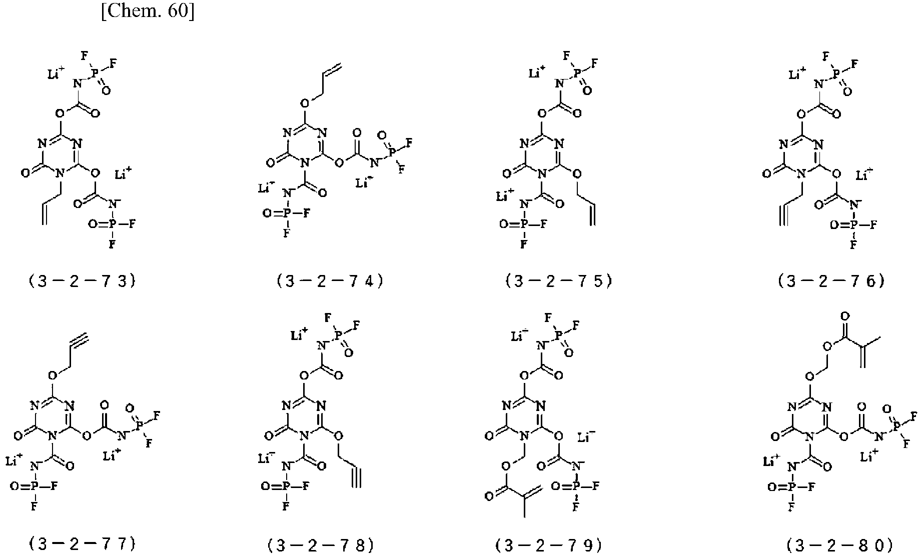

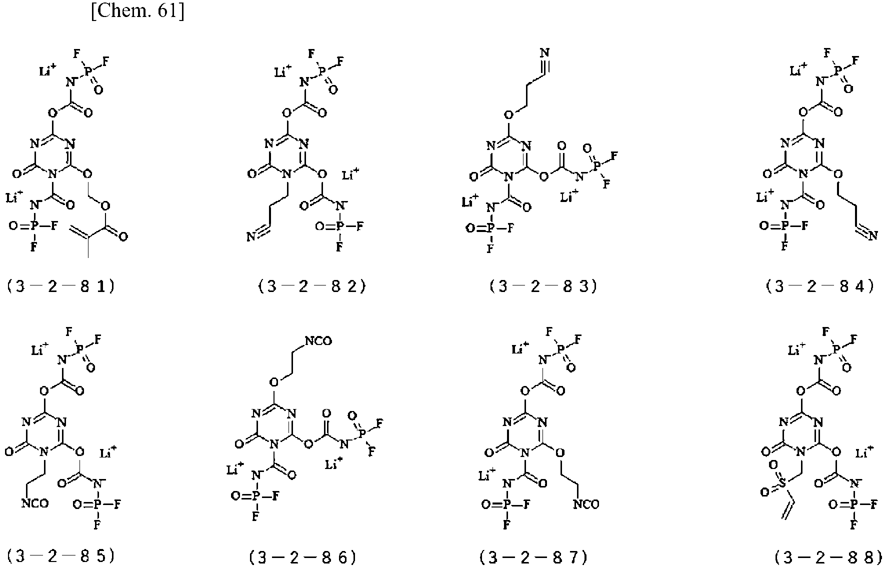

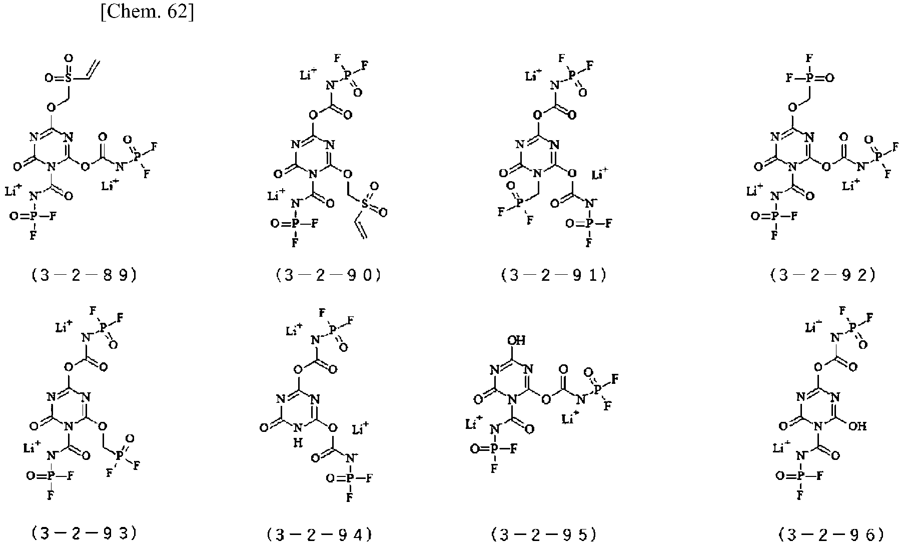

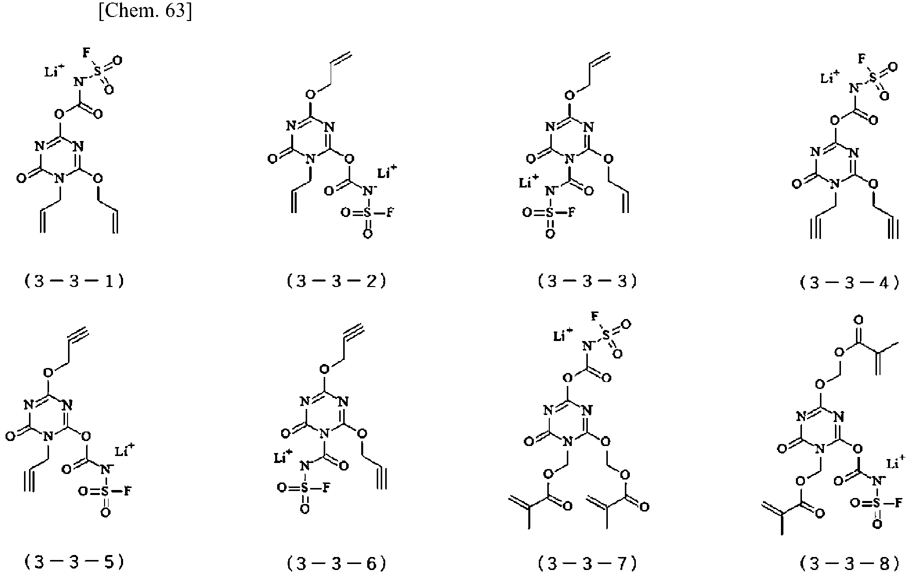

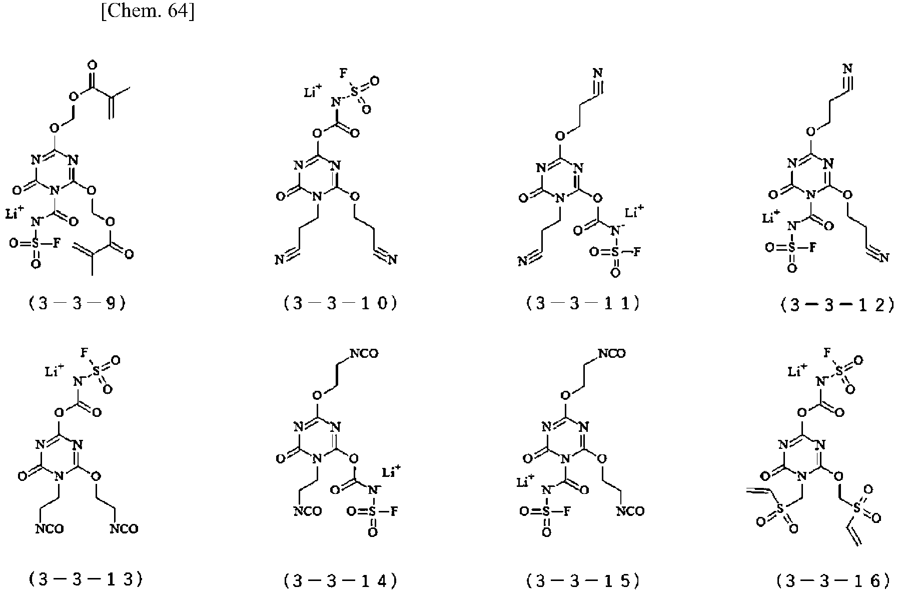

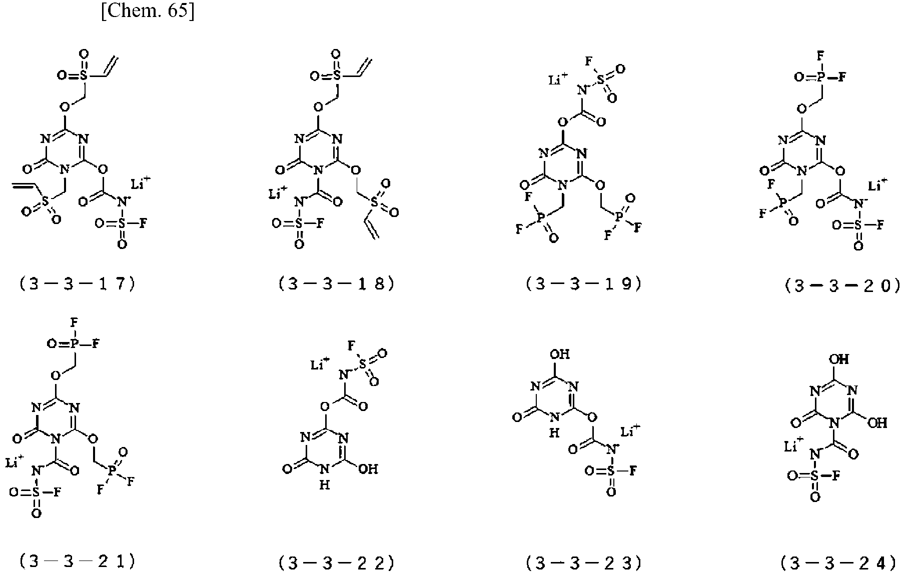

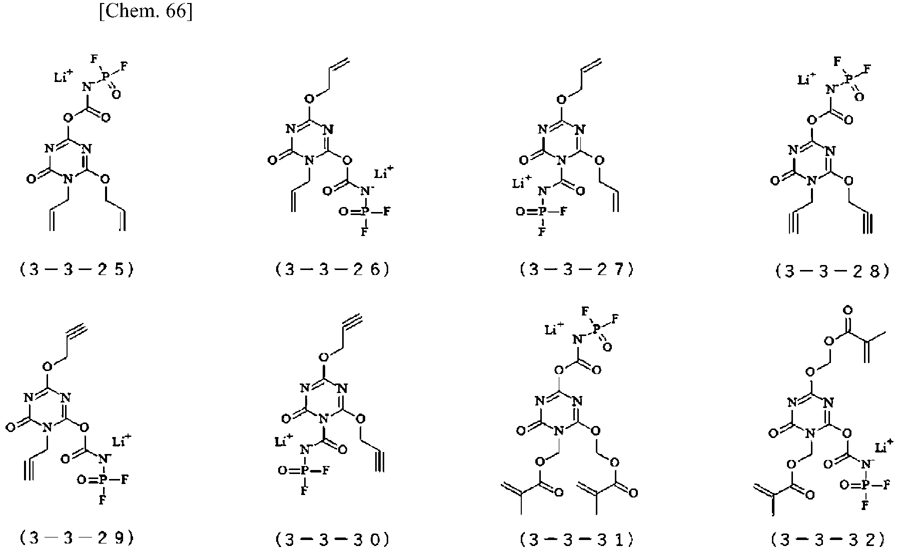

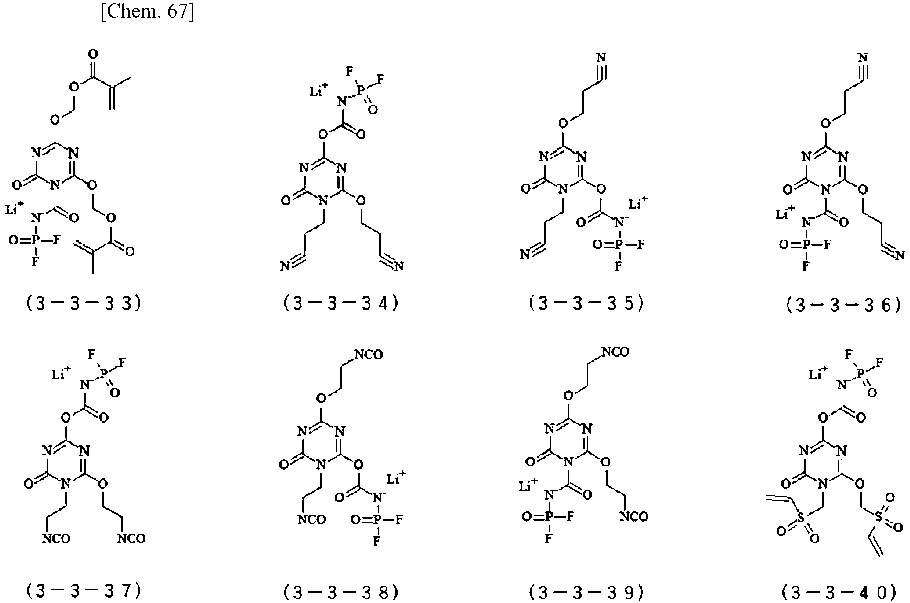

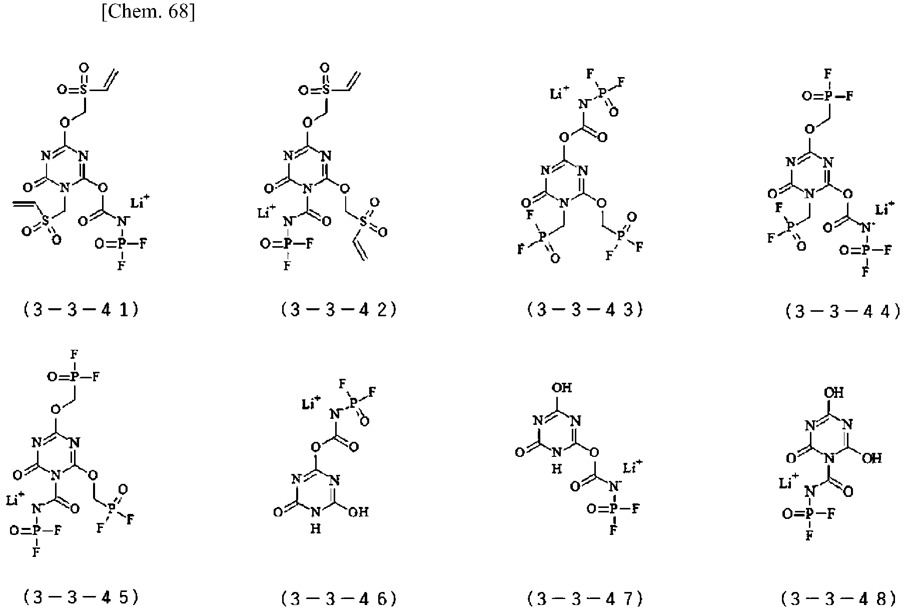

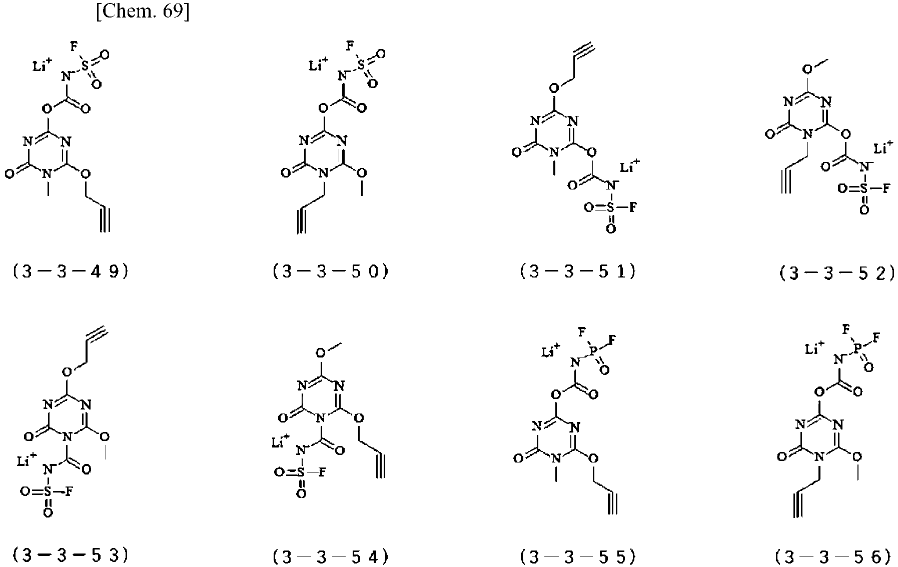

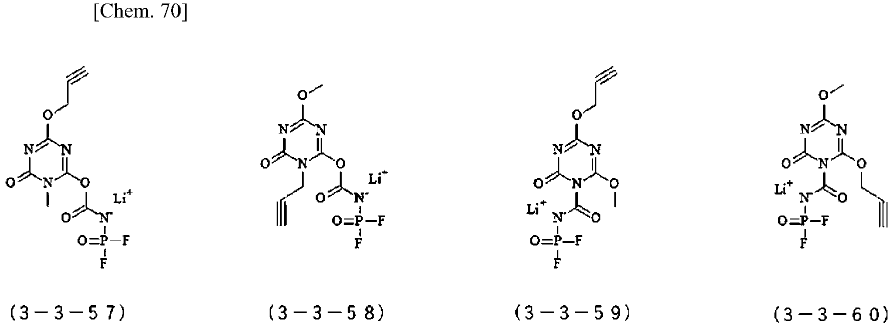

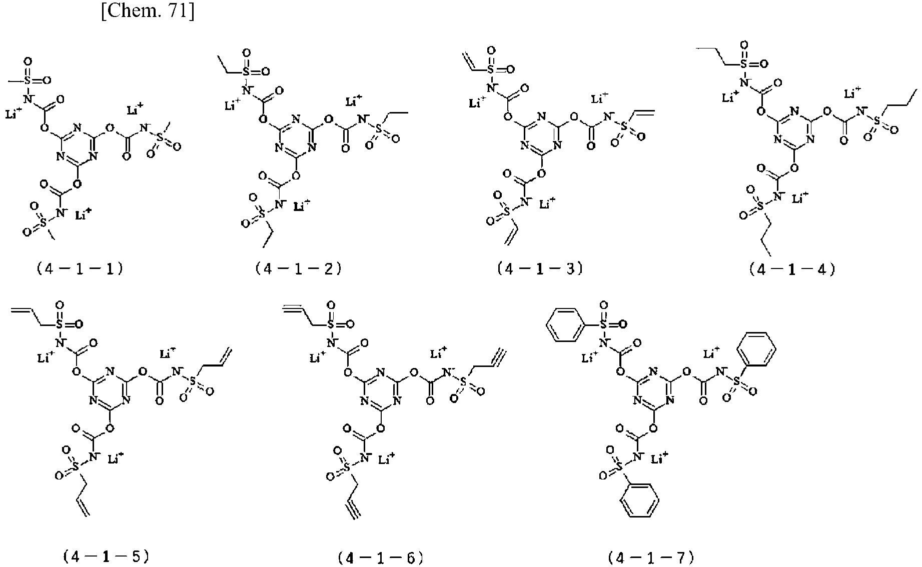

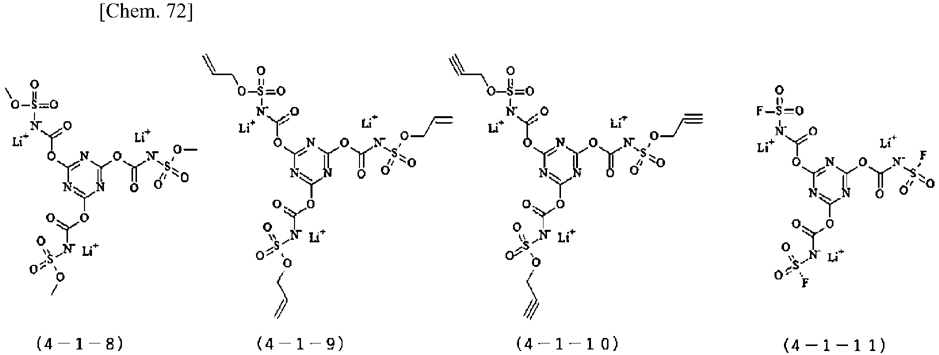

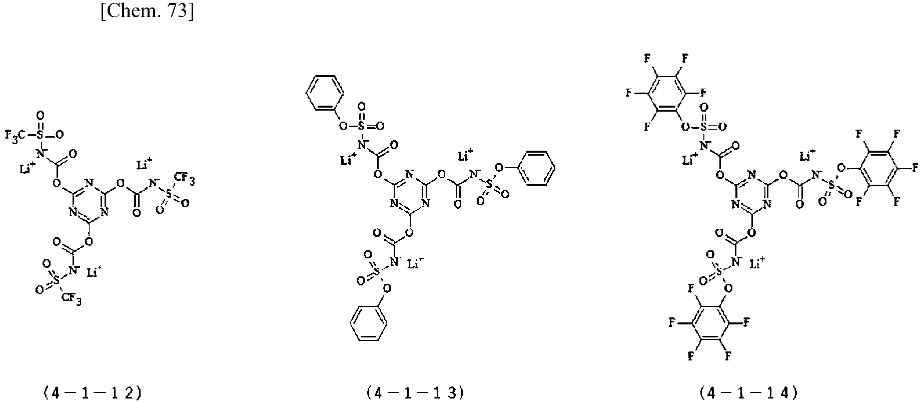

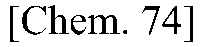

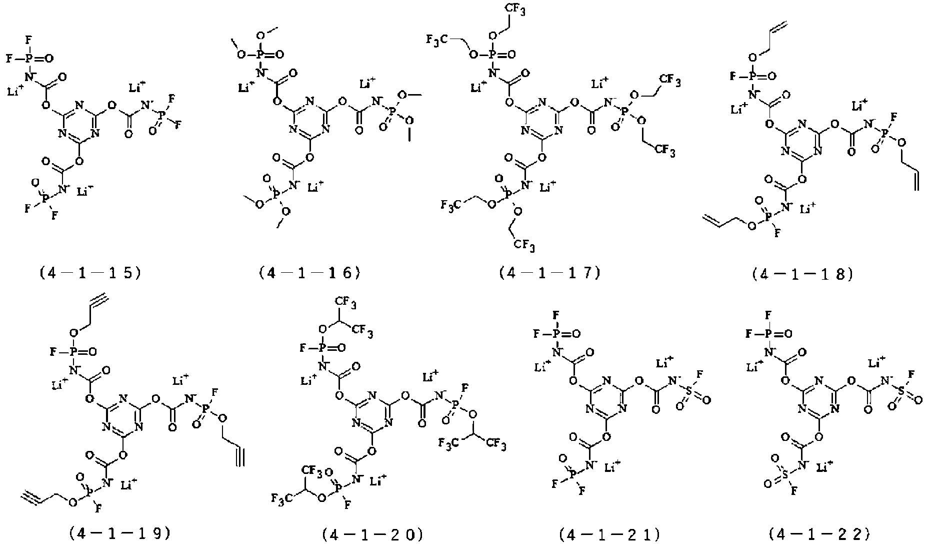

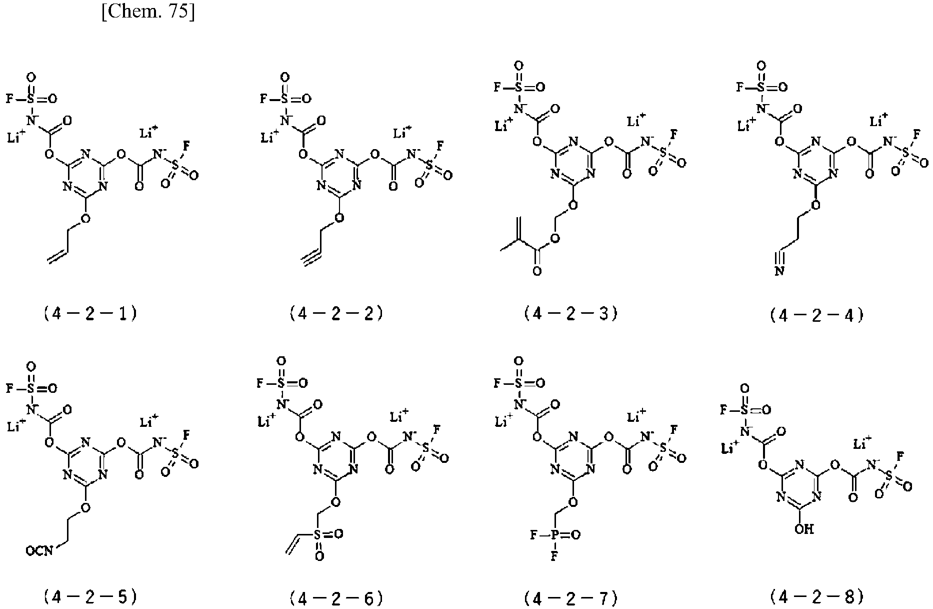

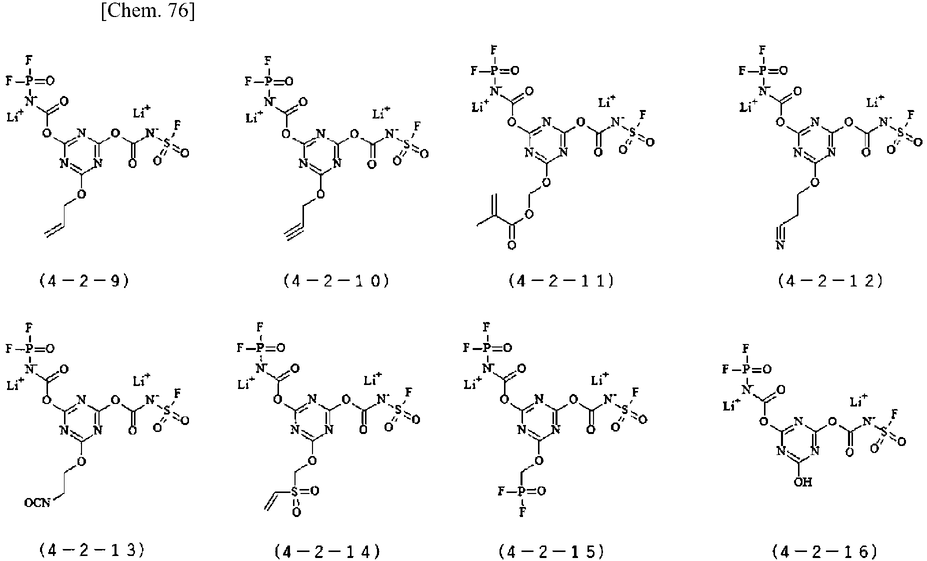

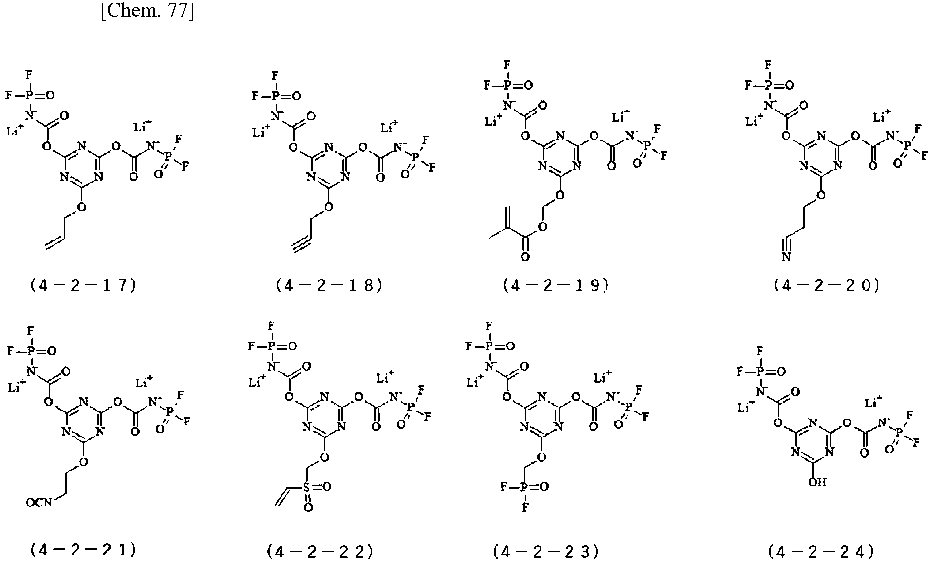

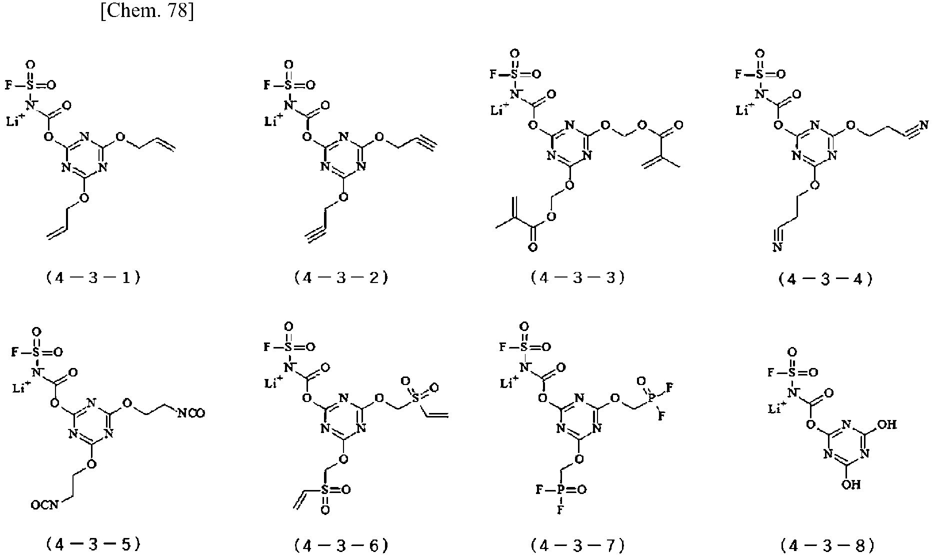

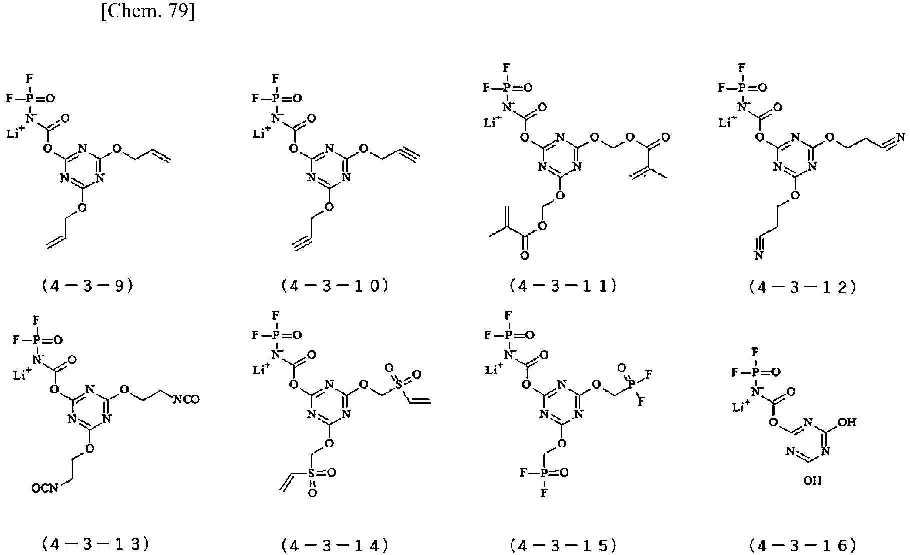

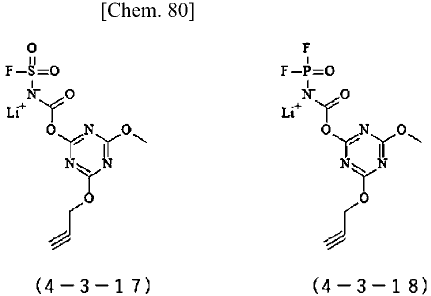

From the viewpoint of exhibiting the excellent initial input-output characteristic (low initial direct current internal resistance), of the above compounds, at least one selected from the group consisting of (1-1-1), (1-1-8), (1-1-11), (1-1-12), (1-1-15), (1-1-16), (1-1-21), (1-1-22), (1-2-8), (1-2-15), (1-2-16), (1-2-23), (1-2-24), (1-3-7), (1-3-8), (1-3-15), (1-3-16), (2-1-1), (2-1-8), (2-1-11), (2-1-12), (2-1-15), (2-1-16), (2-1-21), (2-1-22), (2-1-23), (2-1-24), (2-1-25), (2-1-26), (2-2-19), (2-2-20), (2-2-21), (2-2-22), (2-2-23), (2-2-24), (2-2-43), (2-2-44), (2-2-45), (2-2-46), (2-2-47), (2-2-48), (2-2-67), (2-2-68), (2-2-69), (2-2-70), (2-2-71), (2-2-72), (2-2-91), (2-2-92), (2-2-93), (2-2-94), (2-2-95), (2-2-96), (2-3-19), (2-3-20), (2-3-21), (2-3-22), (2-3-23), (2-3-24), (2-3-43), (2-3-44), (2-3-45), (2-3-46), (2-3-47), (2-3-48), (3-1-1), (3-1-8), (3-1-11), (3-1-12), (3-1-15), (3-1-16), (3-1-21), (3-1-22), (3-1-23), (3-1-24), (3-1-25), (3-1-26), (3-2-19), (3-2-20), (3-2-21), (3-2-22), (3-2-23), (3-2-24), (3-2-43), (3-2-44), (3-2-45), (3-2-46), (3-2-47), (3-2-48), (3-2-67), (3-2-68), (3-2-69), (3-2-70), (3-2-71), (2-2-72), (3-2-91), (2-2-92), (3-2-93), (3-2-94), (3-2-95), (3-2-96), (3-3-19), (3-3-20), (3-3-21), (3-3-22), (3-3-23), (3-3-24), (3-3-43), (3-3-44), (3-3-45), (3-3-46), (3-3-47), (3-3-48), (4-1-1), (4-1-8), (4-1-11), (4-1-12), (4-1-15), (4-1-16), (4-1-21), (4-1-22), (4-2-8), (4-2-15), (4-2-16), (4-2-23), (4-2-24), (4-3-7), (4-3-8), (4-3-15), and (4-3-16) may be adopted, at least one selected from the group consisting of (1-1-1), (1-1-8), (1-1-11), (1-1-15), (1-1-16), (1-1-21), (1-1-22), (1-2-8), (1-2-16), (1-2-24), (1-3-8), (1-3-16), (2-1-1), (2-1-8), (2-1-11), (2-1-15), (2-1-16), (2-1-21), (2-1-22), (2-1-23), (2-1-24), (2-1-25), (2-1-26), (2-2-22), (2-2-23), (2-2-24), (2-2-46), (2-2-47), (2-2-48), (2-2-70), (2-2-71), (2-2-72), (2-2-94), (2-2-95), (2-2-96), (2-3-22), (2-3-23), (2-3-24), (2-3-46), (2-3-47), (2-3-48), (3-1-1), (3-1-8), (3-1-11), (3-1-15), (3-1-16), (3-1-21), (3-1-22), (3-1-23), (3-1-24), (3-1-25), (3-1-26), (3-2-22), (3-2-23), (3-2-24), (3-2-46), (3-2-47), (3-2-48), (3-2-70), (3-2-71), (2-2-72), (3-2-94), (3-2-95), (3-2-96), (3-3-22), (3-3-23), (3-3-24), (3-3-46), (3-3-47), (3-3-48), (4-1-1), (4-1-8), (4-1-11), (4-1-15), (4-1-16), (4-1-21), (4-1-22), (4-2-8), (4-2-16), (4-2-24), (4-3-8), and (4-3-16) may be adopted, or at least one selected from the group consisting of (1-1-1), (1-1-8), (1-1-11), (1-1-15), (1-1-16), (1-1-21), (1-1-22), (1-2-8), (1-2-16), (1-2-24), (2-1-1), (2-1-8), (2-1-11), (2-1-15), (2-1-16), (2-1-21), (2-1-22), (2-1-23), (2-1-24), (2-1-25), (2-1-26), (2-2-22), (2-2-23), (2-2-24), (2-2-46), (2-2-47), (2-2-48), (2-2-70), (2-2-71), (2-2-72), (2-2-94), (2-2-95), (2-2-96), (3-1-1), (3-1-8), (3-1-11), (3-1-15), (3-1-16), (3-1-21), (3-1-22), (3-1-23), (3-1-24), (3-1-25), (3-1-26), (3-2-22), (3-2-23), (3-2-24), (3-2-46), (3-2-47), (3-2-48), (3-2-70), (3-2-71), (3-2-72), (3-2-94), (3-2-95), (3-2-96), (4-1-1), (4-1-8), (4-1-11), (4-1-15), (4-1-16), (4-1-21), (4-1-22), (4-2-8), (4-2-16), and (4-2-24) may be adopted.

From the viewpoint of a small increase rate of the direct current internal resistance after a cycle test, at least one selected from the group consisting of (1-1-5), (1-1-6), (1-1-7), (1-1-9), (1-1-13), (1-1-14), (1-1-18), (1-1-19), (1-2-1), (1-2-2), (1-2-3), (1-2-4), (1-2-9), (1-2-10), (1-2-11), (1-2-12), (1-2-17), (1-2-18), (1-2-19), (1-2-20), (1-3-1), (1-3-2), (1-3-3), (1-3-4), (1-3-9), (1-3-10), (1-3-11), (1-3-12), (2-1-5), (2-1-6), (2-1-7), (2-1-9), (2-1-13), (2-1-14), (2-1-18), (2-1-19), (2-2-1), (2-2-2), (2-2-3), (2-2-4), (2-2-5), (2-2-6), (2-2-7), (2-2-8), (2-2-9), (2-2-10), (2-2-11), (2-2-12), (2-2-25), (2-2-26), (2-2-27), (2-2-28), (2-2-29), (2-2-30), (2-2-31), (2-2-32), (2-2-33), (2-2-34), (2-2-35), (2-2-36), (2-2-49), (2-2-50), (2-2-51), (2-2-52), (2-2-53), (2-2-54), (2-2-55), (2-2-56), (2-2-57), (2-2-58), (2-2-59), (2-2-60), (2-2-73), (2-2-74), (2-2-75), (2-2-76), (2-2-77), (2-2-78), (2-2-79), (2-2-80), (2-2-81), (2-2-82), (2-2-83), (2-2-84), (2-3-1), (2-3-2), (2-3-3), (2-3-4), (2-3-5), (2-3-6), (2-3-7), (2-3-8), (2-3-9), (2-3-10), (2-3-11), (2-3-12), (2-3-13), (2-3-25), (2-3-26), (2-3-27), (2-3-28), (2-3-29), (2-3-30), (2-3-31), (2-3-32), (2-3-33), (2-3-34), (2-3-35), (2-3-36), (3-1-5), (3-1-6), (3-1-7), (3-1-9), (3-1-13), (3-1-14), (3-1-18), (3-1-19), (3-2-1), (3-2-2), (3-2-3), (3-2-4), (3-2-5), (3-2-6), (3-2-7), (3-2-8), (3-2-9), (3-2-10), (3-2-11), (3-2-12), (3-2-25), (3-2-26), (3-2-27), (3-2-28), (3-2-29), (3-2-30), (3-2-31), (3-2-32), (3-2-33), (3-2-34), (3-2-35), (3-2-36), (3-2-49), (3-2-50), (3-2-51), (3-2-52), (3-2-53), (3-2-54), (3-2-55), (3-2-56), (3-2-57), (3-2-58), (3-2-59), (3-2-60), (3-2-73), (3-2-74), (3-2-75), (3-2-76), (3-2-77), (3-2-78), (3-2-79), (3-2-80), (3-2-81), (3-2-82), (3-2-83), (3-2-84), (3-3-1), (3-3-2), (3-3-3), (3-3-4), (3-3-5), (3-3-6), (3-3-7), (3-3-8), (3-3-9), (3-3-10), (3-3-11), (3-3-12), (3-3-13), (3-3-25), (3-3-26), (3-3-27), (3-3-28), (3-3-29), (3-3-30), (3-3-31), (3-3-32), (3-3-33), (3-3-34), (3-3-35), (3-3-36), (4-1-5), (4-1-6), (4-1-7), (4-1-9), (4-1-13), (4-1-14), (4-1-18), (4-1-19), (4-2-1), (4-2-2), (4-2-3), (4-2-4), (4-2-9), (4-2-10), (4-2-11), (4-2-12), (4-2-17), (4-2-18), (4-2-19), (4-2-20), (4-3-1), (4-3-2), (4-3-3), (4-3-4), (4-3-9), (4-3-10), (4-3-11), and (4-3-12) may be adopted, at least one selected from the group consisting of (1-1-5), (1-1-6), (1-1-7), (1-1-9), (1-1-13), (1-1-18), (1-1-19), (1-2-1), (1-2-2), (1-2-4), (1-2-9), (1-2-10), (1-2-12), (1-2-17), (1-2-18), (1-2-20), (1-3-1), (1-3-2), (1-3-4), (1-3-9), (1-3-10), (1-3-12), (2-1-5), (2-1-6), (2-1-7), (2-1-9), (2-1-13), (2-1-18), (2-1-19), (2-2-1), (2-2-2), (2-2-3), (2-2-4), (2-2-5), (2-2-6), (2-2-10), (2-2-11), (2-2-12), (2-2-25), (2-2-26), (2-2-27), (2-2-28), (2-2-29), (2-2-30), (2-2-34), (2-2-35), (2-2-36), (2-2-49), (2-2-50), (2-2-51), (2-2-52), (2-2-53), (2-2-54), (2-2-58), (2-2-59), (2-2-60), (2-2-73), (2-2-74), (2-2-75), (2-2-76), (2-2-77), (2-2-78), (2-2-82), (2-2-83), (2-2-84), (2-3-1), (2-3-2), (2-3-3), (2-3-4), (2-3-5), (2-3-6), (2-3-7), (2-3-8), (2-3-9), (2-3-10), (2-3-11), (2-3-12), (2-3-13), (2-3-25), (2-3-26), (2-3-27), (2-3-28), (2-3-29), (2-3-30), (2-3-34), (2-3-35), (2-3-36), (3-1-5), (3-1-6), (3-1-7), (3-1-9), (3-1-13), (3-1-18), (3-1-19), (3-2-1), (3-2-2), (3-2-3), (3-2-4), (3-2-5), (3-2-6), (3-2-10), (3-2-11), (3-2-12), (3-2-25), (3-2-26), (3-2-27), (3-2-28), (3-2-29), (3-2-30), (3-2-34), (3-2-35), (3-2-36), (3-2-49), (3-2-50), (3-2-51), (3-2-52), (3-2-53), (3-2-54), (3-2-58), (3-2-59), (3-2-60), (3-2-73), (3-2-74), (3-2-75), (3-2-76), (3-2-77), (3-2-78), (3-2-82), (3-2-83), (3-2-84), (3-3-1), (3-3-2), (3-3-3), (3-3-4), (3-3-5), (3-3-6), (3-3-7), (3-3-8), (3-3-9), (3-3-10), (3-3-11), (3-3-12), (3-3-13), (3-3-25), (3-3-26), (3-3-27), (3-3-28), (3-3-29), (3-3-30), (3-3-34), (3-3-35), (3-3-36), (4-1-5), (4-1-6), (4-1-7), (4-1-9), (4-1-13), (4-1-18), (4-1-19), (4-2-1), (4-2-2), (4-2-4), (4-2-9), (4-2-10), (4-2-12), (4-2-17), (4-2-18), (4-2-20), (4-3-1), (4-3-2), (4-3-4), (4-3-9), (4-3-10), and (4-3-12) may be adopted, or at least one selected from the group consisting of (1-1-5), (1-1-6), (1-1-9), (1-1-13), (1-1-18), (1-1-19), (1-2-1), (1-2-2), (1-2-9), (1-2-10), (1-2-17), (1-2-18), (1-3-1), (1-3-2), (1-3-9), (1-3-10), (2-1-5), (2-1-6), (2-1-9), (2-1-13), (2-1-18), (2-1-19), (2-2-1), (2-2-2), (2-2-3), (2-2-4), (2-2-5), (2-2-6), (2-2-25), (2-2-26), (2-2-27), (2-2-28), (2-2-29), (2-2-30), (2-2-49), (2-2-50), (2-2-51), (2-2-52), (2-2-53), (2-2-54), (2-2-73), (2-2-74), (2-2-75), (2-2-76), (2-2-77), (2-2-78), (2-3-1), (2-3-2), (2-3-3), (2-3-4), (2-3-5), (2-3-6), (2-3-7), (2-3-8), (2-3-9), (2-3-13), (2-3-25), (2-3-26), (2-3-27), (2-3-28), (2-3-29), (2-3-30), (3-1-5), (3-1-6), (3-1-9), (3-1-13), (3-1-18), (3-1-19), (3-2-1), (3-2-2), (3-2-3), (3-2-4), (3-2-5), (3-2-6), (3-2-25), (3-2-26), (3-2-27), (3-2-28), (3-2-29), (3-2-30), (3-2-34), (3-2-35), (3-2-36), (3-2-49), (3-2-50), (3-2-51), (3-2-52), (3-2-53), (3-2-54), (3-2-73), (3-2-74), (3-2-75), (3-2-76), (3-2-77), (3-2-78), (3-3-1), (3-3-2), (3-3-3), (3-3-4), (3-3-5), (3-3-6), (3-3-7), (3-3-8), (3-3-9), (3-3-13), (3-3-25), (3-3-26), (3-3-27), (3-3-28), (3-3-29), (3-3-30), (4-1-5), (4-1-6), (4-1-9), (4-1-13), (4-1-18), (4-1-19), (4-2-1), (4-2-2), (4-2-9), (4-2-10), (4-2-17), (4-2-18), (4-3-1), (4-3-2), (4-3-9), and (4-3-10) may be adopted.

Furthermore, considering both viewpoints together, at least one selected from the group consisting of (1-1-2), (1-1-3), (1-1-4), (1-1-11), (1-1-15), (1-1-17), (1-1-20), (1-1-21), (1-1-22), (1-2-5), (1-2-6), (1-2-13), (1-2-14), (1-2-21), (1-2-22), (1-3-5), (1-3-6), (1-3-13), (1-3-14), (1-3-17), (1-3-18), (2-1-2), (2-1-3), (2-1-4), (2-1-11), (2-1-15), (2-1-17), (2-1-20), (2-1-21), (2-1-22), (2-1-23), (2-1-24), (2-1-25), (2-1-26), (2-2-13), (2-2-14), (2-2-15), (2-2-16), (2-2-17), (2-2-18), (2-2-37), (2-2-38), (2-2-39), (2-2-40), (2-2-41), (2-2-42), (2-2-61), (2-2-62), (2-2-63), (2-2-64), (2-2-65), (2-2-66), (2-2-85), (2-2-86), (2-2-87), (2-2-88), (2-2-89), (2-2-90), (2-3-13), (2-3-14), (2-3-15), (2-3-16), (2-3-17), (2-3-18), (2-3-37), (2-3-38), (2-3-39), (2-3-40), (2-3-41), (2-3-42), (2-3-49), (2-3-50), (2-3-51), (2-3-52), (2-3-53), (2-3-54), (2-3-55), (2-3-56), (2-3-57), (2-3-58), (2-3-59), (2-3-60), (4-1-2), (4-1-3), (4-1-4), (4-1-11), (4-1-15), (4-1-17), (4-1-20), (4-1-21), (4-1-22), (4-2-5), (4-2-6), (4-2-13), (4-2-14), (4-2-21), (4-2-22), (4-3-5), (4-3-6), (4-3-13), (4-3-14), (4-3-17), and (4-3-18) may be adopted, at least one selected from the group consisting of (1-1-2), (1-1-3), (1-1-11), (1-1-15), (1-1-17), (1-1-21), (1-1-22), (1-2-6), (1-2-14), (1-2-22), (1-3-6), (1-3-14), (1-3-17), (1-3-18), (2-1-2), (2-1-3), (2-1-11), (2-1-15), (2-1-17), (2-1-21), (2-1-22), (2-1-23), (2-1-24), (2-1-25), (2-1-26), (2-2-16), (2-2-17), (2-2-18), (2-2-40), (2-2-41), (2-2-42), (2-2-64), (2-2-65), (2-2-66), (2-2-88), (2-2-89), (2-2-90), (2-3-16), (2-3-17), (2-3-18), (2-3-40), (2-3-41), (2-3-42), (2-3-49), (2-3-50), (2-3-51), (2-3-52), (2-3-53), (2-3-54), (2-3-55), (2-3-56), (2-3-57), (2-3-58), (2-3-59), (2-3-60), (3-1-2), (3-1-3), (3-1-11), (3-1-15), (3-1-17), (3-1-21), (3-1-22), (3-1-23), (3-1-24), (3-1-25), (3-1-26), (3-2-16), (3-2-17), (3-2-18), (3-2-40), (3-2-41), (3-2-42), (3-2-64), (3-2-65), (3-2-66), (3-2-88), (3-2-89), (3-2-90), (3-3-16), (3-3-17), (3-3-18), (3-3-40), (3-3-41), (3-3-42), (3-3-49), (3-3-50), (3-3-51), (3-3-52), (3-3-53), (3-3-54), (3-3-55), (3-3-56), (3-3-57), (3-3-58), (3-3-59), (3-3-60), (4-1-2), (4-1-3), (4-1-11), (4-1-15), (4-1-17), (4-1-21), (4-1-22), (4-2-6), (4-2-14), (4-2-22), (4-3-6), (4-3-14), (4-3-17), and (4-3-18) may be adopted, or at least one selected from the group consisting of (1-1-3), (1-1-11), (1-1-15), (1-1-21), (1-1-22), (1-2-6), (1-2-14), (1-2-22), (1-3-6), (1-3-14), (1-3-17), (1-3-18), (2-1-3), (2-1-11), (2-1-15), (2-1-21), (2-1-22), (2-1-23), (2-1-24), (2-1-25), (2-1-26), (2-2-16), (2-2-17), (2-2-18), (2-2-40), (2-2-41), (2-2-42), (2-2-64), (2-2-65), (2-2-66), (2-2-88), (2-2-89), (2-2-90), (2-3-16), (2-3-17), (2-3-18), (2-3-40), (2-3-41), (2-3-42), (2-3-54), (3-1-3), (3-1-11), (3-1-15), (3-1-21), (3-1-22), (3-1-23), (3-1-24), (3-1-25), (3-1-26), (3-2-16), (3-2-17), (3-2-18), (3-2-40), (3-2-41), (3-2-42), (3-2-64), (3-2-65), (3-2-66), (3-2-88), (3-2-89), (3-2-90), (3-3-16), (3-3-17), (3-3-18), (3-3-40), (3-3-41), (3-3-42), (3-3-54), (4-1-3), (4-1-11), (4-1-15), (4-1-21), (4-1-22), (4-2-6), (4-2-14), (4-2-22), (4-3-6), (4-3-14), (4-3-17), and (4-3-18) may be adopted.

In the nonaqueous electrolyte solution of the present disclosure, the lower limit of the total amount of the component (I) with respect to the total amount (100% by mass) of the nonaqueous electrolyte solution (also referred to as "concentration of (I)") may be 0.01% by mass or more, 0.08% by mass or more, 0.3% by mass or more, or 0.8% by mass or more. The upper limit of the concentration of (I) may be 10.00% by mass or less, 5.0% by mass or less, or 2.5% by mass or less.

By setting the concentration of (I) to 0.01% by mass or more, an effect of preventing an increase in the initial resistance of the nonaqueous electrolyte solution battery using the nonaqueous electrolyte solution is easily obtained. On the other hand, by setting the concentration of (I) to 10.0% by mass or less, an increase in the viscosity of the nonaqueous electrolyte solution can be prevented, and an effect of preventing an increase in the resistance of the nonaqueous electrolyte solution battery using the nonaqueous electrolyte solution is easily obtained.

In the nonaqueous electrolyte solution of the present disclosure, one type of compound may be used alone as the component (I), or two or more types of compound may be mixed and used in any combination and any ratio according to an application.

The compound represented by the general formula (1) can be produced by various methods. The production method is not particularly limited.

For example, the compound (1-1-11) can be obtained by reacting a cyanate salt with fluorosulfonyl isocyanate and then reacting with lithium chloride.

The compound represented by the general formula (2) can be produced by various methods. The production method is not particularly limited.

For example, the compound (2-2-2) can be obtained by mixing 6-(allyloxy)-1,3,5-triazine-2,4(1H,3H)-dione with lithium hydride and then reacting with fluorosulfonyl isocyanate.

The compound represented by the general formula (3) can be produced by various methods. The production method is not particularly limited.

For example, the compound (3-3-3) can be obtained by mixing 4,6-(allyloxy)-1,3,5-triazin-2(1H)-one with lithium hydride and then reacting with fluorosulfonyl isocyanate.

The compound represented by the general formula (4) can be produced by various methods. The production method is not particularly limited.

For example, the compound (4-1-11) can be obtained by mixing cyanuric acid and lithium hydride and then reacting with fluorosulfonyl isocyanate.

The present disclosure also relates to the compound represented by the general formula (1), (2), (3), or (4).

The above compounds are suitably used as an additive in a nonaqueous electrolyte solution.

### <(II) Solute>

The nonaqueous electrolyte solution of the present disclosure contains a solute.

The solute is not particularly limited but may be an ionic salt or an ionic salt containing fluorine.

The solute may be, for example, an ionic salt containing a pair of at least one cation selected from the group consisting of an alkali metal ion such as a lithium ion and a sodium ion, an alkaline earth metal ion, and quaternary ammonium and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, and a tris(trifluoromethanesulfonyl)methide anion.

The solute may be at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

One type of these solutes may be used alone, or two or more types thereof may be mixed and used in any combination and any ratio according to an application.

Of the solutes, considering the energy density, the output characteristic, the life, and the like of the nonaqueous electrolyte solution battery, the cation may be at least one selected from the group consisting of lithium, sodium, potassium, magnesium, and quaternary ammonium, and the anion may be at least one selected from the group consisting of a hexafluorophosphate anion, a tetrafluoroborate anion, a bis(trifluoromethanesulfonyl)imide anion, and a bis(fluorosulfonyl)imide anion.

The total amount of the solute in the nonaqueous electrolyte solution of the present disclosure (hereinafter also referred to as "solute concentration") is not particularly restricted, but the lower limit may be 0.5 mol/L or more, 0.7 mol/L or more, or 0.9 mol/L or more. The upper limit of the solute concentration may be 5.0 mol/L or less, 4.0 mol/L or less, or 2.0 mol/L or less. By setting the solute concentration to 0.5 mol/L or more, the decrease in the cycle characteristic and the output characteristic of the nonaqueous electrolyte solution battery, due to a decrease in the ionic conductivity, is easily prevented, and by setting the solute concentration to 5.0 mol/L or less, the decrease in the ionic conductivity and the decrease in the cycle characteristic and the output characteristic of the nonaqueous electrolyte solution battery, due to an increase in the viscosity of the nonaqueous electrolyte solution, is easily prevented.

### <(III) Nonaqueous Organic Solvent>

The type of the nonaqueous organic solvent used in the nonaqueous electrolyte solution of the present disclosure is not particularly limited, and any nonaqueous organic solvent can be used.

The nonaqueous organic solvent may contain at least one selected from the group consisting of a cyclic ester, a chain ester, a cyclic ether, a chain ether, a sulfone compound, a sulfoxide compound, and an ionic liquid.

Specifically, the nonaqueous organic solvent may contain at least one selected from the group consisting of ethyl methyl carbonate (hereinafter also referred to as "EMC"), dimethyl carbonate (hereinafter also referred to as "DMC"), diethyl carbonate (hereinafter also referred to as "DEC"), methyl propyl carbonate, ethyl propyl carbonate, methyl butyl carbonate, methyl 2,2,2-trifluoroethyl carbonate, ethyl 2,2,2-trifluoroethyl carbonate, propyl 2,2,2-trifluoroethyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also referred to as "EC"), propylene carbonate (hereinafter also referred to as "PC"), butylene carbonate, fluoroethylene carbonate (hereinafter also referred to as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone.

In the present disclosure, an ionic liquid having a salt structure may be used as the nonaqueous organic solvent.

In terms of the excellent input-output characteristic at low temperature, at least one selected from the group consisting of a cyclic ester and a chain ester may be contained as the nonaqueous organic solvent in the nonaqueous electrolyte solution.

In addition, in terms of the excellent cycle characteristic at high temperature, at least one selected from the group consisting of a cyclic carbonate and a chain carbonate may be contained as the nonaqueous organic solvent in the nonaqueous electrolyte solution.

The nonaqueous organic solvent may contain a cyclic ester, and the cyclic ester may be a cyclic carbonate.

Specific examples of the cyclic carbonate include EC, PC, butylene carbonate, FEC, and the like, and of these, the cyclic carbonate may be at least one selected from the group consisting of EC, PC, and FEC.

The nonaqueous organic solvent may contain a chain ester, and the chain ester may be a chain carbonate.

Specific examples of the chain carbonate include EMC, DMC, DEC, methyl propyl carbonate, ethyl propyl carbonate, methyl 2,2,2-trifluoroethyl carbonate, ethyl 2,2,2-trifluoroethyl carbonate, 1,1,1,3,3,3-hexafluoro-1-propylmethyl carbonate, 1,1,1,3,3,3-hexafluoro-1-propylethyl carbonate, and the like, and of these, the chain carbonate may be at least one selected from the group consisting of EMC, DMC, DEC, and methyl propyl carbonate.

Specific examples of the chain ester include methyl acetate, ethyl acetate, methyl propionate, ethyl propionate, methyl 2-fluoropropionate, ethyl 2-fluoropropionate, and the like.

### <Other Additive>

An additive component which is generally used may be further added to the nonaqueous electrolyte solution of the present disclosure in any ratio as long as the gist of the present disclosure is not impaired.

Specific examples of the other additive include compounds having an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, lithium nitrate, sodium nitrate, potassium nitrate, methanesulfonyl fluoride, ethenesulfonyl fluoride, and phenyl difluorophosphate.

The nonaqueous electrolyte solution of the present disclosure may further contain at least one selected from the group consisting of vinylene carbonate, bis(oxalato)borate, difluorooxalatoborate, difluorobis(oxalato)phosphate, tetrafluorooxalato phosphate, (difluorophosphoryl)(fluorosulfonyl)imide salt, difluorophosphate, fluorosulfonate, nitrate, 1,3-propenesultone, 1,3-propanesultone, 1,6-diisocyanatohexane, dimethyl dicarbonate, ethynyl ethylene carbonate, trans-difluoroethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonic anhydride, methanesulfonyl fluoride, 1,4-dioxane-2,6-dione, tripropagyl phosphate, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, N,N'-carbonylbis(N-methylsulfamoylfluoride), tetravinylsilane, trivinylmethylsilane, t-butylbenzene, t-amylbenzene, fluorobenzene, and cyclohexylbenzene.

The additive content of the nonaqueous electrolyte solution may be 0.01% by mass or more and 5.0% by mass or less with respect to the total amount of the nonaqueous electrolyte solution.

The nonaqueous electrolyte solution of the present disclosure may contain a compound represented by the following general formula (6) as the other additive.

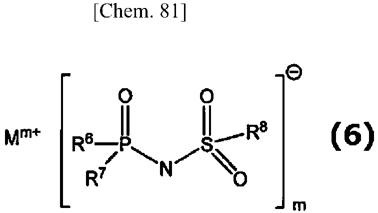

[In the general formula (6), R⁶ to R⁸ are each independently a fluorine atom or an organic group selected from the group consisting of a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group can have a fluorine atom, an oxygen atom, or an unsaturated bond. Here, at least one of R⁶ to R⁸ is a fluorine atom.

M^{m+} is an alkali metal cation, an alkaline earth metal cation, or an onium cation, and m represents the same integer as a valence of the corresponding cation.]

When the compound represented by the general formula (6) (a salt having an imide anion) has at least one P-F bond or S-F bond, an excellent low-temperature characteristic is obtained. As the number of the P-F bonds and the S-F bonds in the salt having the imide anion is larger, the low-temperature characteristic can be further improved, and thus the salt having the imide anion represented by the general formula (6) may be a compound in which all of R⁶ to R⁸ are fluorine atoms.

Moreover, the salt having the imide anion represented by the general formula (6) may be a compound
in which at least one of R⁶ to R⁸ is a fluorine atom and
at least one of R⁶ to R⁸ is selected from a hydrocarbon group having 6 or less carbon atoms which may have a fluorine atom.

In addition, the salt having the imide anion represented by the general formula (6) may be a compound
in which at least one of R⁶ to R³ is a fluorine atom and
at least one of R⁶ to R⁸ is selected from the group consisting of a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

The counter cation M^{m+} of the salt having the imide anion represented by the general formula (6) may be selected from the group consisting of a lithium ion, a sodium ion, a potassium ion, and a tetraalkylammonium ion.

In the general formula (6), examples of the alkyl group and the alkoxy group represented by R⁶ to R⁸ include an alkyl group having 1 to 10 carbon atoms and a fluorine-containing alkyl group, such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group, and an alkoxy group derived from these groups.

Examples of the alkenyl group and the alkenyloxy group include an alkenyl group having 2 to 10 carbon atoms such as a vinyl group, an allyl group, a I-propenyl group, an isopropenyl group, a 2-butenyl group, and a 1,3-butadienyl group, a fluorine-containing alkenyl group, and an alkenyloxy group derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, a fluorine-containing alkynyl group, and an alkynyloxy group derived from these groups.

Examples of the cycloalkyl group and the cycloalkoxy group include a cycloalkyl group having 3 to 10 carbon atoms such as a cyclopentyl group and a cyclohexyl group, a fluorine-containing cycloalkyl group, and a cycloalkoxy group derived from these groups.

Examples of the cycloalkenyl group and the cycloalkenyloxy group include a cycloalkenyl group having 3 to 10 carbon atoms such as a cyclopentenyl group and a cyclohexenyl group, a fluorine-containing cycloalkenyl group, and a cycloalkenyloxy group derived from these groups.

Examples of the aryl group and the aryloxy group include an aryl group having 6 to 10 carbon atoms such as a phenyl group, a tolyl group, and an xylyl group, a fluorine-containing aryl group, and an aryloxy group derived from these groups.

Specific examples and synthesis methods of the salt having the imide anion represented by the general formula (6) include those described in WO2017/111143.

The other additive content of the nonaqueous electrolyte solution may be 0.01% by mass or more and 8.0% by mass or less with respect to the total amount of the nonaqueous electrolyte solution.

When the content of the ionic salt exemplified as the solute in the nonaqueous electrolyte solution is less than 0.5 mol/L, which is the lower limit of the suitable concentration of the solute, the ionic salt can exert a negative electrode film-forming effect and a positive electrode protective effect as the "other additive". In this case, the content in the nonaqueous electrolyte solution is preferably 0.01% by mass to 5.0% by mass.

For example, when the nonaqueous electrolyte solution battery is a lithium ion battery, examples of the ionic salt in this case include lithium hexafluorophosphate, lithium tetrafluoroborate, lithium trifluoromethanesulfonate, lithium bis(trifluoromethanesulfonyl)imide, lithium bis(fluorosulfonyl)imide, lithium (trifluoromethanesulfonyl)(fluorosulfonyl)imide, and the like, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, sodium (trifluoromethanesulfonyl)(fluorosulfonyl)imide, and the like.

In addition, of the ionic salts listed as the solute, a salt compound in which the counter anion is at least one selected from the group consisting of a tetrafluoroborate anion, a chloride anion, and a perchlorate anion and the counter cation is at least one selected from the group consisting of a lithium cation, a sodium cation, a potassium cation, a tetraalkylammonium cation, and a tetraalkylphosphonium cation may be contained in the nonaqueous electrolyte solution as the "other additive" described above.

From the viewpoint of improving the durability (life) of the battery, a salt compound in which the counter anion is a bis(oxalato)borate anion and the counter cation is at least one selected from the group consisting of a lithium cation, a sodium cation, a potassium cation, a tetraalkylammonium cation, and a tetraalkylphosphonium cation may be contained in the nonaqueous electrolyte solution.

The total concentration of the salt compound may be 50 to 5000 ppm by mass, 50 to 3000 ppm by mass, or 50 to 1000 ppm by mass with respect to the total amount of the nonaqueous electrolyte solution.

An alkali metal salt other than the solutes may be used as an additive.

Specific examples include carboxylates such as lithium acrylate, sodium acrylate, lithium methacrylate, and sodium methacrylate, sulfate ester salts such as lithium methyl sulfate, sodium methyl sulfate, lithium ethyl sulfate, and sodium ethyl sulfate, and the like.

From the viewpoint of improving the durability (life) of the battery, when the nonaqueous electrolyte solution battery is a lithium ion battery, the nonaqueous electrolyte solution of the present disclosure may contain, with respect to the total amount of the nonaqueous electrolyte solution, 0.01 to 5.0% by mass of at least one selected from the group consisting of vinylene carbonate, fluoroethylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium (difluorophosphoryl)(fluorosulfonyl)imide, lithium difluorophosphate, lithium fluorosulfonate, 1,3-propenesultone, 1,3-propanesultone, 1,3,2-dioxathiolane-2,2-dioxide, and 4-propyl-1,3,2-dioxathiolane-2,2-dioxide, among the other additives.

From the same viewpoint, when the nonaqueous electrolyte solution battery is a sodium ion battery, the nonaqueous electrolyte solution may contain, with respect to the total amount of the nonaqueous electrolyte solution, 0.01 to 5.0% by mass of at least one selected from the group consisting of vinylene carbonate, fluoroethylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium (difluorophosphoryl)(fluorosulfonyl)imide, sodium difluorophosphate, sodium fluorosulfonate, 1,3-propenesultone, 1,3-propanesultone, 1,3,2-dioxathiolane-2,2-dioxide, and 4-propyl-1,3,2-dioxathiolane-2,2-dioxide.

It is preferable that the nonaqueous electrolyte solution of the present disclosure further contains (IV) at least one selected from the group consisting of a compound represented by the following general formula [1b] and a compound represented by the following general formula [1b'] in addition to (I) to (III) described above and that a content of the (IV) in the nonaqueous electrolyte solution is 10 to 25000 ppm by mass, because the initial direct current internal resistance and the increase rate of the direct current internal resistance after a cycle test are easily improved in a well-balanced manner.

Here, the phrase "the initial direct current internal resistance and the increase rate of the direct current internal resistance after a cycle test are easily improved in a well-balanced manner" using the nonaqueous electrolyte solution means that "the initial direct current internal resistance and the increase rate of the direct current internal resistance after a cycle test are equivalent or better and at least one of the initial direct current internal resistance and the increase rate of the direct current internal resistance after a cycle test is superior" compared to the case in which a content of the (IV) in the nonaqueous electrolyte solution is less than 10 ppm by mass. 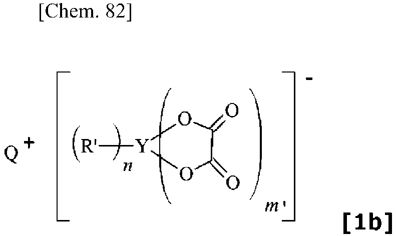
(In the general formula [1b], Y represents a boron atom, and R' represents a fluorine atom. n is 0 to 4, and m' is 0 to 2. Q⁺ represents an alkali metal ion, a tetraalkylammonium cation, or a tetraalkylphosphonium cation.)

   Q⁺[Z]⁻ [1b']
(In the general formula [1b'], the anion moiety represented by [Z]⁻ is the following structure [1b-1] or a chloride anion. Q⁺ represents an alkali metal ion, a tetraalkylammonium cation, or a tetraalkylphosphonium cation.)

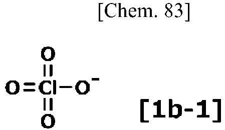

### (IV) Compound Represented by General Formula [1b] and Compound Represented by General Formula [1b']

The (IV) may be a salt compound containing a counter anion selected from a bis(oxalato)borate anion, a tetrafluoroborate anion, a chloride anion, or a perchlorate anion and a counter cation selected from a lithium cation, a sodium cation, a potassium cation, a tetraalkylammonium cation, or a tetraalkylphosphonium cation.

Specific examples of the compound represented by the general formula [1b] and the compound represented by the general formula [1b'] include, for example, the following compounds, but the compounds are not limited to these compounds.
· Lithium bis(oxalato)borate
· Lithium difluorooxalatoborate
· Lithium tetrafluoroborate
· Lithium perchlorate
· Lithium chloride
· Sodium bis(oxalato)borate
· Sodium difluorooxalatoborate
· Sodium tetrafluoroborate
· Sodium perchlorate
· Sodium chloride

Of these, from the viewpoint of easily improving the initial direct current internal resistance and the increase rate of the direct current internal resistance after a cycle test in a well-balanced manner, one from the group consisting of lithium bis(oxalato)borate, lithium difluorooxalatoborate, lithium tetrafluoroborate, lithium perchlorate, sodium bis(oxalato)borate, sodium difluorooxalatoborate, sodium tetrafluoroborate, sodium perchlorate, potassium bis(oxalato)borate, potassium difluorooxalatoborate, potassium tetrafluoroborate, and potassium perchlorate may be employed.

Since lithium or sodium is often used as the cation of a secondary battery, one from the group consisting of lithium bis(oxalato)borate, lithium difluorooxalatoborate, lithium tetrafluoroborate, lithium perchlorate, sodium bis(oxalato)borate, sodium difluorooxalatoborate, sodium tetrafluoroborate, and sodium perchlorate may be employed.

The lower limit of the content of the (IV) in the nonaqueous electrolyte solution may be 10 ppm (parts per million) by mass, 30 ppm by mass, 50 ppm by mass, or 70 ppm by mass with respect to the total amount of the electrolyte solution. The upper limit with respect to the total amount of the electrolyte solution may be 25000 ppm by mass, 15000 ppm by mass, 8000 ppm by mass, 7000 ppm by mass, or 5000 ppm by mass. When the content of the (IV) is 10 ppm by mass or more, the initial direct current internal resistance and the increase rate of the direct current internal resistance after a cycle test are easily improved in a well-balanced manner. When the content is 8000 ppm by mass or less, the initial direct current internal resistance and the increase rate of the direct current internal resistance after a cycle test are easily improved in a well-balanced manner.

The compound represented by the general formula [1b] and the compound represented by the general formula [1b'] can be produced by various methods. The production methods are not particularly limited. For example, commercially available products of KISHIDA CHEMICAL CO., LTD. and the like can be used as lithium bis(oxalato)borate, lithium tetrafluoroborate, lithium perchlorate, and lithium chloride. As lithium difluorooxalatoborate, commercially available products of Merck and the like can be used. As the sodium salt, a commercially available product may be used in the same manner, or a product obtained by cation exchange of a lithium salt may be used.

When the nonaqueous electrolyte solution battery is a lithium ion battery, the content of the cations other than lithium in the nonaqueous electrolyte solution of the present disclosure is preferably 1000 ppm by mass or less from the viewpoint of the initial input-output characteristic or the increase rate of the direct current internal resistance after a cycle test. The content is more preferably 500 ppm by mass or less, and particularly preferably 300 ppm by mass or less.

Even when the content of the cations other than lithium in the nonaqueous electrolyte solution is about 2000 ppm by mass, the initial direct current internal resistance and the increase rate of the direct current internal resistance after a cycle test are easily improved in a well-balanced manner in the nonaqueous electrolyte solution containing the (I) and a predetermined amount (10 to 25000 ppm by mass in the nonaqueous electrolyte solution) of the (IV). This is advantageous in terms of controlling the content of the cations other than lithium contained in the raw material in the preparation of the nonaqueous electrolyte solution. From the above viewpoint, when the nonaqueous electrolyte solution battery is a lithium ion battery, the content of the cations other than lithium in the nonaqueous electrolyte solution containing the (I) and a predetermined amount of the (IV) may be 2000 ppm by mass or less. From the viewpoint of easily improving the initial direct current internal resistance and the increase rate of the direct current internal resistance after a cycle test in a well-balanced manner, the content of the cations other than lithium in the nonaqueous electrolyte solution is preferably as small as possible and is, for example, preferably 1000 ppm by mass or less, more preferably 500 ppm by mass or less, and particularly preferably 300 ppm by mass or less.

When the nonaqueous electrolyte solution battery is a lithium ion battery and when a cation other than lithium is contained in the nonaqueous electrolyte solution, examples of the nonaqueous electrolyte solution include a nonaqueous electrolyte solution composition in which the cation species of the solute is lithium, the cation species of (I) is lithium, and the cation species of the "other salt compound (for example, (IV))" is other than lithium.

The content of the cations other than lithium in the nonaqueous electrolyte solution of the lithium ion battery can be determined by ICP emission spectrometry.

When the nonaqueous electrolyte solution battery is a sodium ion battery, the content of the cations other than sodium in the nonaqueous electrolyte solution of the present disclosure is preferably 500 ppm by mass or less from the viewpoint of both the initial input-output characteristic and the increase rate of the direct current internal resistance after a cycle test together. The content is more preferably 400 ppm by mass or less, and particularly preferably 200 ppm by mass or less.

Even when the content of the cations other than sodium in the nonaqueous electrolyte solution is about 700 ppm by mass, the initial direct current internal resistance and the increase rate of the direct current internal resistance after a cycle test are easily improved in a well-balanced manner in the nonaqueous electrolyte solution containing the (I) and a predetermined amount (10 to 25000 ppm by mass in the nonaqueous electrolyte solution) of the (IV). This is advantageous in terms of controlling the content of the cations other than sodium contained in the raw material in the preparation of the nonaqueous electrolyte solution. From the above viewpoint, when the nonaqueous electrolyte solution battery is a sodium ion battery, the content of the cations other than sodium in the nonaqueous electrolyte solution containing the (I) and a predetermined amount of the (IV) may be 700 pp by mass or less. From the viewpoint of easily improving the initial direct current internal resistance and the increase rate of the direct current internal resistance after a cycle test in a well-balanced manner, the content of the cations other than sodium in the nonaqueous electrolyte solution is preferably as small as possible and is, for example, preferably 500 ppm by mass or less, more preferably 400 ppm by mass or less, and particularly preferably 200 ppm by mass or less.

When the nonaqueous electrolyte solution battery is a sodium ion battery and when a cation other than sodium is contained in the nonaqueous electrolyte solution, examples of the nonaqueous electrolyte solution include a nonaqueous electrolyte solution composition in which the cation species of the solute is sodium, the cation species of (I) is sodium, and the cation species of the "other salt compound (for example, (IV))" is other than sodium.

The content of the cations other than sodium in the nonaqueous electrolyte solution of the sodium ion battery can be determined by ICP emission spectrometry.

In addition, the nonaqueous electrolyte solution of the present disclosure can also contain a polymer, and as in the case of being used in a nonaqueous electrolyte solution battery referred to as a polymer battery, the nonaqueous electrolyte solution can be quasi-solidified with a gelling agent or a cross-linked polymer before use. The polymer solid electrolyte includes one containing a nonaqueous organic solvent as a plasticizer.

The polymer is not particularly limited as long as the polymer is an aprotic polymer capable of dissolving the component (I), the solute, and the other additive. Examples thereof include polymers having polyethylene oxide as a principal chain or a side chain, homopolymers or copolymers of polyvinylidene fluoride, methacrylic acid ester polymers, polyacrylonitrile, and the like. When a plasticizer is added to such a polymer, of the above nonaqueous organic solvents, an aprotic nonaqueous organic solvent may be used.

### [2. Nonaqueous Electrolyte Solution Battery]

The nonaqueous electrolyte solution battery of the present disclosure includes, at least, the nonaqueous electrolyte solution of the present disclosure described above, a negative electrode, and a positive electrode. Further, a separator, an exterior body, and the like may be included.

The nonaqueous electrolyte solution battery of the present disclosure preferably includes at least a positive electrode, a negative electrode, a separator, and the nonaqueous electrolyte solution of the present disclosure.

The nonaqueous electrolyte solution battery of the present disclosure is preferably a nonaqueous electrolyte solution secondary battery.

The negative electrode is not particularly limited, but a material capable of reversibly intercalating and deintercalating alkali metal ions such as lithium ions and sodium ions or alkaline earth metal ions may be used.

For example, in the case of a lithium ion secondary battery in which cations are mainly lithium, the negative electrode active material constituting the negative electrode is one capable of doping and dedoping lithium ions. Examples of the negative electrode active material include a material containing at least one selected from a carbon material in which the d value of the lattice plane (002) plane in X-ray diffraction is 0.340 nm or less, a carbon material in which the d value of the lattice plane (002) plane in X-ray diffraction exceeds 0.340 nm, oxides of one or more metals selected from the group consisting of Si, Sn, and Al, one or more metals selected from the group consisting of Si, Sn, and Al, alloys containing these metals, alloys of the metals or the alloys and lithium, and lithium titanium oxide. One type of these negative electrode active materials can be used alone, or two or more types thereof can be used in combination. Lithium metal, a metal nitride, a tin compound, a conductive polymer, and the like may also be used.

Preferable examples of the negative electrode active material include those containing Si and/or an Si metal oxide and a carbon material. The Si is silicon metal. The Si metal oxide may be a compound represented by SiOx (x is a value of 0.5 to 1.5). Here, the total content of the Si and/or the Si metal oxide contained in the negative electrode active material may be 0.1 to 50% by mass, and preferably 0.1 to 30% by mass, when the total amount of the Si and/or the Si metal oxide and the carbon material contained in the negative electrode active material is 100% by mass. As the carbon material, graphite is preferable, and various types of artificial graphite, natural graphite, and hard carbon (non-graphitizing carbon) can be used. With graphite, the change in crystal structure due to occlusion and release of lithium is very small, and thus a high energy density and an excellent cycle characteristic can be obtained. The shape of the graphite may be any of a fibrous shape, a spherical shape, a granular shape, and a scale-like shape. Amorphous carbon or graphite coated with amorphous carbon on the surface is more preferable because the reactivity between the surface of the material and the electrolyte solution is low.

One type of these negative electrode active materials can be used alone, or two or more types thereof can be used in combination.

For example, in the case of a sodium ion secondary battery in which the cations are mainly sodium, as the negative electrode active material constituting the negative electrode, sodium metal, an alloy of sodium metal and another metal such as tin, an intermetallic compound, various carbon materials such as hard carbon, a metal oxide such as titanium oxide, a metal nitride, (elemental) tin, a tin compound, activated carbon, a conductive polymer, and the like may be used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like may be used. One type of these negative electrode active materials may be used alone, or two or more types thereof may be used in combination.

The positive electrode is not particularly limited, but a material capable of reversibly intercalating and deintercalating alkali metal ions such as lithium ions and sodium ions or alkaline earth metal ions may be used.

For example, when the cation is lithium, lithium-containing transition metal oxide composites such as LiCoO₂, LiNiO₂, LiMnO₂, and LiMn₂O₄, lithium-containing transition metal oxide composites including a mixture of a plurality of transition metals in the lithium-containing transition metal oxide composites such as Co, Mn, and Ni, or lithium-containing transition metal oxide composites in which a part of the transition metals in the lithium-containing transition metal oxide composites is substituted with a metal other than the transition metals may be used as a positive electrode material. Specific examples thereof include Li[Ni_{1/3}Mn_{1/3}Co_{1/3}]O₂, Li[Ni_{0.45}Mn_{0.35}Co_{0.2}]O₂, Li[Ni_{0.5}Mn_{0.3}Co_{0.2}]O₂, Li[Ni_{0.6}Mn_{0.2}Co_{0.2}]O₂ (hereinafter sometimes referred to as "NCM622"), Li[Ni_{0.8}Mn_{0.1}Co_{0.1}]O₂ (hereinafter sometimes referred to as "NCM811"), Li[Ni_{0.49}Mn_{0.3}Co_{0.2}Zr_{0.01}]O₂, Li[Ni_{0.49}Mn_{0.3}Co_{0.2}Mg_{0.01}]O₂, LiNi_{0.8}Co_{0.2}O₂, LiNi_{0.85}Co_{0.10}Al_{0.05}O₂, LiNi_{0.87}Co_{0.10}Al_{0.03}O₂, LiNi_{0.90}Co_{0.07}Al_{0.03}O₂, LiNi_{0.6}Co_{0.3}Al_{0.1}O₂, LiNi_{0.5}Mn_{1.5}O₄, LiNi_{0.5}Mn_{0.5}O₂, LiNi_{0.1}Mn_{1.9}O₄, LiCo_{0.5}Mn_{0.5}O₂, 0.5[LiNi_{0.5}Mn_{0.5}O₂]·0.5[Li₂MnO₃], 0.5[LiNi_{1/3}CO_{1/3}Mn_{1/3}O₂]·0.5[Li₂MnO₃], 0.5[LiNi_{0.375}Co_{0.25}Mn_{0.375}O₂]·0.5[Li₂MnO₃], 0.5[LiNi_{0.375}Co_{0.125}Fe_{0.125}Mn_{0.375}O₂]·0.5[Li₂MnO₃], 0.45[LiNi_{0.375}Co_{0.25}Mn_{0.375}O₂]·0.10[Li₂TiO₃]·0.45[Li₂MnO₃], and the like.

In addition, phosphoric acid compounds of transition metals such as LiFePO₄ called olivine, LiCoPO₄, and LiMnPO₄, oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used.

For example, when the cation is sodium, sodium-containing transition metal oxide composites such as NaCrO₂, NaFe_{0.5}Co_{0.5}O₂, NaFe_{0.4}Mn_{0.3}Ni_{0.3}O₂, NaNi_{0.5}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Ti_{1/6}Mn_{1/2}O₂, and Na_{2/3}Ni_{1/3}Mn_{2/3}O₂, sodium-containing transition metal oxide composites including a mixture of a plurality of transition metals in the sodium-containing transition metal oxide composites such as Co, Mn, and Ni, sodium-containing transition metal oxide composites in which a part of the transition metals in the sodium-containing transition metal oxide composites is substituted with a metal other than the transition metals, polyanion type compounds such as NaFePO₄, NaVPO₄F, Na₃V₂(PO₄)₃, and Na₂Fe₂(SO₄)₃, sodium salts of Prussian Blue analogues represented by a compositional formula NaₐM_{b}[Fe(CN)₆]_{c} (M represents Cr, Mn, Fe, Co, Ni, Cu, or Zn, 0 ≤ a ≤ 2, 0.5 ≤ b ≤ 1.5, and 0.5 ≤ c ≤ 1.5), oxides such as TiO₂, V₂O₃, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material (positive electrode active material).

Acetylene black, Ketjen black, carbon fibers, or graphite as a conductive material and polytetrafluoroethylene, polyvinylidene fluoride, SBR resin, or the like as a binder may be added to the positive electrode and negative electrode materials, and an electrode sheet molded into a sheet shape may also be used.

As a separator for preventing contact between the positive electrode and the negative electrode, a nonwoven fabric or a porous sheet made of polypropylene, polyethylene, paper, glass fibers, or the like may be used.

An electrochemical device having a shape such as a coin shape, a cylindrical shape, a square shape, or an aluminum laminate sheet shape is assembled with the above elements.

### Examples

Hereinafter, the present disclosure will be described in more detail with Examples, but the present disclosure is not restricted by these descriptions.

### <Synthesis Example 1-1>

### Synthesis of Compound (1-1-11)

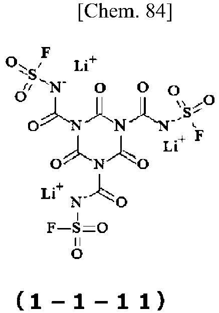

To a 50-ml recovery flask, 30 g of acetonitrile (hereinafter also referred to as "MeCN"), 0.26 g (2 mmol) of isocyanuric acid, and 0.048 g (6 mmol) of lithium hydride were added, and after stirring at 20 to 30°C for an hour, 0.81 g (6.5 mmol) of fluorosulfonyl isocyanate was slowly added. After stirring at 30°C or lower for an hour, concentration was carried out to obtain 0.97 g (recovery rate: 93%) of Compound (1-1-11).

### <Synthesis Example 1-2>

### Synthesis of Compound (1-3-1)

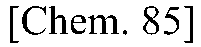

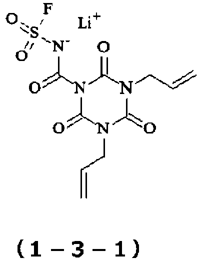

To a 50-ml recovery flask, 30 g of MeCN, 0.21 g (1 mmol) of diallyl isocyanurate, and 0.008 g (1 mmol) of lithium hydride were added, and after stirring at 20 to 30°C for an hour, 0.14 g (1.1 mmol) of fluorosulfonyl isocyanate was slowly added. After stirring at 40°C or lower for 15 hours, concentration was carried out to obtain 0.33 g (recovery rate: 96%) of Compound (1-3-1).
¹H NMR (CD₃CN) σ_{H} 5.91-5.79, 5.23-5.13, 4.42 ppm,
¹⁹F NMR (CD₃CN) σ_{F} 48.0 ppm.

### <Synthesis Example 2-1>

### Synthesis of Compound (2-2-2)

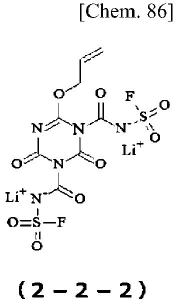

To a 50-ml recovery flask, 30 g of MeCN, 0.17 g (1 mmol) of 6-(allyloxy)-1,3,5-triazine-2,4(1H,3H)-dione, and 0.016 g (2 mmol) of lithium hydride were added, and after stirring at 20 to 30°C for an hour, 0.25 g (2 mmol) of fluorosulfonyl isocyanate was slowly added. After stirring at 40°C or lower for 15 hours, concentration was carried out to obtain 0.41 g (recovery rate: 95%) of Compound (2-2-2). ¹H NMR (CD₃CN) σ_{H} 6.10-5.92, 5.41-5.21, 4.83 ppm,
¹⁹F NMR (CD₃CN) σ_{F} 48.0 ppm, 48.2 ppm

### <Synthesis Example 3-1>

### Synthesis of Compound (3-3-3)

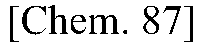

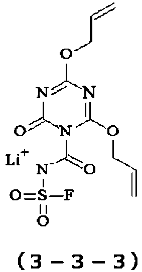

To a 50-ml recovery flask, 30 g of ethyl methyl carbonate (hereinafter also referred to as "EMC"), 0.21 g (1 mmol) of 4,6-(allyloxy)-1,3,5-triazin-2(1H)-one, 0.008 g (1 mmol) of lithium hydride were added, and after stirring at 20 to 30°C for 10 minutes, 0.12 g (1 mmol) of fluorosulfonyl isocyanate was slowly added. After stirring at 40°C or lower for two hours, concentration was carried out to obtain 0.33 g (recovery rate: 97%) of Compound (3-3-3).
¹H NMR (CD₃CN) σ_{H} 6.08-5.95, 5.48-5.25, 4.93 ppm, 4.79 ppm
¹⁹F NMR (CD₃CN) σ_{F} 48.0 ppm

### <Synthesis Example 3-2>

### Synthesis of Compound (3-3-54)

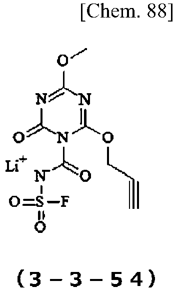

To a 50-ml recovery flask, 30 g of EMC, 0.18 g (1 mmol) of 4-methoxy,6-propargyloxy-1,3,5-triazin-2(1H)-one, and 0.008 g (1 mmol) of lithium hydride were added, and after stirring at 20 to 30°C for 10 minutes, 0.12 g (1 mmol) of fluorosulfonyl isocyanate was slowly added. After stirring at 40°C or lower for three hours, concentration was carried out to obtain 0.29 g (recovery rate: 95%) of Compound (3-3-54).
¹H NMR(CD₃CN) σ_{H} 4.92 ppm, 4.03 ppm, 2.83 ppm,
¹⁹F NMR (CD₃CN) σ_{F} 47.5 ppm

### <Synthesis Example 4-1>

### Synthesis of Compound (4-3-1)

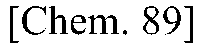

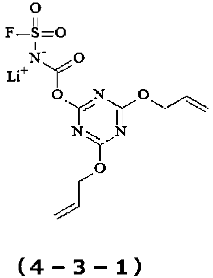

To a 50-ml recovery flask, 30 g of EMC and 0.21 g (1 mmol) of diallyl cyanurate Li salt were added, and after stirring at 20 to 30°C for 10 minutes, 0.19 g (1.5 mmol) of fluorosulfonyl isocyanate was slowly added. After stirring at 40°C or lower for two hours, the production of Compound (4-3-1) was observed by NMR.
¹H NMR (CD₃CN) σ_{H} 6.07-5.96, 5.41-5.27, 4.86 ppm
¹⁹F NMR (CD₃CN) σ_{F} 48.0 ppm

### <Synthesis Example 4-2>

### Synthesis of Compound (4-1-11)

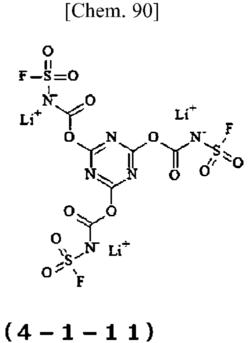

Compound (4-1-11) was synthesized in the same manner as in the synthesis method of Compound (4-3-1) except that the raw materials were changed according to the structure of the target product.

In addition, the following Compounds (1-1-11-Na), (1-3-1-Na), (2-2-2-Na), (3-3-3-Na), (4-1-11-Na), and (4-3-1-Na) were obtained by subjecting the above compounds to cation exchange reaction.

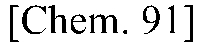

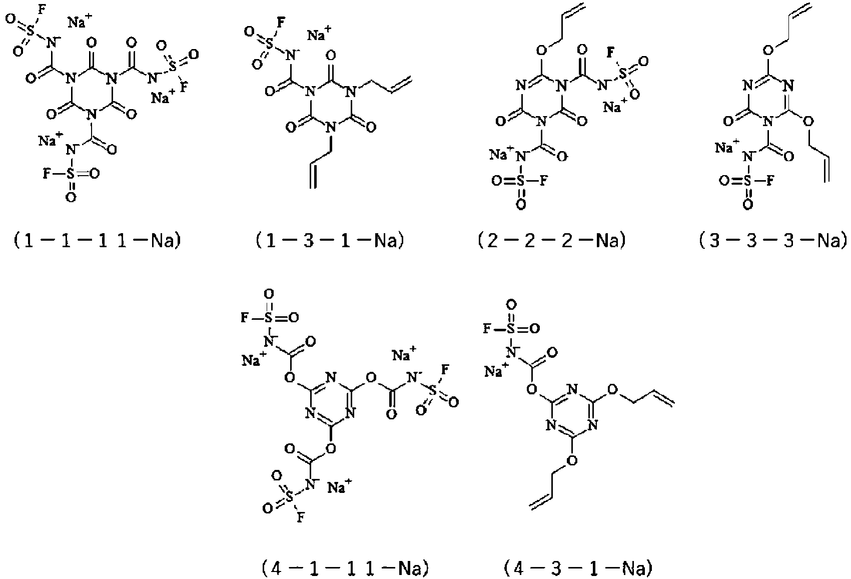

### <Example 1-1>

### (Preparation of Nonaqueous Electrolyte Solution 1-1)

A mixed solvent of ethylene carbonate (hereinafter also referred to as "EC"), dimethyl carbonate (hereinafter also referred to as "DMC"), and EMC at a volume ratio of 3:3:4 was used as a nonaqueous organic solvent, and lithium hexafluorophosphate (hereinafter also referred to as "LiPF₆") at a concentration of 1.0 mol/L as a solute and the compound represented by the formula (1-1-11) as a compound represented by the general formula (1) at a concentration of 0.05% by mass with respect to the total amount of the nonaqueous electrolyte solution were dissolved in the solvent to obtain a nonaqueous electrolyte solution 1-1. The above preparation was performed while maintaining the liquid temperature at 25°C.

### <Examples 1-2 to 1-12>

### (Preparation of Nonaqueous Electrolyte Solutions 1-2 to 1-12)

Nonaqueous electrolyte solutions 1-2 to 1-12 were prepared by dissolving by the same procedure as in the preparation of the nonaqueous electrolyte solution 1-1 except that the type and the concentration of the compound represented by the general formula (1) were changed as shown in Table 1.

### <Examples 2-1 to 2-6>

### (Preparation of Nonaqueous Electrolyte Solutions 2-1 to 2-6)

Nonaqueous electrolyte solutions 2-1 to 2-6 were prepared by dissolving by the same procedure as in the preparation of the nonaqueous electrolyte solution 1-1 except that the compound represented by the general formula (2) was used instead of the compound represented by the general formula (1) and that the concentration thereof was changed as shown in Table 1.

### <Examples 3-1 to 3-6>

### (Preparation of Nonaqueous Electrolyte Solutions 3-1 to 3-6)

Nonaqueous electrolyte solutions 3-1 to 3-6 were prepared by dissolving by the same procedure as in the preparation of the nonaqueous electrolyte solution 1-1 except that the compound represented by the general formula (3) was used instead of the compound represented by the general formula (1) and that the concentration thereof was changed as shown in Table 1.

### <Examples 4-1 to 4-12>

### (Preparation of Nonaqueous Electrolyte Solutions 4-1 to 4-12)

Nonaqueous electrolyte solutions 4-1 to 4-12 were prepared by dissolving by the same procedure as in the preparation of the nonaqueous electrolyte solution 1-1 except that the compound represented by the general formula (4) was used instead of the compound represented by the general formula (1) and that the concentration thereof was changed as shown in Table 1.

### <Examples 5-1 to 5-9>

### (Preparation of Nonaqueous Electrolyte Solutions 5-1 to 5-9)

Nonaqueous electrolyte solutions 5-1 to 5-9 were prepared by dissolving by the same procedure as in the preparation of the nonaqueous electrolyte solution 1-1 except that the type and the concentration of the compound represented by the general formula (1), (2), (3), or (4) were changed as shown in Table 2.

### <Comparative Example 1-1>

### (Preparation of Comparative Nonaqueous Electrolyte Solution 1-1)

A comparative nonaqueous electrolyte solution 1-1 was prepared by dissolving by the same procedure as in the preparation of the nonaqueous electrolyte solution 1-1 except that the compound represented by the general formula (1) was not added.

### <Comparative Examples 1-2 to 1-4>

### (Preparation of Comparative Nonaqueous Electrolyte Solutions 1-2 to 1-4)

Comparative nonaqueous electrolyte solutions 1-2 to 1-4 were prepared by dissolving by the same procedure as in the preparation of the nonaqueous electrolyte solution 1-1 except that the compound represented by the following formula (6-1), (6-2), or (6-3) was used instead of the compound represented by the general formula (1) and that the concentration thereof was changed as shown in Table 1. Compounds (6-1) and (6-2) used were purchased from Tokyo Chemical Industry Co., Ltd. For (6-3), referring to Patent Literature 4, 0.71 g (5.7 mmol) of ethene sulfonyl fluoride and 0.25 g (1.89 mmol) of cyanuric acid were dissolved in 30 ml of ethanol, and then 0.16 g (1.9 mmol) of sodium acetate was added thereto at an ice bath temperature. The mixture was stirred at room temperature for 12 hours. The obtained suspension was filtered and washed with water, ethanol, and diethyl ether. The product was dried under reduced pressure, and a white solid obtained was used.

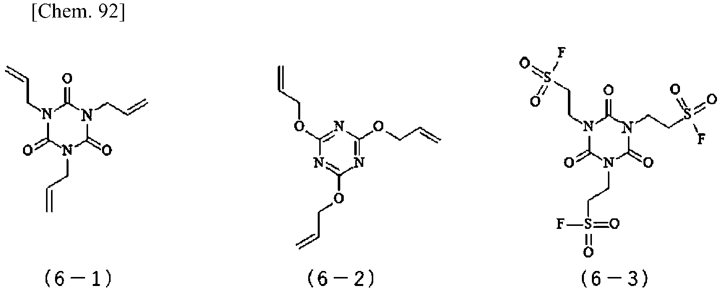

### <Examples 6-1 to 6-6 and Comparative Examples 2-1 to 2-4>

### (Preparation of Nonaqueous Electrolyte Solutions 6-1 to 6-6 and Comparative Nonaqueous Electrolyte Solutions 2-1 to 2-4)

Nonaqueous electrolyte solutions 6-1 to 6-6 and comparative nonaqueous electrolyte solutions 2-1 to 2-4 were obtained in the same manner as in the preparation of the nonaqueous electrolyte solutions 1-4, 1-10, 2-4, 3-4, 4-4, and 4-10 and the comparative nonaqueous electrolyte solutions 1-1 to 1-4 except that vinylene carbonate (hereinafter also referred to as "VC") as the other additive (1) and lithium bis(oxalato)borate (hereinafter also referred to as "BOB") as the other additive (2) were further added and dissolved at the concentrations shown in Table 3.

### <Examples 7-1 to 7-6 and Comparative Examples 3-1 to 3-4> to <Examples 13-1 to 13-6 and Comparative Examples 9-1 to 9-4>

As shown in Tables 3 and 4, the nonaqueous electrolyte solutions and the comparative nonaqueous electrolyte solutions shown in the tables were prepared by dissolving in the same manner as in the preparation of the nonaqueous electrolyte solutions 6-1 to 6-6 and the comparative nonaqueous electrolyte solutions 2-1 to 2-4 except that the other additive (2) was changed from BOB to the respective compounds shown in the tables. Here, "DFBOP" means lithium difluorobis(oxalato)phosphate, "DFOB" means lithium difluorooxalatoborate, "TFOP" means lithium tetrafluorooxalatophosphate, "DTD" means 1,3,2-dioxathiolane-2,2-dioxide, "DFPFSI" means lithium (difluorophosphoryl)(fluorosulfonyl)imide, "FS" means lithium fluorosulfonate, and "TV-Si" means tetravinylsilane.

### <Examples 14-1 to 14-6 and Comparative Examples 10-1 to 10-4> to <Examples 37-1 to 37-6 and Comparative Examples 33-1 to 33-4>

As shown in Tables 5 to 10, the nonaqueous electrolyte solutions and the comparative nonaqueous electrolyte solutions shown in the tables were prepared by dissolving in the same manner as in the preparation of the nonaqueous electrolyte solutions 6-1 to 6-6 and the comparative nonaqueous electrolyte solutions 2-1 to 2-4 except that the compounds shown in "Additional Solute" in the tables in the amounts shown were added as the solute and that the other additive (2) was changed to the respective compounds shown in the tables. Here, "DFP" means lithium difluorophosphate, "FSI" means lithium bis(fluorosulfonyl)imide, and "BF₄" means lithium tetrafluoroborate.

### <Examples 38-1 to 38-12 and Comparative Examples 34-1 to 34-4> to <Examples 66-1 to 66-6 and Comparative Examples 58-1 to 58-4>

The nonaqueous electrolyte solutions and the comparative nonaqueous electrolyte solutions shown in the tables were prepared by dissolving in the same manner as in the preparation of the electrolyte solutions shown in Table 1 to Table 6, Table 9, and Table 10 except for using a mixed solvent of EC, fluoroethylene carbonate (hereinafter also referred to as "FEC"), DMC, and EMC at a volume ratio of 3:0.2:3:3.8 as a nonaqueous organic solvent, dissolving LiPF₆ and FSI as solutes in the solvent at concentrations of 1.0 mol/L and 0.1 mol/L, respectively, changing the other additive (2) to the respective compounds shown in the tables as shown in Table 11 to Table 18, and adding the compounds shown in "Additional Solute" in the tables in the amounts shown as a further solute.

### <Example 67-1>

### (Preparation of Nonaqueous Electrolyte Solution 67-1)

A mixed solvent of EC, propylene carbonate (hereinafter also referred to as "PC"), FEC, and EMC at a volume ratio of 2:1:0.2:6.8 was used as a nonaqueous organic solvent, and sodium hexafluorophosphate (hereinafter also referred to as "NaPF₆") at a concentration of 1.0 mol/L as a solute and the compound represented by the formula (1-1-11-Na) as a compound represented by the general formula (1) at a concentration of 0.05% by mass with respect to the total amount of the nonaqueous electrolyte solution were dissolved in the solvent to obtain a nonaqueous electrolyte solution 67-1. The above preparation was performed while maintaining the liquid temperature at 25°C.

### <Examples 67-2 to 67-12>

### (Preparation of Nonaqueous Electrolyte Solutions 67-2 to 67-12)

Nonaqueous electrolyte solutions 67-2 to 67-12 were prepared by dissolving by the same procedure as in the preparation of the nonaqueous electrolyte solution 67-1 except that the type and the concentration of the compound represented by the general formula (1) were changed as shown in Table 19.

### <Examples 68-1 to 68-6>

### (Preparation of Nonaqueous Electrolyte Solutions 68-1 to 68-6)

Nonaqueous electrolyte solutions 68-1 to 68-6 were prepared by dissolving by the same procedure as in the preparation of the nonaqueous electrolyte solution 67-1 except that the compound represented by the general formula (2) was used instead of the compound represented by the general formula (1) and that the concentration thereof was changed as shown in Table 19.

### <Examples 69-1 to 69-6>

### (Preparation of Nonaqueous Electrolyte Solutions 69-1 to 69-6)

Nonaqueous electrolyte solutions 69-1 to 69-6 were prepared by dissolving by the same procedure as in the preparation of the nonaqueous electrolyte solution 67-1 except that the compound represented by the general formula (3) was used instead of the compound represented by the general formula (1) and that the concentration thereof was changed as shown in Table 19.

### <Examples 70-1 to 70-12>

### (Preparation of Nonaqueous Electrolyte Solutions 70-1 to 70-12)

Nonaqueous electrolyte solutions 70-1 to 70-12 were prepared by dissolving by the same procedure as in the preparation of the nonaqueous electrolyte solution 67-1 except that the compound represented by the general formula (4) was used instead of the compound represented by the general formula (1) and that the concentration thereof was changed as shown in Table 19.

### <Examples 71-1 to 71-9>

### (Preparation of Nonaqueous Electrolyte Solutions 71-1 to 71-9)

Nonaqueous electrolyte solutions 71-1 to 71-9 were prepared by dissolving by the same procedure as in the preparation of the nonaqueous electrolyte solution 67-1 except that the type and the concentration of the compound represented by the general formula (1), (2), (3), or (4) were changed as shown in Table 20.

### <Comparative Example 59-1>

### (Preparation of Comparative Nonaqueous Electrolyte Solution 59-1)

A comparative nonaqueous electrolyte solution 59-1 was prepared by dissolving by the same procedure as in the preparation of the nonaqueous electrolyte solution 67-1 except that the compound represented by the general formula (1) was not added.

### <Comparative Examples 59-2 to 59-4>

### (Preparation of Comparative Nonaqueous Electrolyte Solutions 59-2 to 59-4)

Comparative nonaqueous electrolyte solutions 59-2 to 59-4 were prepared by dissolving by the same procedure as in the preparation of the nonaqueous electrolyte solution 67-1 except that the compound represented by the formula (6-1) or (6-2) was used instead of the compound represented by the general formula (1) and that the concentration thereof was changed as shown in Table 19.

### <Examples 72-1 to 72-6 and Comparative Examples 60-1 to 60-4>

### (Preparation of Nonaqueous Electrolyte Solutions 72-1 to 72-6 and Comparative Nonaqueous Electrolyte Solutions 60-1 to 60-4)

Nonaqueous electrolyte solutions 72-1 to 72-6 and comparative nonaqueous electrolyte solutions 60-1 to 60-4 were obtained in the same manner as in the preparation of the nonaqueous electrolyte solutions 67-4, 67-10, 68-4, 69-4, 70-4, and 70-10 and the comparative nonaqueous electrolyte solutions 59-1 to 59-4 except that sodium difluorobis(oxalato)phosphate (hereinafter also referred to as "DFBOP-Na") was further added and dissolved as the other additive (1) at the concentration shown in Table 21.

### <Examples 73-1 to 73-6 and Comparative Examples 61-1 to 61-4> to <Examples 78-1 to 78-6 and Comparative Examples 66-1 to 66-4>

As shown in Tables 21 and 22, the nonaqueous electrolyte solutions and the comparative nonaqueous electrolyte solutions shown in the tables were prepared by dissolving in the same manner as in the preparation of the nonaqueous electrolyte solutions 72-1 to 72-6 and the comparative nonaqueous electrolyte solutions 60-1 to 60-4 except that the other additive (1) was changed from DFBOP-Na to the respective compounds shown in the tables. Here, "DFOB-Na" means sodium difluorooxalatoborate, "TFOP-Na" means sodium tetrafluorooxalatophosphate, "DFPFSI-Na" means sodium (difluorophosphoryl)(fluorosulfonyl)imide, and "FS-Na" means sodium fluorosulfonate.

### <Examples 79-1 to 79-6 and Comparative Examples 67-1 to 67-4> to <Examples 99-1 to 99-6 and Comparative Examples 87-1 to 87-4>

As shown in Tables 23 to 28, the nonaqueous electrolyte solutions and the comparative nonaqueous electrolyte solutions shown in the tables were prepared by dissolving in the same manner as in the preparation of the nonaqueous electrolyte solutions 72-1 to 72-6 and the comparative nonaqueous electrolyte solutions 60-1 to 60-4 except that the compounds shown in "Additional Solute" in the tables in the amounts shown were added as the solute and that the other additive (I) was changed to the respective compounds shown in the tables. Here, "DFP-Na" means sodium difluorophosphate, "FSI-Na" means sodium bis(fluorosulfonyl)imide, and "BF₄-Na" means sodium tetrafluoroborate.

### <Examples 100-1 to 100-5 (Na, K, Li)> to <Examples 117-1 to 117-5 (Na, K, Li)>

As shown in Tables 29 to 34 and 45 to 47, the nonaqueous electrolyte solutions shown in the tables were prepared by dissolving Na-BOB, K-BOB, Li-BOB, Na-BF4, K-BF4, Li-BF4, Na-ClO4, K-ClO4, and Li-ClO4 at the contents shown in the tables as the other additives in the nonaqueous electrolyte solutions 1-4, 1-10, 2-4, 3-4, 4-4, and 4-10. Here, "Na-BOB" means sodium bisoxalatoborate, "K-BOB" means potassium bisoxalatoborate, "Li-BOB" means lithium bisoxalatoborate, "Na-BF4" means sodium tetrafluoroborate, "K-BF4" means potassium tetrafluoroborate, "Li-BF4" means lithium tetrafluoroborate, "Na-ClO4" means sodium perchlorate, "K-ClO4" means potassium perchlorate, and "Li-ClO4" means lithium perchlorate. The contents of the cations other than Li in the nonaqueous electrolyte solutions shown in Tables 29 to 34 and 45 to 47 were determined by ICP emission spectrometry. The contents of the cations other than Li in the nonaqueous electrolyte solutions shown in Tables 1 to 10 were all less than 1 ppm by mass.

### <Examples 118-1 to 118-5 (Na, K, Li)> to <Examples 135-1 to 135-5 (Na, K, Li)>

As shown in Tables 35 to 40 and 48 to 50, the nonaqueous electrolyte solutions shown in the tables were prepared by dissolving Na-BOB, K-BOB, Li-BOB, Na-BF4, K-BF4, Li-BF4, Na-ClO4, K-ClO4, and Li-ClO4 at the contents shown in the tables as the other additives in the nonaqueous electrolyte solutions 38-4, 38-10, 39-4, 40-4, 41-4, and 41-10. The contents of the cations other than Li in the nonaqueous electrolyte solutions shown in Tables 35 to 40 and 48 to 50 were determined by ICP emission spectrometry. The contents of the cations other than Li in the nonaqueous electrolyte solutions shown in Tables 11 to 18 were all less than 1 ppm by mass.

### <Examples 136-1 to 136-5 (Na, K, Li)> to <Examples 147-1 to 147-5 (Na, K, Li)>

As shown in Tables 41 to 44, 51, and 52, the nonaqueous electrolyte solutions shown in the tables were prepared by dissolving Li-BF4, K-BF4, Na-BF4, Li-ClO4, K-ClO4, and Na-ClO4 at the contents shown in the tables as the other additives in the nonaqueous electrolyte solutions 67-4, 67-10, 68-4, 69-4, 70-4, and 70-10. The contents of the cations other than Na in the nonaqueous electrolyte solutions shown in Tables 41 to 44, 51, and 52 were determined by ICP emission spectrometry. The contents of the cations other than Na in the nonaqueous electrolyte solutions shown in Tables 19 to 28 were all less than 1 ppm by mass.

### [Production of Nonaqueous Electrolyte Solution Battery]

### (Production of NCM622 Positive Electrode)

In 90% by mass of LiNi_{0.6}Co_{0.2}Mn_{0.2}O₂ powder, 5% by mass of polyvinylidene fluoride (hereinafter also referred to as "PVDF") as a binder and 5% by mass of acetylene black as a conductive material were mixed, and N-methyl-2-pyrrolidone (hereinafter also referred to as "NMP") was further added to produce a positive electrode mixture paste. The paste was applied onto both sides of aluminum foil (A1085), dried, pressed and then punched into 4 cm × 5 cm to obtain an NCM622 positive electrode for testing.

### (Production of NCM811 Positive Electrode)

In 92.0% by mass of LiNi_{0.8}Mn_{0.1}Co_{0.1}O₂ powder, 3.5% by mass of PVDF as a binder and 4.5% by mass of acetylene black as a conductive material were mixed, and NMP was further added to produce a positive electrode mixture paste. The paste was applied onto both sides of aluminum foil (A1085), dried, pressed and then punched into 4 cm × 5 cm to obtain an NCM811 positive electrode for testing.

### (Production of Sodium Ion Battery Positive Electrode: NaNi_{0.5}Ti_{0.3}Mn_{0.2}O₂ Positive Electrode)

By mixing 90% by mass of NaNi_{0.5}Ti_{0.3}Mn_{0.2}O₂ as a positive electrode active material, 5% by mass of acetylene black as a conductive agent, and 5% by mass of PVDF as a binder and further adding NMP as a solvent, a positive electrode mixture paste was produced. The paste was applied onto both sides of aluminum foil (A1085), dried, pressed and then punched into 4 cm × 5 cm to obtain an NaNi_{0.5}Ti_{0.3}Mn_{0.2}O₂ positive electrode for testing.

### (Production of LFP Positive Electrode)

In 90.0% by mass of LiFePO₄ powder, 5% by mass of PVDF as a binder and 5% by mass of acetylene black as a conductive material were mixed, and NMP was further added to produce a positive electrode mixture paste. The paste was applied onto both sides of aluminum foil (A1085), dried, pressed and then punched into 4 cm × 5 cm to obtain an LFP positive electrode for testing.

### (Production of Natural Graphite Negative Electrode)

By mixing 92% by mass of natural graphite powder, 3% by mass of a conductive material (HS-100 manufactured by Denka Company Limited), 2% by mass of carbon nanofiber (VGCF manufactured by Showa Denko K.K.), 2% by mass of styrene-butadiene rubber (hereinafter also referred to as "SBR"), 1% by mass of sodium carboxymethyl cellulose (hereinafter also referred to as "CMC"), and water, a negative electrode mixture paste was produced. The paste was applied onto copper foil, dried, pressed and then punched into 4.5 cm × 5.5 cm to obtain a natural graphite negative electrode for testing.

### (Production of Silicon-Containing Graphite Negative Electrode)

In 85% by mass of artificial graphite powder, 7% by mass of nanosilicon, 3% by mass of a conductive material (HS-100 manufactured by Denka Company Limited), 2% by mass of carbon nanofiber (VGCF manufactured by Showa Denko K.K.), 2% by mass of SBR, 1% by mass of CMC, and water were mixed to produce a negative electrode mixture paste. The paste was applied onto copper foil, dried, pressed and then punched into 4.5 cm × 5.5 cm to obtain a silicon-containing graphite negative electrode for testing.

### (Production of Hard Carbon Negative Electrode)

By mixing 90% by mass of a hard carbon powder (Carbotron P manufactured by Kureha Corporation) and 10% by mass of PVDF as a binder and further adding NMP as a solvent, a negative electrode mixture paste was produced. The paste was applied onto aluminum foil (A1085), dried, pressed and then punched into 4.5 cm × 5.5 cm to obtain a hard carbon negative electrode for testing.

### (Production of Nonaqueous Electrolyte Solution Battery)

Under an argon atmosphere at a dew point of -50°C or lower, a terminal was welded to the above NCM622 positive electrode, and both sides of the welded product were then sandwiched between two polyethylene separators (5 cm × 6 cm). The outside of the sandwiched product was sandwiched between two natural graphite negative electrodes to which a terminal had been welded in advance in such a manner that the surface of the negative electrode active material faced the surface of the positive electrode active material. The resultant product was put in an aluminum laminated bag having an opening on one side, and after the nonaqueous electrolyte solution was vacuum-injected into the bag, the opening was sealed with heat. In this manner, the aluminum laminated nonaqueous electrolyte solution batteries (lithium ion batteries) according to the Examples and the Comparative Examples in Tables 1 to 10, 29 to 34, and 45 to 47 were produced.

In the Examples and the Comparative Examples in Tables 11 to 18, 35 to 40, and 48 to 50, nonaqueous electrolyte solution batteries (lithium ion batteries) were similarly produced using NCM811 as the positive electrode and silicon-containing graphite as the negative electrode.

In the Examples and the Comparative Examples in Tables 19 to 28, 41 to 44, 51, and 52, nonaqueous electrolyte solution batteries (sodium ion batteries) were similarly produced using the NaNi_{0.5}Ti_{0.3}Mn_{0.2}O₂ positive electrode as the positive electrode and the hard carbon negative electrode as the negative electrode.

In the Examples and the Comparative Examples in Tables 53 to 65, nonaqueous electrolyte solution batteries (lithium ion batteries -LFP positive electrode-) were similarly produced using the nonaqueous electrolyte solutions shown in the tables and using the LFP positive electrode as the positive electrode and the natural graphite negative electrode as the negative electrode.

### [Evaluation]

### <Initial Charge and Discharge Test: Lithium Ion Battery>

First, the produced cells were conditioned at an ambient temperature of 25°C under the following conditions. That is, as an initial charge and discharge test, a charge and discharge cycle including constant-current constant-voltage charging at an upper limit charge voltage of 4.2 V and at 5 mA, constant-current discharging at 10 mA to a discharge end voltage of 2.5 V, subsequent constant-current constant-voltage charging at an upper limit charge voltage of 4.2 V and at 10 mA, and constant-current discharging at 10 mA to a discharge end voltage of 2.5 V was repeated three times.

### <Initial Charge and Discharge Test: Sodium Ion Battery>

First, the produced cells were conditioned at an ambient temperature of 25°C under the following conditions. That is, as an initial charge and discharge test, a charge and discharge cycle including constant-current constant-voltage charging at an upper limit charge voltage of 4.1 V and at 5 mA, constant-current discharging at 10 mA to a discharge end voltage of 1.5 V, subsequent constant-current constant-voltage charging at an upper limit charge voltage of 4.1 V and at 10 mA, and constant-current discharging at 10 mA to a discharge end voltage of 1.5 V was repeated three times.

### <Initial Charge and Discharge Test: Lithium Ion Battery -LFP Positive Electrode->

First, the produced cells were conditioned at an ambient temperature of 25°C under the following conditions. That is, as an initial charge and discharge test, a charge and discharge cycle including constant-current constant-voltage charging at an upper limit charge voltage of 3.5 V and at 5 mA, constant-current discharging at 10 mA to a discharge end voltage of 2.0 V, subsequent constant-current constant-voltage charging at an upper limit charge voltage of 3.5 V and at 10 mA, and constant-current discharging at 10 mA to a discharge end voltage of 2.0 V was repeated three times.

### <Measurement of Initial Direct Current Internal Resistance: Lithium Ion Battery>

The nonaqueous electrolyte solution battery subjected to the initial charging and discharging was charged at a constant current of 10 mA for 150 minutes at an ambient temperature of 25°C and discharged at a predetermined constant current value (5 mA, 10 mA, 25 mA, 50 mA, and 100 mA) for 10 seconds. The voltage at the tenth second was measured and plotted with respect to the current value. An approximate straight line was determined by applying the least squares method to the plots. The value of the slope of the approximate straight line was defined as the initial direct current internal resistance. A smaller value thereof means a better initial input-output characteristic.

### <Measurement of Initial Direct Current Internal Resistance: Sodium Ion Battery>

Evaluation was performed in the same manner as that for the lithium ion battery. A smaller value thereof means the better initial input-output characteristic.

### <Measurement of Initial Direct Current Internal Resistance: Lithium Ion Battery -LFP Positive Electrode->

Evaluation was performed in the same manner as that for the lithium ion battery. A smaller value thereof means the better initial input-output characteristic.

### <Cycle Test (45°C): Lithium Ion Battery>

The nonaqueous electrolyte solution battery after the completion of the measurement of the initial direct current internal resistance was discharged at a constant current of 10 mA to a discharge end voltage of 2.5V and allowed to stand at an ambient temperature of 45°C for three hours. Thereafter, the battery was subjected to constant-current constant-voltage charging at an upper limit charge voltage of 4.2 V and at 75 mA and constant-current discharging at 75 mA to a discharge end voltage of 2.5 V. The charging and discharging at 75 mA in an environment at 45°C were repeated 1000 cycles. Thereafter, the battery was allowed to stand at an ambient temperature of 25°C for three hours and subjected to constant-current constant-voltage charging at an upper limit charge voltage of 4.2 V and at 10 mA and constant-current discharging at 10 mA to a discharge end voltage of 2.5 V.

### <Cycle Test (45°C): Sodium Ion Battery>

Except that the upper limit charge voltage was changed to 4.1 V and that the discharge end voltage was changed to 1.5 V, evaluation was performed in the same manner as that for the lithium ion battery.

### <Cycle Test (45°C): Lithium Ion Battery -LFP Positive Electrode->

Except that the upper limit charge voltage was changed to 3.5 V and that the discharge end voltage was changed to 2.0 V, evaluation was performed in the same manner as that for the lithium ion battery.

### <Measurement of Direct Current Internal Resistance after Cycle Test: Lithium Ion Battery>

The nonaqueous electrolyte solution battery after the completion of the cycle test (45°C) was charged and discharged in the same manner as in the measurement of the initial direct current internal resistance and charged at a constant current of 10 mA for 150 minutes at an ambient temperature of 25°C, and the voltage at the tenth second was measured and plotted with respect to the current value. An approximate straight line was determined by applying the least squares method to the plots. The value of the slope of the approximate straight line was defined as the direct current internal resistance after the cycle test.

### <Measurement of Direct Current Internal Resistance after Cycle Test: Sodium Ion Battery>

Evaluation was performed in the same manner as that for the lithium ion battery.

### <Measurement of Direct Current Internal Resistance after Cycle Test: Lithium Ion Battery -LFP Positive Electrode->

Evaluation was performed in the same manner as that for the lithium ion battery.

### <Increase Rate of Direct Current Internal Resistance after Cycle Test: Lithium Ion Battery>

The increase rate of the direct current internal resistance after the cycle test was determined by the following equation. A smaller value thereof means the better input-output characteristic after the life characteristic. Increase rate (%) of direct current internal resistance after cycle test = (direct current internal resistance after cycle test/initial direct current internal resistance) × 100

### <Increase Rate of Direct Current Internal Resistance after Cycle Test: Sodium Ion Battery>

Evaluation was performed in the same manner as that for the lithium ion battery. A smaller value thereof means the better input-output characteristic after the life characteristic.

### <Increase Rate of Direct Current Internal Resistance after Cycle Test: Lithium Ion Battery -LFP Positive Electrode->

Evaluation was performed in the same manner as that for the lithium ion battery. A smaller value thereof means better the input-output characteristic after the life characteristic.

In the tables, the initial direct current internal resistances and the increase rates of the direct current internal resistance after the cycle test are shown as relative values, where the evaluation results of the comparative nonaqueous electrolyte solution containing none of the component (I), the compound (6-1), the compound (6-2), and the compound (6-3) are set to 100.

In the tables, "R1" represents the initial direct current internal resistance (relative value), and "R2" represents the increase rate of the direct current internal resistance after the cycle test (relative value).

**[Table 1]**

| Table 1 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 1-1 | Nonaqueous electrolyte solution 1-1 | 1-1-11 | 0.05 | - | - | - | - | - | - | 98 | 84 |
| Example 1-2 | Nonaqueous electrolyte solution 1-2 | | 0.1 | - | - | - | - | - | - | 95 | 80 |
| Example 1-3 | Nonaqueous electrolyte solution 1-3 | | 0.5 | - | - | - | - | - | - | 87 | 76 |
| Example 1-4 | Nonaqueous electrolyte solution 1-4 | | 1.0 | - | - | - | - | - | - | 79 | 71 |
| Example 1-5 | Nonaqueous electrolyte solution 1-5 | | 2.0 | - | - | - | - | - | - | 73 | 69 |
| Example 1-6 | Nonaqueous electrolyte solution 1-6 | | 3.0 | - | - | - | - | - | - | 76 | 65 |
| Example 1-7 | Nonaqueous electrolyte solution 1-7 | 1-3-1 | 0.05 | - | - | - | - | - | - | 97 | 84 |
| Example 1-8 | Nonaqueous electrolyte solution 1-8 | | 0.1 | - | - | - | - | - | - | 94 | 80 |
| Example 1-9 | Nonaqueous electrolyte solution 1-9 | | 0.5 | - | - | - | - | - | - | 87 | 68 |
| Example 1-10 | Nonaqueous electrolyte solution 1-10 | | 1.0 | - | - | - | - | - | - | 93 | 58 |
| Example 1-11 | Nonaqueous electrolyte solution 1-11 | | 2.0 | - | - | - | - | - | - | 95 | 51 |
| Example 1-12 | Nonaqueous electrolyte solution 1-12 | | 3.0 | - | - | - | - | - | - | 97 | 48 |
| Example 2-1 | Nonaqueous electrolyte solution 2-1 | 2-2-2 | 0.05 | - | - | - | - | - | - | 97 | 84 |
| Example 2-2 | Nonaqueous electrolyte solution 2-2 | | 0.1 | - | - | - | - | - | - | 93 | 80 |
| Example 2-3 | Nonaqueous electrolyte solution 2-3 | | 0.5 | - | - | - | - | - | - | 89 | 69 |
| Example 2-4 | Nonaqueous electrolyte solution 2-4 | | 1.0 | - | - | - | - | - | - | 88 | 60 |
| Example 2-5 | Nonaqueous electrolyte solution 2-5 | | 2.0 | - | - | - | - | - | - | 85 | 58 |
| Example 2-6 | Nonaqueous electrolyte solution 2-6 | | 3.0 | - | - | - | - | - | - | 91 | 52 |
| Example 3-1 | Nonaqueous electrolyte solution 3-1 | 3-3-3 | 0.05 | - | - | - | - | - | - | 96 | 84 |
| Example 3-2 | Nonaqueous electrolyte solution 3-2 | | 0.1 | - | - | - | - | - | - | 93 | 81 |
| Example 3-3 | Nonaqueous electrolyte solution 3-3 | | 0.5 | - | - | - | - | - | - | 85 | 70 |
| Example 3-4 | Nonaqueous electrolyte solution 3-4 | | 1.0 | - | - | - | - | - | - | 92 | 59 |
| Example 3-5 | Nonaqueous electrolyte solution 3-5 | | 2.0 | - | - | - | - | - | - | 94 | 52 |
| Example 3-6 | Nonaqueous electrolyte solution 3-6 | | 3.0 | - | - | - | - | - | - | 96 | 49 |
| Example 4-1 | Nonaqueous electrolyte solution 4-1 | 4-3-1 | 0.05 | - | - | - | - | - | - | 95 | 84 |
| Example 4-2 | Nonaqueous electrolyte solution 4-2 | | 0.1 | - | - | - | - | - | - | 93 | 81 |
| Example 4-3 | Nonaqueous electrolyte solution 4-3 | | 0.5 | - | - | - | - | - | - | 84 | 71 |
| Example 4-4 | Nonaqueous electrolyte solution 4-4 | | 1.0 | - | - | - | - | - | - | 91 | 60 |
| Example 4-5 | Nonaqueous electrolyte solution 4-5 | | 2.0 | - | - | - | - | - | - | 93 | 53 |
| Example 4-6 | Nonaqueous electrolyte solution 4-6 | | 3.0 | - | - | - | - | - | - | 95 | 50 |
| Example 4-7 | Nonaqueous electrolyte solution 4-7 | 4-1-11 | 0.05 | - | - | - | - | - | - | 97 | 86 |
| Example 4-8 | Nonaqueous electrolyte solution 4-8 | | 0.1 | - | - | - | - | - | - | 94 | 80 |
| Example 4-9 | Nonaqueous electrolyte solution 4-9 | | 0.5 | - | - | - | - | - | - | 86 | 78 |
| Example 4-10 | Nonaqueous electrolyte solution 4-10 | | 1.0 | - | - | - | - | - | - | 77 | 73 |
| Example 4-11 | Nonaqueous electrolyte solution 4-11 | | 2.0 | - | - | - | - | - | - | 72 | 71 |
| Example 4-12 | Nonaqueous electrolyte solution 4-12 | | 3.0 | - | - | - | - | - | - | 75 | 66 |
| Comparative Example 1-1 | Comparative nonaqueous electrolyte solution 1-1 | - | - | - | - | - | - | - | - | 100 | 100 |
| Comparative Example 1-2 | Comparative nonaqueous electrolyte solution 1-2 | 6-1 | 1.0 | - | - | - | - | - | - | 150 | 85 |
| Comparative Example 1-3 | Comparative nonaqueous electrolyte solution 1-3 | 6-2 | 1.0 | - | - | - | - | - | - | 153 | 87 |
| Comparative Example 1-4 | Comparative nonaqueous electrolyte solution 1-4 | 6-3 | 1.0 | - | - | - | - | - | - | 116 | 93 |

**[Table 2]**

| Table 2 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 5-1 | Nonaqueous electrolyte solution 5-1 | 1-1-11 | 0.5 | - | - | - | - | - | - | 86 | 64 |
| | | 1-3-1 | 0.5 | | | | | | | | |
| Example 5-2 | Nonaqueous electrolyte solution 5-2 | 1-1-11 | 0.5 | - | - | - | - | - | - | 84 | 65 |
| | | 2-2-2 | 0.5 | | | | | | | | |
| Example 5-3 | Nonaqueous electrolyte solution 5-3 | 1-1-11 | 0.5 | - | - | - | - | - | - | 86 | 64 |
| | | 3-3-3 | 0.5 | | | | | | | | |
| Example 5-4 | Nonaqueous electrolyte solution 5-4 | 1-1-11 | 0.5 | - | - | - | - | - | - | 85 | 65 |
| | | 4-3-1 | 0.5 | | | | | | | | |
| Example 5-5 | Nonaqueous electrolyte solution 5-5 | 2-2-2 | 0.5 | - | - | - | - | - | - | 90 | 59 |
| | | 3-3-3 | 0.5 | | | | | | | | |
| Example 5-6 | Nonaqueous electrolyte solution 5-6 | 2-2-2 | 0.5 | - | - | - | - | - | - | 89 | 60 |
| | | 4-3-1 | 0.5 | | | | | | | | |
| Example 5-7 | Nonaqueous electrolyte solution 5-7 | 3-3-3 | 0.5 | - | - | - | - | - | - | 91 | 59 |
| | | 4-3-1 | 0.5 | | | | | | | | |
| Example 5-8 | Nonaqueous electrolyte solution 5-8 | 1-1-11 | 0.25 | - | - | - | - | - | - | 88 | 62 |
| | | 2-2-2 | 0.25 | | | | | | | | |
| | | 3-3-3 | 0.25 | | | | | | | | |
| | | 4-3-1 | 0.25 | | | | | | | | |
| Example 5-9 | Nonaqueous electrolyte solution 5-9 | 1-1-11 | 0.5 | - | - | - | - | - | - | 78 | 72 |
| | | 4-1-11 | 0.5 | | | | | | | | |
| Comparative Example 1-1 | Comparative nonaqueous electrolyte solution 1-1 | - | - | - | - | - | - | - | - | 100 | 100 |
| Comparative Example 1-2 | Comparative nonaqueous electrolyte solution 1-2 | 6-1 | 1.0 | - | - | - | - | - | - | 150 | 85 |
| Comparative Example 1-3 | Comparative nonaqueous electrolyte solution 1-3 | 6-2 | 1.0 | - | - | - | - | - | - | 153 | 87 |
| Comparative Example 1-4 | Comparative nonaqueous electrolyte solution 1-4 | 6-3 | 1.0 | - | - | - | - | - | - | 116 | 93 |

**[Table 3]**

| Table 3 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 6-1 | Nonaqueous electrolyte solution 6-1 | 1-1-11 | 1.0 | - | - | | | | | 67 | 79 |
| Example 6-2 | Nonaqueous electrolyte solution 6-2 | 1-3-1 | 1.0 | - | - | | | | | 81 | 65 |
| Example 6-3 | Nonaqueous electrolyte solution 6-3 | 2-2-2 | 1.0 | - | - | | | | | 77 | 67 |
| Example 6-4 | Nonaqueous electrolyte solution 6-4 | 3-3-3 | 1.0 | - | - | | | | | 80 | 67 |
| Example 6-5 | Nonaqueous electrolyte solution 6-5 | 4-3-1 | 1.0 | - | - | | | | | 79 | 68 |
| Example 6-6 | Nonaqueous electrolyte solution 6-6 | 4-1-11 | 1.0 | - | - | VC | 1.0 | BOB | 1.0 | 66 | 83 |
| Comparative Example 2-1 | Comparative nonaqueous electrolyte solution 2-1 | - | - | - | - | | | | | 100 | 100 |
| Comparative Example 2-2 | Comparative nonaqueous electrolyte solution 2-2 | 6-1 | 1.0 | - | - | | | | | 150 | 86 |
| Comparative Example 2-3 | Comparative nonaqueous electrolyte solution 2-3 | 6-2 | 1.0 | | | | | | | 152 | 89 |
| Comparative Example 2-4 | Comparative nonaqueous electrolyte solution 2-4 | 6-3 | 1.0 | - | - | | | | | 116 | 96 |
| Example 7-1 | Nonaqueous electrolyte solution 7-1 | 1-1-11 | 1.0 | - | - | | | | | 77 | 75 |
| Example 7-2 | Nonaqueous electrolyte solution 7-2 | 1-3-1 | 1.0 | - | - | | | | | 92 | 63 |
| Example 7-3 | Nonaqueous electrolyte solution 7-3 | 2-2-2 | 1.0 | - | - | | | | | 87 | 64 |
| Example 7-4 | Nonaqueous electrolyte solution 7-4 | 3-3-3 | 1.0 | - | - | | | | | 91 | 64 |
| Example 7-5 | Nonaqueous electrolyte solution 7-5 | 4-3-1 | 1.0 | - | - | | | | | 90 | 66 |
| Example 7-6 | Nonaqueous electrolyte solution 7-6 | 4-1-11 | 1.0 | - | - | VC | 1.0 | DFBOP | 1.0 | 75 | 78 |
| Comparative Example 3-1 | Comparative nonaqueous electrolyte solution 3-1 | - | - | - | - | | | | | 100 | 100 |
| Comparative Example 3-2 | Comparative nonaqueous electrolyte solution 3-2 | 6-1 | 1.0 | - | - | | | | | 149 | 83 |
| Comparative Example 3-3 | Comparative nonaqueous electrolyte solution 3-3 | 6-2 | 1.0 | - | - | | | | | 151 | 87 |
| Comparative Example 3-4 | Comparative nonaqueous electrolyte solution 3-4 | 6-3 | 1.0 | - | - | | | | | 114 | 96 |
| Example 8-1 | Nonaqueous electrolyte solution 8-1 | 1-1-11 | 1.0 | - | - | | | | | 75 | 75 |
| Example 8-2 | Nonaqueous electrolyte solution 8-2 | 1-3-1 | 1.0 | - | - | | | | | 92 | 61 |
| Example 8-3 | Nonaqueous electrolyte solution 8-3 | 2-2-2 | 1.0 | - | - | | | | | 87 | 62 |
| Example 8-4 | Nonaqueous electrolyte solution 8-4 | 3-3-3 | 1.0 | - | - | | | | | 90 | 62 |
| Example 8-5 | Nonaqueous electrolyte solution 8-5 | 4-3-1 | 1.0 | - | - | | | | | 89 | 63 |
| Example 8-6 | Nonaqueous electrolyte solution 8-6 | 4-1-11 | 1.0 | - | - | VC | 1.0 | DFOB | 1.0 | 73 | 78 |
| Comparative Example 4-1 | Comparative nonaqueous electrolyte solution 4-1 | - | - | - | - | | | | | 100 | 100 |
| Comparative Example 4-2 | Comparative nonaqueous electrolyte solution 4-2 | 6-1 | 1.0 | - | - | | | | | 147 | 82 |
| Comparative Example 4-3 | Comparative nonaqueous electrolyte solution 4-3 | 6-2 | 1.0 | - | - | | | | | 151 | 85 |
| Comparative Example 4-4 | Comparative nonaqueous electrolyte solution 4-4 | 6-3 | 1.0 | - | - | | | | | 111 | 95 |
| Example 9-1 | Nonaqueous electrolyte solution 9-1 | 1-1-11 | 1.0 | - | - | | | | | 75 | 74 |
| Example 9-2 | Nonaqueous electrolyte solution 9-2 | 1-3-1 | 1.0 | - | - | | | | | 91 | 61 |
| Example 9-3 | Nonaqueous electrolyte solution 9-3 | 2-2-2 | 1.0 | - | - | | | | | 87 | 61 |
| Example 9-4 | Nonaqueous electrolyte solution 9-4 | 3-3-3 | 1.0 | - | - | | | | | 89 | 62 |
| Example 9-5 | Nonaqueous electrolyte solution 9-5 | 4-3-1 | 1.0 | - | - | | | | | 88 | 63 |
| Example 9-6 | Nonaqueous electrolyte solution 9-6 | 4-1-11 | 1.0 | - | - | VC | 1.0 | TFOP | 1.0 | 73 | 77 |
| Comparative Example 5-1 | Comparative nonaqueous electrolyte solution 5-1 | - | - | - | - | | | | | 100 | 100 |
| Comparative Example 5-2 | Comparative nonaqueous electrolyte solution 5-2 | 6-1 | 1.0 | - | - | | | | | 142 | 83 |
| Comparative Example 5-3 | Comparative nonaqueous electrolyte solution 5-3 | 6-2 | 1.0 | - | - | | | | | 148 | 84 |
| Comparative Example 5-4 | Comparative nonaqueous electrolyte solution 5-4 | 6-3 | 1.0 | - | - | | | | | 109 | 96 |

**[Table 4]**

| Table 4 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 10-1 | Nonaqueous electrolyte solution 10-1 | 1-1-11 | 1.0 | - | - | | | | | 70 | 75 |
| Example 10-2 | Nonaqueous electrolyte solution 10-2 | 1-3-1 | 1.0 | - | - | | | | | 85 | 63 |
| Example 10-3 | Nonaqueous electrolyte solution 10-3 | 2-2-2 | 1.0 | - | - | | | | | 81 | 63 |
| Example 10-4 | Nonaqueous electrolyte solution 10-4 | 3-3-3 | 1.0 | - | - | | | | | 84 | 65 |
| Example 10-5 | Nonaqueous electrolyte solution 10-5 | 4-3-1 | 1.0 | - | - | | | | | 82 | 66 |
| Example 10-6 | Nonaqueous electrolyte solution 10-6 | 4-1-11 | 1.0 | - | - | VC | 1.0 | DTD | 1.0 | 69 | 78 |
| Comparative Example 6-1 | Comparative nonaqueous electrolyte solution 6-1 | - | - | - | - | | | | | 100 | 100 |
| Comparative Example 6-2 | Comparative nonaqueous electrolyte solution 6-2 | 6-1 | 1.0 | - | - | | | | | 150 | 81 |
| Comparative Example 6-3 | Comparative nonaqueous electrolyte solution 6-3 | 6-2 | 1.0 | - | - | | | | | 153 | 84 |
| Comparative Example 6-4 | Comparative nonaqueous electrolyte solution 6-4 | 6-3 | 1.0 | - | - | | | | | 114 | 92 |
| Example 11-1 | Nonaqueous electrolyte solution 11-1 | 1-1-11 | 1.0 | - | - | | | | | 76 | 70 |
| Example 11-2 | Nonaqueous electrolyte solution 11-2 | 1-3-1 | 1.0 | - | - | | | | | 90 | 60 |
| Example 11-3 | Nonaqueous electrolyte solution 11-3 | 2-2-2 | 1.0 | - | - | | | | | 85 | 60 |
| Example 11-4 | Nonaqueous electrolyte solution 11-4 | 3-3-3 | 1.0 | - | - | | | | | 89 | 61 |
| Example 11-5 | Nonaqueous electrolyte solution 11-5 | 4-3-1 | 1.0 | - | - | | | | | 88 | 62 |
| Example 11-6 | Nonaqueous electrolyte solution 11-6 | 4-1-11 | 1.0 | - | - | VC | 1.0 | DFPFSI | 1.0 | 74 | 73 |
| Comparative Example 7-1 | Comparative nonaqueous electrolyte solution 7-1 | - | - | - | - | | | | | 100 | 100 |
| Comparative Example 7-2 | Comparative nonaqueous electrolyte solution 7-2 | 6-1 | 1.0 | - | - | | | | | 150 | 82 |
| Comparative Example 7-3 | Comparative nonaqueous electrolyte solution 7-3 | 6-2 | 1.0 | - | - | | | | | 154 | 84 |
| Comparative Example 7-4 | Comparative nonaqueous electrolyte solution 7-4 | 6-3 | 1.0 | - | - | | | | | 113 | 93 |
| Example 12-1 | Nonaqueous electrolyte solution 12-1 | 1-1-11 | 1.0 | - | - | | | | | 77 | 67 |
| Example 12-2 | Nonaqueous electrolyte solution 12-2 | 1-3-1 | 1.0 | - | - | | | | | 90 | 57 |
| Example 12-3 | Nonaqueous electrolyte solution 12-3 | 2-2-2 | 1.0 | - | - | | | | | 85 | 59 |
| Example 12-4 | Nonaqueous electrolyte solution 12-4 | 3-3-3 | 1.0 | - | - | | | | | 89 | 58 |
| Example 12-5 | Nonaqueous electrolyte solution 12-5 | 4-3-1 | 1.0 | - | - | | | | | 88 | 59 |
| Example 12-6 | Nonaqueous electrolyte solution 12-6 | 4-1-11 | 1.0 | - | - | VC | 1.0 | FS | 1.0 | 76 | 69 |
| Comparative Example 8-1 | Comparative nonaqueous electrolyte solution 8-1 | - | - | - | - | | | | | 100 | 100 |
| Comparative Example 8-2 | Comparative nonaqueous electrolyte solution 8-2 | 6-1 | 1.0 | - | - | | | | | 154 | 80 |
| Comparative Example 8-3 | Comparative nonaqueous electrolyte solution 8-3 | 6-2 | 1.0 | - | - | | | | | 155 | 83 |
| Comparative Example 8-4 | Comparative nonaqueous electrolyte solution 8-4 | 6-3 | 1.0 | - | - | | | | | 117 | 91 |
| Example 13-1 | Nonaqueous electrolyte solution 13-1 | 1-1-11 | 1.0 | - | - | | | | | 66 | 77 |
| Example 13-2 | Nonaqueous electrolyte solution 13-2 | 1-3-1 | 1.0 | - | - | | | | | 78 | 62 |
| Example 13-3 | Nonaqueous electrolyte solution 13-3 | 2-2-2 | 1.0 | - | - | | | | | 75 | 64 |
| Example 13-4 | Nonaqueous electrolyte solution 13-4 | 3-3-3 | 1.0 | - | - | | | | | 77 | 63 |
| Example 13-5 | Nonaqueous electrolyte solution 13-5 | 4-3-1 | 1.0 | - | - | | | | | 76 | 65 |
| Example 13-6 | Nonaqueous electrolyte solution 13-6 | 4-1-11 | 1.0 | - | - | VC | 1.0 | TV-Si | 0.5 | 65 | 80 |
| Comparative Example 9-1 | Comparative nonaqueous electrolyte solution 9-1 | - | - | - | - | | | | | 100 | 100 |
| Comparative Example 9-2 | Comparative nonaqueous electrolyte solution 9-2 | 6-1 | 1.0 | - | - | | | | | 153 | 82 |
| Comparative Example 9-3 | Comparative nonaqueous electrolyte solution 9-3 | 6-2 | 1.0 | - | - | | | | | 155 | 84 |
| Comparative Example 9-4 | Comparative nonaqueous electrolyte solution 9-4 | 6-3 | 1.0 | - | - | | | | | 116 | 93 |

**[Table 5]**

| Table 5 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 14-1 | Nonaqueous electrolyte solution 14-1 | 1-1-11 | 1.0 | | | | | | | 65 | 78 |
| Example 14-2 | Nonaqueous electrolyte solution 14-2 | 1-3-1 | 1.0 | | | | | | | 79 | 64 |
| Example 14-3 | Nonaqueous electrolyte solution 14-3 | 2-2-2 | 1.0 | | | | | | | 75 | 65 |
| Example 14-4 | Nonaqueous electrolyte solution 14-4 | 3-3-3 | 1.0 | | | | | | | 78 | 65 |
| Example 14-5 | Nonaqueous electrolyte solution 14-5 | 4-3-1 | 1.0 | | | | | | | 77 | 67 |
| Example 14-6 | Nonaqueous electrolyte solution 14-6 | 4-1-11 | 1.0 | DFP | 0.1 | VC | 1.0 | BOB | 1.0 | 64 | 81 |
| Comparative Example 10-1 | Comparative nonaqueous electrolyte solution 10-1 | - | - | | | | | | | 100 | 100 |
| Comparative Example 10-2 | Comparative nonaqueous electrolyte solution 10-2 | 6-1 | 1.0 | | | | | | | 150 | 86 |
| Comparative Example 10-3 | Comparative nonaqueous electrolyte solution 10-3 | 6-2 | 1.0 | | | | | | | 152 | 89 |
| Comparative Example 10-4 | Comparative nonaqueous electrolyte solution 10-4 | 6-3 | 1.0 | | | | | | | 116 | 96 |
| Example 15-1 | Nonaqueous electrolyte solution 15-1 | 1-1-11 | 1.0 | | | | | | | 75 | 74 |
| Example 15-2 | Nonaqueous electrolyte solution 15-2 | 1-3-1 | 1.0 | | | | | | | 90 | 62 |
| Example 15-3 | Nonaqueous electrolyte solution 15-3 | 2-2-2 | 1.0 | | | | | | | 85 | 63 |
| Example 15-4 | Nonaqueous electrolyte solution 15-4 | 3-3-3 | 1.0 | | | | | | | 88 | 63 |
| Example 15-5 | Nonaqueous electrolyte solution 15-5 | 4-3-1 | 1.0 | | | | | | | 87 | 64 |
| Example 15-6 | Nonaqueous electrolyte solution 15-6 | 4-1-11 | 1.0 | DFP | 0.1 | VC | 1.0 | DFBOP | 1.0 | 73 | 77 |
| Comparative Example 11-1 | Comparative nonaqueous electrolyte solution 11-1 | - | - | | | | | | | 100 | 100 |
| Comparative Example 11-2 | Comparative nonaqueous electrolyte solution 11-2 | 6-1 | 1.0 | | | | | | | 149 | 83 |
| Comparative Example 11-3 | Comparative nonaqueous electrolyte solution 11-3 | 6-2 | 1.0 | | | | | | | 151 | 87 |
| Comparative Example 11-4 | Comparative nonaqueous electrolyte solution 11-4 | 6-3 | 1.0 | | | | | | | 114 | 96 |
| Example 16-1 | Nonaqueous electrolyte solution 16-1 | 1-1-11 | 1.0 | | | | | | | 73 | 74 |
| Example 16-2 | Nonaqueous electrolyte solution 16-2 | 1-3-1 | 1.0 | | | | | | | 89 | 60 |
| Example 16-3 | Nonaqueous electrolyte solution 16-3 | 2-2-2 | 1.0 | | | | | | | 85 | 60 |
| Example 16-4 | Nonaqueous electrolyte solution 16-4 | 3-3-3 | 1.0 | | | | | | | 88 | 61 |
| Example 16-5 | Nonaqueous electrolyte solution 16-5 | 4-3-1 | 1.0 | | | | | | | 87 | 62 |
| Example 16-6 | Nonaqueous electrolyte solution 16-6 | 4-1-11 | 1.0 | DFP | 0.1 | VC | 1.0 | DFOB | 1.0 | 72 | 77 |
| Comparative Example 12-1 | Comparative nonaqueous electrolyte solution 12-1 | - | - | | | | | | | 100 | 100 |
| Comparative Example 12-2 | Comparative nonaqueous electrolyte solution 12-2 | 6-1 | 1.0 | | | | | | | 147 | 82 |
| Comparative Example 12-3 | Comparative nonaqueous electrolyte solution 12-3 | 6-2 | 1.0 | | | | | | | 151 | 85 |
| Comparative Example 12-4 | Comparative nonaqueous electrolyte solution 12-4 | 6-3 | 1.0 | | | | | | | 111 | 95 |
| Example 17-1 | Nonaqueous electrolyte solution 17-1 | 1-1-11 | 1.0 | | | | | | | 73 | 73 |
| Example 17-2 | Nonaqueous electrolyte solution 17-2 | 1-3-1 | 1.0 | | | | | | | 88 | 59 |
| Example 17-3 | Nonaqueous electrolyte solution 17-3 | 2-2-2 | 1.0 | | | | | | | 85 | 60 |
| Example 17-4 | Nonaqueous electrolyte solution 17-4 | 3-3-3 | 1.0 | | | | | | | 87 | 60 |
| Example 17-5 | Nonaqueous electrolyte solution 17-5 | 4-3-1 | 1.0 | | | | | | | 86 | 62 |
| Example 17-6 | Nonaqueous electrolyte solution 17-6 | 4-1-11 | 1.0 | DFP | 0.1 | VC | 1.0 | TFOP | 1.0 | 71 | 76 |
| Comparative Example 13-1 | Comparative nonaqueous electrolyte solution 13-1 | - | - | | | | | | | 100 | 100 |
| Comparative Example 13-2 | Comparative nonaqueous electrolyte solution 13-2 | 6-1 | 1.0 | | | | | | | 142 | 83 |
| Comparative Example 13-3 | Comparative nonaqueous electrolyte solution 13-3 | 6-2 | 1.0 | | | | | | | 148 | 84 |
| Comparative Example 13-4 | Comparative nonaqueous electrolyte solution 13-4 | 6-3 | 1.0 | | | | | | | 109 | 96 |

**[Table 6]**

| Table 6 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 18-1 | Nonaqueous electrolyte solution 18-1 | 1-1-11 | 1.0 | | | | | | | 69 | 74 |
| Example 18-2 | Nonaqueous electrolyte solution 18-2 | 1-3-1 | 1.0 | | | | | | | 83 | 62 |
| Example 18-3 | Nonaqueous electrolyte solution 18-3 | 2-2-2 | 1.0 | | | | | | | 79 | 62 |
| Example 18-4 | Nonaqueous electrolyte solution 18-4 | 3-3-3 | 1.0 | | | | | | | 81 | 63 |
| Example 18-5 | Nonaqueous electrolyte solution 18-5 | 4-3-1 | 1.0 | | | | | | | 80 | 64 |
| Example 18-6 | Nonaqueous electrolyte solution 18-6 | 4-1-11 | 1.0 | DFP | 0.1 | VC | 1.0 | DTD | 1.0 | 67 | 76 |
| Comparative Example 14-1 | Comparative nonaqueous electrolyte solution 14-1 | - | - | | | | | | | 100 | 100 |
| Comparative Example 14-2 | Comparative nonaqueous electrolyte solution 14-2 | 6-1 | 1.0 | | | | | | | 150 | 81 |
| Comparative Example 14-3 | Comparative nonaqueous electrolyte solution 14-3 | 6-2 | 1.0 | | | | | | | 153 | 84 |
| Comparative Example 14-4 | Comparative nonaqueous electrolyte solution 14-4 | 6-3 | 1.0 | | | | | | | 114 | 92 |
| Example 19-1 | Nonaqueous electrolyte solution 19-1 | 1-1-11 | 1.0 | | | | | | | 74 | 69 |
| Example 19-2 | Nonaqueous electrolyte solution 19-2 | 1-3-1 | 1.0 | | | | | | | 88 | 58 |
| Example 19-3 | Nonaqueous electrolyte solution 19-3 | 2-2-2 | 1.0 | | | | | | | 83 | 59 |
| Example 19-4 | Nonaqueous electrolyte solution 19-4 | 3-3-3 | 1.0 | | | | | | | 87 | 59 |
| Example 19-5 | Nonaqueous electrolyte solution 19-5 | 4-3-1 | 1.0 | | | | | | | 86 | 61 |
| Example 19-6 | Nonaqueous electrolyte solution 19-6 | 4-1-11 | 1.0 | DFP | 0.1 | VC | 1.0 | DFPFSI | 1.0 | 72 | 72 |
| Comparative Example 15-1 | Comparative nonaqueous electrolyte solution 15-1 | - | - | | | | | | | 100 | 100 |
| Comparative Example 15-2 | Comparative nonaqueous electrolyte solution 15-2 | 6-1 | 1.0 | | | | | | | 150 | 82 |
| Comparative Example 15-3 | Comparative nonaqueous electrolyte solution 15-3 | 6-2 | 1.0 | | | | | | | 154 | 84 |
| Comparative Example 15-4 | Comparative nonaqueous electrolyte solution 15-4 | 6-3 | 1.0 | | | | | | | 113 | 93 |
| Example 20-1 | Nonaqueous electrolyte solution 20-1 | 1-1-11 | 1.0 | | | | | | | 75 | 65 |
| Example 20-2 | Nonaqueous electrolyte solution 20-2 | 1-3-1 | 1.0 | | | | | | | 88 | 56 |
| Example 20-3 | Nonaqueous electrolyte solution 20-3 | 2-2-2 | 1.0 | | | | | | | 83 | 58 |
| Example 20-4 | Nonaqueous electrolyte solution 20-4 | 3-3-3 | 1.0 | | | | | | | 87 | 57 |
| Example 20-5 | Nonaqueous electrolyte solution 20-5 | 4-3-1 | 1.0 | | | | | | | 85 | 58 |
| Example 20-6 | Nonaqueous electrolyte solution 20-6 | 4-1-11 | 1.0 | DFP | 0.1 | VC | 1.0 | FS | 1.0 | 74 | 68 |
| Comparative Example 16-1 | Comparative nonaqueous electrolyte solution 16-1 | - | - | | | | | | | 100 | 100 |
| Comparative Example 16-2 | Comparative nonaqueous electrolyte solution 16-2 | 6-1 | 1.0 | | | | | | | 154 | 80 |
| Comparative Example 16-3 | Comparative nonaqueous electrolyte solution 16-3 | 6-2 | 1.0 | | | | | | | 155 | 83 |
| Comparative Example 16-4 | Comparative nonaqueous electrolyte solution 16-4 | 6-3 | 1.0 | | | | | | | 117 | 91 |
| Example 21-1 | Nonaqueous electrolyte solution 21-1 | 1-1-11 | 1.0 | | | | | | | 65 | 76 |
| Example 21-2 | Nonaqueous electrolyte solution 21-2 | 1-3-1 | 1.0 | | | | | | | 76 | 61 |
| Example 21-3 | Nonaqueous electrolyte solution 21-3 | 2-2-2 | 1.0 | | | | | | | 73 | 62 |
| Example 21-4 | Nonaqueous electrolyte solution 21-4 | 3-3-3 | 1.0 | | | | | | | 75 | 62 |
| Example 21-5 | Nonaqueous electrolyte solution 21-5 | 4-3-1 | 1.0 | | | | | | | 74 | 63 |
| Example 21-6 | Nonaqueous electrolyte solution 21-6 | 4-1-11 | 1.0 | DFP | 0.1 | VC | 1.0 | TV-Si | 0.5 | 63 | 79 |
| Comparative Example 17-1 | Comparative nonaqueous electrolyte solution 17-1 | - | - | | | | | | | 100 | 100 |
| Comparative Example 17-2 | Comparative nonaqueous electrolyte solution 17-2 | 6-1 | 1.0 | | | | | | | 153 | 82 |
| Comparative Example 17-3 | Comparative nonaqueous electrolyte solution 17-3 | 6-2 | 1.0 | | | | | | | 155 | 84 |
| Comparative Example 17-4 | Comparative nonaqueous electrolyte solution 17-4 | 6-3 | 1.0 | | | | | | | 116 | 93 |

**[Table 7]**

| Table 7 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 22-1 | Nonaqueous electrolyte solution 22-1 | 1-1-11 | 1.0 | | | | | | | 66 | 77 |
| Example 22-2 | Nonaqueous electrolyte solution 22-2 | 1-3-1 | 1.0 | | | | | | | 80 | 64 |
| Example 22-3 | Nonaqueous electrolyte solution 22-3 | 2-2-2 | 1.0 | | | | | | | 75 | 65 |
| Example 22-4 | Nonaqueous electrolyte solution 22-4 | 3-3-3 | 1.0 | | | | | | | 78 | 65 |
| Example 22-5 | Nonaqueous electrolyte solution 22-5 | 4-3-1 | 1.0 | | | | | | | 77 | 66 |
| Example 22-6 | Nonaqueous electrolyte solution 22-6 | 4-1-11 | 1.0 | FSI | 0.1 | VC | 1.0 | BOB | 1.0 | 64 | 80 |
| Comparative Example 18-1 | Comparative nonaqueous electrolyte solution 18-1 | - | - | | | | | | | 100 | 100 |
| Comparative Example 18-2 | Comparative nonaqueous electrolyte solution 18-2 | 6-1 | 1.0 | | | | | | | 150 | 86 |
| Comparative Example 18-3 | Comparative nonaqueous electrolyte solution 18-3 | 6-2 | 1.0 | | | | | | | 152 | 89 |
| Comparative Example 18-4 | Comparative nonaqueous electrolyte solution 18-4 | 6-3 | 1.0 | | | | | | | 116 | 96 |
| Example 23-1 | Nonaqueous electrolyte solution 23-1 | 1-1-11 | 1.0 | | | | | | | 76 | 73 |
| Example 23-2 | Nonaqueous electrolyte solution 23-2 | 1-3-1 | 1.0 | | | | | | | 90 | 61 |
| Example 23-3 | Nonaqueous electrolyte solution 23-3 | 2-2-2 | 1.0 | | | | | | | 86 | 63 |
| Example 23-4 | Nonaqueous electrolyte solution 23-4 | 3-3-3 | 1.0 | | | | | | | 89 | 63 |
| Example 23-5 | Nonaqueous electrolyte solution 23-5 | 4-3-1 | 1.0 | | | | | | | 88 | 64 |
| Example 23-6 | Nonaqueous electrolyte solution 23-6 | 4-1-11 | 1.0 | FSI | 0.1 | VC | 1.0 | DFBOP | 1.0 | 74 | 76 |
| Comparative Example 19-1 | Comparative nonaqueous electrolyte solution 19-1 | - | - | | | | | | | 100 | 100 |
| Comparative Example 19-2 | Comparative nonaqueous electrolyte solution 19-2 | 6-1 | 1.0 | | | | | | | 149 | 83 |
| Comparative Example 19-3 | Comparative nonaqueous electrolyte solution 19-3 | 6-2 | 1.0 | | | | | | | 151 | 87 |
| Comparative Example 19-4 | Comparative nonaqueous electrolyte solution 19-4 | 6-3 | 1.0 | | | | | | | 114 | 96 |
| Example 24-1 | Nonaqueous electrolyte solution 24-1 | 1-1-11 | 1.0 | | | | | | | 74 | 73 |
| Example 24-2 | Nonaqueous electrolyte solution 24-2 | 1-3-1 | 1.0 | | | | | | | 90 | 59 |
| Example 24-3 | Nonaqueous electrolyte solution 24-3 | 2-2-2 | 1.0 | | | | | | | 85 | 60 |
| Example 24-4 | Nonaqueous electrolyte solution 24-4 | 3-3-3 | 1.0 | | | | | | | 88 | 60 |
| Example 24-5 | Nonaqueous electrolyte solution 24-5 | 4-3-1 | 1.0 | | | | | | | 87 | 62 |
| Example 24-6 | Nonaqueous electrolyte solution 24-6 | 4-1-11 | 1.0 | FSI | 0.1 | VC | 1.0 | DFOB | 1.0 | 72 | 76 |
| Comparative Example 20-1 | Comparative nonaqueous electrolyte solution 20-1 | - | - | | | | | | | 100 | 100 |
| Comparative Example 20-2 | Comparative nonaqueous electrolyte solution 20-2 | 6-1 | 1.0 | | | | | | | 147 | 82 |
| Comparative Example 20-3 | Comparative nonaqueous electrolyte solution 20-3 | 6-2 | 1.0 | | | | | | | 151 | 85 |
| Comparative Example 20-4 | Comparative nonaqueous electrolyte solution 20-4 | 6-3 | 1.0 | | | | | | | 111 | 95 |
| Example 25-1 | Nonaqueous electrolyte solution 25-1 | 1-1-11 | 1.0 | | | | | | | 73 | 72 |
| Example 25-2 | Nonaqueous electrolyte solution 25-2 | 1-3-1 | 1.0 | | | | | | | 89 | 59 |
| Example 25-3 | Nonaqueous electrolyte solution 25-3 | 2-2-2 | 1.0 | | | | | | | 85 | 59 |
| Example 25-4 | Nonaqueous electrolyte solution 25-4 | 3-3-3 | 1.0 | | | | | | | 87 | 60 |
| Example 25-5 | Nonaqueous electrolyte solution 25-5 | 4-3-1 | 1.0 | | | | | | | 86 | 61 |
| Example 25-6 | Nonaqueous electrolyte solution 25-6 | 4-1-11 | 1.0 | FSI | 0.1 | VC | 1.0 | TFOP | 1.0 | 72 | 75 |
| Comparative Example 21-1 | Comparative nonaqueous electrolyte solution 21-1 | - | - | | | | | | | 100 | 100 |
| Comparative Example 21-2 | Comparative nonaqueous electrolyte solution 21-2 | 6-1 | 1.0 | | | | | | | 142 | 83 |
| Comparative Example 21-3 | Comparative nonaqueous electrolyte solution 21-3 | 6-2 | 1.0 | | | | | | | 148 | 84 |
| Comparative Example 21-4 | Comparative nonaqueous electrolyte solution 21-4 | 6-3 | 1.0 | | | | | | | 109 | 96 |

**[Table 8]**

| Table 8 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 26-1 | Nonaqueous electrolyte solution 26-1 | 1-1-11 | 1.0 | | | | | | | 69 | 73 |
| Example 26-2 | Nonaqueous electrolyte solution 26-2 | 1-3-1 | 1.0 | | | | | | | 83 | 62 |
| Example 26-3 | Nonaqueous electrolyte solution 26-3 | 2-2-2 | 1.0 | | | | | | | 79 | 61 |
| Example 26-4 | Nonaqueous electrolyte solution 26-4 | 3-3-3 | 1.0 | | | | | | | 82 | 63 |
| Example 26-5 | Nonaqueous electrolyte solution 26-5 | 4-3-1 | 1.0 | | | | | | | 81 | 64 |
| Example 26-6 | Nonaqueous electrolyte solution 26-6 | 4-1-11 | 1.0 | FSI | 0.1 | VC | 1.0 | DTD | 1.0 | 67 | 76 |
| Comparative Example 22-1 | Comparative nonaqueous electrolyte solution 22-1 | - | - | | | | | | | 100 | 100 |
| Comparative Example 22-2 | Comparative nonaqueous electrolyte solution 22-2 | 6-1 | 1.0 | | | | | | | 150 | 81 |
| Comparative Example 22-3 | Comparative nonaqueous electrolyte solution 22-3 | 6-2 | 1.0 | | | | | | | 153 | 84 |
| Comparative Example 22-4 | Comparative nonaqueous electrolyte solution 22-4 | 6-3 | 1.0 | | | | | | | 114 | 92 |
| Example 27-1 | Nonaqueous electrolyte solution 27-1 | 1-1-11 | 1.0 | | | | | | | 74 | 69 |
| Example 27-2 | Nonaqueous electrolyte solution 27-2 | 1-3-1 | 1.0 | | | | | | | 89 | 58 |
| Example 27-3 | Nonaqueous electrolyte solution 27-3 | 2-2-2 | 1.0 | | | | | | | 84 | 59 |
| Example 27-4 | Nonaqueous electrolyte solution 27-4 | 3-3-3 | 1.0 | | | | | | | 87 | 59 |
| Example 27-5 | Nonaqueous electrolyte solution 27-5 | 4-3-1 | 1.0 | | | | | | | 86 | 60 |
| Example 27-6 | Nonaqueous electrolyte solution 27-6 | 4-1-11 | 1.0 | FSI | 0.1 | VC | 1.0 | DFPFSI | 1.0 | 72 | 71 |
| Comparative Example 23-1 | Comparative nonaqueous electrolyte solution 23-1 | - | - | | | | | | | 100 | 100 |
| Comparative Example 23-2 | Comparative nonaqueous electrolyte solution 23-2 | 6-1 | 1.0 | | | | | | | 150 | 82 |
| Comparative Example 23-3 | Comparative nonaqueous electrolyte solution 23-3 | 6-2 | 1.0 | | | | | | | 154 | 84 |
| Comparative Example 23-4 | Comparative nonaqueous electrolyte solution 23-4 | 6-3 | 1.0 | | | | | | | 113 | 93 |
| Example 28-1 | Nonaqueous electrolyte solution 28-1 | 1-1-11 | 1.0 | | | | | | | 76 | 65 |
| Example 28-2 | Nonaqueous electrolyte solution 28-2 | 1-3-1 | 1.0 | | | | | | | 88 | 55 |
| Example 28-3 | Nonaqueous electrolyte solution 28-3 | 2-2-2 | 1.0 | | | | | | | 83 | 58 |
| Example 28-4 | Nonaqueous electrolyte solution 28-4 | 3-3-3 | 1.0 | | | | | | | 87 | 56 |
| Example 28-5 | Nonaqueous electrolyte solution 28-5 | 4-3-1 | 1.0 | | | | | | | 86 | 58 |
| Example 28-6 | Nonaqueous electrolyte solution 28-6 | 4-1-11 | 1.0 | FSI | 0.1 | VC | 1.0 | FS | 1.0 | 74 | 67 |
| Comparative Example 24-1 | Comparative nonaqueous electrolyte solution 24-1 | - | - | | | | | | | 100 | 100 |
| Comparative Example 24-2 | Comparative nonaqueous electrolyte solution 24-2 | 6-1 | 1.0 | | | | | | | 154 | 80 |
| Comparative Example 24-3 | Comparative nonaqueous electrolyte solution 24-3 | 6-2 | 1.0 | | | | | | | 155 | 83 |
| Comparative Example 24-4 | Comparative nonaqueous electrolyte solution 24-4 | 6-3 | 1.0 | | | | | | | 117 | 91 |
| Example 29-1 | Nonaqueous electrolyte solution 29-1 | 1-1-11 | 1.0 | | | | | | | 65 | 75 |
| Example 29-2 | Nonaqueous electrolyte solution 29-2 | 1-3-1 | 1.0 | | | | | | | 76 | 61 |
| Example 29-3 | Nonaqueous electrolyte solution 29-3 | 2-2-2 | 1.0 | | | | | | | 73 | 62 |
| Example 29-4 | Nonaqueous electrolyte solution 29-4 | 3-3-3 | 1.0 | | | | | | | 75 | 62 |
| Example 29-5 | Nonaqueous electrolyte solution 29-5 | 4-3-1 | 1.0 | | | | | | | 74 | 63 |
| Example 29-6 | Nonaqueous electrolyte solution 29-6 | 4-1-11 | 1.0 | FSI | 0.1 | VC | 1.0 | TV-Si | 0.5 | 63 | 78 |
| Comparative Example 25-1 | Comparative nonaqueous electrolyte solution 25-1 | - | - | | | | | | | 100 | 100 |
| Comparative Example 25-2 | Comparative nonaqueous electrolyte solution 25-2 | 6-1 | 1.0 | | | | | | | 153 | 82 |
| Comparative Example 25-3 | Comparative nonaqueous electrolyte solution 25-3 | 6-2 | 1.0 | | | | | | | 155 | 84 |
| Comparative Example 25-4 | Comparative nonaqueous electrolyte solution 25-4 | 6-3 | 1.0 | | | | | | | 116 | 93 |

**[Table 9]**

| Table 9 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 30-1 | Nonaqueous electrolyte solution 30-1 | 1-1-11 | 1.0 | | | | | | | 66 | 77 |
| Example 30-2 | Nonaqueous electrolyte solution 30-2 | 1-3-1 | 1.0 | | | | | | | 79 | 63 |
| Example 30-3 | Nonaqueous electrolyte solution 30-3 | 2-2-2 | 1.0 | | | | | | | 75 | 65 |
| Example 30-4 | Nonaqueous electrolyte solution 30-4 | 3-3-3 | 1.0 | | | | | | | 78 | 65 |
| Example 30-5 | Nonaqueous electrolyte solution 30-5 | 4-3-1 | 1.0 | | | | | | | 77 | 66 |
| Example 30-6 | Nonaqueous electrolyte solution 30-6 | 4-1-11 | 1.0 | BF₄ | 0.025 | VC | 1.0 | BOB | 1.0 | 64 | 80 |
| Comparative Example 26-1 | Comparative nonaqueous electrolyte solution 26-1 | - | - | | | | | | | 100 | 100 |
| Comparative Example 26-2 | Comparative nonaqueous electrolyte solution 26-2 | 6-1 | 1.0 | | | | | | | 150 | 86 |
| Comparative Example 26-3 | Comparative nonaqueous electrolyte solution 26-3 | 6-2 | 1.0 | | | | | | | 152 | 89 |
| Comparative Example 26-4 | Comparative nonaqueous electrolyte solution 26-4 | 6-3 | 1.0 | | | | | | | 116 | 96 |
| Example 31-1 | Nonaqueous electrolyte solution 31-1 | 1-1-11 | 1.0 | | | | | | | 76 | 73 |
| Example 31-2 | Nonaqueous electrolyte solution 31-2 | 1-3-1 | 1.0 | | | | | | | 90 | 61 |
| Example 31-3 | Nonaqueous electrolyte solution 31-3 | 2-2-2 | 1.0 | | | | | | | 85 | 62 |
| Example 31-4 | Nonaqueous electrolyte solution 31-4 | 3-3-3 | 1.0 | | | | | | | 89 | 62 |
| Example 31-5 | Nonaqueous electrolyte solution 31-5 | 4-3-1 | 1.0 | | | | | | | 88 | 64 |
| Example 31-6 | Nonaqueous electrolyte solution 31-6 | 4-1-11 | 1.0 | BF₄ | 0.025 | VC | 1.0 | DFBOP | 1.0 | 74 | 76 |
| Comparative Example 27-1 | Comparative nonaqueous electrolyte solution 27-1 | - | - | | | | | | | 100 | 100 |
| Comparative Example 27-2 | Comparative nonaqueous electrolyte solution 27-2 | 6-1 | 1.0 | | | | | | | 149 | 83 |
| Comparative Example 27-3 | Comparative nonaqueous electrolyte solution 27-3 | 6-2 | 1.0 | | | | | | | 151 | 87 |
| Comparative Example 27-4 | Comparative nonaqueous electrolyte solution 27-4 | 6-3 | 1.0 | | | | | | | 114 | 96 |
| Example 32-1 | Nonaqueous electrolyte solution 32-1 | 1-1-11 | 1.0 | | | | | | | 74 | 73 |
| Example 32-2 | Nonaqueous electrolyte solution 32-2 | 1-3-1 | 1.0 | | | | | | | 90 | 59 |
| Example 32-3 | Nonaqueous electrolyte solution 32-3 | 2-2-2 | 1.0 | | | | | | | 85 | 60 |
| Example 32-4 | Nonaqueous electrolyte solution 32-4 | 3-3-3 | 1.0 | | | | | | | 88 | 60 |
| Example 32-5 | Nonaqueous electrolyte solution 32-5 | 4-3-1 | 1.0 | | | | | | | 87 | 61 |
| Example 32-6 | Nonaqueous electrolyte solution 32-6 | 4-1-11 | 1.0 | BF₄ | 0.025 | VC | 1.0 | DFOB | 1.0 | 72 | 76 |
| Comparative Example 28-1 | Comparative nonaqueous electrolyte solution 28-1 | - | - | | | | | | | 100 | 100 |
| Comparative Example 28-2 | Comparative nonaqueous electrolyte solution 28-2 | 6-1 | 1.0 | | | | | | | 147 | 82 |
| Comparative Example 28-3 | Comparative nonaqueous electrolyte solution 28-3 | 6-2 | 1.0 | | | | | | | 151 | 85 |
| Comparative Example 28-4 | Comparative nonaqueous electrolyte solution 28-4 | 6-3 | 1.0 | | | | | | | 111 | 95 |
| Example 33-1 | Nonaqueous electrolyte solution 33-1 | 1-1-11 | 1.0 | | | | | | | 73 | 72 |
| Example 33-2 | Nonaqueous electrolyte solution 33-2 | 1-3-1 | 1.0 | | | | | | | 89 | 59 |
| Example 33-3 | Nonaqueous electrolyte solution 33-3 | 2-2-2 | 1.0 | | | | | | | 85 | 59 |
| Example 33-4 | Nonaqueous electrolyte solution 33-4 | 3-3-3 | 1.0 | | | | | | | 87 | 60 |
| Example 33-5 | Nonaqueous electrolyte solution 33-5 | 4-3-1 | 1.0 | | | | | | | 86 | 61 |
| Example 33-6 | Nonaqueous electrolyte solution 33-6 | 4-1-11 | 1.0 | BF₄ | 0.025 | VC | 1.0 | TFOP | 1.0 | 71 | 75 |
| Comparative Example 29-1 | Comparative nonaqueous electrolyte solution 29-1 | - | - | | | | | | | 100 | 100 |
| Comparative Example 29-2 | Comparative nonaqueous electrolyte solution 29-2 | 6-1 | 1.0 | | | | | | | 142 | 83 |
| Comparative Example 29-3 | Comparative nonaqueous electrolyte solution 29-3 | 6-2 | 1.0 | | | | | | | 148 | 84 |
| Comparative Example 29-4 | Comparative nonaqueous electrolyte solution 29-4 | 6-3 | 1.0 | | | | | | | 109 | 96 |

**[Table 10]**

| Table 10 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 34-1 | Nonaqueous electrolyte solution 34-1 | 1-1-11 | 1.0 | | | | | | | 69 | 73 |
| Example 34-2 | Nonaqueous electrolyte solution 34-2 | 1-3-1 | 1.0 | | | | | | | 83 | 61 |
| Example 34-3 | Nonaqueous electrolyte solution 34-3 | 2-2-2 | 1.0 | | | | | | | 79 | 61 |
| Example 34-4 | Nonaqueous electrolyte solution 34-4 | 3-3-3 | 1.0 | | | | | | | 82 | 63 |
| Example 34-5 | Nonaqueous electrolyte solution 34-5 | 4-3-1 | 1.0 | | | | | | | 81 | 64 |
| Example 34-6 | Nonaqueous electrolyte solution 34-6 | 4-1-11 | 1.0 | BF₄ | 0.025 | VC | 1.0 | DTD | 1.0 | 67 | 76 |
| Comparative Example 30-1 | Comparative nonaqueous electrolyte solution 30-1 | - | - | | | | | | | 100 | 100 |
| Comparative Example 30-2 | Comparative nonaqueous electrolyte solution 30-2 | 6-1 | 1.0 | | | | | | | 150 | 81 |
| Comparative Example 30-3 | Comparative nonaqueous electrolyte solution 30-3 | 6-2 | 1.0 | | | | | | | 153 | 84 |
| Comparative Example 30-4 | Comparative nonaqueous electrolyte solution 30-4 | 6-3 | 1.0 | | | | | | | 114 | 92 |
| Example 35-1 | Nonaqueous electrolyte solution 35-1 | 1-1-11 | 1.0 | | | | | | | 74 | 68 |
| Example 35-2 | Nonaqueous electrolyte solution 35-2 | 1-3-1 | 1.0 | | | | | | | 88 | 58 |
| Example 35-3 | Nonaqueous electrolyte solution 35-3 | 2-2-2 | 1.0 | | | | | | | 84 | 59 |
| Example 35-4 | Nonaqueous electrolyte solution 35-4 | 3-3-3 | 1.0 | | | | | | | 87 | 59 |
| Example 35-5 | Nonaqueous electrolyte solution 35-5 | 4-3-1 | 1.0 | | | | | | | 86 | 60 |
| Example 35-6 | Nonaqueous electrolyte solution 35-6 | 4-1-11 | 1.0 | BF₄ | 0.025 | VC | 1.0 | DFPFSI | 1.0 | 72 | 71 |
| Comparative Example 31-1 | Comparative nonaqueous electrolyte solution 31-1 | - | - | | | | | | | 100 | 100 |
| Comparative Example 31-2 | Comparative nonaqueous electrolyte solution 31-2 | 6-1 | 1.0 | | | | | | | 150 | 82 |
| Comparative Example 31-3 | Comparative nonaqueous electrolyte solution 31-3 | 6-2 | 1.0 | | | | | | | 154 | 84 |
| Comparative Example 31-4 | Comparative nonaqueous electrolyte solution 31-4 | 6-3 | 1.0 | | | | | | | 113 | 93 |
| Example 36-1 | Nonaqueous electrolyte solution 36-1 | 1-1-11 | 1.0 | | | | | | | 76 | 65 |
| Example 36-2 | Nonaqueous electrolyte solution 36-2 | 1-3-1 | 1.0 | | | | | | | 88 | 55 |
| Example 36-3 | Nonaqueous electrolyte solution 36-3 | 2-2-2 | 1.0 | | | | | | | 83 | 57 |
| Example 36-4 | Nonaqueous electrolyte solution 36-4 | 3-3-3 | 1.0 | | | | | | | 87 | 56 |
| Example 36-5 | Nonaqueous electrolyte solution 36-5 | 4-3-1 | 1.0 | | | | | | | 86 | 57 |
| Example 36-6 | Nonaqueous electrolyte solution 36-6 | 4-1-11 | 1.0 | BF₄ | 0.025 | VC | 1.0 | FS | 1.0 | 74 | 67 |
| Comparative Example 32-1 | Comparative nonaqueous electrolyte solution 32-1 | - | - | | | | | | | 100 | 100 |
| Comparative Example 32-2 | Comparative nonaqueous electrolyte solution 32-2 | 6-1 | 1.0 | | | | | | | 154 | 80 |
| Comparative Example 32-3 | Comparative nonaqueous electrolyte solution 32-3 | 6-2 | 1.0 | | | | | | | 155 | 83 |
| Comparative Example 32-4 | Comparative nonaqueous electrolyte solution 32-4 | 6-3 | 1.0 | | | | | | | 117 | 91 |
| Example 37-1 | Nonaqueous electrolyte solution 37-1 | 1-1-11 | 1.0 | | | | | | | 65 | 75 |
| Example 37-2 | Nonaqueous electrolyte solution 37-2 | 1-3-1 | 1.0 | | | | | | | 76 | 60 |
| Example 37-3 | Nonaqueous electrolyte solution 37-3 | 2-2-2 | 1.0 | | | | | | | 73 | 62 |
| Example 37-4 | Nonaqueous electrolyte solution 37-4 | 3-3-3 | 1.0 | | | | | | | 75 | 61 |
| Example 37-5 | Nonaqueous electrolyte solution 37-5 | 4-3-1 | 1.0 | | | | | | | 74 | 63 |
| Example 37-6 | Nonaqueous electrolyte solution 37-6 | 4-1-11 | 1.0 | BF₄ | 0.025 | VC | 1.0 | TV-Si | 0.5 | 63 | 78 |
| Comparative Example 33-1 | Comparative nonaqueous electrolyte solution 33-1 | - | - | | | | | | | 100 | 100 |
| Comparative Example 33-2 | Comparative nonaqueous electrolyte solution 33-2 | 6-1 | 1.0 | | | | | | | 153 | 82 |
| Comparative Example 33-3 | Comparative nonaqueous electrolyte solution 33-3 | 6-2 | 1.0 | | | | | | | 155 | 84 |
| Comparative Example 33-4 | Comparative nonaqueous electrolyte solution 33-4 | 6-3 | 1.0 | | | | | | | 116 | 93 |

**[Table 11]**

| Table 11 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 38-1 | Nonaqueous electrolyte solution 38-1 | 1-1-11 | 0.05 | - | - | - | - | - | - | 94 | 84 |
| Example 38-2 | Nonaqueous electrolyte solution 38-2 | | 0.1 | - | - | - | - | - | - | 91 | 81 |
| Example 38-3 | Nonaqueous electrolyte solution 38-3 | | 0.5 | - | - | - | - | - | - | 86 | 78 |
| Example 38-4 | Nonaqueous electrolyte solution 38-4 | | 1.0 | - | - | - | - | - | - | 80 | 74 |
| Example 38-5 | Nonaqueous electrolyte solution 38-5 | | 2.0 | - | - | - | - | - | - | 75 | 73 |
| Example 38-6 | Nonaqueous electrolyte solution 38-6 | | 3.0 | - | - | - | - | - | - | 77 | 70 |
| Example 38-7 | Nonaqueous electrolyte solution 38-7 | 1-3-1 | 0.05 | - | - | - | - | - | - | 93 | 84 |
| Example 38-8 | Nonaqueous electrolyte solution 38-8 | | 0.1 | - | - | - | - | - | - | 90 | 81 |
| Example 38-9 | Nonaqueous electrolyte solution 38-9 | | 0.5 | - | - | - | - | - | - | 85 | 72 |
| Example 38-10 | Nonaqueous electrolyte solution 38-10 | | 1.0 | - | - | - | - | - | - | 90 | 64 |
| Example 38-11 | Nonaqueous electrolyte solution 38-11 | | 2.0 | - | - | - | - | - | - | 92 | 59 |
| Example 38-12 | Nonaqueous electrolyte solution 38-12 | | 3.0 | - | - | - | - | - | - | 93 | 56 |
| Example 39-1 | Nonaqueous electrolyte solution 39-1 | 2-2-2 | 0.05 | - | - | - | - | - | - | 93 | 84 |
| Example 39-2 | Nonaqueous electrolyte solution 39-2 | | 0.1 | - | - | - | - | - | - | 90 | 81 |
| Example 39-3 | Nonaqueous electrolyte solution 39-3 | | 0.5 | - | - | - | - | - | - | 87 | 72 |
| Example 39-4 | Nonaqueous electrolyte solution 39-4 | | 1.0 | - | - | - | - | - | - | 86 | 66 |
| Example 39-5 | Nonaqueous electrolyte solution 39-5 | | 2.0 | - | - | - | - | - | - | 84 | 64 |
| Example 39-6 | Nonaqueous electrolyte solution 39-6 | | 3.0 | - | - | - | - | - | - | 89 | 60 |
| Example 40-1 | Nonaqueous electrolyte solution 40-1 | 3-3-3 | 0.05 | - | - | - | - | - | - | 92 | 84 |
| Example 40-2 | Nonaqueous electrolyte solution 40-2 | | 0.1 | - | - | - | - | - | - | 90 | 81 |
| Example 40-3 | Nonaqueous electrolyte solution 40-3 | | 0.5 | - | - | - | - | - | - | 84 | 73 |
| Example 40-4 | Nonaqueous electrolyte solution 40-4 | | 1.0 | - | - | - | - | - | - | 89 | 65 |
| Example 40-5 | Nonaqueous electrolyte solution 40-5 | | 2.0 | - | - | - | - | - | - | 91 | 60 |
| Example 40-6 | Nonaqueous electrolyte solution 40-6 | | 3.0 | - | - | - | - | - | - | 92 | 57 |
| Example 41-1 | Nonaqueous electrolyte solution 41-1 | 4-3-1 | 0.05 | - | - | - | - | - | - | 92 | 84 |
| Example 41-2 | Nonaqueous electrolyte solution 41-2 | | 0.1 | - | - | - | - | - | - | 90 | 81 |
| Example 41-3 | Nonaqueous electrolyte solution 41-3 | | 0.5 | - | - | - | - | - | - | 83 | 74 |
| Example 41-4 | Nonaqueous electrolyte solution 41-4 | | 1.0 | - | - | - | - | - | - | 88 | 65 |
| Example 41-5 | Nonaqueous electrolyte solution 41-5 | | 2.0 | - | - | - | - | - | - | 90 | 60 |
| Example 41-6 | Nonaqueous electrolyte solution 41-6 | | 3.0 | - | - | - | - | - | - | 91 | 58 |
| Example 41-7 | Nonaqueous electrolyte solution 41-7 | 4-1-11 | 0.05 | - | - | - | - | - | - | 93 | 85 |
| Example 41-8 | Nonaqueous electrolyte solution 41-8 | | 0.1 | - | - | - | - | - | - | 91 | 81 |
| Example 41-9 | Nonaqueous electrolyte solution 41-9 | | 0.5 | - | - | - | - | - | - | 85 | 79 |
| Example 41-10 | Nonaqueous electrolyte solution 41-10 | | 1.0 | - | - | - | - | - | - | 78 | 76 |
| Example 41-11 | Nonaqueous electrolyte solution 41-11 | | 2.0 | - | - | - | - | - | - | 74 | 75 |
| Example 41-12 | Nonaqueous electrolyte solution 41-12 | | 3.0 | - | - | - | - | - | - | 77 | 71 |
| Comparative Example 34-1 | Comparative nonaqueous electrolyte solution 34-1 | - | - | - | - | - | - | - | - | 100 | 100 |
| Comparative Example 34-2 | Comparative nonaqueous electrolyte solution 34-2 | 6-1 | 1.0 | - | - | - | - | - | - | 138 | 90 |
| Comparative Example 34-3 | Comparative nonaqueous electrolyte solution 34-3 | 6-2 | 1.0 | - | - | - | - | - | - | 140 | 91 |
| Comparative Example 34-4 | Comparative nonaqueous electrolyte solution 34-4 | 6-3 | 1.0 | - | - | - | - | - | - | 112 | 96 |

**[Table 12]**

| Table 12 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 42-1 | Nonaqueous electrolyte solution 42-1 | 1-1-11 | 0.5 | - | - | - | - | - | - | 85 | 72 |
| | | 1-3-1 | 0.5 | | | | | | | | |
| Example 42-2 | Nonaqueous electrolyte solution 42-2 | 1-1-11 | 0.5 | - | - | - | - | - | - | 83 | 70 |
| | | 2-2-2 | 0.5 | | | | | | | | |
| Example 42-3 | Nonaqueous electrolyte solution 42-3 | 1-1-11 | 0.5 | - | - | - | - | - | - | 84 | 69 |
| | | 3-3-3 | 0.5 | | | | | | | | |
| Example 42-4 | Nonaqueous electrolyte solution 42-4 | 1-1-11 | 0.5 | - | - | - | - | - | - | 84 | 70 |
| | | 4-3-1 | 0.5 | | | | | | | | |
| Example 42-5 | Nonaqueous electrolyte solution 42-5 | 2-2-2 | 0.5 | - | - | - | - | - | - | 88 | 65 |
| | | 3-3-3 | 0.5 | | | | | | | | |
| Example 42-6 | Nonaqueous electrolyte solution 42-6 | 2-2-2 | 0.5 | - | - | - | - | - | - | 87 | 66 |
| | | 4-3-1 | 0.5 | | | | | | | | |
| Example 42-7 | Nonaqueous electrolyte solution 42-7 | 3-3-3 | 0.5 | - | - | - | - | - | - | 89 | 65 |
| | | 4-3-1 | 0.5 | | | | | | | | |
| Example 42-8 | Nonaqueous electrolyte solution 42-8 | 1-1-11 | 0.25 | - | | - | | - | | 86 | 67 |
| | | 2-2-2 | 0.25 | | | | | | | | |
| | | 3-3-3 | 0.25 | | | | | | | | |
| | | 4-3-1 | 0.25 | | | | | | | | |
| Example 42-9 | Nonaqueous electrolyte solution 42-9 | 1-1-11 | 0.5 | - | - | - | - | - | - | 79 | 75 |
| | | 4-1-11 | 0.5 | | | | | | | | |
| Comparative Example 34-1 | Comparative nonaqueous electrolyte solution 34-1 | - | - | - | - | - | - | - | - | 100 | 100 |
| Comparative Example 34-2 | Comparative nonaqueous electrolyte solution 34-2 | 6-1 | 1.0 | - | - | - | - | - | - | 138 | 90 |
| Comparative Example 34-3 | Comparative nonaqueous electrolyte solution 34-3 | 6-2 | 1.0 | - | - | - | - | - | - | 140 | 91 |
| Comparative Example 34-4 | Comparative nonaqueous electrolyte solution 34-4 | 6-3 | 1.0 | - | - | - | - | - | - | 112 | 96 |

**[Table 13]**

| Table 13 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 43-1 | Nonaqueous electrolyte solution 43-1 | 1-1-11 | 1.0 | - | - | | | | | 69 | 82 |
| Example 43-2 | Nonaqueous electrolyte solution 43-2 | 1-3-1 | 1.0 | - | - | | | | | 81 | 70 |
| Example 43-3 | Nonaqueous electrolyte solution 43-3 | 2-2-2 | 1.0 | - | - | | | | | 77 | 72 |
| Example 43-4 | Nonaqueous electrolyte solution 43-4 | 3-3-3 | 1.0 | - | - | | | | | 80 | 71 |
| Example 43-5 | Nonaqueous electrolyte solution 43-5 | 4-3-1 | 1.0 | - | - | | | | | 79 | 73 |
| Example 43-6 | Nonaqueous electrolyte solution 43-6 | 4-1-11 | 1.0 | - | - | VC | 1.0 | BOB | 1.0 | 68 | 85 |
| Comparative Example 35-1 | Comparative nonaqueous electrolyte solution 35-1 | - | - | - | - | | | | | 100 | 100 |
| Comparative Example 35-2 | Comparative nonaqueous electrolyte solution 35-2 | 6-1 | 1.0 | - | - | | | | | 142 | 88 |
| Comparative Example 35-3 | Comparative nonaqueous electrolyte solution 35-3 | 6-2 | 1.0 | - | - | | | | | 143 | 89 |
| Comparative Example 35-4 | Comparative nonaqueous electrolyte solution 35-4 | 6-3 | 1.0 | - | - | | | | | 113 | 97 |
| Example 44-1 | Nonaqueous electrolyte solution 44-1 | 1-1-11 | 1.0 | - | - | | | | | 78 | 78 |
| Example 44-2 | Nonaqueous electrolyte solution 44-2 | 1-3-1 | 1.0 | - | - | | | | | 89 | 68 |
| Example 44-3 | Nonaqueous electrolyte solution 44-3 | 2-2-2 | 1.0 | - | - | | | | | 85 | 70 |
| Example 44-4 | Nonaqueous electrolyte solution 44-4 | 3-3-3 | 1.0 | - | - | | | | | 88 | 69 |
| Example 44-5 | Nonaqueous electrolyte solution 44-5 | 4-3-1 | 1.0 | - | - | | | | | 87 | 70 |
| Example 44-6 | Nonaqueous electrolyte solution 44-6 | 4-1-11 | 1.0 | - | - | VC | 1.0 | DFBOP | 1.0 | 76 | 80 |
| Comparative Example 36-1 | Comparative nonaqueous electrolyte solution 36-1 | - | - | - | - | | | | | 100 | 100 |
| Comparative Example 36-2 | Comparative nonaqueous electrolyte solution 36-2 | 6-1 | 1.0 | - | - | | | | | 136 | 88 |
| Comparative Example 36-3 | Comparative nonaqueous electrolyte solution 36-3 | 6-2 | 1.0 | - | - | | | | | 138 | 90 |
| Comparative Example 36-4 | Comparative nonaqueous electrolyte solution 36-4 | 6-3 | 1.0 | - | - | | | | | 110 | 99 |
| Example 45-1 | Nonaqueous electrolyte solution 45-1 | 1-1-11 | 1.0 | - | - | | | | | 77 | 78 |
| Example 45-2 | Nonaqueous electrolyte solution 45-2 | 1-3-1 | 1.0 | - | - | | | | | 88 | 67 |
| Example 45-3 | Nonaqueous electrolyte solution 45-3 | 2-2-2 | 1.0 | - | - | | | | | 85 | 68 |
| Example 45-4 | Nonaqueous electrolyte solution 45-4 | 3-3-3 | 1.0 | - | - | | | | | 87 | 68 |
| Example 45-5 | Nonaqueous electrolyte solution 45-5 | 4-3-1 | 1.0 | - | - | | | | | 86 | 69 |
| Example 45-6 | Nonaqueous electrolyte solution 45-6 | 4-1-11 | 1.0 | - | - | VC | 1.0 | DFOB | 1.0 | 75 | 80 |
| Comparative Example 37-1 | Comparative nonaqueous electrolyte solution 37-1 | - | - | - | - | | | | | 100 | 100 |
| Comparative Example 37-2 | Comparative nonaqueous electrolyte solution 37-2 | 6-1 | 1.0 | - | - | | | | | 134 | 88 |
| Comparative Example 37-3 | Comparative nonaqueous electrolyte solution 37-3 | 6-2 | 1.0 | - | - | | | | | 137 | 90 |
| Comparative Example 37-4 | Comparative nonaqueous electrolyte solution 37-4 | 6-3 | 1.0 | - | - | | | | | 108 | 98 |
| Example 46-1 | Nonaqueous electrolyte solution 46-1 | 1-1-11 | 1.0 | - | - | | | | | 76 | 78 |
| Example 46-2 | Nonaqueous electrolyte solution 46-2 | 1-3-1 | 1.0 | - | - | | | | | 88 | 66 |
| Example 46-3 | Nonaqueous electrolyte solution 46-3 | 2-2-2 | 1.0 | - | - | | | | | 86 | 67 |
| Example 46-4 | Nonaqueous electrolyte solution 46-4 | 3-3-3 | 1.0 | - | - | | | | | 87 | 67 |
| Example 46-5 | Nonaqueous electrolyte solution 46-5 | 4-3-1 | 1.0 | - | - | | | | | 86 | 68 |
| Example 46-6 | Nonaqueous electrolyte solution 46-6 | 4-1-11 | 1.0 | - | - | VC | 1.0 | TFOP | 1.0 | 74 | 80 |
| Comparative Example 38-1 | Comparative nonaqueous electrolyte solution 38-1 | - | - | - | - | | | | | 100 | 100 |
| Comparative Example 38-2 | Comparative nonaqueous electrolyte solution 38-2 | 6-1 | 1.0 | - | - | | | | | 133 | 88 |
| Comparative Example 38-3 | Comparative nonaqueous electrolyte solution 38-3 | 6-2 | 1.0 | - | - | | | | | 138 | 87 |
| Comparative Example 38-4 | Comparative nonaqueous electrolyte solution 38-4 | 6-3 | 1.0 | - | - | | | | | 107 | 98 |

**[Table 14]**

| Table 14 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 47-1 | Nonaqueous electrolyte solution 47-1 | 1-1-11 | 1.0 | - | - | | | | | 73 | 78 |
| Example 47-2 | Nonaqueous electrolyte solution 47-2 | 1-3-1 | 1.0 | - | - | | | | | 84 | 69 |
| Example 47-3 | Nonaqueous electrolyte solution 47-3 | 2-2-2 | 1.0 | - | - | | | | | 81 | 69 |
| Example 47-4 | Nonaqueous electrolyte solution 47-4 | 3-3-3 | 1.0 | - | - | | | | | 83 | 70 |
| Example 47-5 | Nonaqueous electrolyte solution 47-5 | 4-3-1 | 1.0 | - | - | | | | | 82 | 71 |
| Example 47-6 | Nonaqueous electrolyte solution 47-6 | 4-1-11 | 1.0 | - | - | VC | 1.0 | DTD | 1.0 | 72 | 80 |
| Comparative Example 39-1 | Comparative nonaqueous electrolyte solution 39-1 | - | - | - | - | | | | | 100 | 100 |
| Comparative Example 39-2 | Comparative nonaqueous electrolyte solution 39-2 | 6-1 | 1.0 | - | - | | | | | 137 | 87 |
| Comparative Example 39-3 | Comparative nonaqueous electrolyte solution 39-3 | 6-2 | 1.0 | - | - | | | | | 139 | 88 |
| Comparative Example 39-4 | Comparative nonaqueous electrolyte solution 39-4 | 6-3 | 1.0 | - | - | | | | | 111 | 95 |
| Example 48-1 | Nonaqueous electrolyte solution 48-1 | 1-1-11 | 1.0 | - | - | | | | | 77 | 75 |
| Example 48-2 | Nonaqueous electrolyte solution 48-2 | 1-3-1 | 1.0 | - | - | | | | | 88 | 66 |
| Example 48-3 | Nonaqueous electrolyte solution 48-3 | 2-2-2 | 1.0 | - | - | | | | | 84 | 67 |
| Example 48-4 | Nonaqueous electrolyte solution 48-4 | 3-3-3 | 1.0 | - | - | | | | | 87 | 67 |
| Example 48-5 | Nonaqueous electrolyte solution 48-5 | 4-3-1 | 1.0 | - | - | | | | | 86 | 68 |
| Example 48-6 | Nonaqueous electrolyte solution 48-6 | 4-1-11 | 1.0 | - | - | VC | 1.0 | DFPFSI | 1.0 | 75 | 77 |
| Comparative Example 40-1 | Comparative nonaqueous electrolyte solution 40-1 | - | - | - | - | | | | | 100 | 100 |
| Comparative Example 40-2 | Comparative nonaqueous electrolyte solution 40-2 | 6-1 | 1.0 | - | - | | | | | 136 | 88 |
| Comparative Example 40-3 | Comparative nonaqueous electrolyte solution 40-3 | 6-2 | 1.0 | - | - | | | | | 139 | 89 |
| Comparative Example 40-4 | Comparative nonaqueous electrolyte solution 40-4 | 6-3 | 1.0 | - | - | | | | | 110 | 97 |
| Example 49-1 | Nonaqueous electrolyte solution 49-1 | 1-1-11 | 1.0 | - | - | | | | | 78 | 72 |
| Example 49-2 | Nonaqueous electrolyte solution 49-2 | 1-3-1 | 1.0 | - | - | | | | | 88 | 64 |
| Example 49-3 | Nonaqueous electrolyte solution 49-3 | 2-2-2 | 1.0 | - | - | | | | | 83 | 66 |
| Example 49-4 | Nonaqueous electrolyte solution 49-4 | 3-3-3 | 1.0 | - | - | | | | | 87 | 65 |
| Example 49-5 | Nonaqueous electrolyte solution 49-5 | 4-3-1 | 1.0 | - | - | | | | | 86 | 66 |
| Example 49-6 | Nonaqueous electrolyte solution 49-6 | 4-1-11 | 1.0 | - | - | VC | 1.0 | FS | 1.0 | 77 | 74 |
| Comparative Example 41-1 | Comparative nonaqueous electrolyte solution 41-1 | - | - | - | - | | | | | 100 | 100 |
| Comparative Example 41-2 | Comparative nonaqueous electrolyte solution 41-2 | 6-1 | 1.0 | - | - | | | | | 140 | 86 |
| Comparative Example 41-3 | Comparative nonaqueous electrolyte solution 41-3 | 6-2 | 1.0 | - | - | | | | | 141 | 88 |
| Comparative Example 41-4 | Comparative nonaqueous electrolyte solution 41-4 | 6-3 | 1.0 | - | - | | | | | 113 | 94 |
| Example 50-1 | Nonaqueous electrolyte solution 50-1 | 1-1-11 | 1.0 | - | - | | | | | 69 | 81 |
| Example 50-2 | Nonaqueous electrolyte solution 50-2 | 1-3-1 | 1.0 | - | - | | | | | 78 | 69 |
| Example 50-3 | Nonaqueous electrolyte solution 50-3 | 2-2-2 | 1.0 | - | - | | | | | 76 | 70 |
| Example 50-4 | Nonaqueous electrolyte solution 50-4 | 3-3-3 | 1.0 | - | - | | | | | 77 | 70 |
| Example 50-5 | Nonaqueous electrolyte solution 50-5 | 4-3-1 | 1.0 | - | - | | | | | 77 | 71 |
| Example 50-6 | Nonaqueous electrolyte solution 50-6 | 4-1-11 | 1.0 | - | - | VC | 1.0 | TV-Si | 0.5 | 67 | 83 |
| Comparative Example 42-1 | Comparative nonaqueous electrolyte solution 42-1 | - | - | - | - | | | | | 100 | 100 |
| Comparative Example 42-2 | Comparative nonaqueous electrolyte solution 42-2 | 6-1 | 1.0 | - | - | | | | | 144 | 85 |
| Comparative Example 42-3 | Comparative nonaqueous electrolyte solution 42-3 | 6-2 | 1.0 | - | - | | | | | 145 | 86 |
| Comparative Example 42-4 | Comparative nonaqueous electrolyte solution 42-4 | 6-3 | 1.0 | - | - | | | | | 113 | 95 |

**[Table 15]**

| Table 15 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 51-1 | Nonaqueous electrolyte solution 51-1 | 1-1-11 | 1.0 | | | | | | | 68 | 82 |
| Example 51-2 | Nonaqueous electrolyte solution 51-2 | 1-1-12 | 1.0 | | | | | | | 79 | 70 |
| Example 51-3 | Nonaqueous electrolyte solution 51-3 | 1-1-13 | 1.0 | | | | | | | 76 | 71 |
| Example 51-4 | Nonaqueous electrolyte solution 51-4 | 1-1-14 | 1.0 | | | | | | | 78 | 71 |
| Example 51-5 | Nonaqueous electrolyte solution 51-5 | 1-1-15 | 1.0 | | | | | | | 77 | 72 |
| Example 51-6 | Nonaqueous electrolyte solution 51-6 | 4-1-11 | 1.0 | | | | | | | 67 | 84 |
| Comparative Example 43-1 | Comparative nonaqueous electrolyte solution 43-1 | - | - | DFP | 0.1 | VC | 1.0 | BOB | 1.0 | 100 | 100 |
| Comparative Example 43-2 | Comparative nonaqueous electrolyte solution 43-2 | 6-1 | 1.0 | | | | | | | 141 | 88 |
| Comparative Example 43-3 | Comparative nonaqueous electrolyte solution 43-3 | 6-2 | 1.0 | | | | | | | 143 | 89 |
| Comparative Example 43-4 | Comparative nonaqueous electrolyte solution 43-4 | 6-3 | 1.0 | | | | | | | 113 | 97 |
| Example 52-1 | Nonaqueous electrolyte solution 52-1 | 1-1-11 | 1.0 | | | | | | | 76 | 77 |
| Example 52-2 | Nonaqueous electrolyte solution 52-2 | 1-3-1 | 1.0 | | | | | | | 87 | 68 |
| Example 52-3 | Nonaqueous electrolyte solution 52-3 | 2-2-2 | 1.0 | | | | | | | 84 | 69 |
| Example 52-4 | Nonaqueous electrolyte solution 52-4 | 3-3-3 | 1.0 | | | | | | | 86 | 69 |
| Example 52-5 | Nonaqueous electrolyte solution 52-5 | 4-3-1 | 1.0 | | | | | | | 85 | 70 |
| Example 52-6 | Nonaqueous electrolyte solution 52-6 | 4-1-11 | 1.0 | DFP | 0.1 | VC | 1.0 | DFBOP | 1.0 | 75 | 79 |
| Comparative Example 44-1 | Comparative nonaqueous electrolyte solution 44-1 | - | - | | | | | | | 100 | 100 |
| Comparative Example 44-2 | Comparative nonaqueous electrolyte solution 44-2 | 6-1 | 1.0 | | | | | | | 136 | 89 |
| Comparative Example 44-3 | Comparative nonaqueous electrolyte solution 44-3 | 6-2 | 1.0 | | | | | | | 138 | 91 |
| Comparative Example 44-4 | Comparative nonaqueous electrolyte solution 44-4 | 6-3 | 1.0 | | | | | | | 110 | 99 |
| Example 53-1 | Nonaqueous electrolyte solution 53-1 | 1-1-11 | 1.0 | | | | | | | 75 | 77 |
| Example 53-2 | Nonaqueous electrolyte solution 53-2 | 1-3-1 | 1.0 | | | | | | | 87 | 66 |
| Example 53-3 | Nonaqueous electrolyte solution 53-3 | 2-2-2 | 1.0 | | | | | | | 84 | 67 |
| Example 53-4 | Nonaqueous electrolyte solution 53-4 | 3-3-3 | 1.0 | | | | | | | 86 | 67 |
| Example 53-5 | Nonaqueous electrolyte solution 53-5 | 4-3-1 | 1.0 | | | | | | | 85 | 68 |
| Example 53-6 | Nonaqueous electrolyte solution 53-6 | 4-1-11 | 1.0 | DFP | 0.1 | VC | 1.0 | DFOB | 1.0 | 74 | 79 |
| Comparative Example 45-1 | Comparative nonaqueous electrolyte solution 45-1 | - | - | | | | | | | 100 | 100 |
| Comparative Example 45-2 | Comparative nonaqueous electrolyte solution 45-2 | 6-1 | 1.0 | | | | | | | 134 | 88 |
| Comparative Example 45-3 | Comparative nonaqueous electrolyte solution 45-3 | 6-2 | 1.0 | | | | | | | 136 | 90 |
| Comparative Example 45-4 | Comparative nonaqueous electrolyte solution 45-4 | 6-3 | 1.0 | | | | | | | 108 | 98 |
| Example 54-1 | Nonaqueous electrolyte solution 54-1 | 1-1-11 | 1.0 | | | | | | | 74 | 77 |
| Example 54-2 | Nonaqueous electrolyte solution 54-2 | 1-3-1 | 1.0 | | | | | | | 86 | 65 |
| Example 54-3 | Nonaqueous electrolyte solution 54-3 | 2-2-2 | 1.0 | | | | | | | 84 | 66 |
| Example 54-4 | Nonaqueous electrolyte solution 54-4 | 3-3-3 | 1.0 | | | | | | | 85 | 66 |
| Example 54-5 | Nonaqueous electrolyte solution 54-5 | 4-3-1 | 1.0 | | | | | | | 85 | 67 |
| Example 54-6 | Nonaqueous electrolyte solution 54-6 | 4-1-11 | 1.0 | DFP | 0.1 | VC | 1.0 | TFOP | 1.0 | 73 | 79 |
| Comparative Example 46-1 | Comparative nonaqueous electrolyte solution 46-1 | - | - | | | | | | | 100 | 100 |
| Comparative Example 46-2 | Comparative nonaqueous electrolyte solution 46-2 | 6-1 | 1.0 | | | | | | | 133 | 88 |
| Comparative Example 46-3 | Comparative nonaqueous electrolyte solution 46-3 | 6-2 | 1.0 | | | | | | | 138 | 87 |
| Comparative Example 46-4 | Comparative nonaqueous electrolyte solution 46-4 | 6-3 | 1.0 | | | | | | | 107 | 98 |

**[Table 16]**

| Table 16 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 55-1 | Nonaqueous electrolyte solution 55-1 | 1-1-11 | 1.0 | | | | | | | 72 | 77 |
| Example 55-2 | Nonaqueous electrolyte solution 55-2 | 1-3-1 | 1.0 | | | | | | | 82 | 68 |
| Example 55-3 | Nonaqueous electrolyte solution 55-3 | 2-2-2 | 1.0 | | | | | | | 79 | 68 |
| Example 55-4 | Nonaqueous electrolyte solution 55-4 | 3-3-3 | 1.0 | | | | | | | 81 | 69 |
| Example 55-5 | Nonaqueous electrolyte solution 55-5 | 4-3-1 | 1.0 | DFP | 0.1 | VC | 1.0 | DTD | 1.0 | 80 | 70 |
| Example 55-6 | Nonaqueous electrolyte solution 55-6 | 4-1-11 | 1.0 | | | | | | | 70 | 79 |
| Comparative Example 47-1 | Comparative nonaqueous electrolyte solution 47-1 | - | - | | | | | | | 100 | 100 |
| Comparative Example 47-2 | Comparative nonaqueous electrolyte solution 47-2 | 6-1 | 1.0 | | | | | | | 137 | 87 |
| Comparative Example 47-3 | Comparative nonaqueous electrolyte solution 47-3 | 6-2 | 1.0 | | | | | | | 139 | 89 |
| Comparative Example 47-4 | Comparative nonaqueous electrolyte solution 47-4 | 6-3 | 1.0 | | | | | | | 111 | 95 |
| Example 56-1 | Nonaqueous electrolyte solution 56-1 | 1-1-11 | 1.0 | | | | | | | 75 | 74 |
| Example 56-2 | Nonaqueous electrolyte solution 56-2 | 1-3-1 | 1.0 | | | | | | | 86 | 65 |
| Example 56-3 | Nonaqueous electrolyte solution 56-3 | 2-2-2 | 1.0 | | | | | | | 82 | 66 |
| Example 56-4 | Nonaqueous electrolyte solution 56-4 | 3-3-3 | 1.0 | | | | | | | 85 | 66 |
| Example 56-5 | Nonaqueous electrolyte solution 56-5 | 4-3-1 | 1.0 | | | | | | | 84 | 67 |
| Example 56-6 | Nonaqueous electrolyte solution 56-6 | 4-1-11 | 1.0 | DFP | 0.1 | VC | 1.0 | DFPFSI | 1.0 | 74 | 76 |
| Comparative Example 48-1 | Comparative nonaqueous electrolyte solution 48-1 | - | - | | | | | | | 100 | 100 |
| Comparative Example 48-2 | Comparative nonaqueous electrolyte solution 48-2 | 6-1 | 1.0 | | | | | | | 136 | 88 |
| Comparative Example 48-3 | Comparative nonaqueous electrolyte solution 48-3 | 6-2 | 1.0 | | | | | | | 139 | 89 |
| Comparative Example 48-4 | Comparative nonaqueous electrolyte solution 48-4 | 6-3 | 1.0 | | | | | | | 110 | 97 |
| Example 57-1 | Nonaqueous electrolyte solution 57-1 | 1-1-11 | 1.0 | | | | | | | 77 | 71 |
| Example 57-2 | Nonaqueous electrolyte solution 57-2 | 1-3-1 | 1.0 | | | | | | | 86 | 63 |
| Example 57-3 | Nonaqueous electrolyte solution 57-3 | 2-2-2 | 1.0 | | | | | | | 82 | 65 |
| Example 57-4 | Nonaqueous electrolyte solution 57-4 | 3-3-3 | 1.0 | | | | | | | 85 | 64 |
| Example 57-5 | Nonaqueous electrolyte solution 57-5 | 4-3-1 | 1.0 | | | | | | | 84 | 65 |
| Example 57-6 | Nonaqueous electrolyte solution 57-6 | 4-1-11 | 1.0 | DFP | 0.1 | VC | 1.0 | FS | 1.0 | 75 | 73 |
| Comparative Example 49-1 | Comparative nonaqueous electrolyte solution 49-1 | - | - | | | | | | | 100 | 100 |
| Comparative Example 49-2 | Comparative nonaqueous electrolyte solution 49-2 | 6-1 | 1.0 | | | | | | | 139 | 86 |
| Comparative Example 49-3 | Comparative nonaqueous electrolyte solution 49-3 | 6-2 | 1.0 | | | | | | | 140 | 88 |
| Comparative Example 49-4 | Comparative nonaqueous electrolyte solution 49-4 | 6-3 | 1.0 | | | | | | | 112 | 94 |
| Example 58-1 | Nonaqueous electrolyte solution 58-1 | 1-1-11 | 1.0 | | | | | | | 67 | 80 |
| Example 58-2 | Nonaqueous electrolyte solution 58-2 | 1-3-1 | 1.0 | | | | | | | 77 | 68 |
| Example 58-3 | Nonaqueous electrolyte solution 58-3 | 2-2-2 | 1.0 | | | | | | | 74 | 70 |
| Example 58-4 | Nonaqueous electrolyte solution 58-4 | 3-3-3 | 1.0 | | | | | | | 76 | 69 |
| Example 58-5 | Nonaqueous electrolyte solution 58-5 | 4-3-1 | 1.0 | | | | | | | 75 | 70 |
| Example 58-6 | Nonaqueous electrolyte solution 58-6 | 4-1-11 | 1.0 | DFP | 0.1 | VC | 1.0 | TV-Si | 0.5 | 66 | 83 |
| Comparative Example 50-1 | Comparative nonaqueous electrolyte solution 50-1 | - | - | | | | | | | 100 | 100 |
| Comparative Example 50-2 | Comparative nonaqueous electrolyte solution 50-2 | 6-1 | 1.0 | | | | | | | 143 | 85 |
| Comparative Example 50-3 | Comparative nonaqueous electrolyte solution 50-3 | 6-2 | 1.0 | | | | | | | 145 | 86 |
| Comparative Example 50-4 | Comparative nonaqueous electrolyte solution 50-4 | 6-3 | 1.0 | | | | | | | 113 | 95 |

**[Table 17]**

| Table 17 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 59-1 | Nonaqueous electrolyte solution 59-1 | 1-1-11 | 1.0 | | | | | | | 68 | 81 |
| Example 59-2 | Nonaqueous electrolyte solution 59-2 | 1-3-1 | 1.0 | | | | | | | 80 | 69 |
| Example 59-3 | Nonaqueous electrolyte solution 59-3 | 2-2-2 | 1.0 | | | | | | | 76 | 71 |
| Example 59-4 | Nonaqueous electrolyte solution 59-4 | 3-3-3 | 1.0 | | | | | | | 79 | 70 |
| Example 59-5 | Nonaqueous electrolyte solution 59-5 | 4-3-1 | 1.0 | | | | | | | 78 | 71 |
| Example 59-6 | Nonaqueous electrolyte solution 59-6 | 4-1-11 | 1.0 | BF₄ | 0.025 | VC | 1.0 | BOB | 1.0 | 67 | 83 |
| Comparative Example 51-1 | Comparative nonaqueous electrolyte solution 51-1 | - | - | | | | | | | 100 | 100 |
| Comparative Example 51-2 | Comparative nonaqueous electrolyte solution 51-2 | 6-1 | 1.0 | | | | | | | 141 | 88 |
| Comparative Example 51-3 | Comparative nonaqueous electrolyte solution 51-3 | 6-2 | 1.0 | | | | | | | 143 | 89 |
| Comparative Example 51-4 | Comparative nonaqueous electrolyte solution 51-4 | 6-3 | 1.0 | | | | | | | 113 | 97 |
| Example 60-1 | Nonaqueous electrolyte solution 60-1 | 1-1-11 | 1.0 | | | | | | | 77 | 77 |
| Example 60-2 | Nonaqueous electrolyte solution 60-2 | 1-3-1 | 1.0 | | | | | | | 87 | 67 |
| Example 60-3 | Nonaqueous electrolyte solution 60-3 | 2-2-2 | 1.0 | | | | | | | 84 | 69 |
| Example 60-4 | Nonaqueous electrolyte solution 60-4 | 3-3-3 | 1.0 | | | | | | | 86 | 68 |
| Example 60-5 | Nonaqueous electrolyte solution 60-5 | 4-3-1 | 1.0 | | | | | | | 86 | 69 |
| Example 60-6 | Nonaqueous electrolyte solution 60-6 | 4-1-11 | 1.0 | | | | | | | 75 | 79 |
| Comparative Example 52-1 | Comparative nonaqueous electrolyte solution 52-1 | - | - | BF₄ | 0.025 | VC | 1.0 | DFBOP | 1.0 | 100 | 100 |
| Comparative Example 52-2 | Comparative nonaqueous electrolyte solution 52-2 | 6-1 | 1.0 | | | | | | | 135 | 89 |
| Comparative Example 52-3 | Comparative nonaqueous electrolyte solution 52-3 | 6-2 | 1.0 | | | | | | | 137 | 91 |
| Comparative Example 52-4 | Comparative nonaqueous electrolyte solution 52-4 | 6-3 | 1.0 | | | | | | | 110 | 99 |
| Example 61-1 | Nonaqueous electrolyte solution 61-1 | 1-1-11 | 1.0 | | | | | | | 76 | 76 |
| Example 61-2 | Nonaqueous electrolyte solution 61-2 | 1-3-1 | 1.0 | | | | | | | 87 | 66 |
| Example 61-3 | Nonaqueous electrolyte solution 61-3 | 2-2-2 | 1.0 | | | | | | | 84 | 66 |
| Example 61-4 | Nonaqueous electrolyte solution 61-4 | 3-3-3 | 1.0 | | | | | | | 86 | 66 |
| Example 61-5 | Nonaqueous electrolyte solution 61-5 | 4-3-1 | 1.0 | | | | | | | 85 | 67 |
| Example 61-6 | Nonaqueous electrolyte solution 61-6 | 4-1-11 | 1.0 | | | | | | | 74 | 78 |
| Comparative Example 53-1 | Comparative nonaqueous electrolyte solution 53-1 | - | - | BF₄ | 0.025 | VC | 1.0 | DFOB | 1.0 | 100 | 100 |
| Comparative Example 53-2 | Comparative nonaqueous electrolyte solution 53-2 | 6-1 | 1.0 | | | | | | | 133 | 89 |
| Comparative Example 53-3 | Comparative nonaqueous electrolyte solution 53-3 | 6-2 | 1.0 | | | | | | | 136 | 90 |
| Comparative Example 53-4 | Comparative nonaqueous electrolyte solution 53-4 | 6-3 | 1.0 | | | | | | | 108 | 98 |
| Example 62-1 | Nonaqueous electrolyte solution 62-1 | 1-1-11 | 1.0 | | | | | | | 75 | 76 |
| Example 62-2 | Nonaqueous electrolyte solution 62-2 | 1-3-1 | 1.0 | | | | | | | 87 | 65 |
| Example 62-3 | Nonaqueous electrolyte solution 62-3 | 2-2-2 | 1.0 | | | | | | | 84 | 65 |
| Example 62-4 | Nonaqueous electrolyte solution 62-4 | 3-3-3 | 1.0 | | | | | | | 86 | 66 |
| Example 62-5 | Nonaqueous electrolyte solution 62-5 | 4-3-1 | 1.0 | | | | | | | 85 | 67 |
| Example 62-6 | Nonaqueous electrolyte solution 62-6 | 4-1-11 | 1.0 | BF₄ | 0.025 | VC | 1.0 | TFOP | 1.0 | 73 | 78 |
| Comparative Example 54-1 | Comparative nonaqueous electrolyte solution 54-1 | - | - | | | | | | | 100 | 100 |
| Comparative Example 54-2 | Comparative nonaqueous electrolyte solution 54-2 | 6-1 | 1.0 | | | | | | | 133 | 88 |
| Comparative Example 54-3 | Comparative nonaqueous electrolyte solution 54-3 | 6-2 | 1.0 | | | | | | | 138 | 87 |
| Comparative Example 54-4 | Comparative nonaqueous electrolyte solution 54-4 | 6-3 | 1.0 | | | | | | | 107 | 98 |

**[Table 18]**

| Table 18 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L) | Type | Content [mass%] | Type | Content [mass%] | | |
| Example 63-1 | Nonaqueous electrolyte solution 63-1 | 1-1-11 | 1.0 | | | | | | | 72 | 77 |
| Example 63-2 | Nonaqueous electrolyte solution 63-2 | 1-3-1 | 1.0 | | | | | | | 82 | 68 |
| Example 63-3 | Nonaqueous electrolyte solution 63-3 | 2-2-2 | 1.0 | | | | | | | 79 | 68 |
| Example 63-4 | Nonaqueous electrolyte solution 63-4 | 3-3-3 | 1.0 | | | | | | | 81 | 69 |
| Example 63-5 | Nonaqueous electrolyte solution 63-5 | 4-3-1 | 1.0 | | | | | | | 80 | 70 |
| Example 63-6 | Nonaqueous electrolyte solution 63-6 | 4-1-11 | 1.0 | BF₄ | 0.025 | VC | 1.0 | DTD | 1.0 | 71 | 79 |
| Comparative Example 55-1 | Comparative nonaqueous electrolyte solution 55-1 | - | - | | | | | | | 100 | 100 |
| Comparative Example 55-2 | Comparative nonaqueous electrolyte solution 55-2 | 6-1 | 1.0 | | | | | | | 137 | 87 |
| Comparative Example 55-3 | Comparative nonaqueous electrolyte solution 55-3 | 6-2 | 1.0 | | | | | | | 139 | 89 |
| Comparative Example 55-4 | Comparative nonaqueous electrolyte solution 55-4 | 6-3 | 1.0 | | | | | | | 111 | 95 |
| Example 64-1 | Nonaqueous electrolyte solution 64-1 | 1-1-11 | 1.0 | | | | | | | 76 | 73 |
| Example 64-2 | Nonaqueous electrolyte solution 64-2 | 1-3-1 | 1.0 | | | | | | | 86 | 65 |
| Example 64-3 | Nonaqueous electrolyte solution 64-3 | 2-2-2 | 1.0 | | | | | | | 83 | 66 |
| Example 64-4 | Nonaqueous electrolyte solution 64-4 | 3-3-3 | 1.0 | | | | | | | 85 | 66 |
| Example 64-5 | Nonaqueous electrolyte solution 64-5 | 4-3-1 | 1.0 | | | | | | | 84 | 67 |
| Example 64-6 | Nonaqueous electrolyte solution 64-6 | 4-1-11 | 1.0 | | | | | | | 74 | 75 |
| Comparative Example 56-1 | Comparative nonaqueous electrolyte solution 56-1 | - | - | BF₄ | 0.025 | VC | 1.0 | DFPFSI | 1.0 | 100 | 100 |
| Comparative Example 56-2 | Comparative nonaqueous electrolyte solution 56-2 | 6-1 | 1.0 | | | | | | | 136 | 88 |
| Comparative Example 56-3 | Comparative nonaqueous electrolyte solution 56-3 | 6-2 | 1.0 | | | | | | | 139 | 89 |
| Comparative Example 56-4 | Comparative nonaqueous electrolyte solution 56-4 | 6-3 | 1.0 | | | | | | | 110 | 97 |
| Example 65-1 | Nonaqueous electrolyte solution 65-1 | 1-1-11 | 1.0 | | | | | | | 77 | 71 |
| Example 65-2 | Nonaqueous electrolyte solution 65-2 | 1-3-1 | 1.0 | | | | | | | 86 | 63 |
| Example 65-3 | Nonaqueous electrolyte solution 65-3 | 2-2-2 | 1.0 | | | | | | | 82 | 65 |
| Example 65-4 | Nonaqueous electrolyte solution 65-4 | 3-3-3 | 1.0 | | | | | | | 85 | 64 |
| Example 65-5 | Nonaqueous electrolyte solution 65-5 | 4-3-1 | 1.0 | | | | | | | 84 | 65 |
| Example 65-6 | Nonaqueous electrolyte solution 65-6 | 4-1-11 | 1.0 | | | | | | | 76 | 73 |
| Comparative Example 57-1 | Comparative nonaqueous electrolyte solution 57-1 | - | - | BF₄ | 0.025 | VC | 1.0 | FS | 1.0 | 100 | 100 |
| Comparative Example 57-2 | Comparative nonaqueous electrolyte solution 57-2 | 6-1 | 1.0 | | | | | | | 139 | 87 |
| Comparative Example 57-3 | Comparative nonaqueous electrolyte solution 57-3 | 6-2 | 1.0 | | | | | | | 140 | 88 |
| Comparative Example 57-4 | Comparative nonaqueous electrolyte solution 57-4 | 6-3 | 1.0 | | | | | | | 112 | 95 |
| Example 66-1 | Nonaqueous electrolyte solution 66-1 | 1-1-11 | 1.0 | | | | | | | 68 | 80 |
| Example 66-2 | Nonaqueous electrolyte solution 66-2 | 1-3-1 | 1.0 | | | | | | | 77 | 68 |
| Example 66-3 | Nonaqueous electrolyte solution 66-3 | 2-2-2 | 1.0 | | | | | | | 74 | 69 |
| Example 66-4 | Nonaqueous electrolyte solution 66-4 | 3-3-3 | 1.0 | | | | | | | 76 | 69 |
| Example 66-5 | Nonaqueous electrolyte solution 66-5 | 4-3-1 | 1.0 | | | | | | | 75 | 70 |
| Example 66-6 | Nonaqueous electrolyte solution 66-6 | 4-1-11 | 1.0 | BF₄ | 0.025 | VC | 1.0 | TV-Si | 0.5 | 66 | 82 |
| Comparative Example 58-1 | Comparative nonaqueous electrolyte solution 58-1 | - | - | | | | | | | 100 | 100 |
| Comparative Example 58-2 | Comparative nonaqueous electrolyte solution 58-2 | 6-1 | 1.0 | | | | | | | 143 | 85 |
| Comparative Example 58-3 | Comparative nonaqueous electrolyte solution 58-3 | 6-2 | 1.0 | | | | | | | 145 | 86 |
| Comparative Example 58-4 | Comparative nonaqueous electrolyte solution 58-4 | 6-3 | 1.0 | | | | | | | 113 | 95 |

**[Table 19]**

| Table 19 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 67-1 | Nonaqueous electrolyte solution 67-1 | 1-1-11-Na | 0.05 | - | - | - | - | - | - | 93 | 70 |
| Example 67-2 | Nonaqueous electrolyte solution 67-2 | | 0.1 | - | - | - | - | - | - | 90 | 68 |
| Example 67-3 | Nonaqueous electrolyte solution 67-3 | | 0.5 | - | - | - | - | - | - | 84 | 68 |
| Example 67-4 | Nonaqueous electrolyte solution 67-4 | | 1.0 | - | - | - | - | - | - | 77 | 68 |
| Example 67-5 | Nonaqueous electrolyte solution 67-5 | | 2.0 | - | - | - | - | - | - | 72 | 69 |
| Example 67-6 | Nonaqueous electrolyte solution 67-6 | | 3.0 | - | - | - | - | - | - | 74 | 66 |
| Example 67-7 | Nonaqueous electrolyte solution 67-7 | 1-3-1-Na | 0.05 | - | - | - | - | - | - | 92 | 70 |
| Example 67-8 | Nonaqueous electrolyte solution 67-8 | | 0.1 | - | - | - | - | - | - | 89 | 68 |
| Example 67-9 | Nonaqueous electrolyte solution 67-9 | | 0.5 | - | - | - | - | - | - | 83 | 64 |
| Example 67-10 | Nonaqueous electrolyte solution 67-10 | | 1.0 | - | - | - | - | - | - | 89 | 58 |
| Example 67-11 | Nonaqueous electrolyte solution 67-11 | | 2.0 | - | - | - | - | - | - | 91 | 54 |
| Example 67-12 | Nonaqueous electrolyte solution 67-12 | | 3.0 | - | - | - | - | - | - | 92 | 52 |
| Example 68-1 | Nonaqueous electrolyte solution 68-1 | 2-2-2-Na | 0.05 | - | - | - | - | - | - | 92 | 70 |
| Example 68-2 | Nonaqueous electrolyte solution 68-2 | | 0.1 | - | - | - | - | - | - | 89 | 69 |
| Example 68-3 | Nonaqueous electrolyte solution 68-3 | | 0.5 | - | - | - | - | - | - | 86 | 64 |
| Example 68-4 | Nonaqueous electrolyte solution 68-4 | | 1.0 | - | - | - | - | - | - | 84 | 60 |
| Example 68-5 | Nonaqueous electrolyte solution 68-5 | | 2.0 | - | - | - | - | - | - | 82 | 60 |
| Example 68-6 | Nonaqueous electrolyte solution 68-6 | | 3.0 | - | - | - | - | - | - | 87 | 55 |
| Example 69-1 | Nonaqueous electrolyte solution 69-1 | 3-3-3-Na | 0.05 | - | - | - | - | - | - | 91 | 70 |
| Example 69-2 | Nonaqueous electrolyte solution 69-2 | | 0.1 | - | - | - | - | - | - | 88 | 69 |
| Example 69-3 | Nonaqueous electrolyte solution 69-3 | | 0.5 | - | - | - | - | - | - | 82 | 66 |
| Example 69-4 | Nonaqueous electrolyte solution 69-4 | | 1.0 | - | - | - | - | - | - | 88 | 58 |
| Example 69-5 | Nonaqueous electrolyte solution 69-5 | | 2.0 | - | - | - | - | - | - | 89 | 55 |
| Example 69-6 | Nonaqueous electrolyte solution 69-6 | | 3.0 | - | - | - | - | - | - | 91 | 53 |
| Example 70-1 | Nonaqueous electrolyte solution 70-1 | 4-3-1-Na | 0.05 | - | - | - | - | - | - | 91 | 70 |
| Example 70-2 | Nonaqueous electrolyte solution 70-2 | | 0.1 | - | - | - | - | - | - | 88 | 69 |
| Example 70-3 | Nonaqueous electrolyte solution 70-3 | | 0.5 | - | - | - | - | - | - | 81 | 66 |
| Example 70-4 | Nonaqueous electrolyte solution 70-4 | | 1.0 | - | - | - | - | - | - | 87 | 59 |
| Example 70-5 | Nonaqueous electrolyte solution 70-5 | | 2.0 | - | - | - | - | - | - | 88 | 56 |
| Example 70-6 | Nonaqueous electrolyte solution 70-6 | | 3.0 | - | - | - | - | - | - | 90 | 53 |
| Example 70-7 | Nonaqueous electrolyte solution 70-7 | 4-1-11-Na | 0.05 | - | - | - | - | - | - | 92 | 71 |
| Example 70-8 | Nonaqueous electrolyte solution 70-8 | | 0.1 | - | - | - | - | - | - | 89 | 69 |
| Example 70-9 | Nonaqueous electrolyte solution 70-9 | | 0.5 | - | - | - | - | - | - | 83 | 69 |
| Example 70-10 | Nonaqueous electrolyte solution 70-10 | | 1.0 | - | - | - | - | - | - | 76 | 70 |
| Example 70-11 | Nonaqueous electrolyte solution 70-11 | | 2.0 | - | - | - | - | - | - | 71 | 70 |
| Example 70-12 | Nonaqueous electrolyte solution 70-12 | | 3.0 | - | - | - | - | - | - | 74 | 67 |
| Comparative Example 59-1 | Comparative nonaqueous electrolyte solution 59-1 | - | - | - | - | - | - | - | - | 100 | 100 |
| Comparative Example 59-2 | Comparative nonaqueous electrolyte solution 59-2 | 6-1 | 1.0 | - | - | - | - | - | - | 142 | 81 |
| Comparative Example 59-3 | Comparative nonaqueous electrolyte solution 59-3 | 6-2 | 1.0 | - | - | - | - | - | - | 144 | 82 |
| Comparative Example 59-4 | Comparative nonaqueous electrolyte solution 59-4 | 6-3 | 1.0 | - | - | - | - | - | - | 113 | 92 |

**[Table 20]**

| Table 20 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 71-1 | Nonaqueous electrolyte solution 71-1 | 1-1-11-Na | 0.5 | - | - | - | - | - | - | 83 | 62 |
| | | 1-3-1-Na | 0.5 | | | | | | | | |
| Example 71-2 | Nonaqueous electrolyte solution 71-2 | 1-1-11-Na | 0.5 | - | - | - | - | - | - | 81 | 64 |
| | | 2-2-2-Na | 0.5 | | | | | | | | |
| Example 71-3 | Nonaqueous electrolyte solution 71-3 | 1-1-11-Na | 0.5 | - | - | - | - | - | - | 83 | 63 |
| | | 3-3-3-Na | 0.5 | | | | | | | | |
| Example 71-4 | Nonaqueous electrolyte solution 71-4 | 1-1-11-Na | 0.5 | - | - | - | - | - | - | 82 | 63 |
| | | 4-3-1-Na | 0.5 | | | | | | | | |
| Example 71-5 | Nonaqueous electrolyte solution 71-5 | 2-2-2-Na | 0.5 | - | - | - | - | - | - | 86 | 59 |
| | | 3-3-3-Na | 0.5 | | | | | | | | |
| Example 71-6 | Nonaqueous electrolyte solution 71-6 | 2-2-2-Na | 0.5 | - | - | - | - | - | - | 86 | 60 |
| | | 4-3-1-Na | 0.5 | | | | | | | | |
| Example 71-7 | Nonaqueous electrolyte solution 71-7 | 3-3-3-Na | 0.5 | - | - | - | - | - | - | 87 | 59 |
| | | 4-3-1-Na | 0.5 | | | | | | | | |
| Example 71-8 | Nonaqueous electrolyte solution 71-8 | 1-1-11-Na | 0.25 | - | - | - | - | - | - | 84 | 61 |
| | | 2-2-2-Na | 0.25 | | | | | | | | |
| | | 3-3-3-Na | 0.25 | | | | | | | | |
| | | 4-3-1-Na | 0.25 | | | | | | | | |
| Example 71-9 | Nonaqueous electrolyte solution 71-9 | 1-1-11-Na | 0.5 | - | - | - | - | - | - | 76 | 69 |
| | | 4-1-11-Na | 0.5 | | | | | | | | |
| Comparative Example 59-1 | Comparative nonaqueous electrolyte solution 59-1 | - | - | - | - | - | - | - | - | 100 | 100 |
| Comparative Example 59-2 | Comparative nonaqueous electrolyte solution 59-2 | 6-1 | 1.0 | - | - | - | - | - | - | 142 | 81 |
| Comparative Example 59-3 | Comparative nonaqueous electrolyte solution 59-3 | 6-2 | 1.0 | - | - | - | - | - | - | 144 | 82 |
| Comparative Example 59-4 | Comparative nonaqueous electrolyte solution 59-4 | 6-3 | 1.0 | - | - | - | - | - | - | 113 | 92 |

**[Table 21]**

| Table 21 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 72-1 | Nonaqueous electrolyte solution 72-1 | 1-1-11-Na | 1.0 | - | - | | | - | - | 75 | 70 |
| Example 72-2 | Nonaqueous electrolyte solution 72-2 | 1-3-1-Na | 1.0 | - | - | | | - | - | 88 | 60 |
| Example 72-3 | Nonaqueous electrolyte solution 72-3 | 2-2-2-Na | 1.0 | - | - | | | - | - | 84 | 62 |
| Example 72-4 | Nonaqueous electrolyte solution 72-4 | 3-3-3-Na | 1.0 | - | - | | | - | - | 87 | 61 |
| Example 72-5 | Nonaqueous electrolyte solution 72-5 | 4-3-1-Na | 1.0 | - | - | | | - | - | 85 | 62 |
| Example 72-6 | Nonaqueous electrolyte solution 72-6 | 4-1-11-Na | 1.0 | - | - | DFBOP-Na | 1.0 | - | - | 74 | 72 |
| Comparative Example 60-1 | Comparative nonaqueous electrolyte solution 60-1 | - | - | - | - | | | - | - | 100 | 100 |
| Comparative Example 60-2 | Comparative nonaqueous electrolyte solution 60-2 | 6-1 | 1.0 | - | - | | | - | - | 140 | 83 |
| Comparative Example 60-3 | Comparative nonaqueous electrolyte solution 60-3 | 6-2 | 1.0 | - | - | | | - | - | 142 | 84 |
| Comparative Example 60-4 | Comparative nonaqueous electrolyte solution 60-4 | 6-3 | 1.0 | - | - | | | - | - | 111 | 95 |
| Example 73-1 | Nonaqueous Electrolyte Solution 73-1 | 1-1-11-Na | 1.0 | - | - | | | - | - | 74 | 71 |
| Example 73-2 | Nonaqueous Electrolyte Solution 73-2 | 1-3-1-Na | 1.0 | - | - | | | - | - | 87 | 60 |
| Example 73-3 | Nonaqueous Electrolyte Solution 73-3 | 2-2-2-Na | 1.0 | - | - | | | - | - | 83 | 61 |
| Example 73-4 | Nonaqueous Electrolyte Solution 73-4 | 3-3-3-Na | 1.0 | - | - | | | - | - | 86 | 61 |
| Example 73-5 | Nonaqueous Electrolyte Solution 73-5 | 4-3-1-Na | 1.0 | - | - | | | - | - | 85 | 62 |
| Example 73-6 | Nonaqueous Electrolyte Solution 73-6 | 4-1-11-Na | 1.0 | - | - | | | - | - | 72 | 73 |
| Comparative Example 61-1 | Comparative nonaqueous electrolyte solution 61-1 | - | - | - | - | DFOB-Na | 1.0 | - | - | 100 | 100 |
| Comparative Example 61-2 | Comparative nonaqueous electrolyte solution 61-2 | 6-1 | 1.0 | - | - | | | - | - | 138 | 83 |
| Comparative Example 61-3 | Comparative nonaqueous electrolyte solution 61-3 | 6-2 | 1.0 | - | - | | | - | - | 141 | 84 |
| Comparative Example 61-4 | Comparative nonaqueous electrolyte solution 61-4 | 6-3 | 1.0 | - | - | | | - | - | 109 | 95 |
| Example 74-1 | Nonaqueous Electrolyte Solution 74-1 | 1-1-11-Na | 1.0 | - | - | | | - | - | 74 | 70 |
| Example 74-2 | Nonaqueous Electrolyte Solution 74-2 | 1-3-1-Na | 1.0 | - | - | | | - | - | 87 | 59 |
| Example 74-3 | Nonaqueous Electrolyte Solution 74-3 | 2-2-2-Na | 1.0 | - | - | | | - | - | 84 | 60 |
| Example 74-4 | Nonaqueous Electrolyte Solution 74-4 | 3-3-3-Na | 1.0 | - | - | | | - | - | 86 | 60 |
| Example 74-5 | Nonaqueous Electrolyte Solution 74-5 | 4-3-1-Na | 1.0 | - | - | | | - | - | 85 | 61 |
| Example 74-6 | Nonaqueous Electrolyte Solution 74-6 | 4-1-11-Na | 1.0 | - | - | | | - | - | 72 | 72 |
| Comparative Example 62-1 | Comparative nonaqueous electrolyte solution 62-1 | - | - | - | - | TFOP-Na | 1.0 | - | - | 100 | 100 |
| Comparative Example 62-2 | Comparative nonaqueous electrolyte solution 62-2 | 6-1 | 1.0 | - | - | | | - | - | 136 | 84 |
| Comparative Example 62-3 | Comparative nonaqueous electrolyte solution 62-3 | 6-2 | 1.0 | - | - | | | - | - | 142 | 83 |
| Comparative Example 62-4 | Comparative nonaqueous electrolyte solution 62-4 | 6-3 | 1.0 | - | - | | | - | - | 108 | 96 |
| Example 75-1 | Nonaqueous electrolyte solution 75-1 | 1-1-11-Na | 1.0 | - | - | | | - | - | 70 | 73 |
| Example 75-2 | Nonaqueous electrolyte solution 75-2 | 1-3-1-Na | 1.0 | - | - | | | - | - | 82 | 62 |
| Example 75-3 | Nonaqueous electrolyte solution 75-3 | 2-2-2-Na | 1.0 | - | - | | | - | - | 78 | 63 |
| Example 75-4 | Nonaqueous electrolyte solution 75-4 | 3-3-3-Na | 1.0 | - | - | | | - | - | 81 | 63 |
| Example 75-5 | Nonaqueous electrolyte solution 75-5 | 4-3-1-Na | 1.0 | - | - | | | - | - | 80 | 64 |
| Example 75-6 | Nonaqueous electrolyte solution 75-6 | 4-1-11-Na | 1.0 | - | - | DTD | 1.0 | - | - | 68 | 75 |
| Comparative Example 63-1 | Comparative nonaqueous electrolyte solution 63-1 | - | - | - | - | | | - | - | 100 | 100 |
| Comparative Example 63-2 | Comparative nonaqueous electrolyte solution 63-2 | 6-1 | 1.0 | - | - | | | - | - | 142 | 82 |
| Comparative Example 63-3 | Comparative nonaqueous electrolyte solution 63-3 | 6-2 | 1.0 | - | - | | | - | - | 144 | 83 |
| Comparative Example 63-4 | Comparative nonaqueous electrolyte solution 63-4 | 6-3 | 1.0 | - | - | | | - | - | 112 | 92 |

**[Table 22]**

| Table 22 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 76-1 | Nonaqueous electrolyte solution 76-1 | 1-1-11-Na | 1.0 | - | - | | | - | - | 74 | 69 |
| Example 76-2 | Nonaqueous electrolyte solution 76-2 | 1-3-1-Na | 1.0 | - | - | | | - | - | 86 | 59 |
| Example 76-3 | Nonaqueous electrolyte solution 76-3 | 2-2-2-Na | 1.0 | - | - | | | - | - | 82 | 61 |
| Example 76-4 | Nonaqueous electrolyte solution 76-4 | 3-3-3-Na | 1.0 | - | - | | | - | - | 85 | 60 |
| Example 76-5 | Nonaqueous electrolyte solution 76-5 | 4-3-1-Na | 1.0 | - | - | DFPFSI-Na | 1.0 | - | - | 84 | 61 |
| Example 76-6 | Nonaqueous electrolyte solution 76-6 | 4-1-11-Na | 1.0 | - | - | | | - | - | 72 | 71 |
| Comparative Example 64-1 | Comparative nonaqueous electrolyte solution 64-1 | - | - | - | - | | | - | - | 100 | 100 |
| Comparative Example 64-2 | Comparative nonaqueous electrolyte solution 64-2 | 6-1 | 1.0 | - | - | | | - | - | 141 | 83 |
| Comparative Example 64-3 | Comparative nonaqueous electrolyte solution 64-3 | 6-2 | 1.0 | - | - | | | - | - | 144 | 83 |
| Comparative Example 64-4 | Comparative nonaqueous electrolyte solution 64-4 | 6-3 | 1.0 | - | - | | | - | - | 111 | 94 |
| Example 77-1 | Nonaqueous electrolyte solution 77-1 | 1-1-11-Na | 1.0 | - | - | | | - | - | 76 | 66 |
| Example 77-2 | Nonaqueous electrolyte solution 77-2 | 1-3-1-Na | 1.0 | - | - | | | - | - | 86 | 58 |
| Example 77-3 | Nonaqueous electrolyte solution 77-3 | 2-2-2-Na | 1.0 | - | - | | | - | - | 82 | 61 |
| Example 77-4 | Nonaqueous electrolyte solution 77-4 | 3-3-3-Na | 1.0 | - | - | | | - | - | 85 | 59 |
| Example 77-5 | Nonaqueous electrolyte solution 77-5 | 4-3-1-Na | 1.0 | - | - | | | - | - | 84 | 60 |
| Example 77-6 | Nonaqueous electrolyte solution 77-6 | 4-1-11-Na | 1.0 | - | - | FS-Na | 1.0 | - | - | 74 | 68 |
| Comparative Example 65-1 | Comparative nonaqueous electrolyte solution 65-1 | - | - | - | - | | | - | - | 100 | 100 |
| Comparative Example 65-2 | Comparative nonaqueous electrolyte solution 65-2 | 6-1 | 1.0 | - | - | | | - | - | 144 | 81 |
| Comparative Example 65-3 | Comparative nonaqueous electrolyte solution 65-3 | 6-2 | 1.0 | - | - | | | - | - | 145 | 82 |
| Comparative Example 65-4 | Comparative nonaqueous electrolyte solution 65-4 | 6-3 | 1.0 | - | - | | | - | - | 114 | 91 |
| Example 78-1 | Nonaqueous electrolyte solution 78-1 | 1-1-11-Na | 1.0 | - | - | | | - | - | 66 | 75 |
| Example 78-2 | Nonaqueous electrolyte solution 78-2 | 1-3-1-Na | 1.0 | - | - | | | - | - | 77 | 63 |
| Example 78-3 | Nonaqueous electrolyte solution 78-3 | 2-2-2-Na | 1.0 | - | - | | | - | - | 74 | 65 |
| Example 78-4 | Nonaqueous electrolyte solution 78-4 | 3-3-3-Na | 1.0 | - | - | | | - | - | 76 | 64 |
| Example 78-5 | Nonaqueous electrolyte solution 78-5 | 4-3-1-Na | 1.0 | - | - | | | - | - | 75 | 65 |
| Example 78-6 | Nonaqueous electrolyte solution 78-6 | 4-1-11-Na | 1.0 | - | - | TV-Si | 0.5 | - | - | 65 | 77 |
| Comparative Example 66-1 | Comparative nonaqueous electrolyte solution 66-1 | - | - | - | - | | | - | - | 100 | 100 |
| Comparative Example 66-2 | Comparative nonaqueous electrolyte solution 66-2 | 6-1 | 1.0 | - | - | | | - | - | 147 | 80 |
| Comparative Example 66-3 | Comparative nonaqueous electrolyte solution 66-3 | 6-2 | 1.0 | - | - | | | - | - | 149 | 81 |
| Comparative Example 66-4 | Comparative nonaqueous electrolyte solution 66-4 | 6-3 | 1.0 | - | - | | | - | - | 114 | 92 |

**[Table 23]**

| Table 23 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 79-1 | Nonaqueous electrolyte solution 79-1 | 1-1-11-Na | 1.0 | | | | | - | - | 74 | 70 |
| Example 79-2 | Nonaqueous electrolyte solution 79-2 | 1-3-1-Na | 1.0 | | | | | - | - | 86 | 60 |
| Example 79-3 | Nonaqueous electrolyte solution 79-3 | 2-2-2-Na | 1.0 | | | | | - | - | 82 | 62 |
| Example 79-4 | Nonaqueous electrolyte solution 79-4 | 3-3-3-Na | 1.0 | | | | | - | - | 85 | 61 |
| Example 79-5 | Nonaqueous electrolyte solution 79-5 | 4-3-1-Na | 1.0 | | | | | - | - | 83 | 62 |
| Example 79-6 | Nonaqueous electrolyte solution 79-6 | 4-1-11-Na | 1.0 | DFP-Na | 0.1 | DFBOP-Na | 1.0 | - | - | 72 | 72 |
| Comparative Example 67-1 | Comparative nonaqueous electrolyte solution 67-1 | - | - | | | | | - | - | 100 | 100 |
| Comparative Example 67-2 | Comparative nonaqueous electrolyte solution 67-2 | 6-1 | 1.0 | | | | | - | - | 140 | 83 |
| Comparative Example 67-3 | Comparative nonaqueous electrolyte solution 67-3 | 6-2 | 1.0 | | | | | - | - | 142 | 84 |
| Comparative Example 67-4 | Comparative nonaqueous electrolyte solution 67-4 | 6-3 | 1.0 | | | | | - | - | 111 | 95 |
| Example 80-1 | Nonaqueous electrolyte solution 80-1 | 1-1-11-Na | 1.0 | | | | | - | - | 72 | 71 |
| Example 80-2 | Nonaqueous electrolyte solution 80-2 | 1-3-1-Na | 1.0 | | | | | - | - | 85 | 60 |
| Example 80-3 | Nonaqueous electrolyte solution 80-3 | 2-2-2-Na | 1.0 | | | | | - | - | 81 | 61 |
| Example 80-4 | Nonaqueous electrolyte solution 80-4 | 3-3-3-Na | 1.0 | | | | | - | - | 84 | 61 |
| Example 80-5 | Nonaqueous electrolyte solution 80-5 | 4-3-1-Na | 1.0 | | | | | - | - | 83 | 62 |
| Example 80-6 | Nonaqueous electrolyte solution 80-6 | 4-1-11-Na | 1.0 | DFP-Na | 0.1 | DFOB-Na | 1.0 | - | - | 71 | 73 |
| Comparative Example 68-1 | Comparative nonaqueous electrolyte solution 68-1 | - | - | | | | | - | - | 100 | 100 |
| Comparative Example 68-2 | Comparative nonaqueous electrolyte solution 68-2 | 6-1 | 1.0 | | | | | - | - | 138 | 83 |
| Comparative Example 68-3 | Comparative nonaqueous electrolyte solution 68-3 | 6-2 | 1.0 | | | | | - | - | 141 | 84 |
| Comparative Example 68-4 | Comparative nonaqueous electrolyte solution 68-4 | 6-3 | 1.0 | | | | | - | - | 109 | 95 |
| Example 81-1 | Nonaqueous electrolyte solution 81-1 | 1-1-11-Na | 1.0 | | | | | - | - | 72 | 70 |
| Example 81-2 | Nonaqueous electrolyte solution 81-2 | 1-3-1-Na | 1.0 | | | | | - | - | 85 | 59 |
| Example 81-3 | Nonaqueous electrolyte solution 81-3 | 2-2-2-Na | 1.0 | | | | | - | - | 82 | 60 |
| Example 81-4 | Nonaqueous electrolyte solution 81-4 | 3-3-3-Na | 1.0 | | | | | - | - | 84 | 60 |
| Example 81-5 | Nonaqueous electrolyte solution 81-5 | 4-3-1-Na | 1.0 | | | | | - | - | 83 | 61 |
| Example 81-6 | Nonaqueous electrolyte solution 81-6 | 4-1-11-Na | 1.0 | DFP-Na | 0.1 | TFOP-Na | 1.0 | - | - | 70 | 72 |
| Comparative Example 69-1 | Comparative nonaqueous electrolyte solution 69-1 | - | - | | | | | - | - | 100 | 100 |
| Comparative Example 69-2 | Comparative nonaqueous electrolyte solution 69-2 | 6-1 | 1.0 | | | | | - | - | 136 | 84 |
| Comparative Example 69-3 | Comparative nonaqueous electrolyte solution 69-3 | 6-2 | 1.0 | | | | | - | - | 141 | 83 |
| Comparative Example 69-4 | Comparative nonaqueous electrolyte solution 69-4 | 6-3 | 1.0 | | | | | - | - | 108 | 96 |
| Example 82-1 | Nonaqueous electrolyte solution 82-1 | 1-1-11-Na | 1.0 | | | | | - | - | 68 | 73 |
| Example 82-2 | Nonaqueous electrolyte solution 82-2 | 1-3-1-Na | 1.0 | | | | | - | - | 80 | 63 |
| Example 82-3 | Nonaqueous electrolyte solution 82-3 | 2-2-2-Na | 1.0 | | | | | - | - | 77 | 64 |
| Example 82-4 | Nonaqueous electrolyte solution 82-4 | 3-3-3-Na | 1.0 | | | | | - | - | 79 | 63 |
| Example 82-5 | Nonaqueous electrolyte solution 82-5 | 4-3-1-Na | 1.0 | | | | | - | - | 78 | 64 |
| Example 82-6 | Nonaqueous electrolyte solution 82-6 | 4-1-11-Na | 1.0 | DFP-Na | 0.1 | DTD | 1.0 | - | - | 67 | 75 |
| Comparative Example 70-1 | Comparative nonaqueous electrolyte solution 70-1 | - | - | | | | | - | - | 100 | 100 |
| Comparative Example 70-2 | Comparative nonaqueous electrolyte solution 70-2 | 6-1 | 1.0 | | | | | - | - | 141 | 82 |
| Comparative Example 70-3 | Comparative nonaqueous electrolyte solution 70-3 | 6-2 | 1.0 | | | | | - | - | 144 | 83 |
| Comparative Example 70-4 | Comparative nonaqueous electrolyte solution 70-4 | 6-3 | 1.0 | | | | | - | - | 112 | 92 |

**[Table 24]**

| Table 24 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 83-1 | Nonaqueous electrolyte solution 83-1 | 1-1-11-Na | 1.0 | | | | | - | - | 72 | 69 |
| Example 83-2 | Nonaqueous electrolyte solution 83-2 | 1-3-1-Na | 1.0 | | | | | - | - | 84 | 59 |
| Example 83-3 | Nonaqueous electrolyte solution 83-3 | 2-2-2-Na | 1.0 | | | | | - | - | 80 | 61 |
| Example 83-4 | Nonaqueous electrolyte solution 83-4 | 3-3-3-Na | 1.0 | | | | | - | - | 83 | 60 |
| Example 83-5 | Nonaqueous electrolyte solution 83-5 | 4-3-1-Na | 1.0 | | | | | - | - | 82 | 61 |
| Example 83-6 | Nonaqueous electrolyte solution 83-6 | 4-1-11-Na | 1.0 | DFP-Na | 0.1 | DFPFSI-Na | 1.0 | - | - | 71 | 71 |
| Comparative Example 71-1 | Comparative nonaqueous electrolyte solution 71-1 | - | - | | | | | - | - | 100 | 100 |
| Comparative Example 71-2 | Comparative nonaqueous electrolyte solution 71-2 | 6-1 | 1.0 | | | | | - | - | 140 | 83 |
| Comparative Example 71-3 | Comparative nonaqueous electrolyte solution 71-3 | 6-2 | 1.0 | | | | | - | - | 144 | 83 |
| Comparative Example 71-4 | Comparative nonaqueous electrolyte solution 71-4 | 6-3 | 1.0 | | | | | - | - | 111 | 94 |
| Example 84-1 | Nonaqueous electrolyte solution 84-1 | 1-1-11-Na | 1.0 | | | | | - | - | 74 | 66 |
| Example 84-2 | Nonaqueous electrolyte solution 84-2 | 1-3-1-Na | 1.0 | | | | | - | - | 84 | 58 |
| Example 84-3 | Nonaqueous electrolyte solution 84-3 | 2-2-2-Na | 1.0 | | | | | - | - | 80 | 61 |
| Example 84-4 | Nonaqueous electrolyte solution 84-4 | 3-3-3-Na | 1.0 | | | | | - | - | 83 | 59 |
| Example 84-5 | Nonaqueous electrolyte solution 84-5 | 4-3-1-Na | 1.0 | | | | | - | - | 82 | 60 |
| Example 84-6 | Nonaqueous electrolyte solution 84-6 | 4-1-11-Na | 1.0 | DFP-Na | 0.1 | FS-Na | 1.0 | - | - | 72 | 68 |
| Comparative Example 72-1 | Comparative nonaqueous electrolyte solution 72-1 | - | - | | | | | - | - | 100 | 100 |
| Comparative Example 72-2 | Comparative nonaqueous electrolyte solution 72-2 | 6-1 | 1.0 | | | | | - | - | 144 | 81 |
| Comparative Example 72-3 | Comparative nonaqueous electrolyte solution 72-3 | 6-2 | 1.0 | | | | | - | - | 145 | 82 |
| Comparative Example 72-4 | Comparative nonaqueous electrolyte solution 72-4 | 6-3 | 1.0 | | | | | - | - | 114 | 91 |
| Example 85-1 | Nonaqueous electrolyte solution 85-1 | 1-1-11-Na | 1.0 | | | | | - | - | 65 | 75 |
| Example 85-2 | Nonaqueous electrolyte solution 85-2 | 1-3-1-Na | 1.0 | | | | | - | - | 75 | 64 |
| Example 85-3 | Nonaqueous electrolyte solution 85-3 | 2-2-2-Na | 1.0 | | | | | - | - | 72 | 66 |
| Example 85-4 | Nonaqueous electrolyte solution 85-4 | 3-3-3-Na | 1.0 | | | | | - | - | 74 | 65 |
| Example 85-5 | Nonaqueous electrolyte solution 85-5 | 4-3-1-Na | 1.0 | | | | | - | - | 73 | 66 |
| Example 85-6 | Nonaqueous electrolyte solution 85-6 | 4-1-11-Na | 1.0 | DFP-Na | 0.1 | TV-Si | 0.5 | - | - | 63 | 77 |
| Comparative Example 73-1 | Comparative nonaqueous electrolyte solution 73-1 | - | - | | | | | - | - | 100 | 100 |
| Comparative Example 73-2 | Comparative nonaqueous electrolyte solution 73-2 | 6-1 | 1.0 | | | | | - | - | 147 | 80 |
| Comparative Example 73-3 | Comparative nonaqueous electrolyte solution 73-3 | 6-2 | 1.0 | | | | | - | - | 149 | 81 |
| Comparative Example 73-4 | Comparative nonaqueous electrolyte solution 73-4 | 6-3 | 1.0 | | | | | - | - | 114 | 92 |

**[Table 25]**

| Table 25 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 86-1 | Nonaqueous electrolyte solution 86-1 | 1-1-11-Na | 1.0 | | | | | - | - | 74 | 70 |
| Example 86-2 | Nonaqueous electrolyte solution 86-2 | 1-3-1-Na | 1.0 | | | | | - | - | 86 | 60 |
| Example 86-3 | Nonaqueous electrolyte solution 86-3 | 2-2-2-Na | 1.0 | | | | | - | - | 82 | 62 |
| Example 86-4 | Nonaqueous electrolyte solution 86-4 | 3-3-3-Na | 1.0 | | | | | - | - | 85 | 61 |
| Example 86-5 | Nonaqueous electrolyte solution 86-5 | 4-3-1-Na | 1.0 | | | | | - | - | 84 | 62 |
| Example 86-6 | Nonaqueous electrolyte solution 86-6 | 4-1-11-Na | 1.0 | FSI-Na | 0.1 | DFBOP-Na | 1.0 | - | - | 72 | 72 |
| Comparative Example 74-1 | Comparative nonaqueous electrolyte solution 74-1 | - | - | | | | | - | - | 100 | 100 |
| Comparative Example 74-2 | Comparative nonaqueous electrolyte solution 74-2 | 6-1 | 1.0 | | | | | - | - | 140 | 83 |
| Comparative Example 74-3 | Comparative nonaqueous electrolyte solution 74-3 | 6-2 | 1.0 | | | | | - | - | 142 | 84 |
| Comparative Example 74-4 | Comparative nonaqueous electrolyte solution 74-4 | 6-3 | 1.0 | | | | | - | - | 111 | 95 |
| Example 87-1 | Nonaqueous electrolyte solution 87-1 | 1-1-11-Na | 1.0 | | | | | - | - | 73 | 71 |
| Example 87-2 | Nonaqueous electrolyte solution 87-2 | 1-3-1-Na | 1.0 | | | | | - | - | 85 | 59 |
| Example 87-3 | Nonaqueous electrolyte solution 87-3 | 2-2-2-Na | 1.0 | | | | | - | - | 82 | 61 |
| Example 87-4 | Nonaqueous electrolyte solution 87-4 | 3-3-3-Na | 1.0 | | | | | - | - | 84 | 60 |
| Example 87-5 | Nonaqueous electrolyte solution 87-5 | 4-3-1-Na | 1.0 | | | | | - | - | 83 | 61 |
| Example 87-6 | Nonaqueous electrolyte solution 87-6 | 4-1-11-Na | 1.0 | FSI-Na | 0.1 | DFOB-Na | 1.0 | - | - | 71 | 73 |
| Comparative Example 75-1 | Comparative nonaqueous electrolyte solution 75-1 | - | - | | | | | - | - | 100 | 100 |
| Comparative Example 75-2 | Comparative nonaqueous electrolyte solution 75-2 | 6-1 | 1.0 | | | | | - | - | 138 | 83 |
| Comparative Example 75-3 | Comparative nonaqueous electrolyte solution 75-3 | 6-2 | 1.0 | | | | | - | - | 141 | 84 |
| Comparative Example 75-4 | Comparative nonaqueous electrolyte solution 75-4 | 6-3 | 1.0 | | | | | - | - | 109 | 95 |
| Example 88-1 | Nonaqueous electrolyte solution 88-1 | 1-1-11-Na | 1.0 | | | | | - | - | 72 | 70 |
| Example 88-2 | Nonaqueous electrolyte solution 88-2 | 1-3-1-Na | 1.0 | | | | | - | - | 86 | 59 |
| Example 88-3 | Nonaqueous electrolyte solution 88-3 | 2-2-2-Na | 1.0 | | | | | - | - | 83 | 60 |
| Example 88-4 | Nonaqueous electrolyte solution 88-4 | 3-3-3-Na | 1.0 | | | | | - | - | 85 | 60 |
| Example 88-5 | Nonaqueous electrolyte solution 88-5 | 4-3-1-Na | 1.0 | | | | | - | - | 84 | 61 |
| Example 88-6 | Nonaqueous electrolyte solution 88-6 | 4-1-11-Na | 1.0 | | | | | - | - | 71 | 72 |
| Comparative Example 76-1 | Comparative nonaqueous electrolyte solution 76-1 | - | - | FSI-Na | 0.1 | TFOP-Na | 1.0 | - | - | 100 | 100 |
| Comparative Example 76-2 | Comparative nonaqueous electrolyte solution 76-2 | 6-1 | 1.0 | | | | | - | - | 136 | 84 |
| Comparative Example 76-3 | Comparative nonaqueous electrolyte solution 76-3 | 6-2 | 1.0 | | | | | - | - | 142 | 83 |
| Comparative Example 76-4 | Comparative nonaqueous electrolyte solution 76-4 | 6-3 | 1.0 | | | | | - | - | 108 | 96 |
| Example 89-1 | Nonaqueous electrolyte solution 89-1 | 1-1-11-Na | 1.0 | | | | | - | - | 69 | 72 |
| Example 89-2 | Nonaqueous electrolyte solution 89-2 | 1-3-1-Na | 1.0 | | | | | - | - | 80 | 62 |
| Example 89-3 | Nonaqueous electrolyte solution 89-3 | 2-2-2-Na | 1.0 | | | | | - | - | 77 | 63 |
| Example 89-4 | Nonaqueous electrolyte solution 89-4 | 3-3-3-Na | 1.0 | | | | | - | - | 79 | 63 |
| Example 89-5 | Nonaqueous electrolyte solution 89-5 | 4-3-1-Na | 1.0 | | | | | - | - | 78 | 64 |
| Example 89-6 | Nonaqueous electrolyte solution 89-6 | 4-1-11-Na | 1.0 | FSI-Na | 0.1 | DTD | 1.0 | - | - | 67 | 74 |
| Comparative Example 77-1 | Comparative nonaqueous electrolyte solution 77-1 | - | - | | | | | - | - | 100 | 100 |
| Comparative Example 77-2 | Comparative nonaqueous electrolyte solution 77-2 | 6-1 | 1.0 | | | | | - | - | 142 | 82 |
| Comparative Example 77-3 | Comparative nonaqueous electrolyte solution 77-3 | 6-2 | 1.0 | | | | | - | - | 144 | 83 |
| Comparative Example 77-4 | Comparative nonaqueous electrolyte solution 77-4 | 6-3 | 1.0 | | | | | - | - | 112 | 92 |

**[Table 26]**

| Table 26 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 90-1 | Nonaqueous electrolyte solution 90-1 | 1-1-11-Na | 1.0 | | | | | - | - | 73 | 68 |
| Example 90-2 | Nonaqueous electrolyte solution 90-2 | 1-3-1-Na | 1.0 | | | | | - | - | 85 | 59 |
| Example 90-3 | Nonaqueous electrolyte solution 90-3 | 2-2-2-Na | 1.0 | | | | | - | - | 81 | 61 |
| Example 90-4 | Nonaqueous electrolyte solution 90-4 | 3-3-3-Na | 1.0 | | | | | - | - | 84 | 60 |
| Example 90-5 | Nonaqueous electrolyte solution 90-5 | 4-3-1-Na | 1.0 | | | | | - | - | 82 | 61 |
| Example 90-6 | Nonaqueous electrolyte solution 90-6 | 4-1-11-Na | 1.0 | FSI-Na | 0.1 | DFPFSI-Na | 1.0 | - | - | 71 | 70 |
| Comparative Example 78-1 | Comparative nonaqueous electrolyte solution 78-1 | - | - | | | | | - | - | 100 | 100 |
| Comparative Example 78-2 | Comparative nonaqueous electrolyte solution 78-2 | 6-1 | 1.0 | | | | | - | - | 141 | 83 |
| Comparative Example 78-3 | Comparative nonaqueous electrolyte solution 78-3 | 6-2 | 1.0 | | | | | - | - | 144 | 83 |
| Comparative Example 78-4 | Comparative nonaqueous electrolyte solution 78-4 | 6-3 | 1.0 | | | | | - | - | 111 | 94 |
| Example 91-1 | Nonaqueous electrolyte solution 91-1 | 1-1-11-Na | 1.0 | | | | | - | - | 74 | 66 |
| Example 91-2 | Nonaqueous electrolyte solution 91-2 | 1-3-1-Na | 1.0 | | | | | - | - | 85 | 58 |
| Example 91-3 | Nonaqueous electrolyte solution 91-3 | 2-2-2-Na | 1.0 | | | | | - | - | 80 | 60 |
| Example 91-4 | Nonaqueous electrolyte solution 91-4 | 3-3-3-Na | 1.0 | | | | | - | - | 83 | 59 |
| Example 91-5 | Nonaqueous electrolyte solution 91-5 | 4-3-1-Na | 1.0 | FSI-Na | 0.1 | FS | 1.0 | - | - | 82 | 60 |
| Example 91-6 | Nonaqueous electrolyte solution 91-6 | 4-1-11-Na | 1.0 | | | | | - | - | 73 | 68 |
| Comparative Example 79-1 | Comparative nonaqueous electrolyte solution 79-1 | - | - | | | | | - | - | 100 | 100 |
| Comparative Example 79-2 | Comparative nonaqueous electrolyte solution 79-2 | 6-1 | 1.0 | | | | | - | - | 144 | 81 |
| Comparative Example 79-3 | Comparative nonaqueous electrolyte solution 79-3 | 6-2 | 1.0 | | | | | - | - | 145 | 82 |
| Comparative Example 79-4 | Comparative nonaqueous electrolyte solution 79-4 | 6-3 | 1.0 | | | | | - | - | 114 | 91 |
| Example 92-1 | Nonaqueous electrolyte solution 92-1 | 1-1-11-Na | 1.0 | | | | | - | - | 65 | 75 |
| Example 92-2 | Nonaqueous electrolyte solution 92-2 | 1-3-1-Na | 1.0 | | | | | - | - | 75 | 63 |
| Example 92-3 | Nonaqueous electrolyte solution 92-3 | 2-2-2-Na | 1.0 | | | | | - | - | 72 | 65 |
| Example 92-4 | Nonaqueous electrolyte solution 92-4 | 3-3-3-Na | 1.0 | | | | | - | - | 74 | 64 |
| Example 92-5 | Nonaqueous electrolyte solution 92-5 | 4-3-1-Na | 1.0 | | | | | - | - | 73 | 65 |
| Example 92-6 | Nonaqueous electrolyte solution 92-6 | 4-1-11-Na | 1.0 | | | | | - | - | 64 | 77 |
| Comparative Example 80-1 | Comparative nonaqueous electrolyte solution 80-1 | - | - | FSI-Na | 0.1 | TV-Si | 0.5 | - | - | 100 | 100 |
| Comparative Example 80-2 | Comparative nonaqueous electrolyte solution 80-2 | 6-1 | 1.0 | | | | | - | - | 147 | 80 |
| Comparative Example 80-3 | Comparative nonaqueous electrolyte solution 80-3 | 6-2 | 1.0 | | | | | - | - | 149 | 80 |
| Comparative Example 80-4 | Comparative nonaqueous electrolyte solution 80-4 | 6-3 | 1.0 | | | | | - | - | 114 | 92 |

**[Table 27]**

| Table 27 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 93-1 | Nonaqueous electrolyte solution 93-1 | 1-1-11-Na | 1.0 | | | | | - | - | 74 | 70 |
| Example 93-2 | Nonaqueous electrolyte solution 93-2 | 1-3-1-Na | 1.0 | | | | | - | - | 86 | 60 |
| Example 93-3 | Nonaqueous electrolyte solution 93-3 | 2-2-2-Na | 1.0 | | | | | - | - | 82 | 62 |
| Example 93-4 | Nonaqueous electrolyte solution 93-4 | 3-3-3-Na | 1.0 | | | | | - | - | 85 | 61 |
| Example 93-5 | Nonaqueous electrolyte solution 93-5 | 4-3-1-Na | 1.0 | BF₄-Na | 0.025 | DFBOP-Na | 1.0 | - | - | 84 | 62 |
| Example 93-6 | Nonaqueous electrolyte solution 93-6 | 4-1-11-Na | 1.0 | | | | | - | - | 72 | 72 |
| Comparative Example 81-1 | Comparative nonaqueous electrolyte solution 81-1 | - | - | | | | | - | - | 100 | 100 |
| Comparative Example 81-2 | Comparative nonaqueous electrolyte solution 81-2 | 6-1 | 1.0 | | | | | - | - | 140 | 83 |
| Comparative Example 81-3 | Comparative nonaqueous electrolyte solution 81-3 | 6-2 | 1.0 | | | | | - | - | 142 | 84 |
| Comparative Example 81-4 | Comparative nonaqueous electrolyte solution 81-4 | 6-3 | 1.0 | | | | | - | - | 111 | 95 |
| Example 94-1 | Nonaqueous electrolyte solution 94-1 | 1-1-11-Na | 1.0 | | | | | - | - | 72 | 71 |
| Example 94-2 | Nonaqueous electrolyte solution 94-2 | 1-3-1-Na | 1.0 | | | | | - | - | 85 | 59 |
| Example 94-3 | Nonaqueous electrolyte solution 94-3 | 2-2-2-Na | 1.0 | | | | | - | - | 82 | 61 |
| Example 94-4 | Nonaqueous electrolyte solution 94-4 | 3-3-3-Na | 1.0 | | | | | - | - | 84 | 60 |
| Example 94-5 | Nonaqueous electrolyte solution 94-5 | 4-3-1-Na | 1.0 | | | | | - | - | 83 | 61 |
| Example 94-6 | Nonaqueous electrolyte solution 94-6 | 4-1-11-Na | 1.0 | BF₄-Na | 0.025 | DFOB-Na | 1.0 | - | - | 71 | 73 |
| Comparative Example 82-1 | Comparative nonaqueous electrolyte solution 82-1 | - | - | | | | | - | - | 100 | 100 |
| Comparative Example 82-2 | Comparative nonaqueous electrolyte solution 82-2 | 6-1 | 1.0 | | | | | - | - | 138 | 84 |
| Comparative Example 82-3 | Comparative nonaqueous electrolyte solution 82-3 | 6-2 | 1.0 | | | | | - | - | 141 | 84 |
| Comparative Example 82-4 | Comparative nonaqueous electrolyte solution 82-4 | 6-3 | 1.0 | | | | | - | - | 109 | 95 |
| Example 95-1 | Nonaqueous electrolyte solution 95-1 | 1-1-11-Na | 1.0 | | | | | - | - | 72 | 70 |
| Example 95-2 | Nonaqueous electrolyte solution 95-2 | 1-3-1-Na | 1.0 | | | | | - | - | 86 | 59 |
| Example 95-3 | Nonaqueous electrolyte solution 95-3 | 2-2-2-Na | 1.0 | | | | | - | - | 83 | 60 |
| Example 95-4 | Nonaqueous electrolyte solution 95-4 | 3-3-3-Na | 1.0 | | | | | - | - | 84 | 60 |
| Example 95-5 | Nonaqueous electrolyte solution 95-5 | 4-3-1-Na | 1.0 | | | | | - | - | 83 | 61 |
| Example 95-6 | Nonaqueous electrolyte solution 95-6 | 4-1-11-Na | 1.0 | | | | | - | - | 71 | 72 |
| Comparative Example 83-1 | Comparative nonaqueous electrolyte solution 83-1 | - | - | BF₄-Na | 0.025 | TFOP-Na | 1.0 | - | - | 100 | 100 |
| Comparative Example 83-2 | Comparative nonaqueous electrolyte solution 83-2 | 6-1 | 1.0 | | | | | - | - | 136 | 84 |
| Comparative Example 83-3 | Comparative nonaqueous electrolyte solution 83-3 | 6-2 | 1.0 | | | | | - | - | 141 | 83 |
| Comparative Example 83-4 | Comparative nonaqueous electrolyte solution 83-4 | 6-3 | 1.0 | | | | | - | - | 108 | 96 |
| Example 96-1 | Nonaqueous electrolyte solution 96-1 | 1-1-11-Na | 1.0 | | | | | - | - | 68 | 72 |
| Example 96-2 | Nonaqueous electrolyte solution 96-2 | 1-3-1-Na | 1.0 | | | | | - | - | 80 | 62 |
| Example 96-3 | Nonaqueous electrolyte solution 96-3 | 2-2-2-Na | 1.0 | | | | | - | - | 77 | 63 |
| Example 96-4 | Nonaqueous electrolyte solution 96-4 | 3-3-3-Na | 1.0 | | | | | - | - | 79 | 63 |
| Example 96-5 | Nonaqueous electrolyte solution 96-5 | 4-3-1-Na | 1.0 | | | | | - | - | 78 | 64 |
| Example 96-6 | Nonaqueous electrolyte solution 96-6 | 4-1-11-Na | 1.0 | BF₄-Na | 0.025 | DTD | 1.0 | - | - | 67 | 74 |
| Comparative Example 84-1 | Comparative nonaqueous electrolyte solution 84-1 | - | - | | | | | - | - | 100 | 100 |
| Comparative Example 84-2 | Comparative nonaqueous electrolyte solution 84-2 | 6-1 | 1.0 | | | | | - | - | 141 | 82 |
| Comparative Example 84-3 | Comparative nonaqueous electrolyte solution 84-3 | 6-2 | 1.0 | | | | | - | - | 144 | 83 |
| Comparative Example 84-4 | Comparative nonaqueous electrolyte solution 84-4 | 6-3 | 1.0 | | | | | - | - | 112 | 92 |

**[Table 28]**

| Table 28 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 97-1 | Nonaqueous electrolyte solution 97-1 | 1-1-11-Na | 1.0 | | | | | - | - | 73 | 68 |
| Example 97-2 | Nonaqueous electrolyte solution 97-2 | 1-3-1-Na | 1.0 | | | | | - | - | 84 | 59 |
| Example 97-3 | Nonaqueous electrolyte solution 97-3 | 2-2-2-Na | 1.0 | | | | | - | - | 80 | 61 |
| Example 97-4 | Nonaqueous electrolyte solution 97-4 | 3-3-3-Na | 1.0 | | | | | - | - | 83 | 60 |
| Example 97-5 | Nonaqueous electrolyte solution 97-5 | 4-3-1-Na | 1.0 | | | | | - | - | 82 | 61 |
| Example 97-6 | Nonaqueous electrolyte solution 97-6 | 4-1-11-Na | 1.0 | BF₄-Na | 0.025 | DFPFSI-Na | 1.0 | - | - | 71 | 70 |
| Comparative Example 85-1 | Comparative nonaqueous electrolyte solution 85-1 | - | - | | | | | - | - | 100 | 100 |
| Comparative Example 85-2 | Comparative nonaqueous electrolyte solution 85-2 | 6-1 | 1.0 | | | | | - | - | 140 | 83 |
| Comparative Example 85-3 | Comparative nonaqueous electrolyte solution 85-3 | 6-2 | 1.0 | | | | | - | - | 152 | 78 |
| Comparative Example 85-4 | Comparative nonaqueous electrolyte solution 85-4 | 6-3 | 1.0 | | | | | - | - | 111 | 94 |
| Example 98-1 | Nonaqueous electrolyte solution 98-1 | 1-1-11-Na | 1.0 | | | | | - | - | 74 | 66 |
| Example 98-2 | Nonaqueous electrolyte solution 98-2 | 1-3-1-Na | 1.0 | | | | | - | - | 84 | 58 |
| Example 98-3 | Nonaqueous electrolyte solution 98-3 | 2-2-2-Na | 1.0 | | | | | - | - | 80 | 60 |
| Example 98-4 | Nonaqueous electrolyte solution 98-4 | 3-3-3-Na | 1.0 | | | | | - | - | 83 | 59 |
| Example 98-5 | Nonaqueous electrolyte solution 98-5 | 4-3-1-Na | 1.0 | | | | | - | - | 82 | 60 |
| Example 98-6 | Nonaqueous electrolyte solution 98-6 | 4-1-11-Na | 1.0 | | | | | - | - | 73 | 68 |
| Comparative Example 86-1 | Comparative nonaqueous electrolyte solution 86-1 | - | - | BF₄-Na | 0.025 | FS-Na | 1.0 | - | - | 100 | 100 |
| Comparative Example 86-2 | Comparative nonaqueous electrolyte solution 86-2 | 6-1 | 1.0 | | | | | - | - | 144 | 81 |
| Comparative Example 86-3 | Comparative nonaqueous electrolyte solution 86-3 | 6-2 | 1.0 | | | | | - | - | 145 | 82 |
| Comparative Example 86-4 | Comparative nonaqueous electrolyte solution 86-4 | 6-3 | 1.0 | | | | | - | - | 114 | 91 |
| Example 99-1 | Nonaqueous electrolyte solution 99-1 | 1-1-11-Na | 1.0 | | | | | - | - | 65 | 75 |
| Example 99-2 | Nonaqueous electrolyte solution 99-2 | 1-3-1-Na | 1.0 | | | | | - | - | 75 | 63 |
| Example 99-3 | Nonaqueous electrolyte solution 99-3 | 2-2-2-Na | 1.0 | | | | | - | - | 72 | 65 |
| Example 99-4 | Nonaqueous electrolyte solution 99-4 | 3-3-3-Na | 1.0 | | | | | - | - | 74 | 64 |
| Example 99-5 | Nonaqueous electrolyte solution 99-5 | 4-3-1-Na | 1.0 | | | | | - | - | 73 | 65 |
| Example 99-6 | Nonaqueous electrolyte solution 99-6 | 4-1-11-Na | 1.0 | BF₄-Na | 0.025 | TV-Si | 0.5 | - | - | 64 | 77 |
| Comparative Example 87-1 | Comparative nonaqueous electrolyte solution 87-1 | - | - | | | | | - | - | 100 | 100 |
| Comparative Example 87-2 | Comparative nonaqueous electrolyte solution 87-2 | 6-1 | 1.0 | | | | | - | - | 147 | 80 |
| Comparative Example 87-3 | Comparative nonaqueous electrolyte solution 87-3 | 6-2 | 1.0 | | | | | - | - | 148 | 81 |
| Comparative Example 87-4 | Comparative nonaqueous electrolyte solution 87-4 | 6-3 | 1.0 | | | | | - | - | 114 | 92 |

**[Table 29]**

| Table 29 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 71 | 71 |
| Example 100-1Na | Nonaqueous electrolyte solution 100-1Na | | | Na-BOB | 0.005 | 5 | 70 | 70 |
| Example 100-2Na | Nonaqueous electrolyte solution 100-2Na | | | | 0.05 | 56 | 68 | 68 |
| Example 100-3Na | Nonaqueous electrolyte solution 100-3Na | | | | 0.1 | 111 | 71 | 66 |
| Example 100-4Na | Nonaqueous electrolyte solution 100-4Na | | | | 0.3 | 330 | 75 | 62 |
| Example 100-5Na | Nonaqueous electrolyte solution 100-5Na | | | | 0.5 | 550 | 78 | 58 |
| Example 1-10 | Nonaqueous electrolyte solution 1-10 | 1-3-1 | 1.0 | - | - | <1 | 93 | 58 |
| Example 101-1Na | Nonaqueous electrolyte solution 101-1Na | | | Na-BOB | 0.005 | 5 | 90 | 58 |
| Example 101-2Na | Nonaqueous electrolyte solution 101-2Na | | | | 0.05 | 54 | 91 | 55 |
| Example 101-3Na | Nonaqueous electrolyte solution 101-3Na | | | | 0.1 | 108 | 93 | 54 |
| Example 101-4Na | Nonaqueous electrolyte solution 101-4Na | | | | 0.3 | 328 | 96 | 50 |
| Example 101-5Na | Nonaqueous electrolyte solution 101-5Na | | | | 0.5 | 547 | 98 | 47 |
| Example 2-4 | Nonaqueous electrolyte solution 2-4 | 2-2-2 | 1.0 | - | - | <1 | 88 | 60 |
| Example 102-1Na | Nonaqueous electrolyte solution 102-1Na | | | Na-BOB | 0.005 | 5 | 85 | 60 |
| Example 102-2Na | Nonaqueous electrolyte solution 102-2Na | | | | 0.05 | 55 | 86 | 57 |
| Example 102-3Na | Nonaqueous electrolyte solution 102-3Na | | | | 0.1 | 111 | 87 | 56 |
| Example 102-4Na | Nonaqueous electrolyte solution 102-4Na | | | | 0.3 | 329 | 90 | 52 |
| Example 102-5Na | Nonaqueous electrolyte solution 102-5Na | | | | 0.5 | 549 | 95 | 49 |
| Example 3-4 | Nonaqueous electrolyte solution 3-4 | 3-3-3 | 1.0 | - | - | <1 | 92 | 59 |
| Example 103-1Na | Nonaqueous electrolyte solution 103-1Na | | | Na-BOB | 0.005 | 5 | 89 | 59 |
| Example 103-2Na | Nonaqueous electrolyte solution 103-2Na | | | | 0.05 | 55 | 90 | 56 |
| Example 103-3Na | Nonaqueous electrolyte solution 103-3Na | | | | 0.1 | 113 | 91 | 55 |
| Example 103-4Na | Nonaqueous electrolyte solution 103-4Na | | | | 0.3 | 330 | 94 | 51 |
| Example 103-5Na | Nonaqueous electrolyte solution 103-5Na | | | | 0.5 | 551 | 99 | 48 |
| Example 4-4 | Nonaqueous electrolyte solution 4-4 | 4-3-1 | 1.0 | - | - | <1 | 91 | 60 |
| Example 104-1Na | Nonaqueous electrolyte solution 104-1Na | | | Na-BOB | 0.005 | 5 | 88 | 60 |
| Example 104-2Na | Nonaqueous electrolyte solution 104-2Na | | | | 0.05 | 55 | 88 | 58 |
| Example 104-3Na | Nonaqueous electrolyte solution 104-3Na | | | | 0.1 | 109 | 90 | 56 |
| Example 104-4Na | Nonaqueous electrolyte solution 104-4Na | | | | 0.3 | 328 | 93 | 52 |
| Example 104-5Na | Nonaqueous electrolyte solution 104-5Na | | | | 0.5 | 549 | 98 | 49 |
| Example 4-10 | Nonaqueous electrolyte solution 4-10 | 4-1-11 | 1.0 | - | - | <1 | 77 | 73 |
| Example 105-1Na | Nonaqueous electrolyte solution 105-1Na | | | Na-BOB | 0.005 | 5 | 75 | 73 |
| Example 105-2Na | Nonaqueous electrolyte solution 105-2Na | | | | 0.05 | 53 | 75 | 71 |
| Example 105-3Na | Nonaqueous electrolyte solution 105-3Na | | | | 0.1 | 108 | 77 | 68 |
| Example 105-4Na | Nonaqueous electrolyte solution 105-4Na | | | | 0.3 | 331 | 79 | 64 |
| Example 105-5Na | Nonaqueous electrolyte solution 105-5Na | | | | 0.5 | 548 | 83 | 60 |
| Comparative Example 1-1 | Comparative nonaqueous electrolyte solution 1-1 | - | - | - | - | <1 | 100 | 100 |

**[Table 30]**

| Table 30 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 71 | 71 |
| Example 100-1K | Nonaqueous electrolyte solution 100-1K | | | K-BOB | 0.005 | 10 | 71 | 70 |
| Example 100-2K | Nonaqueous electrolyte solution 100-2K | | | | 0.05 | 85 | 69 | 69 |
| Example 100-3K | Nonaqueous electrolyte solution 100-3K | | | | 0.1 | 175 | 72 | 67 |
| Example 100-4K | Nonaqueous electrolyte solution 100-4K | | | | 0.3 | 520 | 76 | 63 |
| Example 100-5K | Nonaqueous electrolyte solution 100-5K | | | | 0.5 | 865 | 79 | 59 |
| Example 1-10 | Nonaqueous electrolyte solution 1-10 | 1-3-1 | 1.0 | - | - | <1 | 93 | 58 |
| Example 101-1K | Nonaqueous electrolyte solution 101-1K | | | K-BOB | 0.005 | 9 | 91 | 59 |
| Example 101-2K | Nonaqueous electrolyte solution 101-2K | | | | 0.05 | 88 | 92 | 56 |
| Example 101-3K | Nonaqueous electrolyte solution 101-3K | | | | 0.1 | 174 | 94 | 54 |
| Example 101-4K | Nonaqueous electrolyte solution 101-4K | | | | 0.3 | 521 | 97 | 51 |
| Example 101-5K | Nonaqueous electrolyte solution 101-5K | | | | 0.5 | 864 | 99 | 48 |
| Example 2-4 | Nonaqueous electrolyte solution 2-4 | 2-2-2 | 1.0 | - | - | <1 | 88 | 60 |
| Example 102-1K | Nonaqueous electrolyte solution 102-1K | | | K-BOB | 0.005 | 8 | 86 | 60 |
| Example 102-2K | Nonaqueous electrolyte solution 102-2K | | | | 0.05 | 85 | 87 | 58 |
| Example 102-3K | Nonaqueous electrolyte solution 102-3K | | | | 0.1 | 173 | 89 | 57 |
| Example 102-4K | Nonaqueous electrolyte solution 102-4K | | | | 0.3 | 518 | 91 | 53 |
| Example 102-5K | Nonaqueous electrolyte solution 102-5K | | | | 0.5 | 866 | 96 | 50 |
| Example 3-4 | Nonaqueous electrolyte solution 3-4 | 3-3-3 | 1.0 | - | - | <1 | 92 | 59 |
| Example 103-1K | Nonaqueous electrolyte solution 103-1K | | | K-BOB | 0.005 | 9 | 90 | 59 |
| Example 103-2K | Nonaqueous electrolyte solution 103-2K | | | | 0.05 | 88 | 91 | 57 |
| Example 103-3K | Nonaqueous electrolyte solution 103-3K | | | | 0.1 | 174 | 93 | 56 |
| Example 103-4K | Nonaqueous electrolyte solution 103-4K | | | | 0.3 | 520 | 95 | 52 |
| Example 103-5K | Nonaqueous electrolyte solution 103-5K | | | | 0.5 | 863 | 100 | 49 |
| Example 4-4 | Nonaqueous electrolyte solution 4-4 | 4-3-1 | 1.0 | - | - | <1 | 91 | 60 |
| Example 104-1K | Nonaqueous electrolyte solution 104-1K | | | K-BOB | 0.005 | 10 | 89 | 60 |
| Example 104-2K | Nonaqueous electrolyte solution 104-2K | | | | 0.05 | 88 | 89 | 58 |
| Example 104-3K | Nonaqueous electrolyte solution 104-3K | | | | 0.1 | 175 | 91 | 57 |
| Example 104-4K | Nonaqueous electrolyte solution 104-4K | | | | 0.3 | 521 | 94 | 53 |
| Example 104-5K | Nonaqueous electrolyte solution 104-5K | | | | 0.5 | 868 | 99 | 50 |
| Example 4-10 | Nonaqueous electrolyte solution 4-10 | 4-1-11 | 1.0 | - | - | <1 | 77 | 73 |
| Example 105-1K | Nonaqueous electrolyte solution 105-1K | | | K-BOB | 0.005 | 11 | 76 | 73 |
| Example 105-2K | Nonaqueous electrolyte solution 105-2K | | | | 0.05 | 89 | 76 | 71 |
| Example 105-3K | Nonaqueous electrolyte solution 105-3K | | | | 0.1 | 173 | 78 | 70 |
| Example 105-4K | Nonaqueous electrolyte solution 105-4K | | | | 0.3 | 517 | 80 | 65 |
| Example 105-5K | Nonaqueous electrolyte solution 105-5K | | | | 0.5 | 866 | 84 | 62 |
| Comparative Example 1-1 | Comparative nonaqueous electrolyte solution 1-1 | - | - | - | - | <1 | 100 | 100 |

**[Table 31]**

| Table 31 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 79 | 71 |
| Example 106-1Na | Nonaqueous electrolyte solution 106-1Na | | | Na-BF4 | 0.005 | 11 | 79 | 70 |
| Example 106-2Na | Nonaqueous electrolyte solution 106-2Na | | | | 0.05 | 104 | 78 | 68 |
| Example 106-3Na | Nonaqueous electrolyte solution 106-3Na | | | | 0.1 | 210 | 76 | 68 |
| Example 106-4Na | Nonaqueous electrolyte solution 106-4Na | | | | 0.3 | 629 | 72 | 71 |
| Example 106-5Na | Nonaqueous electrolyte solution 106-5Na | | | | 0.5 | 1052 | 70 | 73 |
| Example 1-10 | Nonaqueous electrolyte solution 1-10 | 1-3-1 | 1.0 | - | - | <1 | 93 | 58 |
| Example 107-1Na | Nonaqueous electrolyte solution 107-1Na | | | Na-BF4 | 0.005 | 10 | 93 | 57 |
| Example 107-2Na | Nonaqueous electrolyte solution 107-2Na | | | | 0.05 | 103 | 92 | 55 |
| Example 107-3Na | Nonaqueous electrolyte solution 107-3Na | | | | 0.1 | 211 | 89 | 56 |
| Example 107-4Na | Nonaqueous electrolyte solution 107-4Na | | | | 0.3 | 628 | 85 | 58 |
| Example 107-5Na | Nonaqueous electrolyte solution 107-5Na | | | | 0.5 | 1050 | 82 | 59 |
| Example 2-4 | Nonaqueous electrolyte solution 2-4 | 2-2-2 | 1.0 | - | - | <1 | 88 | 60 |
| Example 108-1Na | Nonaqueous electrolyte solution 108-1Na | | | Na-BF4 | 0.005 | 9 | 87 | 59 |
| Example 108-2Na | Nonaqueous electrolyte solution 108-2Na | | | | 0.05 | 102 | 87 | 57 |
| Example 108-3Na | Nonaqueous electrolyte solution 108-3Na | | | | 0.1 | 208 | 84 | 58 |
| Example 108-4Na | Nonaqueous electrolyte solution 108-4Na | | | | 0.3 | 627 | 80 | 60 |
| Example 108-5Na | Nonaqueous electrolyte solution 108-5Na | | | | 0.5 | 1045 | 78 | 61 |
| Example 3-4 | Nonaqueous electrolyte solution 3-4 | 3-3-3 | 1.0 | - | - | <1 | 92 | 59 |
| Example 109-1Na | Nonaqueous electrolyte solution 109-1Na | | | Na-BF4 | 0.005 | 10 | 91 | 58 |
| Example 109-2Na | Nonaqueous electrolyte solution 109-2Na | | | | 0.05 | 104 | 90 | 57 |
| Example 109-3Na | Nonaqueous electrolyte solution 109-3Na | | | | 0.1 | 210 | 88 | 57 |
| Example 109-4Na | Nonaqueous electrolyte solution 109-4Na | | | | 0.3 | 629 | 83 | 59 |
| Example 109-5Na | Nonaqueous electrolyte solution 109-5Na | | | | 0.5 | 1048 | 81 | 60 |
| Example 4-4 | Nonaqueous electrolyte solution 4-4 | 4-3-1 | 1.0 | - | - | <1 | 91 | 60 |
| Example 110-1Na | Nonaqueous electrolyte solution 110-1Na | | | Na-BF4 | 0.005 | 11 | 90 | 60 |
| Example 110-2Na | Nonaqueous electrolyte solution 110-2Na | | | | 0.05 | 107 | 89 | 58 |
| Example 110-3Na | Nonaqueous electrolyte solution 110-3Na | | | | 0.1 | 209 | 87 | 58 |
| Example 110-4Na | Nonaqueous electrolyte solution 110-4Na | | | | 0.3 | 630 | 82 | 60 |
| Example 110-5Na | Nonaqueous electrolyte solution 110-5Na | | | | 0.5 | 1049 | 80 | 61 |
| Example 4-10 | Nonaqueous electrolyte solution 4-10 | 4-1-11 | 1.0 | - | - | <1 | 77 | 73 |
| Example 111-1Na | Nonaqueous electrolyte solution 111-1Na | | | Na-BF4 | 0.005 | 10 | 76 | 72 |
| Example 111-2Na | Nonaqueous electrolyte solution 111-2Na | | | | 0.05 | 105 | 76 | 71 |
| Example 111-3Na | Nonaqueous electrolyte solution 111-3Na | | | | 0.1 | 210 | 74 | 71 |
| Example 111-4Na | Nonaqueous electrolyte solution 111-4Na | | | | 0.3 | 628 | 70 | 74 |
| Example 111-5Na | Nonaqueous electrolyte solution 111-5Na | | | | 0.5 | 1050 | 68 | 75 |
| Comparative Example 1-1 | Comparative nonaqueous electrolyte solution 1-1 | - | - | - | - | <1 | 100 | 100 |

**[Table 32]**

| Table 32 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 79 | 71 |
| Example 106-1K | Nonaqueous electrolyte solution 106-1K | | | K-BF4 | 0.005 | 16 | 79 | 71 |
| Example 106-2K | Nonaqueous electrolyte solution 106-2K | | | | 0.05 | 156 | 79 | 69 |
| Example 106-3K | Nonaqueous electrolyte solution 106-3K | | | | 0.1 | 321 | 77 | 69 |
| Example 106-4K | Nonaqueous electrolyte solution 106-4K | | | | 0.3 | 934 | 73 | 72 |
| Example 106-5K | Nonaqueous electrolyte solution 106-5K | | | | 0.5 | 1555 | 71 | 74 |
| Example 1-10 | Nonaqueous electrolyte solution 1-10 | 1-3-1 | 1.0 | - | - | <1 | 93 | 58 |
| Example 107-1K | Nonaqueous electrolyte solution 107-1K | | | K-BF4 | 0.005 | 17 | 93 | 58 |
| Example 107-2K | Nonaqueous electrolyte solution 107-2K | | | | 0.05 | 156 | 93 | 56 |
| Example 107-3K | Nonaqueous electrolyte solution 107-3K | | | | 0.1 | 313 | 91 | 57 |
| Example 107-4K | Nonaqueous electrolyte solution 107-4K | | | | 0.3 | 935 | 86 | 59 |
| Example 107-5K | Nonaqueous electrolyte solution 107-5K | | | | 0.5 | 1556 | 83 | 60 |
| Example 2-4 | Nonaqueous electrolyte solution 2-4 | 2-2-2 | 1.0 | - | - | <1 | 88 | 60 |
| Example 108-1K | Nonaqueous electrolyte solution 108-1K | | | K-BF4 | 0.005 | 14 | 88 | 59 |
| Example 108-2K | Nonaqueous electrolyte solution 108-2K | | | | 0.05 | 153 | 87 | 58 |
| Example 108-3K | Nonaqueous electrolyte solution 108-3K | | | | 0.1 | 309 | 86 | 59 |
| Example 108-4K | Nonaqueous electrolyte solution 108-4K | | | | 0.3 | 930 | 81 | 61 |
| Example 108-5K | Nonaqueous electrolyte solution 108-5K | | | | 0.5 | 1551 | 78 | 62 |
| Example 3-4 | Nonaqueous electrolyte solution 3-4 | 3-3-3 | 1.0 | - | - | <1 | 92 | 59 |
| Example 109-1K | Nonaqueous electrolyte solution 109-1K | | | K-BF4 | 0.005 | 15 | 92 | 58 |
| Example 109-2K | Nonaqueous electrolyte solution 109-2K | | | | 0.05 | 155 | 91 | 57 |
| Example 109-3K | Nonaqueous electrolyte solution 109-3K | | | | 0.1 | 312 | 89 | 58 |
| Example 109-4K | Nonaqueous electrolyte solution 109-4K | | | | 0.3 | 932 | 84 | 60 |
| Example 109-5K | Nonaqueous electrolyte solution 109-5K | | | | 0.5 | 1552 | 82 | 61 |
| Example 4-4 | Nonaqueous electrolyte solution 4-4 | 4-3-1 | 1.0 | - | - | <1 | 91 | 60 |
| Example 110-1K | Nonaqueous electrolyte solution 110-1K | | | K-BF4 | 0.005 | 16 | 91 | 60 |
| Example 110-2K | Nonaqueous electrolyte solution 110-2K | | | | 0.05 | 154 | 90 | 58 |
| Example 110-3K | Nonaqueous electrolyte solution 110-3K | | | | 0.1 | 311 | 88 | 59 |
| Example 110-4K | Nonaqueous electrolyte solution 110-4K | | | | 0.3 | 933 | 83 | 61 |
| Example 110-5K | Nonaqueous electrolyte solution 110-5K | | | | 0.5 | 1553 | 81 | 62 |
| Example 4-10 | Nonaqueous electrolyte solution 4-10 | 4-1-11 | 1.0 | - | - | <1 | 77 | 73 |
| Example 111-1K | Nonaqueous electrolyte solution 111-1K | | | K-BF4 | 0.005 | 15 | 77 | 72 |
| Example 111-2K | Nonaqueous electrolyte solution 111-2K | | | | 0.05 | 153 | 77 | 71 |
| Example 111-3K | Nonaqueous electrolyte solution 111-3K | | | | 0.1 | 309 | 75 | 73 |
| Example 111-4K | Nonaqueous electrolyte solution 111-4K | | | | 0.3 | 930 | 71 | 75 |
| Example 111-5K | Nonaqueous electrolyte solution 111-5K | | | | 0.5 | 1550 | 69 | 77 |
| Comparative Example 1-1 | Comparative nonaqueous electrolyte solution 1-1 | - | - | - | - | <1 | 100 | 100 |

**[Table 33]**

| Table 33 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 79 | 71 |
| Example 112-1Na | Nonaqueous electrolyte solution 112-1Na | | | Na-ClO4 | 0.005 | 9 | 78 | 71 |
| Example 112-2Na | Nonaqueous electrolyte solution 112-2Na | | | | 0.05 | 95 | 77 | 70 |
| Example 112-3Na | Nonaqueous electrolyte solution 112-3Na | | | | 0.1 | 187 | 75 | 69 |
| Example 112-4Na | Nonaqueous electrolyte solution 112-4Na | | | | 0.3 | 565 | 73 | 69 |
| Example 112-5Na | Nonaqueous electrolyte solution 112-5Na | | | | 0.5 | 940 | 72 | 72 |
| Example 1-10 | Nonaqueous electrolyte solution 1-10 | 1-3-1 | 1.0 | - | - | <1 | 93 | 58 |
| Example 113-1Na | Nonaqueous electrolyte solution 113-1Na | | | Na-ClO4 | 0.005 | 10 | 92 | 58 |
| Example 113-2Na | Nonaqueous electrolyte solution 113-2Na | | | | 0.05 | 96 | 91 | 57 |
| Example 113-3Na | Nonaqueous electrolyte solution 113-3Na | | | | 0.1 | 186 | 89 | 56 |
| Example 113-4Na | Nonaqueous electrolyte solution 113-4Na | | | | 0.3 | 562 | 86 | 56 |
| Example 113-5Na | Nonaqueous electrolyte solution 113-5Na | | | | 0.5 | 938 | 85 | 58 |
| Example 2-4 | Nonaqueous electrolyte solution 2-4 | 2-2-2 | 1.0 | - | - | <1 | 88 | 60 |
| Example 114-1Na | Nonaqueous electrolyte solution 114-1Na | | | Na-ClO4 | 0.005 | 8 | 87 | 60 |
| Example 114-2Na | Nonaqueous electrolyte solution 114-2Na | | | | 0.05 | 95 | 86 | 59 |
| Example 114-3Na | Nonaqueous electrolyte solution 114-3Na | | | | 0.1 | 188 | 84 | 58 |
| Example 114-4Na | Nonaqueous electrolyte solution 114-4Na | | | | 0.3 | 562 | 81 | 58 |
| Example 114-5Na | Nonaqueous electrolyte solution 114-5Na | | | | 0.5 | 940 | 80 | 60 |
| Example 3-4 | Nonaqueous electrolyte solution 3-4 | 3-3-3 | 1.0 | - | - | <1 | 92 | 59 |
| Example 115-1Na | Nonaqueous electrolyte solution 115-1Na | | | Na-ClO4 | 0.005 | 9 | 90 | 59 |
| Example 115-2Na | Nonaqueous electrolyte solution 115-2Na | | | | 0.05 | 95 | 90 | 58 |
| Example 115-3Na | Nonaqueous electrolyte solution 115-3Na | | | | 0.1 | 188 | 87 | 57 |
| Example 115-4Na | Nonaqueous electrolyte solution 115-4Na | | | | 0.3 | 563 | 85 | 57 |
| Example 115-5Na | Nonaqueous electrolyte solution 115-5Na | | | | 0.5 | 941 | 83 | 59 |
| Example 4-4 | Nonaqueous electrolyte solution 4-4 | 4-3-1 | 1.0 | - | - | <1 | 91 | 60 |
| Example 116-1Na | Nonaqueous electrolyte solution 116-1Na | | | Na-ClO4 | 0.005 | 8 | 89 | 60 |
| Example 116-2Na | Nonaqueous electrolyte solution 116-2Na | | | | 0.05 | 94 | 88 | 59 |
| Example 116-3Na | Nonaqueous electrolyte solution 116-3Na | | | | 0.1 | 189 | 86 | 59 |
| Example 116-4Na | Nonaqueous electrolyte solution 116-4Na | | | | 0.3 | 564 | 84 | 58 |
| Example 116-5Na | Nonaqueous electrolyte solution 116-5Na | | | | 0.5 | 939 | 82 | 61 |
| Example 4-10 | Nonaqueous electrolyte solution 4-10 | 4-1-11 | 1.0 | - | - | <1 | 77 | 73 |
| Example 117-1Na | Nonaqueous electrolyte solution 117-1Na | | | Na-ClO4 | 0.005 | 9 | 76 | 72 |
| Example 117-2Na | Nonaqueous electrolyte solution 117-2Na | | | | 0.05 | 95 | 75 | 72 |
| Example 117-3Na | Nonaqueous electrolyte solution 117-3Na | | | | 0.1 | 189 | 73 | 72 |
| Example 117-4Na | Nonaqueous electrolyte solution 117-4Na | | | | 0.3 | 563 | 71 | 72 |
| Example 117-5Na | Nonaqueous electrolyte solution 117-5Na | | | | 0.5 | 938 | 70 | 74 |
| Comparative Example 1-1 | Comparative nonaqueous electrolyte solution 1-1 | - | - | - | - | <1 | 100 | 100 |

**[Table 34]**

| Table 34 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 1-4 | Nonaqueous electrolyte solution 1-4 | 1-1-11 | 1.0 | - | - | <1 | 79 | 71 |
| Example 112-1K | Nonaqueous electrolyte solution 112-1K | | | K-ClO4 | 0.005 | 14 | 78 | 71 |
| Example 112-2K | Nonaqueous electrolyte solution 112-2K | | | | 0.05 | 140 | 78 | 70 |
| Example 112-3K | Nonaqueous electrolyte solution 112-3K | | | | 0.1 | 280 | 76 | 70 |
| Example 112-4K | Nonaqueous electrolyte solution 112-4K | | | | 0.3 | 848 | 74 | 70 |
| Example 112-5K | Nonaqueous electrolyte solution 112-5K | | | | 0.5 | 1410 | 73 | 73 |
| Example 1-10 | Nonaqueous electrolyte solution 1-10 | 1-3-1 | 1.0 | - | - | <1 | 93 | 58 |
| Example 113-1K | Nonaqueous electrolyte solution 113-1K | | | K-ClO4 | 0.005 | 15 | 92 | 58 |
| Example 113-2K | Nonaqueous electrolyte solution 113-2K | | | | 0.05 | 143 | 92 | 57 |
| Example 113-3K | Nonaqueous electrolyte solution 113-3K | | | | 0.1 | 284 | 90 | 57 |
| Example 113-4K | Nonaqueous electrolyte solution 113-4K | | | | 0.3 | 849 | 87 | 57 |
| Example 113-5K | Nonaqueous electrolyte solution 113-5K | | | | 0.5 | 1413 | 86 | 59 |
| Example 2-4 | Nonaqueous electrolyte solution 2-4 | 2-2-2 | 1.0 | - | - | <1 | 88 | 60 |
| Example 114-1K | Nonaqueous electrolyte solution 114-1K | | | K-ClO4 | 0.005 | 15 | 87 | 60 |
| Example 114-2K | Nonaqueous electrolyte solution 114-2K | | | | 0.05 | 142 | 87 | 59 |
| Example 114-3K | Nonaqueous electrolyte solution 114-3K | | | | 0.1 | 283 | 85 | 59 |
| Example 114-4K | Nonaqueous electrolyte solution 114-4K | | | | 0.3 | 847 | 82 | 60 |
| Example 114-5K | Nonaqueous electrolyte solution 114-5K | | | | 0.5 | 1411 | 81 | 61 |
| Example 3-4 | Nonaqueous electrolyte solution 3-4 | 3-3-3 | 1.0 | - | - | <1 | 92 | 59 |
| Example 115-1K | Nonaqueous electrolyte solution 115-1K | | | K-ClO4 | 0.005 | 14 | 91 | 59 |
| Example 115-2K | Nonaqueous electrolyte solution 115-2K | | | | 0.05 | 142 | 91 | 58 |
| Example 115-3K | Nonaqueous electrolyte solution 115-3K | | | | 0.1 | 282 | 89 | 58 |
| Example 115-4K | Nonaqueous electrolyte solution 115-4K | | | | 0.3 | 846 | 86 | 59 |
| Example 115-5K | Nonaqueous electrolyte solution 115-5K | | | | 0.5 | 1409 | 84 | 60 |
| Example 4-4 | Nonaqueous electrolyte solution 4-4 | 4-3-1 | 1.0 | - | - | <1 | 91 | 60 |
| Example 116-1K | Nonaqueous electrolyte solution 116-1K | | | K-ClO4 | 0.005 | 16 | 90 | 60 |
| Example 116-2K | Nonaqueous electrolyte solution 116-2K | | | | 0.05 | 144 | 89 | 59 |
| Example 116-3K | Nonaqueous electrolyte solution 116-3K | | | | 0.1 | 285 | 87 | 59 |
| Example 116-4K | Nonaqueous electrolyte solution 116-4K | | | | 0.3 | 849 | 85 | 60 |
| Example 116-5K | Nonaqueous electrolyte solution 116-5K | | | | 0.5 | 1414 | 84 | 62 |
| Example 4-10 | Nonaqueous electrolyte solution 4-10 | 4-1-11 | 1.0 | - | - | <1 | 77 | 73 |
| Example 117-1K | Nonaqueous electrolyte solution 117-1K | | | K-ClO4 | 0.005 | 14 | 76 | 73 |
| Example 117-2K | Nonaqueous electrolyte solution 117-2K | | | | 0.05 | 145 | 76 | 73 |
| Example 117-3K | Nonaqueous electrolyte solution 117-3K | | | | 0.1 | 283 | 75 | 72 |
| Example 117-4K | Nonaqueous electrolyte solution 117-4K | | | | 0.3 | 848 | 72 | 73 |
| Example 117-5K | Nonaqueous electrolyte solution 117-5K | | | | 0.5 | 1413 | 71 | 76 |
| Comparative Example 1-1 | Comparative nonaqueous electrolyte solution 1-1 | - | - | - | - | <1 | 100 | 100 |

**[Table 35]**

| Table 35 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 118-1Na | Nonaqueous electrolyte solution 118-1Na | | | Na-BOB | 0.005 | 6 | 77 | 74 |
| Example 118-2Na | Nonaqueous electrolyte solution 118-2Na | | | | 0.05 | 54 | 77 | 71 |
| Example 118-3Na | Nonaqueous electrolyte solution 118-3Na | | | | 0.1 | 112 | 79 | 69 |
| Example 118-4Na | Nonaqueous electrolyte solution 118-4Na | | | | 0.3 | 330 | 82 | 65 |
| Example 118-5Na | Nonaqueous electrolyte solution 118-5Na | | | | 0.5 | 548 | 86 | 61 |
| Example 38-10 | Nonaqueous electrolyte solution 38-10 | 1-3-1 | 1.0 | - | - | <1 | 90 | 64 |
| Example 119-1Na | Nonaqueous electrolyte solution 119-1Na | | | Na-BOB | 0.005 | 5 | 87 | 64 |
| Example 119-2Na | Nonaqueous electrolyte solution 119-2Na | | | | 0.05 | 55 | 88 | 61 |
| Example 119-3Na | Nonaqueous electrolyte solution 119-3Na | | | | 0.1 | 111 | 89 | 59 |
| Example 119-4Na | Nonaqueous electrolyte solution 119-4Na | | | | 0.3 | 330 | 92 | 56 |
| Example 119-5Na | Nonaqueous electrolyte solution 119-5Na | | | | 0.5 | 549 | 97 | 52 |
| Example 39-4 | Nonaqueous electrolyte solution 39-4 | 2-2-2 | 1.0 | - | - | <1 | 86 | 66 |
| Example 120-1Na | Nonaqueous electrolyte solution 120-1 Na | | | Na-BOB | 0.005 | 6 | 83 | 66 |
| Example 120-2Na | Nonaqueous electrolyte solution 120-2Na | | | | 0.05 | 54 | 84 | 63 |
| Example 120-3Na | Nonaqueous electrolyte solution 120-3Na | | | | 0.1 | 108 | 85 | 61 |
| Example 120-4Na | Nonaqueous electrolyte solution 120-4Na | | | | 0.3 | 331 | 88 | 57 |
| Example 120-5Na | Nonaqueous electrolyte solution 120-5Na | | | | 0.5 | 549 | 93 | 54 |
| Example 40-4 | Nonaqueous electrolyte solution 40-4 | 3-3-3 | 1.0 | - | - | <1 | 89 | 65 |
| Example 121-1Na | Nonaqueous electrolyte solution 121-1Na | | | Na-BOB | 0.005 | 5 | 86 | 65 |
| Example 121-2Na | Nonaqueous electrolyte solution 121-2Na | | | | 0.05 | 58 | 87 | 62 |
| Example 121-3Na | Nonaqueous electrolyte solution 121-3Na | | | | 0.1 | 110 | 88 | 60 |
| Example 121-4Na | Nonaqueous electrolyte solution 121-4Na | | | | 0.3 | 332 | 91 | 57 |
| Example 121-5Na | Nonaqueous electrolyte solution 121-5Na | | | | 0.5 | 550 | 96 | 53 |
| Example 41-4 | Nonaqueous electrolyte solution 41-4 | 4-3-1 | 1.0 | - | - | <1 | 88 | 65 |
| Example 122-1Na | Nonaqueous electrolyte solution 122-1 Na | | | Na-BOB | 0.005 | 6 | 85 | 65 |
| Example 122-2Na | Nonaqueous electrolyte solution 122-2Na | | | | 0.05 | 57 | 86 | 63 |
| Example 122-3Na | Nonaqueous electrolyte solution 122-3Na | | | | 0.1 | 109 | 87 | 61 |
| Example 122-4Na | Nonaqueous electrolyte solution 122-4Na | | | | 0.3 | 328 | 90 | 57 |
| Example 122-5Na | Nonaqueous electrolyte solution 122-5Na | | | | 0.5 | 547 | 95 | 54 |
| Example 41-10 | Nonaqueous electrolyte solution 41-10 | 4-1-11 | 1.0 | - | - | <1 | 78 | 76 |
| Example 123-1Na | Nonaqueous electrolyte solution 123-1 Na | | | Na-BOB | 0.005 | 4 | 75 | 76 |
| Example 123-2Na | Nonaqueous electrolyte solution 123-2Na | | | | 0.05 | 53 | 76 | 73 |
| Example 123-3Na | Nonaqueous electrolyte solution 123-3Na | | | | 0.1 | 109 | 77 | 71 |
| Example 123-4Na | Nonaqueous electrolyte solution 123-4Na | | | | 0.3 | 327 | 80 | 67 |
| Example 123-5Na | Nonaqueous electrolyte solution 123-5Na | | | | 0.5 | 548 | 84 | 62 |
| Comparative Example 34-1 | Comparative nonaqueous electrolyte solution 34-1 | - | - | - | - | <1 | 100 | 100 |

**[Table 36]**

| Table 36 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 118-1K | Nonaqueous electrolyte solution 118-1K | | | K-BOB | 0.005 | 8 | 79 | 74 |
| Example 118-2K | Nonaqueous electrolyte solution 118-2K | | | | 0.05 | 85 | 78 | 72 |
| Example 118-3K | Nonaqueous electrolyte solution 118-3K | | | | 0.1 | 172 | 80 | 70 |
| Example 118-4K | Nonaqueous electrolyte solution 118-4K | | | | 0.3 | 519 | 82 | 66 |
| Example 118-5K | Nonaqueous electrolyte solution 118-5K | | | | 0.5 | 866 | 87 | 62 |
| Example 38-10 | Nonaqueous electrolyte solution 38-10 | 1-3-1 | 1.0 | - | - | <1 | 90 | 64 |
| Example 119-1K | Nonaqueous electrolyte solution 119-1K | | | K-BOB | 0.005 | 9 | 89 | 65 |
| Example 119-2K | Nonaqueous electrolyte solution 119-2K | | | | 0.05 | 88 | 89 | 62 |
| Example 119-3K | Nonaqueous electrolyte solution 119-3K | | | | 0.1 | 176 | 91 | 60 |
| Example 119-4K | Nonaqueous electrolyte solution 119-4K | | | | 0.3 | 520 | 93 | 57 |
| Example 119-5K | Nonaqueous electrolyte solution 119-5K | | | | 0.5 | 865 | 98 | 53 |
| Example 39-4 | Nonaqueous electrolyte solution 39-4 | 2-2-2 | 1.0 | - | - | <1 | 86 | 66 |
| Example 120-1K | Nonaqueous electrolyte solution 120-1K | | | K-BOB | 0.005 | 10 | 85 | 66 |
| Example 120-2K | Nonaqueous electrolyte solution 120-2K | | | | 0.05 | 89 | 85 | 64 |
| Example 120-3K | Nonaqueous electrolyte solution 120-3K | | | | 0.1 | 175 | 87 | 62 |
| Example 120-4K | Nonaqueous electrolyte solution 120-4K | | | | 0.3 | 521 | 89 | 58 |
| Example 120-5K | Nonaqueous electrolyte solution 120-5K | | | | 0.5 | 867 | 94 | 55 |
| Example 40-4 | Nonaqueous electrolyte solution 40-4 | 3-3-3 | 1.0 | - | - | <1 | 89 | 65 |
| Example 121-1K | Nonaqueous electrolyte solution 121-1K | | | K-BOB | 0.005 | 7 | 88 | 65 |
| Example 121-2K | Nonaqueous electrolyte solution 121-2K | | | | 0.05 | 85 | 88 | 63 |
| Example 121-3K | Nonaqueous electrolyte solution 121-3K | | | | 0.1 | 172 | 90 | 61 |
| Example 121-4K | Nonaqueous electrolyte solution 121-4K | | | | 0.3 | 517 | 92 | 57 |
| Example 121-5K | Nonaqueous electrolyte solution 121-5K | | | | 0.5 | 863 | 97 | 54 |
| Example 41-4 | Nonaqueous electrolyte solution 41-4 | 4-3-1 | 1.0 | - | - | <1 | 88 | 65 |
| Example 122-1K | Nonaqueous electrolyte solution 122-1K | | | K-BOB | 0.005 | 9 | 87 | 65 |
| Example 122-2K | Nonaqueous electrolyte solution 122-2K | | | | 0.05 | 88 | 87 | 64 |
| Example 122-3K | Nonaqueous electrolyte solution 122-3K | | | | 0.1 | 175 | 89 | 62 |
| Example 122-4K | Nonaqueous electrolyte solution 122-4K | | | | 0.3 | 518 | 91 | 58 |
| Example 122-5K | Nonaqueous electrolyte solution 122-5K | | | | 0.5 | 864 | 96 | 55 |
| Example 41-10 | Nonaqueous electrolyte solution 41-10 | 4-1-11 | 1.0 | - | - | <1 | 78 | 76 |
| Example 123-1K | Nonaqueous electrolyte solution 123-1K | | | K-BOB | 0.005 | 8 | 77 | 76 |
| Example 123-2K | Nonaqueous electrolyte solution 123-2K | | | | 0.05 | 85 | 77 | 74 |
| Example 123-3K | Nonaqueous electrolyte solution 123-3K | | | | 0.1 | 171 | 79 | 72 |
| Example 123-4K | Nonaqueous electrolyte solution 123-4K | | | | 0.3 | 516 | 81 | 68 |
| Example 123-5K | Nonaqueous electrolyte solution 123-5K | | | | 0.5 | 862 | 85 | 64 |
| Comparative Example 34-1 | Comparative nonaqueous electrolyte solution 34-1 | - | - | - | - | <1 | 100 | 100 |

**[Table 37]**

| Table 37 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 124-1Na | Nonaqueous electrolyte solution 124-1 Na | | | Na-BF4 | 0.005 | 10 | 79 | 74 |
| Example 124-2Na | Nonaqueous electrolyte solution 124-2Na | | | | 0.05 | 106 | 78 | 71 |
| Example 124-3Na | Nonaqueous electrolyte solution 124-3Na | | | | 0.1 | 211 | 76 | 72 |
| Example 124-4Na | Nonaqueous electrolyte solution 124-4Na | | | | 0.3 | 628 | 72 | 74 |
| Example 124-5Na | Nonaqueous electrolyte solution 124-5Na | | | | 0.5 | 1050 | 70 | 76 |
| Example 38-10 | Nonaqueous electrolyte solution 38-10 | 1-3-1 | 1.0 | - | - | <1 | 90 | 64 |
| Example 125-1Na | Nonaqueous electrolyte solution 125-1 Na | | | Na-BF4 | 0.005 | 11 | 89 | 63 |
| Example 125-2Na | Nonaqueous electrolyte solution 125-2Na | | | | 0.05 | 108 | 88 | 61 |
| Example 125-3Na | Nonaqueous electrolyte solution 125-3Na | | | | 0.1 | 210 | 86 | 62 |
| Example 125-4Na | Nonaqueous electrolyte solution 125-4Na | | | | 0.3 | 631 | 82 | 64 |
| Example 125-5Na | Nonaqueous electrolyte solution 125-5Na | | | | 0.5 | 1049 | 79 | 65 |
| Example 39-4 | Nonaqueous electrolyte solution 39-4 | 2-2-2 | 1.0 | - | - | <1 | 86 | 66 |
| Example 126-1Na | Nonaqueous electrolyte solution 126-1 Na | | | Na-BF4 | 0.005 | 9 | 85 | 65 |
| Example 126-2Na | Nonaqueous electrolyte solution 126-2Na | | | | 0.05 | 103 | 85 | 63 |
| Example 126-3Na | Nonaqueous electrolyte solution 126-3Na | | | | 0.1 | 207 | 82 | 64 |
| Example 126-4Na | Nonaqueous electrolyte solution 126-4Na | | | | 0.3 | 626 | 78 | 66 |
| Example 126-5Na | Nonaqueous electrolyte solution 126-5Na | | | | 0.5 | 1045 | 76 | 67 |
| Example 40-4 | Nonaqueous electrolyte solution 40-4 | 3-3-3 | 1.0 | - | - | <1 | 89 | 65 |
| Example 127-1Na | Nonaqueous electrolyte solution 127-1 Na | | | Na-BF4 | 0.005 | 9 | 88 | 64 |
| Example 127-2Na | Nonaqueous electrolyte solution 127-2Na | | | | 0.05 | 104 | 88 | 62 |
| Example 127-3Na | Nonaqueous electrolyte solution 127-3Na | | | | 0.1 | 209 | 85 | 63 |
| Example 127-4Na | Nonaqueous electrolyte solution 127-4Na | | | | 0.3 | 627 | 81 | 65 |
| Example 127-5Na | Nonaqueous electrolyte solution 127-5Na | | | | 0.5 | 1048 | 79 | 66 |
| Example 41-4 | Nonaqueous electrolyte solution 41-4 | 4-3-1 | 1.0 | - | - | <1 | 88 | 65 |
| Example 128-1Na | Nonaqueous electrolyte solution 128-1 Na | | | Na-BF4 | 0.005 | 10 | 87 | 64 |
| Example 128-2Na | Nonaqueous electrolyte solution 128-2Na | | | | 0.05 | 106 | 87 | 63 |
| Example 128-3Na | Nonaqueous electrolyte solution 128-3Na | | | | 0.1 | 210 | 84 | 63 |
| Example 128-4Na | Nonaqueous electrolyte solution 128-4Na | | | | 0.3 | 628 | 80 | 66 |
| Example 128-5Na | Nonaqueous electrolyte solution 128-5Na | | | | 0.5 | 1047 | 78 | 67 |
| Example 41-10 | Nonaqueous electrolyte solution 41-10 | 4-1-11 | 1.0 | - | - | <1 | 78 | 76 |
| Example 129-1Na | Nonaqueous electrolyte solution 129-1 Na | | | Na-BF4 | 0.005 | 10 | 77 | 75 |
| Example 129-2Na | Nonaqueous electrolyte solution 129-2Na | | | | 0.05 | 104 | 77 | 73 |
| Example 129-3Na | Nonaqueous electrolyte solution 129-3Na | | | | 0.1 | 209 | 75 | 74 |
| Example 129-4Na | Nonaqueous electrolyte solution 129-4Na | | | | 0.3 | 628 | 71 | 76 |
| Example 129-5Na | Nonaqueous electrolyte solution 129-5Na | | | | 0.5 | 1048 | 69 | 78 |
| Comparative Example 34-1 | Comparative nonaqueous electrolyte solution 34-1 | - | - | - | - | <1 | 100 | 100 |

**[Table 38]**

| Table 38 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 124-1K | Nonaqueous electrolyte solution 124-1K | | | K-BF4 | 0.005 | 17 | 79 | 74 |
| Example 124-2K | Nonaqueous electrolyte solution 124-2K | | | | 0.05 | 158 | 79 | 72 |
| Example 124-3K | Nonaqueous electrolyte solution 124-3K | | | | 0.1 | 316 | 77 | 73 |
| Example 124-4K | Nonaqueous electrolyte solution 124-4K | | | | 0.3 | 937 | 73 | 75 |
| Example 124-5K | Nonaqueous electrolyte solution 124-5K | | | | 0.5 | 1560 | 71 | 77 |
| Example 38-10 | Nonaqueous electrolyte solution 38-10 | 1-3-1 | 1.0 | - | - | <1 | 90 | 64 |
| Example 125-1K | Nonaqueous electrolyte solution 125-1K | | | K-BF4 | 0.005 | 15 | 90 | 63 |
| Example 125-2K | Nonaqueous electrolyte solution 125-2K | | | | 0.05 | 155 | 89 | 62 |
| Example 125-3K | Nonaqueous electrolyte solution 125-3K | | | | 0.1 | 310 | 88 | 63 |
| Example 125-4K | Nonaqueous electrolyte solution 125-4K | | | | 0.3 | 931 | 82 | 65 |
| Example 125-5K | Nonaqueous electrolyte solution 125-5K | | | | 0.5 | 1550 | 80 | 66 |
| Example 39-4 | Nonaqueous electrolyte solution 39-4 | 2-2-2 | 1.0 | - | - | <1 | 86 | 66 |
| Example 126-1K | Nonaqueous electrolyte solution 126-1K | | | K-BF4 | 0.005 | 15 | 86 | 65 |
| Example 126-2K | Nonaqueous electrolyte solution 126-2K | | | | 0.05 | 153 | 85 | 64 |
| Example 126-3K | Nonaqueous electrolyte solution 126-3K | | | | 0.1 | 310 | 84 | 65 |
| Example 126-4K | Nonaqueous electrolyte solution 126-4K | | | | 0.3 | 932 | 79 | 67 |
| Example 126-5K | Nonaqueous electrolyte solution 126-5K | | | | 0.5 | 1552 | 77 | 68 |
| Example 40-4 | Nonaqueous electrolyte solution 40-4 | 3-3-3 | 1.0 | - | - | <1 | 89 | 65 |
| Example 127-1K | Nonaqueous electrolyte solution 127-1K | | | K-BF4 | 0.005 | 16 | 89 | 64 |
| Example 127-2K | Nonaqueous electrolyte solution 127-2K | | | | 0.05 | 156 | 88 | 63 |
| Example 127-3K | Nonaqueous electrolyte solution 127-3K | | | | 0.1 | 313 | 87 | 64 |
| Example 127-4K | Nonaqueous electrolyte solution 127-4K | | | | 0.3 | 933 | 82 | 66 |
| Example 127-5K | Nonaqueous electrolyte solution 127-5K | | | | 0.5 | 1553 | 79 | 67 |
| Example 41-4 | Nonaqueous electrolyte solution 41-4 | 4-3-1 | 1.0 | - | - | <1 | 88 | 65 |
| Example 128-1K | Nonaqueous electrolyte solution 128-1K | | | K-BF4 | 0.005 | 16 | 87 | 65 |
| Example 128-2K | Nonaqueous electrolyte solution 128-2K | | | | 0.05 | 158 | 87 | 64 |
| Example 128-3K | Nonaqueous electrolyte solution 128-3K | | | | 0.1 | 314 | 86 | 64 |
| Example 128-4K | Nonaqueous electrolyte solution 128-4K | | | | 0.3 | 934 | 81 | 67 |
| Example 128-5K | Nonaqueous electrolyte solution 128-5K | | | | 0.5 | 1555 | 79 | 68 |
| Example 41-10 | Nonaqueous electrolyte solution 41-10 | 4-1-11 | 1.0 | - | - | <1 | 78 | 76 |
| Example 129-1K | Nonaqueous electrolyte solution 129-1K | | | K-BF4 | 0.005 | 15 | 78 | 75 |
| Example 129-2K | Nonaqueous electrolyte solution 129-2K | | | | 0.05 | 152 | 77 | 74 |
| Example 129-3K | Nonaqueous electrolyte solution 129-3K | | | | 0.1 | 309 | 76 | 75 |
| Example 129-4K | Nonaqueous electrolyte solution 129-4K | | | | 0.3 | 929 | 71 | 78 |
| Example 129-5K | Nonaqueous electrolyte solution 129-5K | | | | 0.5 | 1550 | 70 | 80 |
| Comparative Example 34-1 | Comparative nonaqueous electrolyte solution 34-1 | - | - | - | - | <1 | 100 | 100 |

**[Table 39]**

| Table 39 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 130-1Na | Nonaqueous electrolyte solution 130-1Na | | | Na-ClO4 | 0.005 | 8 | 78 | 74 |
| Example 130-2Na | Nonaqueous electrolyte solution 130-2Na | | | | 0.05 | 93 | 77 | 73 |
| Example 130-3Na | Nonaqueous electrolyte solution 130-3Na | | | | 0.1 | 186 | 75 | 72 |
| Example 130-4Na | Nonaqueous electrolyte solution 130-4Na | | | | 0.3 | 561 | 73 | 72 |
| Example 130-5Na | Nonaqueous electrolyte solution 130-5Na | | | | 0.5 | 938 | 72 | 75 |
| Example 38-10 | Nonaqueous electrolyte solution 38-10 | 1-3-1 | 1.0 | - | - | <1 | 90 | 64 |
| Example 131-1Na | Nonaqueous electrolyte solution 131-1Na | | | Na-ClO4 | 0.005 | 9 | 88 | 64 |
| Example 131-2Na | Nonaqueous electrolyte solution 131-2Na | | | | 0.05 | 95 | 88 | 63 |
| Example 131-3Na | Nonaqueous electrolyte solution 131-3Na | | | | 0.1 | 189 | 85 | 62 |
| Example 131-4Na | Nonaqueous electrolyte solution 131-4Na | | | | 0.3 | 565 | 83 | 62 |
| Example 131-5Na | Nonaqueous electrolyte solution 131-5Na | | | | 0.5 | 941 | 82 | 64 |
| Example 39-4 | Nonaqueous electrolyte solution 39-4 | 2-2-2 | 1.0 | - | - | <1 | 86 | 66 |
| Example 132-1Na | Nonaqueous electrolyte solution 132-1Na | | | Na-ClO4 | 0.005 | 9 | 85 | 65 |
| Example 132-2Na | Nonaqueous electrolyte solution 132-2Na | | | | 0.05 | 94 | 84 | 65 |
| Example 132-3Na | Nonaqueous electrolyte solution 132-3Na | | | | 0.1 | 187 | 82 | 64 |
| Example 132-4Na | Nonaqueous electrolyte solution 132-4Na | | | | 0.3 | 564 | 79 | 64 |
| Example 132-5Na | Nonaqueous electrolyte solution 132-5Na | | | | 0.5 | 940 | 78 | 66 |
| Example 40-4 | Nonaqueous electrolyte solution 40-4 | 3-3-3 | 1.0 | - | - | <1 | 89 | 65 |
| Example 133-1Na | Nonaqueous electrolyte solution 133-1Na | | | Na-ClO4 | 0.005 | 8 | 88 | 64 |
| Example 133-2Na | Nonaqueous electrolyte solution 133-2Na | | | | 0.05 | 93 | 87 | 64 |
| Example 133-3Na | Nonaqueous electrolyte solution 133-3Na | | | | 0.1 | 186 | 85 | 63 |
| Example 133-4Na | Nonaqueous electrolyte solution 133-4Na | | | | 0.3 | 564 | 82 | 63 |
| Example 133-5Na | Nonaqueous electrolyte solution 133-5Na | | | | 0.5 | 940 | 81 | 65 |
| Example 41-4 | Nonaqueous electrolyte solution 41-4 | 4-3-1 | 1.0 | - | - | <1 | 88 | 65 |
| Example 134-1Na | Nonaqueous electrolyte solution 134-1Na | | | Na-ClO4 | 0.005 | 10 | 87 | 64 |
| Example 134-2Na | Nonaqueous electrolyte solution 134-2Na | | | | 0.05 | 96 | 86 | 64 |
| Example 134-3Na | Nonaqueous electrolyte solution 134-3Na | | | | 0.1 | 190 | 84 | 64 |
| Example 134-4Na | Nonaqueous electrolyte solution 134-4Na | | | | 0.3 | 565 | 81 | 64 |
| Example 134-5Na | Nonaqueous electrolyte solution 134-5Na | | | | 0.5 | 941 | 80 | 66 |
| Example 41-10 | Nonaqueous electrolyte solution 41-10 | 4-1-11 | 1.0 | - | - | <1 | 78 | 76 |
| Example 135-1Na | Nonaqueous electrolyte solution 135-1Na | | | Na-ClO4 | 0.005 | 10 | 77 | 76 |
| Example 135-2Na | Nonaqueous electrolyte solution 135-2Na | | | | 0.05 | 96 | 76 | 75 |
| Example 135-3Na | Nonaqueous electrolyte solution 135-3Na | | | | 0.1 | 190 | 74 | 75 |
| Example 135-4Na | Nonaqueous electrolyte solution 135-4Na | | | | 0.3 | 561 | 72 | 74 |
| Example 135-5Na | Nonaqueous electrolyte solution 135-5Na | | | | 0.5 | 938 | 71 | 77 |
| Comparative Example 34-1 | Comparative nonaqueous electrolyte solution 34-1 | - | - | - | - | <1 | 100 | 100 |

**[Table 40]**

| Table 40 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 38-4 | Nonaqueous electrolyte solution 38-4 | 1-1-11 | 1.0 | - | - | <1 | 80 | 74 |
| Example 130-1K | Nonaqueous electrolyte solution 130-1K | | | K-ClO4 | 0.005 | 16 | 79 | 74 |
| Example 130-2K | Nonaqueous electrolyte solution 130-2K | | | | 0.05 | 144 | 78 | 74 |
| Example 130-3K | Nonaqueous electrolyte solution 130-3K | | | | 0.1 | 285 | 77 | 73 |
| Example 130-4K | Nonaqueous electrolyte solution 130-4K | | | | 0.3 | 850 | 74 | 73 |
| Example 130-5K | Nonaqueous electrolyte solution 130-5K | | | | 0.5 | 1414 | 73 | 76 |
| Example 38-10 | Nonaqueous electrolyte solution 38-10 | 1-3-1 | 1.0 | - | - | <1 | 90 | 64 |
| Example 131-1K | Nonaqueous electrolyte solution 131-1K | | | K-ClO4 | 0.005 | 15 | 89 | 64 |
| Example 131-2K | Nonaqueous electrolyte solution 131-2K | | | | 0.05 | 143 | 89 | 63 |
| Example 131-3K | Nonaqueous electrolyte solution 131-3K | | | | 0.1 | 283 | 87 | 63 |
| Example 131-4K | Nonaqueous electrolyte solution 131-4K | | | | 0.3 | 849 | 84 | 63 |
| Example 131-5K | Nonaqueous electrolyte solution 131-5K | | | | 0.5 | 1411 | 82 | 65 |
| Example 39-4 | Nonaqueous electrolyte solution 39-4 | 2-2-2 | 1.0 | - | - | <1 | 86 | 66 |
| Example 132-1K | Nonaqueous electrolyte solution 132-1K | | | K-ClO4 | 0.005 | 14 | 85 | 65 |
| Example 132-2K | Nonaqueous electrolyte solution 132-2K | | | | 0.05 | 142 | 85 | 65 |
| Example 132-3K | Nonaqueous electrolyte solution 132-3K | | | | 0.1 | 282 | 83 | 65 |
| Example 132-4K | Nonaqueous electrolyte solution 132-4K | | | | 0.3 | 848 | 80 | 66 |
| Example 132-5K | Nonaqueous electrolyte solution 132-5K | | | | 0.5 | 1410 | 79 | 67 |
| Example 40-4 | Nonaqueous electrolyte solution 40-4 | 3-3-3 | 1.0 | - | - | <1 | 89 | 65 |
| Example 133-1K | Nonaqueous electrolyte solution 133-1K | | | K-ClO4 | 0.005 | 14 | 88 | 64 |
| Example 133-2K | Nonaqueous electrolyte solution 133-2K | | | | 0.05 | 140 | 88 | 64 |
| Example 133-3K | Nonaqueous electrolyte solution 133-3K | | | | 0.1 | 281 | 86 | 64 |
| Example 133-4K | Nonaqueous electrolyte solution 133-4K | | | | 0.3 | 845 | 83 | 65 |
| Example 133-5K | Nonaqueous electrolyte solution 133-5K | | | | 0.5 | 1409 | 82 | 66 |
| Example 41-4 | Nonaqueous electrolyte solution 41-4 | 4-3-1 | 1.0 | - | - | <1 | 88 | 65 |
| Example 134-1K | Nonaqueous electrolyte solution 134-1K | | | K-ClO4 | 0.005 | 16 | 87 | 65 |
| Example 134-2K | Nonaqueous electrolyte solution 134-2K | | | | 0.05 | 143 | 87 | 65 |
| Example 134-3K | Nonaqueous electrolyte solution 134-3K | | | | 0.1 | 284 | 85 | 64 |
| Example 134-4K | Nonaqueous electrolyte solution 134-4K | | | | 0.3 | 850 | 82 | 65 |
| Example 134-5K | Nonaqueous electrolyte solution 134-5K | | | | 0.5 | 1415 | 81 | 67 |
| Example 41-10 | Nonaqueous electrolyte solution 41-10 | 4-1-11 | 1.0 | - | - | <1 | 78 | 76 |
| Example 135-1K | Nonaqueous electrolyte solution 135-1K | | | K-ClO4 | 0.005 | 13 | 77 | 76 |
| Example 135-2K | Nonaqueous electrolyte solution 135-2K | | | | 0.05 | 139 | 77 | 76 |
| Example 135-3K | Nonaqueous electrolyte solution 135-3K | | | | 0.1 | 279 | 75 | 75 |
| Example 135-4K | Nonaqueous electrolyte solution 135-4K | | | | 0.3 | 844 | 73 | 75 |
| Example 135-5K | Nonaqueous electrolyte solution 135-5K | | | | 0.5 | 1408 | 72 | 79 |
| Comparative Example 34-1 | Comparative nonaqueous electrolyte solution 34-1 | - | - | - | - | <1 | 100 | 100 |

**[Table 41]**

| Table 41 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Example 67-4 | Nonaqueous electrolyte solution 67-4 | 1-1-11-Na | 1.0 | - | - | <1 | 77 | 68 |
| Example 136-1Li | Nonaqueous electrolyte solution 136-1Li | | | Li-BF4 | 0.005 | 5 | 76 | 67 |
| Example 136-2Li | Nonaqueous electrolyte solution 136-2Li | | | | 0.05 | 36 | 76 | 65 |
| Example 136-3Li | Nonaqueous electrolyte solution 136-3Li | | | | 0.1 | 75 | 74 | 66 |
| Example 136-4Li | Nonaqueous electrolyte solution 136-4Li | | | | 0.3 | 222 | 70 | 68 |
| Example 136-5Li | Nonaqueous electrolyte solution 136-5Li | | | | 0.5 | 371 | 68 | 69 |
| Example 67-10 | Nonaqueous electrolyte solution 67-10 | 1-3-1-Na | 1.0 | - | - | <1 | 89 | 58 |
| Example 137-1Li | Nonaqueous electrolyte solution 137-1Li | | | Li-BF4 | 0.005 | 4 | 88 | 57 |
| Example 137-2Li | Nonaqueous electrolyte solution 137-2Li | | | | 0.05 | 38 | 87 | 55 |
| Example 137-3Li | Nonaqueous electrolyte solution 137-3Li | | | | 0.1 | 74 | 85 | 56 |
| Example 137-4Li | Nonaqueous electrolyte solution 137-4Li | | | | 0.3 | 221 | 81 | 58 |
| Example 137-5Li | Nonaqueous electrolyte solution 137-5Li | | | | 0.5 | 369 | 78 | 59 |
| Example 68-4 | Nonaqueous electrolyte solution 68-4 | 2-2-2-Na | 1.0 | - | - | <1 | 84 | 60 |
| Example 138-1Li | Nonaqueous electrolyte solution 138-1Li | | | Li-BF4 | 0.005 | 6 | 84 | 58 |
| Example 138-2Li | Nonaqueous electrolyte solution 138-2Li | | | | 0.05 | 38 | 83 | 58 |
| Example 138-3Li | Nonaqueous electrolyte solution 138-3Li | | | | 0.1 | 76 | 81 | 58 |
| Example 138-4Li | Nonaqueous electrolyte solution 138-4Li | | | | 0.3 | 222 | 77 | 60 |
| Example 138-5Li | Nonaqueous electrolyte solution 138-5Li | | | | 0.5 | 370 | 75 | 62 |
| Example 69-4 | Nonaqueous electrolyte solution 69-4 | 3-3-3-Na | 1.0 | - | - | <1 | 88 | 58 |
| Example 139-1Li | Nonaqueous electrolyte solution 139-1Li | | | Li-BF4 | 0.005 | 5 | 87 | 57 |
| Example 139-2Li | Nonaqueous electrolyte solution 139-2Li | | | | 0.05 | 38 | 86 | 56 |
| Example 139-3Li | Nonaqueous electrolyte solution 139-3Li | | | | 0.1 | 76 | 84 | 57 |
| Example 139-4Li | Nonaqueous electrolyte solution 139-4Li | | | | 0.3 | 221 | 80 | 59 |
| Example 139-5Li | Nonaqueous electrolyte solution 139-5Li | | | | 0.5 | 369 | 77 | 60 |
| Example 70-4 | Nonaqueous electrolyte solution 70-4 | 4-3-1-Na | 1.0 | - | - | <1 | 87 | 59 |
| Example 140-1Li | Nonaqueous electrolyte solution 140-1Li | | | Li-BF4 | 0.005 | 4 | 86 | 58 |
| Example 140-2Li | Nonaqueous electrolyte solution 140-2Li | | | | 0.05 | 38 | 85 | 57 |
| Example 140-3Li | Nonaqueous electrolyte solution 140-3Li | | | | 0.1 | 74 | 83 | 58 |
| Example 140-4Li | Nonaqueous electrolyte solution 140-4Li | | | | 0.3 | 221 | 79 | 60 |
| Example 140-5Li | Nonaqueous electrolyte solution 140-5Li | | | | 0.5 | 370 | 76 | 61 |
| Example 70-10 | Nonaqueous electrolyte solution 70-10 | 4-1-11-Na | 1.0 | - | - | <1 | 76 | 70 |
| Example 141-1Li | Nonaqueous electrolyte solution 141-1Li | | | Li-BF4 | 0.005 | 4 | 75 | 69 |
| Example 141-2Li | Nonaqueous electrolyte solution 141-2Li | | | | 0.05 | 37 | 74 | 67 |
| Example 141-3Li | Nonaqueous electrolyte solution 141-3Li | | | | 0.1 | 76 | 72 | 67 |
| Example 141-4Li | Nonaqueous electrolyte solution 141-4Li | | | | 0.3 | 220 | 69 | 70 |
| Example 141-5Li | Nonaqueous electrolyte solution 141-5Li | | | | 0.5 | 371 | 67 | 71 |
| Comparative Example 59-1 | Comparative nonaqueous electrolyte solution 59-1 | - | - | - | - | <1 | 100 | 100 |

**[Table 42]**

| Table 42 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Example 67-4 | Nonaqueous electrolyte solution 67-4 | 1-1-11-Na | 1.0 | - | - | <1 | 77 | 68 |
| Example 136-1K | Nonaqueous electrolyte solution 136-1K | | | K-BF4 | 0.005 | 16 | 77 | 67 |
| Example 136-2K | Nonaqueous electrolyte solution 136-2K | | | | 0.05 | 156 | 76 | 66 |
| Example 136-3K | Nonaqueous electrolyte solution 136-3K | | | | 0.1 | 313 | 75 | 67 |
| Example 136-4K | Nonaqueous electrolyte solution 136-4K | | | | 0.3 | 935 | 73 | 69 |
| Example 136-5K | Nonaqueous electrolyte solution 136-5K | | | | 0.5 | 1556 | 70 | 71 |
| Example 67-10 | Nonaqueous electrolyte solution 67-10 | 1-3-1-Na | 1.0 | - | - | <1 | 89 | 58 |
| Example 137-1K | Nonaqueous electrolyte solution 137-1K | | | K-BF4 | 0.005 | 16 | 89 | 57 |
| Example 137-2K | Nonaqueous electrolyte solution 137-2K | | | | 0.05 | 155 | 88 | 56 |
| Example 137-3K | Nonaqueous electrolyte solution 137-3K | | | | 0.1 | 314 | 86 | 57 |
| Example 137-4K | Nonaqueous electrolyte solution 137-4K | | | | 0.3 | 932 | 83 | 59 |
| Example 137-5K | Nonaqueous electrolyte solution 137-5K | | | | 0.5 | 1557 | 80 | 60 |
| Example 68-4 | Nonaqueous electrolyte solution 68-4 | 2-2-2-Na | 1.0 | - | - | <1 | 84 | 60 |
| Example 138-1K | Nonaqueous electrolyte solution 138-1K | | | K-BF4 | 0.005 | 15 | 84 | 59 |
| Example 138-2K | Nonaqueous electrolyte solution 138-2K | | | | 0.05 | 154 | 84 | 58 |
| Example 138-3K | Nonaqueous electrolyte solution 138-3K | | | | 0.1 | 308 | 82 | 59 |
| Example 138-4K | Nonaqueous electrolyte solution 138-4K | | | | 0.3 | 928 | 79 | 61 |
| Example 138-5K | Nonaqueous electrolyte solution 138-5K | | | | 0.5 | 1549 | 76 | 64 |
| Example 69-4 | Nonaqueous electrolyte solution 69-4 | 3-3-3-Na | 1.0 | - | - | <1 | 88 | 58 |
| Example 139-1K | Nonaqueous electrolyte solution 139-1K | | | K-BF4 | 0.005 | 14 | 88 | 57 |
| Example 139-2K | Nonaqueous electrolyte solution 139-2K | | | | 0.05 | 150 | 87 | 57 |
| Example 139-3K | Nonaqueous electrolyte solution 139-3K | | | | 0.1 | 307 | 85 | 58 |
| Example 139-4K | Nonaqueous electrolyte solution 139-4K | | | | 0.3 | 926 | 82 | 60 |
| Example 139-5K | Nonaqueous electrolyte solution 139-5K | | | | 0.5 | 1548 | 79 | 62 |
| Example 70-4 | Nonaqueous electrolyte solution 70-4 | 4-3-1-Na | 1.0 | - | - | <1 | 87 | 59 |
| Example 140-1K | Nonaqueous electrolyte solution 140-1 K | | | K-BF4 | 0.005 | 17 | 87 | 58 |
| Example 140-2K | Nonaqueous electrolyte solution 140-2K | | | | 0.05 | 157 | 86 | 58 |
| Example 140-3K | Nonaqueous electrolyte solution 140-3K | | | | 0.1 | 315 | 84 | 58 |
| Example 140-4K | Nonaqueous electrolyte solution 140-4K | | | | 0.3 | 936 | 81 | 60 |
| Example 140-5K | Nonaqueous electrolyte solution 140-5K | | | | 0.5 | 1557 | 78 | 63 |
| Example 70-10 | Nonaqueous electrolyte solution 70-10 | 4-1-11-Na | 1.0 | - | - | <1 | 76 | 70 |
| Example 141-1K | Nonaqueous electrolyte solution 141-1 K | | | K-BF4 | 0.005 | 17 | 76 | 69 |
| Example 141-2K | Nonaqueous electrolyte solution 141-2K | | | | 0.05 | 158 | 75 | 68 |
| Example 141-3K | Nonaqueous electrolyte solution 141-3K | | | | 0.1 | 314 | 73 | 69 |
| Example 141-4K | Nonaqueous electrolyte solution 141-4K | | | | 0.3 | 935 | 71 | 71 |
| Example 141-5K | Nonaqueous electrolyte solution 141-5K | | | | 0.5 | 1556 | 68 | 74 |
| Comparative Example 59-1 | Comparative nonaqueous electrolyte solution 59-1 | - | - | - | - | <1 | 100 | 100 |

**[Table 43]**

| Table 43 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Example 67-4 | Nonaqueous electrolyte solution 67-4 | 1-1-11-Na | 1.0 | - | - | <1 | 77 | 68 |
| Example 142-1Li | Nonaqueous electrolyte solution 142-1Li | | | Li-ClO4 | 0.005 | 4 | 76 | 68 |
| Example 142-2Li | Nonaqueous electrolyte solution 142-2Li | | | | 0.05 | 33 | 75 | 67 |
| Example 142-3Li | Nonaqueous electrolyte solution 142-3Li | | | | 0.1 | 66 | 73 | 66 |
| Example 142-4Li | Nonaqueous electrolyte solution 142-4Li | | | | 0.3 | 197 | 71 | 66 |
| Example 142-5Li | Nonaqueous electrolyte solution 142-5Li | | | | 0.5 | 328 | 70 | 69 |
| Example 67-10 | Nonaqueous electrolyte solution 67-10 | 1-3-1-Na | 1.0 | - | - | <1 | 89 | 58 |
| Example 143-1Li | Nonaqueous electrolyte solution 143-1Li | | | Li-ClO4 | 0.005 | 5 | 87 | 58 |
| Example 143-2Li | Nonaqueous electrolyte solution 143-2Li | | | | 0.05 | 35 | 87 | 57 |
| Example 143-3Li | Nonaqueous electrolyte solution 143-3Li | | | | 0.1 | 64 | 84 | 56 |
| Example 143-4Li | Nonaqueous electrolyte solution 143-4Li | | | | 0.3 | 195 | 82 | 56 |
| Example 143-5Li | Nonaqueous electrolyte solution 143-5Li | | | | 0.5 | 325 | 81 | 58 |
| Example 68-4 | Nonaqueous electrolyte solution 68-4 | 2-2-2-Na | 1.0 | - | - | <1 | 84 | 60 |
| Example 144-1Li | Nonaqueous electrolyte solution 144-1Li | | | Li-ClO4 | 0.005 | 3 | 83 | 59 |
| Example 144-2Li | Nonaqueous electrolyte solution 144-2Li | | | | 0.05 | 32 | 82 | 59 |
| Example 144-3Li | Nonaqueous electrolyte solution 144-3Li | | | | 0.1 | 64 | 80 | 59 |
| Example 144-4Li | Nonaqueous electrolyte solution 144-4Li | | | | 0.3 | 197 | 78 | 58 |
| Example 144-5Li | Nonaqueous electrolyte solution 144-5Li | | | | 0.5 | 326 | 77 | 61 |
| Example 69-4 | Nonaqueous electrolyte solution 69-4 | 3-3-3-Na | 1.0 | - | - | <1 | 88 | 58 |
| Example 145-1Li | Nonaqueous electrolyte solution 145-1Li | | | Li-ClO4 | 0.005 | 4 | 86 | 58 |
| Example 145-2Li | Nonaqueous electrolyte solution 145-2Li | | | | 0.05 | 33 | 86 | 58 |
| Example 145-3Li | Nonaqueous electrolyte solution 145-3Li | | | | 0.1 | 64 | 83 | 57 |
| Example 145-4Li | Nonaqueous electrolyte solution 145-4Li | | | | 0.3 | 197 | 81 | 57 |
| Example 145-5Li | Nonaqueous electrolyte solution 145-5Li | | | | 0.5 | 327 | 80 | 59 |
| Example 70-4 | Nonaqueous electrolyte solution 70-4 | 4-3-1-Na | 1.0 | - | - | <1 | 87 | 59 |
| Example 146-1Li | Nonaqueous electrolyte solution 146-1Li | | | Li-ClO4 | 0.005 | 4 | 85 | 59 |
| Example 146-2Li | Nonaqueous electrolyte solution 146-2Li | | | | 0.05 | 34 | 84 | 58 |
| Example 146-3Li | Nonaqueous electrolyte solution 146-3Li | | | | 0.1 | 64 | 82 | 58 |
| Example 146-4Li | Nonaqueous electrolyte solution 146-4Li | | | | 0.3 | 197 | 80 | 58 |
| Example 146-5Li | Nonaqueous electrolyte solution 146-5Li | | | | 0.5 | 325 | 79 | 60 |
| Example 70-10 | Nonaqueous electrolyte solution 70-10 | 4-1-11-Na | 1.0 | - | - | <1 | 76 | 70 |
| Example 147-1Li | Nonaqueous electrolyte solution 147-1Li | | | Li-ClO4 | 0.005 | 3 | 74 | 70 |
| Example 147-2Li | Nonaqueous electrolyte solution 147-2Li | | | | 0.05 | 32 | 74 | 69 |
| Example 147-3Li | Nonaqueous electrolyte solution 147-3Li | | | | 0.1 | 66 | 72 | 68 |
| Example 147-4Li | Nonaqueous electrolyte solution 147-4Li | | | | 0.3 | 194 | 70 | 68 |
| Example 147-5Li | Nonaqueous electrolyte solution 147-5Li | | | | 0.5 | 328 | 69 | 71 |
| Comparative Example 59-1 | Comparative nonaqueous electrolyte solution 59-1 | - | - | - | - | <1 | 100 | 100 |

**[Table 44]**

| Table 44 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Example 67-4 | Nonaqueous electrolyte solution 67-4 | 1-1-11-Na | 1.0 | - | - | <1 | 77 | 68 |
| Example 142-1K | Nonaqueous electrolyte solution 142-1 K | | | K-ClO4 | 0.005 | 14 | 76 | 68 |
| Example 142-2K | Nonaqueous electrolyte solution 142-2K | | | | 0.05 | 139 | 76 | 67 |
| Example 142-3K | Nonaqueous electrolyte solution 142-3K | | | | 0.1 | 280 | 74 | 67 |
| Example 142-4K | Nonaqueous electrolyte solution 142-4K | | | | 0.3 | 846 | 73 | 67 |
| Example 142-5K | Nonaqueous electrolyte solution 142-5K | | | | 0.5 | 1412 | 72 | 70 |
| Example 67-10 | Nonaqueous electrolyte solution 67-10 | 1-3-1-Na | 1.0 | - | - | <1 | 89 | 58 |
| Example 143-1K | Nonaqueous electrolyte solution 143-1 K | | | K-ClO4 | 0.005 | 13 | 88 | 58 |
| Example 143-2K | Nonaqueous electrolyte solution 143-2K | | | | 0.05 | 138 | 88 | 57 |
| Example 143-3K | Nonaqueous electrolyte solution 143-3K | | | | 0.1 | 281 | 86 | 57 |
| Example 143-4K | Nonaqueous electrolyte solution 143-4K | | | | 0.3 | 845 | 84 | 57 |
| Example 143-5K | Nonaqueous electrolyte solution 143-5K | | | | 0.5 | 1411 | 83 | 59 |
| Example 68-4 | Nonaqueous electrolyte solution 68-4 | 2-2-2-Na | 1.0 | - | - | <1 | 84 | 60 |
| Example 144-1K | Nonaqueous electrolyte solution 144-1 K | | | K-ClO4 | 0.005 | 15 | 83 | 60 |
| Example 144-2K | Nonaqueous electrolyte solution 144-2K | | | | 0.05 | 142 | 83 | 60 |
| Example 144-3K | Nonaqueous electrolyte solution 144-3K | | | | 0.1 | 282 | 81 | 59 |
| Example 144-4K | Nonaqueous electrolyte solution 144-4K | | | | 0.3 | 848 | 80 | 60 |
| Example 144-5K | Nonaqueous electrolyte solution 144-5K | | | | 0.5 | 1413 | 79 | 62 |
| Example 69-4 | Nonaqueous electrolyte solution 69-4 | 3-3-3-Na | 1.0 | - | - | <1 | 88 | 58 |
| Example 145-1K | Nonaqueous electrolyte solution 145-1 K | | | K-ClO4 | 0.005 | 15 | 87 | 58 |
| Example 145-2K | Nonaqueous electrolyte solution 145-2K | | | | 0.05 | 141 | 86 | 58 |
| Example 145-3K | Nonaqueous electrolyte solution 145-3K | | | | 0.1 | 284 | 85 | 58 |
| Example 145-4K | Nonaqueous electrolyte solution 145-4K | | | | 0.3 | 848 | 83 | 58 |
| Example 145-5K | Nonaqueous electrolyte solution 145-5K | | | | 0.5 | 1415 | 82 | 60 |
| Example 70-4 | Nonaqueous electrolyte solution 70-4 | 4-3-1-Na | 1.0 | - | - | <1 | 87 | 59 |
| Example 146-1K | Nonaqueous electrolyte solution 146-1 K | | | K-ClO4 | 0.005 | 13 | 86 | 59 |
| Example 146-2K | Nonaqueous electrolyte solution 146-2K | | | | 0.05 | 140 | 85 | 59 |
| Example 146-3K | Nonaqueous electrolyte solution 146-3K | | | | 0.1 | 280 | 84 | 58 |
| Example 146-4K | Nonaqueous electrolyte solution 146-4K | | | | 0.3 | 844 | 82 | 59 |
| Example 146-5K | Nonaqueous electrolyte solution 146-5K | | | | 0.5 | 1408 | 81 | 61 |
| Example 70-10 | Nonaqueous electrolyte solution 70-10 | 4-1-11-Na | 1.0 | - | - | <1 | 76 | 70 |
| Example 147-1K | Nonaqueous electrolyte solution 147-1 K | | | K-ClO4 | 0.005 | 15 | 75 | 70 |
| Example 147-2K | Nonaqueous electrolyte solution 147-2K | | | | 0.05 | 144 | 74 | 69 |
| Example 147-3K | Nonaqueous electrolyte solution 147-3K | | | | 0.1 | 285 | 73 | 69 |
| Example 147-4K | Nonaqueous electrolyte solution 147-4K | | | | 0.3 | 850 | 71 | 69 |
| Example 147-5K | Nonaqueous electrolyte solution 147-5K | | | | 0.5 | 1415 | 70 | 72 |
| Comparative Example 59-1 | Comparative nonaqueous electrolyte solution 59-1 | - | - | - | - | <1 | 100 | 100 |

**[Table 45]**

| Table 45 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Comparative Example 1-1 | Comparative nonaqueous electrolyte solution 1-1 | - | - | - | - | <1 | 100 | 100 |
| Example 100-5Li | Nonaqueous electrolyte solution 100-5Li | 1-1-11 | 1.0 | Li-BOB | 0.5 | <1 | 77 | 57 |
| Example 100-5Na | Nonaqueous electrolyte solution 100-5Na | | | Na-BOB | 0.5 | 550 | 78 | 58 |
| Example 100-5K | Nonaqueous electrolyte solution 100-5K | | | K-BOB | 0.5 | 865 | 79 | 59 |
| Example 101-5Li | Nonaqueous electrolyte solution 101-5Li | 1-3-1 | 1.0 | Li-BOB | 0.5 | <1 | 97 | 46 |
| Example 101-5Na | Nonaqueous electrolyte solution 101-5Na | | | Na-BOB | 0.5 | 547 | 98 | 47 |
| Example 101-5K | Nonaqueous electrolyte solution 101-5K | | | K-BOB | 0.5 | 864 | 99 | 48 |
| Example 102-5Li | Nonaqueous electrolyte solution 102-5Li | 2-2-2 | 1.0 | Li-BOB | 0.5 | <1 | 94 | 47 |
| Example 102-5Na | Nonaqueous electrolyte solution 102-5Na | | | Na-BOB | 0.5 | 549 | 95 | 49 |
| Example 102-5K | Nonaqueous electrolyte solution 102-5K | | | K-BOB | 0.5 | 866 | 96 | 50 |
| Example 103-5Li | Nonaqueous electrolyte solution 103-5Li | 3-3-3 | 1.0 | Li-BOB | 0.5 | <1 | 98 | 47 |
| Example 103-5Na | Nonaqueous electrolyte solution 103-5Na | | | Na-BOB | 0.5 | 551 | 99 | 48 |
| Example 103-5K | Nonaqueous electrolyte solution 103-5K | | | K-BOB | 0.5 | 863 | 100 | 49 |
| Example 104-5Li | Nonaqueous electrolyte solution 104-5Li | 4-3-1 | 1.0 | Li-BOB | 0.5 | <1 | 97 | 47 |
| Example 104-5Na | Nonaqueous electrolyte solution 104-5Na | | | Na-BOB | 0.5 | 549 | 98 | 49 |
| Example 104-5K | Nonaqueous electrolyte solution 104-5K | | | K-BOB | 0.5 | 868 | 99 | 50 |
| Example 105-5Li | Nonaqueous electrolyte solution 105-5Li | 4-1-11 | 1.0 | Li-BOB | 0.5 | <1 | 82 | 58 |
| Example 105-5Na | Nonaqueous electrolyte solution 105-5Na | | | Na-BOB | 0.5 | 548 | 83 | 60 |
| Example 105-5K | Nonaqueous electrolyte solution 105-5K | | | K-BOB | 0.5 | 866 | 84 | 62 |

**[Table 46]**

| Table 46 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Comparative Example 1-1 | Comparative nonaqueous electrolyte solution 1-1 | - | - | - | - | <1 | 100 | 100 |
| Example 106-5Li | Nonaqueous electrolyte solution 106-5Li | 1-1-11 | 1.0 | Li-BF4 | 0.5 | <1 | 69 | 71 |
| Example 106-5Na | Nonaqueous electrolyte solution 106-5Na | | | Na-BF4 | 0.5 | 1052 | 70 | 73 |
| Example 106-5K | Nonaqueous electrolyte solution 106-5K | | | K-BF4 | 0.5 | 1555 | 71 | 74 |
| Example 107-5Li | Nonaqueous electrolyte solution 107-5Li | 1-3-1 | 1.0 | Li-BF4 | 0.5 | <1 | 82 | 58 |
| Example 107-5Na | Nonaqueous electrolyte solution 107-5Na | | | Na-BF4 | 0.5 | 1050 | 82 | 59 |
| Example 107-5K | Nonaqueous electrolyte solution 107-5K | | | K-BF4 | 0.5 | 1556 | 83 | 60 |
| Example 108-5Li | Nonaqueous electrolyte solution 108-5Li | 2-2-2 | 1.0 | Li-BF4 | 0.5 | <1 | 77 | 59 |
| Example 108-5Na | Nonaqueous electrolyte solution 108-5Na | | | Na-BF4 | 0.5 | 1045 | 78 | 61 |
| Example 108-5K | Nonaqueous electrolyte solution 108-5K | | | K-BF4 | 0.5 | 1551 | 78 | 62 |
| Example 109-5Li | Nonaqueous electrolyte solution 109-5Li | 3-3-3 | 1.0 | Li-BF4 | 0.5 | <1 | 80 | 58 |
| Example 109-5Na | Nonaqueous electrolyte solution 109-5Na | | | Na-BF4 | 0.5 | 1048 | 81 | 60 |
| Example 109-5K | Nonaqueous electrolyte solution 109-5K | | | K-BF4 | 0.5 | 1552 | 82 | 61 |
| Example 110-5Li | Nonaqueous electrolyte solution 110-5Li | 4-3-1 | 1.0 | Li-BF4 | 0.5 | <1 | 79 | 60 |
| Example 110-5Na | Nonaqueous electrolyte solution 110-5Na | | | Na-BF4 | 0.5 | 1049 | 80 | 61 |
| Example 110-5K | Nonaqueous electrolyte solution 110-5K | | | K-BF4 | 0.5 | 1553 | 81 | 62 |
| Example 111-5Li | Nonaqueous electrolyte solution 111-5Li | 4-1-11 | 1.0 | Li-BF4 | 0.5 | <1 | 67 | 73 |
| Example 111-5Na | Nonaqueous electrolyte solution 111-5Na | | | Na-BF4 | 0.5 | 1050 | 68 | 75 |
| Example 111-5K | Nonaqueous electrolyte solution 111-5K | | | K-BF4 | 0.5 | 1550 | 69 | 77 |

**[Table 47]**

| Table 47 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Comparative Example 1-1 | Comparative nonaqueous electrolyte solution 1-1 | - | - | - | - | <1 | 100 | 100 |
| Example 112-5Li | Nonaqueous electrolyte solution 112-5Li | 1-1-11 | 1.0 | Li-ClO4 | 0.5 | <1 | 71 | 70 |
| Example 112-5Na | Nonaqueous electrolyte solution 112-5Na | | | Na-ClO4 | 0.5 | 940 | 72 | 72 |
| Example 112-5K | Nonaqueous electrolyte solution 112-5K | | | K-ClO4 | 0.5 | 1410 | 73 | 73 |
| Example 113-5Li | Nonaqueous electrolyte solution 113-5Li | 1-3-1 | 1.0 | Li-ClO4 | 0.5 | <1 | 84 | 57 |
| Example 113-5Na | Nonaqueous electrolyte solution 113-5Na | | | Na-ClO4 | 0.5 | 938 | 85 | 58 |
| Example 113-5K | Nonaqueous electrolyte solution 113-5K | | | K-ClO4 | 0.5 | 1413 | 86 | 59 |
| Example 114-5Li | Nonaqueous electrolyte solution 114-5Li | 2-2-2 | 1.0 | Li-ClO4 | 0.5 | <1 | 79 | 59 |
| Example 114-5Na | Nonaqueous electrolyte solution 114-5Na | | | Na-ClO4 | 0.5 | 940 | 80 | 60 |
| Example 114-5K | Nonaqueous electrolyte solution 114-5K | | | K-ClO4 | 0.5 | 1411 | 81 | 61 |
| Example 115-5Li | Nonaqueous electrolyte solution 115-5Li | 3-3-3 | 1.0 | Li-ClO4 | 0.5 | <1 | 82 | 58 |
| Example 115-5Na | Nonaqueous electrolyte solution 115-5Na | | | Na-ClO4 | 0.5 | 941 | 83 | 59 |
| Example 115-5K | Nonaqueous electrolyte solution 115-5K | | | K-ClO4 | 0.5 | 1409 | 84 | 60 |
| Example 116-5Li | Nonaqueous electrolyte solution 116-5Li | 4-3-1 | 1.0 | Li-ClO4 | 0.5 | <1 | 81 | 59 |
| Example 116-5Na | Nonaqueous electrolyte solution 116-5Na | | | Na-ClO4 | 0.5 | 939 | 82 | 61 |
| Example 116-5K | Nonaqueous electrolyte solution 116-5K | | | K-ClO4 | 0.5 | 1414 | 84 | 62 |
| Example 117-5Li | Nonaqueous electrolyte solution 117-5Li | 4-1-11 | 1.0 | Li-ClO4 | 0.5 | <1 | 69 | 72 |
| Example 117-5Na | Nonaqueous electrolyte solution 117-5Na | | | Na-ClO4 | 0.5 | 938 | 70 | 74 |
| Example 117-5K | Nonaqueous electrolyte solution 117-5K | | | K-ClO4 | 0.5 | 1413 | 71 | 76 |

**[Table 48]**

| Table 48 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Comparative Example 34-1 | Comparative nonaqueous electrolyte solution 34-1 | - | - | - | - | <1 | 100 | 100 |
| Example 118-5Li | Nonaqueous electrolyte solution 118-5Li | 1-1-11 | 1.0 | Li-BOB | 0.5 | <1 | 85 | 59 |
| Example 118-5Na | Nonaqueous electrolyte solution 118-5Na | | | Na-BOB | 0.5 | 548 | 86 | 61 |
| Example 118-5K | Nonaqueous electrolyte solution 118-5K | | | K-BOB | 0.5 | 866 | 87 | 62 |
| Example 119-5Li | Nonaqueous electrolyte solution 119-5Li | 1-3-1 | 1.0 | Li-BOB | 0.5 | <1 | 96 | 51 |
| Example 119-5Na | Nonaqueous electrolyte solution 119-5Na | | | Na-BOB | 0.5 | 549 | 97 | 52 |
| Example 119-5K | Nonaqueous electrolyte solution 119-5K | | | K-BOB | 0.5 | 865 | 98 | 53 |
| Example 120-5Li | Nonaqueous electrolyte solution 120-5Li | 2-2-2 | 1.0 | Li-BOB | 0.5 | <1 | 92 | 52 |
| Example 120-5Na | Nonaqueous electrolyte solution 120-5Na | | | Na-BOB | 0.5 | 549 | 93 | 54 |
| Example 120-5K | Nonaqueous electrolyte solution 120-5K | | | K-BOB | 0.5 | 867 | 94 | 55 |
| Example 121-5Li | Nonaqueous electrolyte solution 121-5Li | 3-3-3 | 1.0 | Li-BOB | 0.5 | <1 | 94 | 51 |
| Example 121-5Na | Nonaqueous electrolyte solution 121-5Na | | | Na-BOB | 0.5 | 550 | 96 | 53 |
| Example 121-5K | Nonaqueous electrolyte solution 121-5K | | | K-BOB | 0.5 | 863 | 97 | 54 |
| Example 122-5Li | Nonaqueous electrolyte solution 122-5Li | 4-3-1 | 1.0 | Li-BOB | 0.5 | <1 | 94 | 52 |
| Example 122-5Na | Nonaqueous electrolyte solution 122-5Na | | | Na-BOB | 0.5 | 547 | 95 | 54 |
| Example 122-5K | Nonaqueous electrolyte solution 122-5K | | | K-BOB | 0.5 | 864 | 96 | 55 |
| Example 123-5Li | Nonaqueous electrolyte solution 123-5Li | 4-1-11 | 1.0 | Li-BOB | 0.5 | <1 | 83 | 60 |
| Example 123-5Na | Nonaqueous electrolyte solution 123-5Na | | | Na-BOB | 0.5 | 548 | 84 | 62 |
| Example 123-5K | Nonaqueous electrolyte solution 123-5K | | | K-BOB | 0.5 | 862 | 85 | 64 |

**[Table 49]**

| Table 49 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Comparative Example 34-1 | Comparative nonaqueous electrolyte solution 34-1 | - | - | - | - | <1 | 100 | 100 |
| Example 124-5Li | Nonaqueous electrolyte solution 124-5Li | 1-1-11 | 1.0 | Li-BF4 | 0.5 | <1 | 69 | 75 |
| Example 124-5Na | Nonaqueous electrolyte solution 124-5Na | | | Na-BF4 | 0.5 | 1050 | 70 | 76 |
| Example 124-5K | Nonaqueous electrolyte solution 124-5K | | | K-BF4 | 0.5 | 1560 | 71 | 77 |
| Example 125-5Li | Nonaqueous electrolyte solution 125-5Li | 1-3-1 | 1.0 | Li-BF4 | 0.5 | <1 | 78 | 64 |
| Example 125-5Na | Nonaqueous electrolyte solution 125-5Na | | | Na-BF4 | 0.5 | 1049 | 79 | 65 |
| Example 125-5K | Nonaqueous electrolyte solution 125-5K | | | K-BF4 | 0.5 | 1550 | 80 | 66 |
| Example 126-5Li | Nonaqueous electrolyte solution 126-5Li | 2-2-2 | 1.0 | Li-BF4 | 0.5 | <1 | 75 | 65 |
| Example 126-5Na | Nonaqueous electrolyte solution 126-5Na | | | Na-BF4 | 0.5 | 1045 | 76 | 67 |
| Example 126-5K | Nonaqueous electrolyte solution 126-5K | | | K-BF4 | 0.5 | 1552 | 77 | 68 |
| Example 127-5Li | Nonaqueous electrolyte solution 127-5Li | 3-3-3 | 1.0 | Li-BF4 | 0.5 | <1 | 77 | 64 |
| Example 127-5Na | Nonaqueous electrolyte solution 127-5Na | | | Na-BF4 | 0.5 | 1048 | 79 | 66 |
| Example 127-5K | Nonaqueous electrolyte solution 127-5K | | | K-BF4 | 0.5 | 1553 | 79 | 67 |
| Example 128-5Li | Nonaqueous electrolyte solution 128-5Li | 4-3-1 | 1.0 | Li-BF4 | 0.5 | <1 | 77 | 65 |
| Example 128-5Na | Nonaqueous electrolyte solution 128-5Na | | | Na-BF4 | 0.5 | 1047 | 78 | 67 |
| Example 128-5K | Nonaqueous electrolyte solution 128-5K | | | K-BF4 | 0.5 | 1555 | 79 | 68 |
| Example 129-5Li | Nonaqueous electrolyte solution 129-5Li | 4-1-11 | 1.0 | Li-BF4 | 0.5 | <1 | 68 | 76 |
| Example 129-5Na | Nonaqueous electrolyte solution 129-5Na | | | Na-BF4 | 0.5 | 1048 | 69 | 78 |
| Example 129-5K | Nonaqueous electrolyte solution 129-5K | | | K-BF4 | 0.5 | 1550 | 70 | 80 |

**[Table 50]**

| Table 50 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Comparative Example 34-1 | Comparative nonaqueous electrolyte solution 34-1 | - | - | - | - | <1 | 100 | 100 |
| Example 130-5Li | Nonaqueous electrolyte solution 130-5Li | 1-1-11 | 1.0 | Li-ClO4 | 0.5 | <1 | 71 | 74 |
| Example 130-5Na | Nonaqueous electrolyte solution 130-5Na | | | Na-ClO4 | 0.5 | 938 | 72 | 75 |
| Example 130-5K | Nonaqueous electrolyte solution 130-5K | | | K-ClO4 | 0.5 | 1414 | 73 | 76 |
| Example 131-5Li | Nonaqueous electrolyte solution 131-5Li | 1-3-1 | 1.0 | Li-ClO4 | 0.5 | <1 | 80 | 63 |
| Example 131-5Na | Nonaqueous electrolyte solution 131-5Na | | | Na-ClO4 | 0.5 | 941 | 82 | 64 |
| Example 131-5K | Nonaqueous electrolyte solution 131-5K | | | K-ClO4 | 0.5 | 1411 | 82 | 65 |
| Example 132-5Li | Nonaqueous electrolyte solution 132-5Li | 2-2-2 | 1.0 | Li-ClO4 | 0.5 | <1 | 77 | 64 |
| Example 132-5Na | Nonaqueous electrolyte solution 132-5Na | | | Na-ClO4 | 0.5 | 940 | 78 | 66 |
| Example 132-5K | Nonaqueous electrolyte solution 132-5K | | | K-ClO4 | 0.5 | 1410 | 79 | 67 |
| Example 133-5Li | Nonaqueous electrolyte solution 133-5Li | 3-3-3 | 1.0 | Li-ClO4 | 0.5 | <1 | 80 | 63 |
| Example 133-5Na | Nonaqueous electrolyte solution 133-5Na | | | Na-ClO4 | 0.5 | 940 | 81 | 65 |
| Example 133-5K | Nonaqueous electrolyte solution 133-5K | | | K-ClO4 | 0.5 | 1409 | 82 | 66 |
| Example 134-5Li | Nonaqueous electrolyte solution 134-5Li | 4-3-1 | 1.0 | Li-ClO4 | 0.5 | <1 | 79 | 64 |
| Example 134-5Na | Nonaqueous electrolyte solution 134-5Na | | | Na-ClO4 | 0.5 | 941 | 80 | 66 |
| Example 134-5K | Nonaqueous electrolyte solution 134-5K | | | K-ClO4 | 0.5 | 1415 | 81 | 67 |
| Example 135-5Li | Nonaqueous electrolyte solution 135-5Li | 4-1-11 | 1.0 | Li-ClO4 | 0.5 | <1 | 70 | 75 |
| Example 135-5Na | Nonaqueous electrolyte solution 135-5Na | | | Na-ClO4 | 0.5 | 938 | 71 | 77 |
| Example 135-5K | Nonaqueous electrolyte solution 135-5K | | | K-ClO4 | 0.5 | 1408 | 72 | 79 |

**[Table 51]**

| Table 51 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Comparative Example 59-1 | Comparative nonaqueous electrolyte solution 59-1 | - | - | - | - | <1 | 100 | 100 |
| Example 136-5Na | Nonaqueous electrolyte solution 136-5Na | 1-1-11-Na | 1.0 | Na-BF4 | 0.5 | <1 | 67 | 68 |
| Example 136-5Li | Nonaqueous electrolyte solution 136-5Li | | | Li-BF4 | 0.5 | 371 | 68 | 69 |
| Example 136-5K | Nonaqueous electrolyte solution 136-5K | | | K-BF4 | 0.5 | 1556 | 70 | 71 |
| Example 137-5Na | Nonaqueous electrolyte solution 137-5Na | 1-3-1-Na | 1.0 | Na-BF4 | 0.5 | <1 | 77 | 58 |
| Example 137-5Li | Nonaqueous electrolyte solution 137-5Li | | | Li-BF4 | 0.5 | 369 | 78 | 59 |
| Example 137-5K | Nonaqueous electrolyte solution 137-5K | | | K-BF4 | 0.5 | 1557 | 80 | 60 |
| Example 138-5Na | Nonaqueous electrolyte solution 138-5Na | 2-2-2-Na | 1.0 | Na-BF4 | 0.5 | <1 | 74 | 60 |
| Example 138-5Li | Nonaqueous electrolyte solution 138-5Li | | | Li-BF4 | 0.5 | 370 | 75 | 62 |
| Example 138-5K | Nonaqueous electrolyte solution 138-5K | | | K-BF4 | 0.5 | 1549 | 76 | 64 |
| Example 139-5Na | Nonaqueous electrolyte solution 139-5Na | 3-3-3-Na | 1.0 | Na-BF4 | 0.5 | <1 | 76 | 58 |
| Example 139-5Li | Nonaqueous electrolyte solution 139-5Li | | | Li-BF4 | 0.5 | 369 | 77 | 60 |
| Example 139-5K | Nonaqueous electrolyte solution 139-5K | | | K-BF4 | 0.5 | 1548 | 79 | 62 |
| Example 140-5Na | Nonaqueous electrolyte solution 140-5Na | 4-3-1-Na | 1.0 | Na-BF4 | 0.5 | <1 | 76 | 59 |
| Example 140-5Li | Nonaqueous electrolyte solution 140-5Li | | | Li-BF4 | 0.5 | 370 | 76 | 61 |
| Example 140-5K | Nonaqueous electrolyte solution 140-5K | | | K-BF4 | 0.5 | 1557 | 78 | 63 |
| Example 141-5Na | Nonaqueous electrolyte solution 141-5Na | 4-1-11-Na | 1.0 | Na-BF4 | 0.5 | <1 | 66 | 69 |
| Example 141-5Li | Nonaqueous electrolyte solution 141-5Li | | | Li-BF4 | 0.5 | 371 | 67 | 71 |
| Example 141-5K | Nonaqueous electrolyte solution 141-5K | | | K-BF4 | 0.5 | 1556 | 68 | 74 |

**[Table 52]**

| Table 52 | Nonaqueous Electrolyte Solution | Component (I) | | Other Additive = Component (IV) | | Content of Cations Other Than Na in Electrolyte Solution [ppm by mass] | R1 | R2 |
|---|---|---|---|---|---|---|---|---|
| | | Type | Content [mass%] | Type | Content [mass%] | | | |
| Comparative Example 59-1 | Comparative nonaqueous electrolyte solution 59-1 | - | - | - | - | <1 | 100 | 100 |
| Example 142-5Na | Nonaqueous electrolyte solution 142-5Na | 1-1-11-Na | 1.0 | Na-ClO4 | 0.5 | <1 | 69 | 67 |
| Example 142-5Li | Nonaqueous electrolyte solution 142-5Li | | | Li-ClO4 | 0.5 | 328 | 70 | 69 |
| Example 142-5K | Nonaqueous electrolyte solution 142-5K | | | K-ClO4 | 0.5 | 1412 | 72 | 70 |
| Example 143-5Na | Nonaqueous electrolyte solution 143-5Na | 1-3-1-Na | 1.0 | Na-ClO4 | 0.5 | <1 | 79 | 57 |
| Example 143-5Li | Nonaqueous electrolyte solution 143-5Li | | | Li-ClO4 | 0.5 | 325 | 81 | 58 |
| Example 143-5K | Nonaqueous electrolyte solution 143-5K | | | K-ClO4 | 0.5 | 1411 | 83 | 59 |
| Example 144-5Na | Nonaqueous electrolyte solution 144-5Na | 2-2-2-Na | 1.0 | Na-ClO4 | 0.5 | <1 | 76 | 59 |
| Example 144-5Li | Nonaqueous electrolyte solution 144-5Li | | | Li-ClO4 | 0.5 | 326 | 77 | 61 |
| Example 144-5K | Nonaqueous electrolyte solution 144-5K | | | K-ClO4 | 0.5 | 1413 | 79 | 62 |
| Example 145-5Na | Nonaqueous electrolyte solution 145-5Na | 3-3-3-Na | 1.0 | Na-ClO4 | 0.5 | <1 | 78 | 57 |
| Example 145-5Li | Nonaqueous electrolyte solution 145-5Li | | | Li-ClO4 | 0.5 | 327 | 80 | 59 |
| Example 145-5K | Nonaqueous electrolyte solution 145-5K | | | K-ClO4 | 0.5 | 1415 | 82 | 60 |
| Example 146-5Na | Nonaqueous electrolyte solution 146-5Na | 4-3-1-Na | 1.0 | Na-ClO4 | 0.5 | <1 | 78 | 58 |
| Example 146-5Li | Nonaqueous electrolyte solution 146-5Li | | | Li-ClO4 | 0.5 | 325 | 79 | 60 |
| Example 146-5K | Nonaqueous electrolyte solution 146-5K | | | K-ClO4 | 0.5 | 1408 | 81 | 61 |
| Example 147-5Na | Nonaqueous electrolyte solution 147-5Na | 4-1-11-Na | 1.0 | Na-ClO4 | 0.5 | <1 | 68 | 68 |
| Example 147-5Li | Nonaqueous electrolyte solution 147-5Li | | | Li-ClO4 | 0.5 | 328 | 69 | 71 |
| Example 147-5K | Nonaqueous electrolyte solution 147-5K | | | K-ClO4 | 0.5 | 1415 | 70 | 72 |

**[Table 53]**

| Table 53 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 148-1 | Nonaqueous electrolyte solution 1-1 | 1-1-11 | 0.05 | - | - | - | - | - | - | 96 | 79 |
| Example 148-2 | Nonaqueous electrolyte solution 1-2 | | 0.1 | - | - | - | - | - | - | 93 | 77 |
| Example 148-3 | Nonaqueous electrolyte solution 1-3 | | 0.5 | - | - | - | - | - | - | 86 | 76 |
| Example 148-4 | Nonaqueous electrolyte solution 1-4 | | 1.0 | - | - | - | - | - | - | 79 | 75 |
| Example 148-5 | Nonaqueous electrolyte solution 1-5 | | 2.0 | - | - | - | - | - | - | 73 | 76 |
| Example 148-6 | Nonaqueous electrolyte solution 1-6 | | 3.0 | - | - | - | - | - | - | 76 | 73 |
| Example 148-7 | Nonaqueous electrolyte solution 1-7 | 1-3-1 | 0.05 | - | - | - | - | - | - | 95 | 79 |
| Example 148-8 | Nonaqueous electrolyte solution 1-8 | | 0.1 | - | - | - | - | - | - | 92 | 77 |
| Example 148-9 | Nonaqueous electrolyte solution 1-9 | | 0.5 | - | - | - | - | - | - | 85 | 71 |
| Example 148-10 | Nonaqueous electrolyte solution 1-10 | | 1.0 | - | - | - | - | - | - | 91 | 63 |
| Example 148-11 | Nonaqueous electrolyte solution 1-11 | | 2.0 | - | - | - | - | - | - | 93 | 59 |
| Example 148-12 | Nonaqueous electrolyte solution 1-12 | | 3.0 | - | - | - | - | - | - | 95 | 56 |
| Example 149-1 | Nonaqueous electrolyte solution 2-1 | 2-2-2 | 0.05 | - | - | - | - | - | - | 95 | 79 |
| Example 149-2 | Nonaqueous electrolyte solution 2-2 | | 0.1 | - | - | - | - | - | - | 91 | 77 |
| Example 149-3 | Nonaqueous electrolyte solution 2-3 | | 0.5 | - | - | - | - | - | - | 88 | 71 |
| Example 149-4 | Nonaqueous electrolyte solution 2-4 | | 1.0 | - | - | - | - | - | - | 87 | 66 |
| Example 149-5 | Nonaqueous electrolyte solution 2-5 | | 2.0 | - | - | - | - | - | - | 84 | 66 |
| Example 149-6 | Nonaqueous electrolyte solution 2-6 | | 3.0 | - | - | - | - | - | - | 90 | 60 |
| Example 150-1 | Nonaqueous electrolyte solution 3-1 | 3-3-3 | 0.05 | - | - | - | - | - | - | 94 | 79 |
| Example 150-2 | Nonaqueous electrolyte solution 3-2 | | 0.1 | - | - | - | - | - | - | 91 | 78 |
| Example 150-3 | Nonaqueous electrolyte solution 3-3 | | 0.5 | - | - | - | - | - | - | 84 | 73 |
| Example 150-4 | Nonaqueous electrolyte solution 3-4 | | 1.0 | - | - | - | - | - | - | 90 | 64 |
| Example 150-5 | Nonaqueous electrolyte solution 3-5 | | 2.0 | - | - | - | - | - | - | 92 | 60 |
| Example 150-6 | Nonaqueous electrolyte solution 3-6 | | 3.0 | - | - | - | - | - | - | 94 | 57 |
| Example 151-1 | Nonaqueous electrolyte solution 4-1 | 4-3-1 | 0.05 | - | - | - | - | - | - | 93 | 79 |
| Example 151-2 | Nonaqueous electrolyte solution 4-2 | | 0.1 | - | - | - | - | - | - | 91 | 78 |
| Example 151-3 | Nonaqueous electrolyte solution 4-3 | | 0.5 | - | - | - | - | - | - | 83 | 74 |
| Example 151-4 | Nonaqueous electrolyte solution 4-4 | | 1.0 | - | - | - | - | - | - | 89 | 65 |
| Example 151-5 | Nonaqueous electrolyte solution 4-5 | | 2.0 | - | - | - | - | - | - | 91 | 60 |
| Example 151-6 | Nonaqueous electrolyte solution 4-6 | | 3.0 | - | - | - | - | - | - | 93 | 58 |
| Example 151-7 | Nonaqueous electrolyte solution 4-7 | 4-1-11 | 0.05 | - | - | - | - | - | - | 95 | 80 |
| Example 151-8 | Nonaqueous electrolyte solution 4-8 | | 0.1 | - | - | - | - | - | - | 92 | 77 |
| Example 151-9 | Nonaqueous electrolyte solution 4-9 | | 0.5 | - | - | - | - | - | - | 85 | 78 |
| Example 151-10 | Nonaqueous electrolyte solution 4-10 | | 1.0 | - | - | - | - | - | - | 77 | 77 |
| Example 151-11 | Nonaqueous electrolyte solution 4-11 | | 2.0 | - | - | - | - | - | - | 72 | 78 |
| Example 151-12 | Nonaqueous electrolyte solution 4-12 | | 3.0 | - | - | - | - | - | - | 75 | 74 |
| Comparative Example 88-1 | Comparative nonaqueous electrolyte solution 1-1 | - | - | - | - | - | - | - | - | 100 | 100 |
| Comparative Example 88-2 | Comparative nonaqueous electrolyte solution 1-2 | 6-1 | 1.0 | - | - | - | - | - | - | 146 | 81 |
| Comparative Example 88-3 | Comparative nonaqueous electrolyte solution 1-3 | 6-2 | 1.0 | - | - | - | - | - | - | 149 | 82 |
| Comparative Example 88-4 | Comparative nonaqueous electrolyte solution 1-4 | 6-3 | 1.0 | - | - | - | - | - | - | 115 | 93 |

**[Table 54]**

| Table 54 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 152-1 | Nonaqueous electrolyte solution 5-1 | 1-1-11 | 0.5 | - | - | - | - | - | - | 85 | 69 |
| | | 1-3-1 | 0.5 | | | | | | | | |
| Example 152-2 | Nonaqueous electrolyte solution 5-2 | 1-1-11 | 0.5 | - | - | - | - | - | - | 83 | 70 |
| | | 2-2-2 | 0.5 | | | | | | | | |
| Example 152-3 | Nonaqueous electrolyte solution 5-3 | 1-1-11 | 0.5 | - | - | - | - | - | - | 84 | 69 |
| | | 3-3-3 | 0.5 | | | | | | | | |
| Example 152-4 | Nonaqueous electrolyte solution 5-4 | 1-1-11 | 0.5 | - | - | - | - | - | - | 84 | 70 |
| | | 4-3-1 | 0.5 | | | | | | | | |
| Example 152-5 | Nonaqueous electrolyte solution 5-5 | 2-2-2 | 0.5 | - | - | - | - | - | - | 88 | 65 |
| | | 3-3-3 | 0.5 | | | | | | | | |
| Example 152-6 | Nonaqueous electrolyte solution 5-6 | 2-2-2 | 0.5 | - | - | - | - | - | - | 88 | 65 |
| | | 4-3-1 | 0.5 | | | | | | | | |
| Example 152-7 | Nonaqueous electrolyte solution 5-7 | 3-3-3 | 0.5 | - | - | - | - | - | - | 90 | 65 |
| | | 4-3-1 | 0.5 | | | | | | | | |
| Example 152-8 | Nonaqueous electrolyte solution 5-8 | 1-1-11 | 0.25 | | | | | | | 86 | 67 |
| | | 2-2-2 | 0.25 | | | | | | | | |
| | | 3-3-3 | 0.25 | | | | | | | | |
| | | 4-3-1 | 0.25 | | | | | | | | |
| Example 152-9 | Nonaqueous electrolyte solution 5-9 | 1-1-11 | 0.5 | - | - | - | - | - | - | 78 | 76 |
| | | 4-1-11 | 0.5 | | | | | | | | |
| Comparative Example 88-1 | Comparative nonaqueous electrolyte solution 1-1 | - | - | - | - | - | - | - | - | 100 | 100 |
| Comparative Example 88-2 | Comparative nonaqueous electrolyte solution 1-2 | 6-1 | 1.0 | - | - | - | - | - | - | 146 | 81 |
| Comparative Example 88-3 | Comparative nonaqueous electrolyte solution 1-3 | 6-2 | 1.0 | - | - | - | - | - | - | 149 | 82 |
| Comparative Example 88-4 | Comparative nonaqueous electrolyte solution 1-4 | 6-3 | 1.0 | - | - | - | - | - | - | 115 | 93 |

**[Table 55]**

| Table 55 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 153-1 | Nonaqueous electrolyte solution 6-1 | 1-1-11 | 1.0 | - | - | | | | | 84 | 67 |
| Example 153-2 | Nonaqueous electrolyte solution 6-2 | 1-3-1 | 1.0 | - | - | | | | | 91 | 60 |
| Example 153-3 | Nonaqueous electrolyte solution 6-3 | 2-2-2 | 1.0 | - | - | | | | | 89 | 61 |
| Example 153-4 | Nonaqueous electrolyte solution 6-4 | 3-3-3 | 1.0 | - | - | | | | | 91 | 60 |
| Example 153-5 | Nonaqueous electrolyte solution 6-5 | 4-3-1 | 1.0 | - | - | | | | | 90 | 61 |
| Example 153-6 | Nonaqueous electrolyte solution 6-6 | 4-1-11 | 1.0 | - | - | VC | 1.0 | BOB | 1.0 | 83 | 68 |
| Comparative Example 89-1 | Comparative nonaqueous electrolyte solution 2-1 | - | - | - | - | | | | | 100 | 100 |
| Comparative Example 89-2 | Comparative nonaqueous electrolyte solution 2-2 | 6-1 | 1.0 | - | - | | | | | 147 | 83 |
| Comparative Example 89-3 | Comparative nonaqueous electrolyte solution 2-3 | 6-2 | 1.0 | | | | | | | 148 | 85 |
| Comparative Example 89-4 | Comparative nonaqueous electrolyte solution 2-4 | 6-3 | 1.0 | - | - | | | | | 110 | 97 |
| Example 154-1 | Nonaqueous electrolyte solution 7-1 | 1-1-11 | 1.0 | - | - | | | | | 72 | 74 |
| Example 154-2 | Nonaqueous electrolyte solution 7-2 | 1-3-1 | 1.0 | - | - | | | | | 86 | 60 |
| Example 154-3 | Nonaqueous electrolyte solution 7-3 | 2-2-2 | 1.0 | - | - | | | | | 81 | 63 |
| Example 154-4 | Nonaqueous electrolyte solution 7-4 | 3-3-3 | 1.0 | - | - | | | | | 85 | 61 |
| Example 154-5 | Nonaqueous electrolyte solution 7-5 | 4-3-1 | 1.0 | - | - | | | | | 83 | 63 |
| Example 154-6 | Nonaqueous electrolyte solution 7-6 | 4-1-11 | 1.0 | - | - | VC | 1.0 | DFBOP | 1.0 | 70 | 77 |
| Comparative Example 90-1 | Comparative nonaqueous electrolyte solution 3-1 | - | - | - | - | | | | | 100 | 100 |
| Comparative Example 90-2 | Comparative nonaqueous electrolyte solution 3-2 | 6-1 | 1.0 | - | - | | | | | 147 | 79 |
| Comparative Example 90-3 | Comparative nonaqueous electrolyte solution 3-3 | 6-2 | 1.0 | - | - | | | | | 150 | 80 |
| Comparative Example 90-4 | Comparative nonaqueous electrolyte solution 3-4 | 6-3 | 1.0 | - | - | | | | | 114 | 94 |
| Example 155-1 | Nonaqueous electrolyte solution 8-1 | 1-1-11 | 1.0 | - | - | | | | | 78 | 72 |
| Example 155-2 | Nonaqueous electrolyte solution 8-2 | 1-3-1 | 1.0 | - | - | | | | | 93 | 58 |
| Example 155-3 | Nonaqueous electrolyte solution 8-3 | 2-2-2 | 1.0 | - | - | | | | | 87 | 61 |
| Example 155-4 | Nonaqueous electrolyte solution 8-4 | 3-3-3 | 1.0 | - | - | | | | | 92 | 59 |
| Example 155-5 | Nonaqueous electrolyte solution 8-5 | 4-3-1 | 1.0 | - | - | | | | | 90 | 61 |
| Example 155-6 | Nonaqueous electrolyte solution 8-6 | 4-1-11 | 1.0 | - | - | VC | 1.0 | DFOB | 1.0 | 75 | 75 |
| Comparative Example 91-1 | Comparative nonaqueous electrolyte solution 4-1 | - | - | - | - | | | | | 100 | 100 |
| Comparative Example 91-2 | Comparative nonaqueous electrolyte solution 4-2 | 6-1 | 1.0 | - | - | | | | | 151 | 76 |
| Comparative Example 91-3 | Comparative nonaqueous electrolyte solution 4-3 | 6-2 | 1.0 | - | - | | | | | 155 | 77 |
| Comparative Example 91-4 | Comparative nonaqueous electrolyte solution 4-4 | 6-3 | 1.0 | - | - | | | | | 110 | 95 |
| Example 156-1 | Nonaqueous electrolyte solution 9-1 | 1-1-11 | 1.0 | - | - | | | | | 77 | 70 |
| Example 156-2 | Nonaqueous electrolyte solution 9-2 | 1-3-1 | 1.0 | - | - | | | | | 86 | 61 |
| Example 156-3 | Nonaqueous electrolyte solution 9-3 | 2-2-2 | 1.0 | - | - | | | | | 82 | 63 |
| Example 156-4 | Nonaqueous electrolyte solution 9-4 | 3-3-3 | 1.0 | - | - | | | | | 85 | 62 |
| Example 156-5 | Nonaqueous electrolyte solution 9-5 | 4-3-1 | 1.0 | - | - | | | | | 84 | 63 |
| Example 156-6 | Nonaqueous electrolyte solution 9-6 | 4-1-11 | 1.0 | - | - | VC | 1.0 | TFOP | 1.0 | 76 | 72 |
| Comparative Example 92-1 | Comparative nonaqueous electrolyte solution 5-1 | - | - | - | - | | | | | 100 | 100 |
| Comparative Example 92-2 | Comparative nonaqueous electrolyte solution 5-2 | 6-1 | 1.0 | - | - | | | | | 133 | 84 |
| Comparative Example 92-3 | Comparative nonaqueous electrolyte solution 5-3 | 6-2 | 1.0 | - | - | | | | | 137 | 85 |
| Comparative Example 92-4 | Comparative nonaqueous electrolyte solution 5-4 | 6-3 | 1.0 | - | - | | | | | 108 | 95 |

**[Table 56]**

| Table 56 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 157-1 | Nonaqueous electrolyte solution 10-1 | 1-1-11 | 1.0 | - | - | | | | | 73 | 73 |
| Example 157-2 | Nonaqueous electrolyte solution 10-2 | 1-3-1 | 1.0 | - | - | | | | | 86 | 60 |
| Example 157-3 | Nonaqueous electrolyte solution 10-3 | 2-2-2 | 1.0 | - | - | | | | | 81 | 63 |
| Example 157-4 | Nonaqueous electrolyte solution 10-4 | 3-3-3 | 1.0 | - | - | | | | | 85 | 61 |
| Example 157-5 | Nonaqueous electrolyte solution 10-5 | 4-3-1 | 1.0 | - | - | | | | | 84 | 62 |
| Example 157-6 | Nonaqueous electrolyte solution 10-6 | 4-1-11 | 1.0 | - | - | VC | 1.0 | DTD | 1.0 | 71 | 76 |
| Comparative Example 93-1 | Comparative nonaqueous electrolyte solution 6-1 | - | - | - | - | | | | | 100 | 100 |
| Comparative Example 93-2 | Comparative nonaqueous electrolyte solution 6-2 | 6-1 | 1.0 | - | - | | | | | 147 | 77 |
| Comparative Example 93-3 | Comparative nonaqueous electrolyte solution 6-3 | 6-2 | 1.0 | - | - | | | | | 149 | 80 |
| Comparative Example 93-4 | Comparative nonaqueous electrolyte solution 6-4 | 6-3 | 1.0 | - | - | | | | | 112 | 91 |
| Example 158-1 | Nonaqueous electrolyte solution 11-1 | 1-1-11 | 1.0 | - | - | | | | | 77 | 67 |
| Example 158-2 | Nonaqueous electrolyte solution 11-2 | 1-3-1 | 1.0 | - | - | | | | | 92 | 54 |
| Example 158-3 | Nonaqueous electrolyte solution 11-3 | 2-2-2 | 1.0 | - | - | | | | | 87 | 57 |
| Example 158-4 | Nonaqueous electrolyte solution 11-4 | 3-3-3 | 1.0 | - | - | | | | | 91 | 56 |
| Example 158-5 | Nonaqueous electrolyte solution 11-5 | 4-3-1 | 1.0 | - | - | | | | | 89 | 57 |
| Example 158-6 | Nonaqueous electrolyte solution 11-6 | 4-1-11 | 1.0 | - | - | VC | 1.0 | DFPFSI | 1.0 | 75 | 70 |
| Comparative Example 94-1 | Comparative nonaqueous electrolyte solution 7-1 | - | - | - | - | | | | | 100 | 100 |
| Comparative Example 94-2 | Comparative nonaqueous electrolyte solution 7-2 | 6-1 | 1.0 | - | - | | | | | 147 | 76 |
| Comparative Example 94-3 | Comparative nonaqueous electrolyte solution 7-3 | 6-2 | 1.0 | - | - | | | | | 151 | 79 |
| Comparative Example 94-4 | Comparative nonaqueous electrolyte solution 7-4 | 6-3 | 1.0 | - | - | | | | | 113 | 90 |
| Example 159-1 | Nonaqueous electrolyte solution 12-1 | 1-1-11 | 1.0 | - | - | | | | | 77 | 61 |
| Example 159-2 | Nonaqueous electrolyte solution 12-2 | 1-3-1 | 1.0 | - | - | | | | | 90 | 50 |
| Example 159-3 | Nonaqueous electrolyte solution 12-3 | 2-2-2 | 1.0 | - | - | | | | | 85 | 52 |
| Example 159-4 | Nonaqueous electrolyte solution 12-4 | 3-3-3 | 1.0 | - | - | | | | | 89 | 51 |
| Example 159-5 | Nonaqueous electrolyte solution 12-5 | 4-3-1 | 1.0 | - | - | | | | | 88 | 52 |
| Example 159-6 | Nonaqueous electrolyte solution 12-6 | 4-1-11 | 1.0 | - | - | VC | 1.0 | FS | 1.0 | 75 | 64 |
| Comparative Example 95-1 | Comparative nonaqueous electrolyte solution 8-1 | - | - | - | - | | | | | 100 | 100 |
| Comparative Example 95-2 | Comparative nonaqueous electrolyte solution 8-2 | 6-1 | 1.0 | - | - | | | | | 147 | 82 |
| Comparative Example 95-3 | Comparative nonaqueous electrolyte solution 8-3 | 6-2 | 1.0 | - | - | | | | | 149 | 84 |
| Comparative Example 95-4 | Comparative nonaqueous electrolyte solution 8-4 | 6-3 | 1.0 | - | - | | | | | 112 | 95 |
| Example 160-1 | Nonaqueous electrolyte solution 13-1 | 1-1-11 | 1.0 | - | - | | | | | 79 | 70 |
| Example 160-2 | Nonaqueous electrolyte solution 13-2 | 1-3-1 | 1.0 | - | - | | | | | 87 | 62 |
| Example 160-3 | Nonaqueous electrolyte solution 13-3 | 2-2-2 | 1.0 | - | - | | | | | 84 | 63 |
| Example 160-4 | Nonaqueous electrolyte solution 13-4 | 3-3-3 | 1.0 | - | - | | | | | 86 | 63 |
| Example 160-5 | Nonaqueous electrolyte solution 13-5 | 4-3-1 | 1.0 | - | - | VC | 1.0 | TV-Si | 0.5 | 86 | 63 |
| Example 160-6 | Nonaqueous electrolyte solution 13-6 | 4-1-11 | 1.0 | - | - | | | | | 78 | 71 |
| Comparative Example 96-1 | Comparative nonaqueous electrolyte solution 9-1 | - | - | - | - | | | | | 100 | 100 |
| Comparative Example 96-2 | Comparative nonaqueous electrolyte solution 9-2 | 6-1 | 1.0 | - | - | | | | | 140 | 83 |
| Comparative Example 96-3 | Comparative nonaqueous electrolyte solution 9-3 | 6-2 | 1.0 | - | - | | | | | 143 | 83 |
| Comparative Example 96-4 | Comparative nonaqueous electrolyte solution 9-4 | 6-3 | 1.0 | - | - | | | | | 113 | 92 |

**[Table 57]**

| Table 57 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 161-1 | Nonaqueous electrolyte solution 14-1 | 1-1-11 | 1.0 | | | | | | | 82 | 65 |
| Example 161-2 | Nonaqueous electrolyte solution 14-2 | 1-3-1 | 1.0 | | | | | | | 89 | 58 |
| Example 161-3 | Nonaqueous electrolyte solution 14-3 | 2-2-2 | 1.0 | | | | | | | 86 | 59 |
| Example 161-4 | Nonaqueous electrolyte solution 14-4 | 3-3-3 | 1.0 | | | | | | | 88 | 59 |
| Example 161-5 | Nonaqueous electrolyte solution 14-5 | 4-3-1 | 1.0 | DFP | 0.1 | VC | 1.0 | BOB | 1.0 | 88 | 60 |
| Example 161-6 | Nonaqueous electrolyte solution 14-6 | 4-1-11 | 1.0 | | | | | | | 81 | 67 |
| Comparative Example 97-1 | Comparative nonaqueous electrolyte solution 10-1 | - | - | | | | | | | 100 | 100 |
| Comparative Example 97-2 | Comparative nonaqueous electrolyte solution 10-2 | 6-1 | 1.0 | | | | | | | 147 | 83 |
| Comparative Example 97-3 | Comparative nonaqueous electrolyte solution 10-3 | 6-2 | 1.0 | | | | | | | 148 | 85 |
| Comparative Example 97-4 | Comparative nonaqueous electrolyte solution 10-4 | 6-3 | 1.0 | | | | | | | 110 | 97 |
| Example 162-1 | Nonaqueous electrolyte solution 15-1 | 1-1-11 | 1.0 | | | | | | | 70 | 73 |
| Example 162-2 | Nonaqueous electrolyte solution 15-2 | 1-3-1 | 1.0 | | | | | | | 84 | 59 |
| Example 162-3 | Nonaqueous electrolyte solution 15-3 | 2-2-2 | 1.0 | | | | | | | 78 | 62 |
| Example 162-4 | Nonaqueous electrolyte solution 15-4 | 3-3-3 | 1.0 | | | | | | | 82 | 60 |
| Example 162-5 | Nonaqueous electrolyte solution 15-5 | 4-3-1 | 1.0 | DFP | 0.1 | VC | 1.0 | DFBOP | 1.0 | 81 | 61 |
| Example 162-6 | Nonaqueous electrolyte solution 15-6 | 4-1-11 | 1.0 | | | | | | | 68 | 76 |
| Comparative Example 98-1 | Comparative nonaqueous electrolyte solution 11-1 | - | - | | | | | | | 100 | 100 |
| Comparative Example 98-2 | Comparative nonaqueous electrolyte solution 11-2 | 6-1 | 1.0 | | | | | | | 147 | 79 |
| Comparative Example 98-3 | Comparative nonaqueous electrolyte solution 11-3 | 6-2 | 1.0 | | | | | | | 150 | 80 |
| Comparative Example 98-4 | Comparative nonaqueous electrolyte solution 11-4 | 6-3 | 1.0 | | | | | | | 114 | 94 |
| Example 163-1 | Nonaqueous electrolyte solution 16-1 | 1-1-11 | 1.0 | | | | | | | 76 | 70 |
| Example 163-2 | Nonaqueous electrolyte solution 16-2 | 1-3-1 | 1.0 | | | | | | | 91 | 57 |
| Example 163-3 | Nonaqueous electrolyte solution 16-3 | 2-2-2 | 1.0 | | | | | | | 85 | 60 |
| Example 163-4 | Nonaqueous electrolyte solution 16-4 | 3-3-3 | 1.0 | | | | | | | 89 | 58 |
| Example 163-5 | Nonaqueous electrolyte solution 16-5 | 4-3-1 | 1.0 | DFP | 0.1 | VC | 1.0 | DFOB | 1.0 | 88 | 59 |
| Example 163-6 | Nonaqueous electrolyte solution 16-6 | 4-1-11 | 1.0 | | | | | | | 73 | 73 |
| Comparative Example 99-1 | Comparative nonaqueous electrolyte solution 12-1 | - | - | | | | | | | 100 | 100 |
| Comparative Example 99-2 | Comparative nonaqueous electrolyte solution 12-2 | 6-1 | 1.0 | | | | | | | 151 | 76 |
| Comparative Example 99-3 | Comparative nonaqueous electrolyte solution 12-3 | 6-2 | 1.0 | | | | | | | 155 | 77 |
| Comparative Example 99-4 | Comparative nonaqueous electrolyte solution 12-4 | 6-3 | 1.0 | | | | | | | 110 | 95 |
| Example 164-1 | Nonaqueous electrolyte solution 17-1 | 1-1-11 | 1.0 | | | | | | | 75 | 69 |
| Example 164-2 | Nonaqueous electrolyte solution 17-2 | 1-3-1 | 1.0 | | | | | | | 84 | 60 |
| Example 164-3 | Nonaqueous electrolyte solution 17-3 | 2-2-2 | 1.0 | | | | | | | 80 | 61 |
| Example 164-4 | Nonaqueous electrolyte solution 17-4 | 3-3-3 | 1.0 | | | | | | | 83 | 61 |
| Example 164-5 | Nonaqueous electrolyte solution 17-5 | 4-3-1 | 1.0 | DFP | 0.1 | VC | 1.0 | TFOP | 1.0 | 82 | 61 |
| Example 164-6 | Nonaqueous electrolyte solution 17-6 | 4-1-11 | 1.0 | | | | | | | 74 | 70 |
| Comparative Example 100-1 | Comparative nonaqueous electrolyte solution 13-1 | - | - | | | | | | | 100 | 100 |
| Comparative Example 100-2 | Comparative nonaqueous electrolyte solution 13-2 | 6-1 | 1.0 | | | | | | | 133 | 84 |
| Comparative Example 100-3 | Comparative nonaqueous electrolyte solution 13-3 | 6-2 | 1.0 | | | | | | | 137 | 85 |
| Comparative Example 100-4 | Comparative nonaqueous electrolyte solution 13-4 | 6-3 | 1.0 | | | | | | | 108 | 95 |

**[Table 58]**

| Table 58 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 165-1 | Nonaqueous electrolyte solution 18-1 | 1-1-11 | 1.0 | | | | | | | 71 | 72 |
| Example 165-2 | Nonaqueous electrolyte solution 18-2 | 1-3-1 | 1.0 | | | | | | | 84 | 59 |
| Example 165-3 | Nonaqueous electrolyte solution 18-3 | 2-2-2 | 1.0 | | | | | | | 79 | 61 |
| Example 165-4 | Nonaqueous electrolyte solution 18-4 | 3-3-3 | 1.0 | | | | | | | 83 | 60 |
| Example 165-5 | Nonaqueous electrolyte solution 18-5 | 4-3-1 | 1.0 | | | | | | | 82 | 61 |
| Example 165-6 | Nonaqueous electrolyte solution 18-6 | 4-1-11 | 1.0 | DFP | 0.1 | VC | 1.0 | DTD | 1.0 | 69 | 75 |
| Comparative Example 101-1 | Comparative nonaqueous electrolyte solution 14-1 | - | - | | | | | | | 100 | 100 |
| Comparative Example 101-2 | Comparative nonaqueous electrolyte solution 14-2 | 6-1 | 1.0 | | | | | | | 147 | 77 |
| Comparative Example 101-3 | Comparative nonaqueous electrolyte solution 14-3 | 6-2 | 1.0 | | | | | | | 149 | 80 |
| Comparative Example 101-4 | Comparative nonaqueous electrolyte solution 14-4 | 6-3 | 1.0 | | | | | | | 112 | 91 |
| Example 166-1 | Nonaqueous electrolyte solution 19-1 | 1-1-11 | 1.0 | | | | | | | 75 | 66 |
| Example 166-2 | Nonaqueous electrolyte solution 19-2 | 1-3-1 | 1.0 | | | | | | | 90 | 53 |
| Example 166-3 | Nonaqueous electrolyte solution 19-3 | 2-2-2 | 1.0 | | | | | | | 84 | 56 |
| Example 166-4 | Nonaqueous electrolyte solution 19-4 | 3-3-3 | 1.0 | | | | | | | 89 | 54 |
| Example 166-5 | Nonaqueous electrolyte solution 19-5 | 4-3-1 | 1.0 | | | | | | | 87 | 55 |
| Example 166-6 | Nonaqueous electrolyte solution 19-6 | 4-1-11 | 1.0 | DFP | 0.1 | VC | 1.0 | DFPFSI | 1.0 | 73 | 68 |
| Comparative Example 102-1 | Comparative nonaqueous electrolyte solution 15-1 | - | - | | | | | | | 100 | 100 |
| Comparative Example 102-2 | Comparative nonaqueous electrolyte solution 15-2 | 6-1 | 1.0 | | | | | | | 147 | 76 |
| Comparative Example 102-3 | Comparative nonaqueous electrolyte solution 15-3 | 6-2 | 1.0 | | | | | | | 151 | 79 |
| Comparative Example 102-4 | Comparative nonaqueous electrolyte solution 15-4 | 6-3 | 1.0 | | | | | | | 113 | 90 |
| Example 167-1 | Nonaqueous electrolyte solution 20-1 | 1-1-11 | 1.0 | | | | | | | 75 | 60 |
| Example 167-2 | Nonaqueous electrolyte solution 20-2 | 1-3-1 | 1.0 | | | | | | | 88 | 49 |
| Example 167-3 | Nonaqueous electrolyte solution 20-3 | 2-2-2 | 1.0 | | | | | | | 83 | 51 |
| Example 167-4 | Nonaqueous electrolyte solution 20-4 | 3-3-3 | 1.0 | | | | | | | 87 | 50 |
| Example 167-5 | Nonaqueous electrolyte solution 20-5 | 4-3-1 | 1.0 | | | | | | | 85 | 51 |
| Example 167-6 | Nonaqueous electrolyte solution 20-6 | 4-1-11 | 1.0 | DFP | 0.1 | VC | 1.0 | FS | 1.0 | 73 | 62 |
| Comparative Example 103-1 | Comparative nonaqueous electrolyte solution 16-1 | - | - | | | | | | | 100 | 100 |
| Comparative Example 103-2 | Comparative nonaqueous electrolyte solution 16-2 | 6-1 | 1.0 | | | | | | | 147 | 82 |
| Comparative Example 103-3 | Comparative nonaqueous electrolyte solution 16-3 | 6-2 | 1.0 | | | | | | | 149 | 84 |
| Comparative Example 103-4 | Comparative nonaqueous electrolyte solution 16-4 | 6-3 | 1.0 | | | | | | | 112 | 95 |
| Example 168-1 | Nonaqueous electrolyte solution 21-1 | 1-1-11 | 1.0 | | | | | | | 77 | 68 |
| Example 168-2 | Nonaqueous electrolyte solution 21-2 | 1-3-1 | 1.0 | | | | | | | 85 | 61 |
| Example 168-3 | Nonaqueous electrolyte solution 21-3 | 2-2-2 | 1.0 | | | | | | | 82 | 62 |
| Example 168-4 | Nonaqueous electrolyte solution 21-4 | 3-3-3 | 1.0 | | | | | | | 84 | 61 |
| Example 168-5 | Nonaqueous electrolyte solution 21-5 | 4-3-1 | 1.0 | DFP | 0.1 | VC | 1.0 | TV-Si | 0.5 | 83 | 62 |
| Example 168-6 | Nonaqueous electrolyte solution 21-6 | 4-1-11 | 1.0 | | | | | | | 76 | 70 |
| Comparative Example 104-1 | Comparative nonaqueous electrolyte solution 17-1 | - | - | | | | | | | 100 | 100 |
| Comparative Example 104-2 | Comparative nonaqueous electrolyte solution 17-2 | 6-1 | 1.0 | | | | | | | 140 | 83 |
| Comparative Example 104-3 | Comparative nonaqueous electrolyte solution 17-3 | 6-2 | 1.0 | | | | | | | 143 | 83 |
| Comparative Example 104-4 | Comparative nonaqueous electrolyte solution 17-4 | 6-3 | 1.0 | | | | | | | 113 | 92 |

**[Table 59]**

| Table 59 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 169-1 | Nonaqueous electrolyte solution 22-1 | 1-1-11 | 1.0 | | | | | | | 82 | 65 |
| Example 169-2 | Nonaqueous electrolyte solution 22-2 | 1-3-1 | 1.0 | | | | | | | 90 | 58 |
| Example 169-3 | Nonaqueous electrolyte solution 22-3 | 2-2-2 | 1.0 | | | | | | | 87 | 59 |
| Example 169-4 | Nonaqueous electrolyte solution 22-4 | 3-3-3 | 1.0 | | | | | | | 89 | 59 |
| Example 169-5 | Nonaqueous electrolyte solution 22-5 | 4-3-1 | 1.0 | FSI | 0.1 | VC | 1.0 | BOB | 1.0 | 88 | 59 |
| Example 169-6 | Nonaqueous electrolyte solution 22-6 | 4-1-11 | 1.0 | | | | | | | 81 | 66 |
| Comparative Example 105-1 | Comparative nonaqueous electrolyte solution 18-1 | - | - | | | | | | | 100 | 100 |
| Comparative Example 105-2 | Comparative nonaqueous electrolyte solution 18-2 | 6-1 | 1.0 | | | | | | | 147 | 83 |
| Comparative Example 105-3 | Comparative nonaqueous electrolyte solution 18-3 | 6-2 | 1.0 | | | | | | | 148 | 85 |
| Comparative Example 105-4 | Comparative nonaqueous electrolyte solution 18-4 | 6-3 | 1.0 | | | | | | | 110 | 97 |
| Example 170-1 | Nonaqueous electrolyte solution 23-1 | 1-1-11 | 1.0 | | | | | | | 70 | 72 |
| Example 170-2 | Nonaqueous electrolyte solution 23-2 | 1-3-1 | 1.0 | | | | | | | 84 | 59 |
| Example 170-3 | Nonaqueous electrolyte solution 23-3 | 2-2-2 | 1.0 | | | | | | | 79 | 62 |
| Example 170-4 | Nonaqueous electrolyte solution 23-4 | 3-3-3 | 1.0 | | | | | | | 83 | 60 |
| Example 170-5 | Nonaqueous electrolyte solution 23-5 | 4-3-1 | 1.0 | FSI | 0.1 | VC | 1.0 | DFBOP | 1.0 | 82 | 61 |
| Example 170-6 | Nonaqueous electrolyte solution 23-6 | 4-1-11 | 1.0 | | | | | | | 68 | 75 |
| Comparative Example 106-1 | Comparative nonaqueous electrolyte solution 19-1 | - | - | | | | | | | 100 | 100 |
| Comparative Example 106-2 | Comparative nonaqueous electrolyte solution 19-2 | 6-1 | 1.0 | | | | | | | 147 | 79 |
| Comparative Example 106-3 | Comparative nonaqueous electrolyte solution 19-3 | 6-2 | 1.0 | | | | | | | 150 | 80 |
| Comparative Example 106-4 | Comparative nonaqueous electrolyte solution 19-4 | 6-3 | 1.0 | | | | | | | 114 | 94 |
| Example 171-1 | Nonaqueous electrolyte solution 24-1 | 1-1-11 | 1.0 | | | | | | | 76 | 70 |
| Example 171-2 | Nonaqueous electrolyte solution 24-2 | 1-3-1 | 1.0 | | | | | | | 91 | 57 |
| Example 171-3 | Nonaqueous electrolyte solution 24-3 | 2-2-2 | 1.0 | | | | | | | 86 | 60 |
| Example 171-4 | Nonaqueous electrolyte solution 24-4 | 3-3-3 | 1.0 | | | | | | | 90 | 58 |
| Example 171-5 | Nonaqueous electrolyte solution 24-5 | 4-3-1 | 1.0 | | | | | | | 88 | 59 |
| Example 171-6 | Nonaqueous electrolyte solution 24-6 | 4-1-11 | 1.0 | FSI | 0.1 | VC | 1.0 | DFOB | 1.0 | 74 | 73 |
| Comparative Example 107-1 | Comparative nonaqueous electrolyte solution 20-1 | - | - | | | | | | | 100 | 100 |
| Comparative Example 107-2 | Comparative nonaqueous electrolyte solution 20-2 | 6-1 | 1.0 | | | | | | | 151 | 76 |
| Comparative Example 107-3 | Comparative nonaqueous electrolyte solution 20-3 | 6-2 | 1.0 | | | | | | | 155 | 77 |
| Comparative Example 107-4 | Comparative nonaqueous electrolyte solution 20-4 | 6-3 | 1.0 | | | | | | | 110 | 95 |
| Example 172-1 | Nonaqueous electrolyte solution 25-1 | 1-1-11 | 1.0 | | | | | | | 75 | 68 |
| Example 172-2 | Nonaqueous electrolyte solution 25-2 | 1-3-1 | 1.0 | | | | | | | 84 | 59 |
| Example 172-3 | Nonaqueous electrolyte solution 25-3 | 2-2-2 | 1.0 | | | | | | | 81 | 61 |
| Example 172-4 | Nonaqueous electrolyte solution 25-4 | 3-3-3 | 1.0 | | | | | | | 83 | 60 |
| Example 172-5 | Nonaqueous electrolyte solution 25-5 | 4-3-1 | 1.0 | | | | | | | 82 | 61 |
| Example 172-6 | Nonaqueous electrolyte solution 25-6 | 4-1-11 | 1.0 | FSI | 0.1 | VC | 1.0 | TFOP | 1.0 | 74 | 70 |
| Comparative Example 108-1 | Comparative nonaqueous electrolyte solution 21-1 | - | - | | | | | | | 100 | 100 |
| Comparative Example 108-2 | Comparative nonaqueous electrolyte solution 21-2 | 6-1 | 1.0 | | | | | | | 133 | 84 |
| Comparative Example 108-3 | Comparative nonaqueous electrolyte solution 21-3 | 6-2 | 1.0 | | | | | | | 137 | 85 |
| Comparative Example 108-4 | Comparative nonaqueous electrolyte solution 21-4 | 6-3 | 1.0 | | | | | | | 108 | 95 |

**[Table 60]**

| Table 60 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 173-1 | Nonaqueous electrolyte solution 26-1 | 1-1-11 | 1.0 | | | | | | | 71 | 71 |
| Example 173-2 | Nonaqueous electrolyte solution 26-2 | 1-3-1 | 1.0 | | | | | | | 85 | 58 |
| Example 173-3 | Nonaqueous electrolyte solution 26-3 | 2-2-2 | 1.0 | | | | | | | 80 | 61 |
| Example 173-4 | Nonaqueous electrolyte solution 26-4 | 3-3-3 | 1.0 | | | | | | | 83 | 59 |
| Example 173-5 | Nonaqueous electrolyte solution 26-5 | 4-3-1 | 1.0 | FSI | 0.1 | VC | 1.0 | DTD | 1.0 | 82 | 61 |
| Example 173-6 | Nonaqueous electrolyte solution 26-6 | 4-1-11 | 1.0 | | | | | | | 69 | 74 |
| Comparative Example 109-1 | Comparative nonaqueous electrolyte solution 22-1 | - | - | | | | | | | 100 | 100 |
| Comparative Example 109-2 | Comparative nonaqueous electrolyte solution 22-2 | 6-1 | 1.0 | | | | | | | 147 | 77 |
| Comparative Example 109-3 | Comparative nonaqueous electrolyte solution 22-3 | 6-2 | 1.0 | | | | | | | 149 | 80 |
| Comparative Example 109-4 | Comparative nonaqueous electrolyte solution 22-4 | 6-3 | 1.0 | | | | | | | 112 | 91 |
| Example 174-1 | Nonaqueous electrolyte solution 27-1 | 1-1-11 | 1.0 | | | | | | | 76 | 65 |
| Example 174-2 | Nonaqueous electrolyte solution 27-2 | 1-3-1 | 1.0 | | | | | | | 90 | 53 |
| Example 174-3 | Nonaqueous electrolyte solution 27-3 | 2-2-2 | 1.0 | | | | | | | 85 | 55 |
| Example 174-4 | Nonaqueous electrolyte solution 27-4 | 3-3-3 | 1.0 | | | | | | | 89 | 54 |
| Example 174-5 | Nonaqueous electrolyte solution 27-5 | 4-3-1 | 1.0 | | | | | | | 88 | 55 |
| Example 174-6 | Nonaqueous electrolyte solution 27-6 | 4-1-11 | 1.0 | FSI | 0.1 | VC | 1.0 | DFPFSI | 1.0 | 74 | 68 |
| Comparative Example 110-1 | Comparative nonaqueous electrolyte solution 23-1 | - | - | | | | | | | 100 | 100 |
| Comparative Example 110-2 | Comparative nonaqueous electrolyte solution 23-2 | 6-1 | 1.0 | | | | | | | 147 | 76 |
| Comparative Example 110-3 | Comparative nonaqueous electrolyte solution 23-3 | 6-2 | 1.0 | | | | | | | 151 | 79 |
| Comparative Example 110-4 | Comparative nonaqueous electrolyte solution 23-4 | 6-3 | 1.0 | | | | | | | 113 | 90 |
| Example 175-1 | Nonaqueous electrolyte solution 28-1 | 1-1-11 | 1.0 | | | | | | | 75 | 60 |
| Example 175-2 | Nonaqueous electrolyte solution 28-2 | 1-3-1 | 1.0 | | | | | | | 88 | 49 |
| Example 175-3 | Nonaqueous electrolyte solution 28-3 | 2-2-2 | 1.0 | | | | | | | 83 | 51 |
| Example 175-4 | Nonaqueous electrolyte solution 28-4 | 3-3-3 | 1.0 | | | | | | | 87 | 50 |
| Example 175-5 | Nonaqueous electrolyte solution 28-5 | 4-3-1 | 1.0 | | | | | | | 86 | 51 |
| Example 175-6 | Nonaqueous electrolyte solution 28-6 | 4-1-11 | 1.0 | FSI | 0.1 | VC | 1.0 | FS | 1.0 | 73 | 62 |
| Comparative Example 111-1 | Comparative nonaqueous electrolyte solution 24-1 | - | - | | | | | | | 100 | 100 |
| Comparative Example 111-2 | Comparative nonaqueous electrolyte solution 24-2 | 6-1 | 1.0 | | | | | | | 147 | 82 |
| Comparative Example 111-3 | Comparative nonaqueous electrolyte solution 24-3 | 6-2 | 1.0 | | | | | | | 149 | 84 |
| Comparative Example 111-4 | Comparative nonaqueous electrolyte solution 24-4 | 6-3 | 1.0 | | | | | | | 112 | 95 |
| Example 176-1 | Nonaqueous electrolyte solution 29-1 | 1-1-11 | 1.0 | | | | | | | 78 | 68 |
| Example 176-2 | Nonaqueous electrolyte solution 29-2 | 1-3-1 | 1.0 | | | | | | | 85 | 60 |
| Example 176-3 | Nonaqueous electrolyte solution 29-3 | 2-2-2 | 1.0 | | | | | | | 82 | 61 |
| Example 176-4 | Nonaqueous electrolyte solution 29-4 | 3-3-3 | 1.0 | | | | | | | 85 | 61 |
| Example 176-5 | Nonaqueous electrolyte solution 29-5 | 4-3-1 | 1.0 | FSI | 0.1 | VC | 1.0 | TV-Si | 0.5 | 84 | 62 |
| Example 176-6 | Nonaqueous electrolyte solution 29-6 | 4-1-11 | 1.0 | | | | | | | 76 | 70 |
| Comparative Example 112-1 | Comparative nonaqueous electrolyte solution 25-1 | - | - | | | | | | | 100 | 100 |
| Comparative Example 112-2 | Comparative nonaqueous electrolyte solution 25-2 | 6-1 | 1.0 | | | | | | | 140 | 83 |
| Comparative Example 112-3 | Comparative nonaqueous electrolyte solution 25-3 | 6-2 | 1.0 | | | | | | | 143 | 83 |
| Comparative Example 112-4 | Comparative nonaqueous electrolyte solution 25-4 | 6-3 | 1.0 | | | | | | | 113 | 92 |

**[Table 61]**

| Table 61 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 177-1 | Nonaqueous electrolyte solution 30-1 | 1-1-11 | 1.0 | | | | | | | 82 | 65 |
| Example 177-2 | Nonaqueous electrolyte solution 30-2 | 1-3-1 | 1.0 | | | | | | | 89 | 58 |
| Example 177-3 | Nonaqueous electrolyte solution 30-3 | 2-2-2 | 1.0 | | | | | | | 87 | 59 |
| Example 177-4 | Nonaqueous electrolyte solution 30-4 | 3-3-3 | 1.0 | | | | | | | 89 | 58 |
| Example 177-5 | Nonaqueous electrolyte solution 30-5 | 4-3-1 | 1.0 | BF₄ | 0.025 | VC | 1.0 | BOB | 1.0 | 88 | 59 |
| Example 177-6 | Nonaqueous electrolyte solution 30-6 | 4-1-11 | 1.0 | | | | | | | 81 | 66 |
| Comparative Example 113-1 | Comparative nonaqueous electrolyte solution 26-1 | - | - | | | | | | | 100 | 100 |
| Comparative Example 113-2 | Comparative nonaqueous electrolyte solution 26-2 | 6-1 | 1.0 | | | | | | | 147 | 83 |
| Comparative Example 113-3 | Comparative nonaqueous electrolyte solution 26-3 | 6-2 | 1.0 | | | | | | | 148 | 85 |
| Comparative Example 113-4 | Comparative nonaqueous electrolyte solution 26-4 | 6-3 | 1.0 | | | | | | | 110 | 97 |
| Example 178-1 | Nonaqueous electrolyte solution 31-1 | 1-1-11 | 1.0 | | | | | | | 70 | 72 |
| Example 178-2 | Nonaqueous electrolyte solution 31-2 | 1-3-1 | 1.0 | | | | | | | 84 | 58 |
| Example 178-3 | Nonaqueous electrolyte solution 31-3 | 2-2-2 | 1.0 | | | | | | | 79 | 61 |
| Example 178-4 | Nonaqueous electrolyte solution 31-4 | 3-3-3 | 1.0 | | | | | | | 83 | 60 |
| Example 178-5 | Nonaqueous electrolyte solution 31-5 | 4-3-1 | 1.0 | | | | | | | 81 | 61 |
| Example 178-6 | Nonaqueous electrolyte solution 31-6 | 4-1-11 | 1.0 | BF₄ | 0.025 | VC | 1.0 | DFBOP | 1.0 | 68 | 75 |
| Comparative Example 114-1 | Comparative nonaqueous electrolyte solution 27-1 | - | - | | | | | | | 100 | 100 |
| Comparative Example 114-2 | Comparative nonaqueous electrolyte solution 27-2 | 6-1 | 1.0 | | | | | | | 147 | 79 |
| Comparative Example 114-3 | Comparative nonaqueous electrolyte solution 27-3 | 6-2 | 1.0 | | | | | | | 150 | 80 |
| Comparative Example 114-4 | Comparative nonaqueous electrolyte solution 27-4 | 6-3 | 1.0 | | | | | | | 114 | 94 |
| Example 179-1 | Nonaqueous electrolyte solution 32-1 | 1-1-11 | 1.0 | | | | | | | 76 | 70 |
| Example 179-2 | Nonaqueous electrolyte solution 32-2 | 1-3-1 | 1.0 | | | | | | | 91 | 56 |
| Example 179-3 | Nonaqueous electrolyte solution 32-3 | 2-2-2 | 1.0 | | | | | | | 85 | 59 |
| Example 179-4 | Nonaqueous electrolyte solution 32-4 | 3-3-3 | 1.0 | | | | | | | 90 | 58 |
| Example 179-5 | Nonaqueous electrolyte solution 32-5 | 4-3-1 | 1.0 | | | | | | | 88 | 59 |
| Example 179-6 | Nonaqueous electrolyte solution 32-6 | 4-1-11 | 1.0 | BF₄ | 0.025 | VC | 1.0 | DFOB | 1.0 | 74 | 73 |
| Comparative Example 115-1 | Comparative nonaqueous electrolyte solution 28-1 | - | - | | | | | | | 100 | 100 |
| Comparative Example 115-2 | Comparative nonaqueous electrolyte solution 28-2 | 6-1 | 1.0 | | | | | | | 151 | 76 |
| Comparative Example 115-3 | Comparative nonaqueous electrolyte solution 28-3 | 6-2 | 1.0 | | | | | | | 155 | 77 |
| Comparative Example 115-4 | Comparative nonaqueous electrolyte solution 28-4 | 6-3 | 1.0 | | | | | | | 110 | 95 |
| Example 180-1 | Nonaqueous electrolyte solution 33-1 | 1-1-11 | 1.0 | | | | | | | 75 | 68 |
| Example 180-2 | Nonaqueous electrolyte solution 33-2 | 1-3-1 | 1.0 | | | | | | | 84 | 59 |
| Example 180-3 | Nonaqueous electrolyte solution 33-3 | 2-2-2 | 1.0 | | | | | | | 81 | 61 |
| Example 180-4 | Nonaqueous electrolyte solution 33-4 | 3-3-3 | 1.0 | | | | | | | 83 | 60 |
| Example 180-5 | Nonaqueous electrolyte solution 33-5 | 4-3-1 | 1.0 | BF₄ | 0.025 | VC | 1.0 | TFOP | 1.0 | 82 | 61 |
| Example 180-6 | Nonaqueous electrolyte solution 33-6 | 4-1-11 | 1.0 | | | | | | | 74 | 70 |
| Comparative Example 116-1 | Comparative nonaqueous electrolyte solution 29-1 | - | - | | | | | | | 100 | 100 |
| Comparative Example 116-2 | Comparative nonaqueous electrolyte solution 29-2 | 6-1 | 1.0 | | | | | | | 133 | 84 |
| Comparative Example 116-3 | Comparative nonaqueous electrolyte solution 29-3 | 6-2 | 1.0 | | | | | | | 137 | 85 |
| Comparative Example 116-4 | Comparative nonaqueous electrolyte solution 29-4 | 6-3 | 1.0 | | | | | | | 108 | 95 |

**[Table 62]**

| Table 62 | Nonaqueous Electrolyte Solution | Component (I) or Comparative Compound | | Additional Solute | | Other Additive (1) | | Other Additive (2) | | R1 | R2 |
|---|---|---|---|---|---|---|---|---|---|---|---|
| | | Type | Content [mass%] | Type | Content [mol/L] | Type | Content [mass%] | Type | Content [mass%] | | |
| Example 181-1 | Nonaqueous electrolyte solution 34-1 | 1-1-11 | 1.0 | | | | | | | 71 | 71 |
| Example 181-2 | Nonaqueous electrolyte solution 34-2 | 1-3-1 | 1.0 | | | | | | | 85 | 58 |
| Example 181-3 | Nonaqueous electrolyte solution 34-3 | 2-2-2 | 1.0 | | | | | | | 79 | 61 |
| Example 181-4 | Nonaqueous electrolyte solution 34-4 | 3-3-3 | 1.0 | | | | | | | 83 | 59 |
| Example 181-5 | Nonaqueous electrolyte solution 34-5 | 4-3-1 | 1.0 | | | | | | | 82 | 60 |
| Example 181-6 | Nonaqueous electrolyte solution 34-6 | 4-1-11 | 1.0 | BF₄ | 0.025 | VC | 1.0 | DTD | 1.0 | 69 | 74 |
| Comparative Example 117-1 | Comparative nonaqueous electrolyte solution 30-1 | - | - | | | | | | | 100 | 100 |
| Comparative Example 117-2 | Comparative nonaqueous electrolyte solution 30-2 | 6-1 | 1.0 | | | | | | | 147 | 77 |
| Comparative Example 117-3 | Comparative nonaqueous electrolyte solution 30-3 | 6-2 | 1.0 | | | | | | | 149 | 80 |
| Comparative Example 117-4 | Comparative nonaqueous electrolyte solution 30-4 | 6-3 | 1.0 | | | | | | | 112 | 91 |
| Example 182-1 | Nonaqueous electrolyte solution 35-1 | 1-1-11 | 1.0 | | | | | | | 76 | 65 |
| Example 182-2 | Nonaqueous electrolyte solution 35-2 | 1-3-1 | 1.0 | | | | | | | 90 | 53 |
| Example 182-3 | Nonaqueous electrolyte solution 35-3 | 2-2-2 | 1.0 | | | | | | | 85 | 55 |
| Example 182-4 | Nonaqueous electrolyte solution 35-4 | 3-3-3 | 1.0 | | | | | | | 89 | 54 |
| Example 182-5 | Nonaqueous electrolyte solution 35-5 | 4-3-1 | 1.0 | | | | | | | 88 | 55 |
| Example 182-6 | Nonaqueous electrolyte solution 35-6 | 4-1-11 | 1.0 | BF₄ | 0.025 | VC | 1.0 | DFPFSI | 1.0 | 74 | 67 |
| Comparative Example 118-1 | Comparative nonaqueous electrolyte solution 31-1 | - | - | | | | | | | 100 | 100 |
| Comparative Example 118-2 | Comparative nonaqueous electrolyte solution 31-2 | 6-1 | 1.0 | | | | | | | 147 | 76 |
| Comparative Example 118-3 | Comparative nonaqueous electrolyte solution 31-3 | 6-2 | 1.0 | | | | | | | 151 | 79 |
| Comparative Example 118-4 | Comparative nonaqueous electrolyte solution 31-4 | 6-3 | 1.0 | | | | | | | 113 | 90 |
| Example 183-1 | Nonaqueous electrolyte solution 36-1 | 1-1-11 | 1.0 | | | | | | | 75 | 60 |
| Example 183-2 | Nonaqueous electrolyte solution 36-2 | 1-3-1 | 1.0 | | | | | | | 88 | 49 |
| Example 183-3 | Nonaqueous electrolyte solution 36-3 | 2-2-2 | 1.0 | | | | | | | 83 | 51 |
| Example 183-4 | Nonaqueous electrolyte solution 36-4 | 3-3-3 | 1.0 | | | | | | | 87 | 50 |
| Example 183-5 | Nonaqueous electrolyte solution 36-5 | 4-3-1 | 1.0 | BF₄ | 0.025 | VC | 1.0 | FS | 1.0 | 86 | 51 |
| Example 183-6 | Nonaqueous electrolyte solution 36-6 | 4-1-11 | 1.0 | | | | | | | 73 | 62 |
| Comparative Example 119-1 | Comparative nonaqueous electrolyte solution 32-1 | - | - | | | | | | | 100 | 100 |
| Comparative Example 119-2 | Comparative nonaqueous electrolyte solution 32-2 | 6-1 | 1.0 | | | | | | | 147 | 82 |
| Comparative Example 119-3 | Comparative nonaqueous electrolyte solution 32-3 | 6-2 | 1.0 | | | | | | | 149 | 84 |
| Comparative Example 119-4 | Comparative nonaqueous electrolyte solution 32-4 | 6-3 | 1.0 | | | | | | | 112 | 95 |
| Example 184-1 | Nonaqueous electrolyte solution 37-1 | 1-1-11 | 1.0 | | | | | | | 78 | 68 |
| Example 184-2 | Nonaqueous electrolyte solution 37-2 | 1-3-1 | 1.0 | | | | | | | 85 | 60 |
| Example 184-3 | Nonaqueous electrolyte solution 37-3 | 2-2-2 | 1.0 | | | | | | | 82 | 61 |
| Example 184-4 | Nonaqueous electrolyte solution 37-4 | 3-3-3 | 1.0 | | | | | | | 85 | 61 |
| Example 184-5 | Nonaqueous electrolyte solution 37-5 | 4-3-1 | 1.0 | BF₄ | 0.025 | VC | 1.0 | TV-Si | 0.5 | 84 | 61 |
| Example 184-6 | Nonaqueous electrolyte solution 37-6 | 4-1-11 | 1.0 | | | | | | | 76 | 69 |
| Comparative Example 120-1 | Comparative nonaqueous electrolyte solution 33-1 | - | - | | | | | | | 100 | 100 |
| Comparative Example 120-2 | Comparative nonaqueous electrolyte solution 33-2 | 6-1 | 1.0 | | | | | | | 140 | 83 |
| Comparative Example 120-3 | Comparative nonaqueous electrolyte solution 33-3 | 6-2 | 1.0 | | | | | | | 143 | 83 |
| Comparative Example 120-4 | Comparative nonaqueous electrolyte solution 33-4 | 6-3 | 1.0 | | | | | | | 113 | 92 |

**[Table 63]**

| Table 63 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 148-4 | Nonaqueous electrolyte solution 1-4 | 1-1-11 | 1.0 | - | - | <1 | 71 | 75 |
| Example 185-1 Na | Nonaqueous electrolyte solution 100-1 Na | | | Na-BOB | 0.005 | 5 | 70 | 76 |
| Example 185-2Na | Nonaqueous electrolyte solution 100-2Na | | | | 0.05 | 56 | 68 | 72 |
| Example 185-3Na | Nonaqueous electrolyte solution 100-3Na | | | | 0.1 | 111 | 71 | 70 |
| Example 185-4Na | Nonaqueous electrolyte solution 100-4Na | | | | 0.3 | 330 | 75 | 66 |
| Example 185-5Na | Nonaqueous electrolyte solution 100-5Na | | | | 0.5 | 550 | 78 | 61 |
| Example 148-10 | Nonaqueous electrolyte solution 1-10 | 1-3-1 | 1.0 | - | - | <1 | 91 | 63 |
| Example 186-1Na | Nonaqueous electrolyte solution 101-1Na | | | Na-BOB | 0.005 | 5 | 88 | 64 |
| Example 186-2Na | Nonaqueous electrolyte solution 101-2Na | | | | 0.05 | 54 | 89 | 61 |
| Example 186-3Na | Nonaqueous electrolyte solution 101-3Na | | | | 0.1 | 108 | 91 | 59 |
| Example 186-4Na | Nonaqueous electrolyte solution 101-4Na | | | | 0.3 | 328 | 94 | 55 |
| Example 186-5Na | Nonaqueous electrolyte solution 101-5Na | | | | 0.5 | 547 | 98 | 52 |
| Example 149-4 | Nonaqueous electrolyte solution 2-4 | 2-2-2 | 1.0 | - | - | <1 | 87 | 66 |
| Example 187-1Na | Nonaqueous electrolyte solution 102-1Na | | | Na-BOB | 0.005 | 5 | 84 | 67 |
| Example 187-2Na | Nonaqueous electrolyte solution 102-2Na | | | | 0.05 | 55 | 84 | 63 |
| Example 187-3Na | Nonaqueous electrolyte solution 102-3Na | | | | 0.1 | 111 | 86 | 61 |
| Example 187-4Na | Nonaqueous electrolyte solution 102-4Na | | | | 0.3 | 329 | 89 | 58 |
| Example 187-5Na | Nonaqueous electrolyte solution 102-5Na | | | | 0.5 | 549 | 93 | 54 |
| Example 150-4 | Nonaqueous electrolyte solution 3-4 | 3-3-3 | 1.0 | - | - | <1 | 90 | 64 |
| Example 188-1Na | Nonaqueous electrolyte solution 103-1Na | | | Na-BOB | 0.005 | 5 | 87 | 65 |
| Example 188-2Na | Nonaqueous electrolyte solution 103-2Na | | | | 0.05 | 55 | 88 | 62 |
| Example 188-3Na | Nonaqueous electrolyte solution 103-3Na | | | | 0.1 | 113 | 89 | 60 |
| Example 188-4Na | Nonaqueous electrolyte solution 103-4Na | | | | 0.3 | 330 | 93 | 56 |
| Example 188-5Na | Nonaqueous electrolyte solution 103-5Na | | | | 0.5 | 551 | 97 | 52 |
| Example 151-4 | Nonaqueous electrolyte solution 4-4 | 4-3-1 | 1.0 | - | - | <1 | 89 | 65 |
| Example 189-1Na | Nonaqueous electrolyte solution 104-1Na | | | Na-BOB | 0.005 | 5 | 86 | 66 |
| Example 189-2Na | Nonaqueous electrolyte solution 104-2Na | | | | 0.05 | 55 | 87 | 63 |
| Example 189-3Na | Nonaqueous electrolyte solution 104-3Na | | | | 0.1 | 109 | 88 | 61 |
| Example 189-4Na | Nonaqueous electrolyte solution 104-4Na | | | | 0.3 | 328 | 91 | 57 |
| Example 189-5Na | Nonaqueous electrolyte solution 104-5Na | | | | 0.5 | 549 | 96 | 53 |
| Example 151-10 | Nonaqueous electrolyte solution 4-10 | 4-1-11 | 1.0 | - | - | <1 | 77 | 77 |
| Example 190-1Na | Nonaqueous electrolyte solution 105-1Na | | | Na-BOB | 0.005 | 5 | 74 | 78 |
| Example 190-2Na | Nonaqueous electrolyte solution 105-2Na | | | | 0.05 | 53 | 75 | 75 |
| Example 190-3Na | Nonaqueous electrolyte solution 105-3Na | | | | 0.1 | 108 | 76 | 72 |
| Example 190-4Na | Nonaqueous electrolyte solution 105-4Na | | | | 0.3 | 331 | 79 | 68 |
| Example 190-5Na | Nonaqueous electrolyte solution 105-5Na | | | | 0.5 | 548 | 83 | 63 |
| Comparative Example 88-1 | Comparative nonaqueous electrolyte solution 1-1 | - | - | - | - | <1 | 100 | 100 |

**[Table 64]**

| Table 64 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 148-4 | Nonaqueous electrolyte solution 1-4 | 1-1-11 | 1.0 | - | - | <1 | 79 | 75 |
| Example 191-1 Na | Nonaqueous electrolyte solution 106-1 Na | | | Na-BF4 | 0.005 | 11 | 78 | 75 |
| Example 191-2Na | Nonaqueous electrolyte solution 106-2Na | | | | 0.05 | 104 | 77 | 72 |
| Example 191-3Na | Nonaqueous electrolyte solution 106-3Na | | | | 0.1 | 210 | 75 | 73 |
| Example 191-4Na | Nonaqueous electrolyte solution 106-4Na | | | | 0.3 | 629 | 71 | 75 |
| Example 191-5Na | Nonaqueous electrolyte solution 106-5Na | | | | 0.5 | 1052 | 69 | 77 |
| Example 148-10 | Nonaqueous electrolyte solution 1-10 | 1-3-1 | 1.0 | - | - | <1 | 91 | 63 |
| Example 192-1Na | Nonaqueous electrolyte solution 107-1Na | | | Na-BF4 | 0.005 | 10 | 91 | 63 |
| Example 192-2Na | Nonaqueous electrolyte solution 107-2Na | | | | 0.05 | 103 | 90 | 61 |
| Example 192-3Na | Nonaqueous electrolyte solution 107-3Na | | | | 0.1 | 211 | 88 | 61 |
| Example 192-4Na | Nonaqueous electrolyte solution 107-4Na | | | | 0.3 | 628 | 83 | 63 |
| Example 192-5Na | Nonaqueous electrolyte solution 107-5Na | | | | 0.5 | 1050 | 81 | 65 |
| Example 149-4 | Nonaqueous electrolyte solution 2-4 | 2-2-2 | 1.0 | - | - | <1 | 87 | 66 |
| Example 193-1Na | Nonaqueous electrolyte solution 108-1Na | | | Na-BF4 | 0.005 | 9 | 86 | 65 |
| Example 193-2Na | Nonaqueous electrolyte solution 108-2Na | | | | 0.05 | 102 | 85 | 63 |
| Example 193-3Na | Nonaqueous electrolyte solution 108-3Na | | | | 0.1 | 208 | 83 | 64 |
| Example 193-4Na | Nonaqueous electrolyte solution 108-4Na | | | | 0.3 | 627 | 79 | 66 |
| Example 193-5Na | Nonaqueous electrolyte solution 108-5Na | | | | 0.5 | 1045 | 76 | 68 |
| Example 150-4 | Nonaqueous electrolyte solution 3-4 | 3-3-3 | 1.0 | - | - | <1 | 90 | 64 |
| Example 194-1Na | Nonaqueous electrolyte solution 109-1Na | | | Na-BF4 | 0.005 | 10 | 89 | 64 |
| Example 194-2Na | Nonaqueous electrolyte solution 109-2Na | | | | 0.05 | 104 | 89 | 62 |
| Example 194-3Na | Nonaqueous electrolyte solution 109-3Na | | | | 0.1 | 210 | 86 | 62 |
| Example 194-4Na | Nonaqueous electrolyte solution 109-4Na | | | | 0.3 | 629 | 82 | 64 |
| Example 194-5Na | Nonaqueous electrolyte solution 109-5Na | | | | 0.5 | 1048 | 80 | 66 |
| Example 151-4 | Nonaqueous electrolyte solution 4-4 | 4-3-1 | 1.0 | - | - | <1 | 89 | 65 |
| Example 195-1 Na | Nonaqueous electrolyte solution 110-1Na | | | Na-BF4 | 0.005 | 11 | 88 | 65 |
| Example 195-2Na | Nonaqueous electrolyte solution 110-2Na | | | | 0.05 | 107 | 88 | 63 |
| Example 195-3Na | Nonaqueous electrolyte solution 110-3Na | | | | 0.1 | 209 | 85 | 63 |
| Example 195-4Na | Nonaqueous electrolyte solution 110-4Na | | | | 0.3 | 630 | 81 | 65 |
| Example 195-5Na | Nonaqueous electrolyte solution 110-5Na | | | | 0.5 | 1049 | 79 | 67 |
| Example 151-10 | Nonaqueous electrolyte solution 4-10 | 4-1-11 | 1.0 | - | - | <1 | 77 | 77 |
| Example 196-1 Na | Nonaqueous electrolyte solution 111-1Na | | | Na-BF4 | 0.005 | 10 | 76 | 77 |
| Example 196-2Na | Nonaqueous electrolyte solution 111-2Na | | | | 0.05 | 105 | 76 | 75 |
| Example 196-3Na | Nonaqueous electrolyte solution 111-3Na | | | | 0.1 | 210 | 74 | 75 |
| Example 196-4Na | Nonaqueous electrolyte solution 111-4Na | | | | 0.3 | 628 | 70 | 78 |
| Example 196-5Na | Nonaqueous electrolyte solution 111-5Na | | | | 0.5 | 1050 | 68 | 80 |
| Comparative Example 88-1 | Comparative nonaqueous electrolyte solution 1-1 | - | - | - | - | <1 | 100 | 100 |

**[Table 65]**

| Table 65 | Nonaqueous Electrolyte Solution | Component (I) | | Other Additive = Component (IV) | | Content of Cations Other Than Li in Electrolyte Solution [ppm by mass] | R1 | R2 |
|---|---|---|---|---|---|---|---|---|
| | | Type | Content [mass%] | Type | Content [mass%] | | | |
| Example 148-4 | Nonaqueous electrolyte solution 1-4 | 1-1-11 | 1.0 | - | - | <1 | 79 | 75 |
| Example 197-1Na | Nonaqueous electrolyte solution 112-1Na | | | Na-ClO4 | 0.005 | 9 | 77 | 75 |
| Example 197-2Na | Nonaqueous electrolyte solution 112-2Na | | | | 0.05 | 95 | 77 | 74 |
| Example 197-3Na | Nonaqueous electrolyte solution 112-3Na | | | | 0.1 | 187 | 75 | 73 |
| Example 197-4Na | Nonaqueous electrolyte solution 112-4Na | | | | 0.3 | 565 | 73 | 73 |
| Example 197-5Na | Nonaqueous electrolyte solution 112-5Na | | | | 0.5 | 940 | 71 | 76 |
| Example 148-10 | Nonaqueous electrolyte solution 1-10 | 1-3-1 | 1.0 | - | - | <1 | 91 | 63 |
| Example 198-1 Na | Nonaqueous electrolyte solution 113-1Na | | | Na-ClO4 | 0.005 | 10 | 90 | 63 |
| Example 198-2Na | Nonaqueous electrolyte solution 113-2Na | | | | 0.05 | 96 | 89 | 62 |
| Example 198-3Na | Nonaqueous electrolyte solution 113-3Na | | | | 0.1 | 186 | 87 | 62 |
| Example 198-4Na | Nonaqueous electrolyte solution 113-4Na | | | | 0.3 | 562 | 85 | 62 |
| Example 198-5Na | Nonaqueous electrolyte solution 113-5Na | | | | 0.5 | 938 | 83 | 64 |
| Example 149-4 | Nonaqueous electrolyte solution 2-4 | 2-2-2 | 1.0 | - | - | <1 | 87 | 66 |
| Example 199-1 Na | Nonaqueous electrolyte solution 114-1Na | | | Na-ClO4 | 0.005 | 8 | 85 | 66 |
| Example 199-2Na | Nonaqueous electrolyte solution 114-2Na | | | | 0.05 | 95 | 84 | 65 |
| Example 199-3Na | Nonaqueous electrolyte solution 114-3Na | | | | 0.1 | 188 | 82 | 64 |
| Example 199-4Na | Nonaqueous electrolyte solution 114-4Na | | | | 0.3 | 562 | 80 | 64 |
| Example 199-5Na | Nonaqueous electrolyte solution 114-5Na | | | | 0.5 | 940 | 79 | 67 |
| Example 150-4 | Nonaqueous electrolyte solution 3-4 | 3-3-3 | 1.0 | - | - | <1 | 90 | 64 |
| Example 200-1Na | Nonaqueous electrolyte solution 115-1Na | | | Na-ClO4 | 0.005 | 9 | 89 | 64 |
| Example 200-2Na | Nonaqueous electrolyte solution 115-2Na | | | | 0.05 | 95 | 88 | 63 |
| Example 200-3Na | Nonaqueous electrolyte solution 115-3Na | | | | 0.1 | 188 | 86 | 63 |
| Example 200-4Na | Nonaqueous electrolyte solution 115-4Na | | | | 0.3 | 563 | 83 | 63 |
| Example 200-5Na | Nonaqueous electrolyte solution 115-5Na | | | | 0.5 | 941 | 82 | 65 |
| Example 151-4 | Nonaqueous electrolyte solution 4-4 | 4-3-1 | 1.0 | - | - | <1 | 89 | 65 |
| Example 201-1 Na | Nonaqueous electrolyte solution 116-1Na | | | Na-ClO4 | 0.005 | 8 | 88 | 65 |
| Example 201-2Na | Nonaqueous electrolyte solution 116-2Na | | | | 0.05 | 94 | 87 | 64 |
| Example 201-3Na | Nonaqueous electrolyte solution 116-3Na | | | | 0.1 | 189 | 85 | 64 |
| Example 201-4Na | Nonaqueous electrolyte solution 116-4Na | | | | 0.3 | 564 | 82 | 64 |
| Example 201-5Na | Nonaqueous electrolyte solution 116-5Na | | | | 0.5 | 939 | 81 | 66 |
| Example 151-10 | Nonaqueous electrolyte solution 4-10 | 4-1-11 | 1.0 | - | - | <1 | 77 | 77 |
| Example 202-1Na | Nonaqueous electrolyte solution 117-1Na | | | Na-ClO4 | 0.005 | 9 | 76 | 77 |
| Example 202-2Na | Nonaqueous electrolyte solution 117-2Na | | | | 0.05 | 95 | 75 | 76 |
| Example 202-3Na | Nonaqueous electrolyte solution 117-3Na | | | | 0.1 | 189 | 73 | 76 |
| Example 202-4Na | Nonaqueous electrolyte solution 117-4Na | | | | 0.3 | 563 | 71 | 76 |
| Example 202-5Na | Nonaqueous electrolyte solution 117-5Na | | | | 0.5 | 938 | 70 | 79 |
| Comparative Example 88-1 | Comparative nonaqueous electrolyte solution 1-1 | - | - | - | - | <1 | 100 | 100 |

From Tables 1 to 44 and 53 to 62, it can be seen that the nonaqueous electrolyte solution batteries using the nonaqueous electrolyte solutions containing the component (I) to the component (III) of the present disclosure have low initial direct current internal resistance and can exhibit the excellent initial input-output characteristic. In particular, it can be seen that, when the component (I) is (1-3-1), (2-2-2), (3-3-3), or (4-3-1), the increase rate of the direct current internal resistance after the cycle test is small and excellent.

From Tables 45 to 52 and 63 to 65, it can be seen from comparison under the condition where the type and the concentration of component (I) are the same and the type of the anion of component (IV) is the same, that the effect of well-balanced improvement in the initial direct current internal resistance and the increase rate of the direct current internal resistance after the cycle test can be exhibited more as the content of the cations other than lithium in the nonaqueous electrolyte solution is smaller in the case of a lithium ion battery. Similarly, in the case of a sodium ion battery, it can be seen that an effect of easily improving the initial direct current internal resistance and the increase rate of the direct current internal resistance after the cycle test in a well-balanced manner can be exhibited more as the content of the cations other than sodium in the nonaqueous electrolyte solution is smaller.

### INDUSTRIAL APPLICABILITY

According to the present disclosure, a nonaqueous electrolyte solution capable of exhibiting an excellent initial input-output characteristic when used for a nonaqueous electrolyte solution battery, a nonaqueous electrolyte solution battery capable of exhibiting an excellent initial input-output characteristic, and a compound suitably used for the nonaqueous electrolyte solution can be provided.

Although the present disclosure has been explained in detail and referring to specific embodiments, it will be apparent to a person skilled in the art that various changes and modifications can be made without departing from the spirit and the scope of the present disclosure.

The present application is based on a Japanese Patent Application (No. 2023-016464) filed on February 6, 2023, the contents of which are incorporated herein by reference.

## Claims

1. A nonaqueous electrolyte solution, comprising:
(I) at least one compound selected from the group consisting of a compound represented by the following general formula (1), a compound represented by the following general formula (2), a compound represented by the following general formula (3), and a compound represented by the following general formula (4);
(II) a solute; and
(III) a nonaqueous organic solvent: 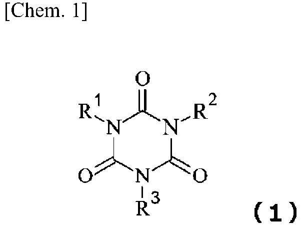
[wherein in the general formula (1), R¹ to R³ may be the same or different from each other and are a hydrogen atom or an organic group having 1 to 20 carbon atoms which may have a substituent, wherein at least one of R¹ to R³ has a group represented by the general formula (5)] 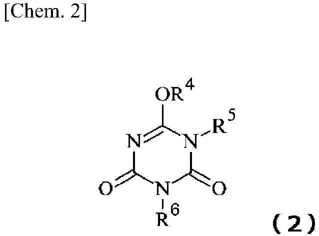
[wherein in the general formula (2), R⁴ to R⁶ may be the same or different from each other and are a hydrogen atom or an organic group having 1 to 20 carbon atoms which may have a substituent, wherein at least one of R⁴ to R⁶ has a group represented by the general formula (5)] 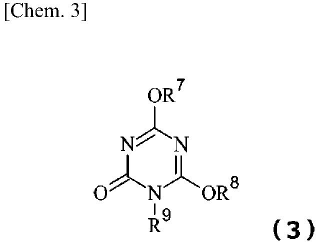
[wherein in the general formula (3), R⁷ to R⁹ may be the same or different from each other and are a hydrogen atom or an organic group having 1 to 20 carbon atoms which may have a substituent, wherein at least one of R⁷ to R⁹ has a group represented by the general formula (5)] 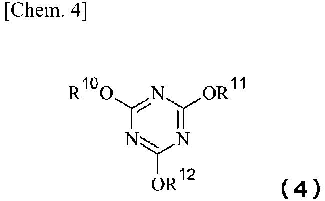
[wherein in the general formula (4), R¹⁰ to R¹² may be the same or different from each other and are a hydrogen atom or an organic group having 1 to 20 carbon atoms which may have a substituent, wherein at least one of R¹⁰ to R¹² has a group represented by the general formula (5)] 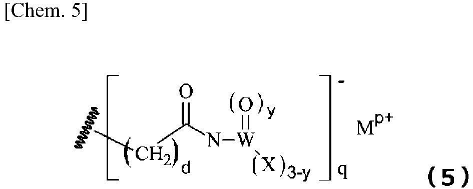
[wherein in the general formula (5), W represents a phosphorus atom or a sulfur atom; y is 1 when W is a phosphorus atom and y is 2 when W is a sulfur atom;
X is each independently a halogen atom, an alkyl group having 1 to 20 carbon atoms which may be substituted with a halogen atom, a cycloalkyl group having 5 to 20 carbon atoms which may be substituted with a halogen atom, an alkenyl group having 2 to 20 carbon atoms which may be substituted with a halogen atom, an alkynyl group having 2 to 20 carbon atoms which may be substituted with a halogen atom, an aryl group having 6 to 40 carbon atoms which may be substituted with a halogen atom, a heteroaryl group having 2 to 40 carbon atoms which may be substituted with a halogen atom, an alkoxy group having 1 to 20 carbon atoms which may be substituted with a halogen atom, a cycloalkoxy group having 5 to 20 carbon atoms which may be substituted with a halogen atom, an alkenyloxy group having 2 to 20 carbon atoms which may be substituted with a halogen atom, an alkynyloxy group having 2 to 20 carbon atoms which may be substituted with a halogen atom, an aryloxy group having 6 to 40 carbon atoms which may be substituted with a halogen atom, or a heteroaryloxy group having 2 to 40 carbon atoms which may be substituted with a halogen atom;
d represents 0 to 5;
M^{p+} represents a proton, a metal cation, or an onium cation;
p represents a valence of the cation; and
q represents a number satisfying p x q = 1.]

2. The nonaqueous electrolyte solution according to claim 1, wherein X in the general formula (5) is each independently a fluorine atom, a methyl group, an ethyl group, an n-propyl group, an isopropyl group, an n-butyl group, a tert-butyl group, an n-pentyl group, an n-hexyl group, a trifluoromethyl group, a trifluoroethyl group, an ethenyl group, a 2-propenyl group, a 2-propynyl group, a phenyl group, a naphthyl group, a pentafluorophenyl group, a methoxy group, an ethoxy group, an n-propoxy group, an isopropoxy group, an n-butoxy group, a tert-butoxy group, an n-pentyloxy group, an n-hexyloxy group, a trifluoromethoxy group, a trifluoroethoxy group, a hexafluoroisopropoxy group, an ethenyloxy group, a 2-propenyloxy group, a 2-propynyloxy group, a phenoxy group, a naphthyloxy group, a pentafluorophenoxy group, a pyrrolyl group, or a pyridinyl group.

3. The nonaqueous electrolyte solution according to claim 1, wherein X in the general formula (5) is each independently a fluorine atom, a methyl group, an ethyl group, an n-propyl group, an isopropyl group, an n-butyl group, a tert-butyl group, a trifluoromethyl group, a phenyl group, a naphthyl group, a pentafluorophenyl group, a pyrrolyl group, or a pyridinyl group.

4. The nonaqueous electrolyte solution according to any one of claims 1 to 3, wherein M^{p+} in the general formula (5) is a proton, a lithium cation, a sodium cation, a potassium cation, a tetraalkylammonium cation, or a tetraalkylphosphonium cation.

5. The nonaqueous electrolyte solution according to any one of claims 1 to 3, wherein the concentration of the (1) is 0.01 to 10.00% by mass with respect to the total amount of the nonaqueous electrolyte solution.

6. The nonaqueous electrolyte solution according to any one of claims 1 to 3, wherein the (II) is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and Nal.

7. The nonaqueous electrolyte solution according to any one of claims 1 to 3, wherein the (III) contains at least one selected from the group consisting of a cyclic ester, a chain ester, a cyclic ether, a chain ether, a sulfone compound, a sulfoxide compound, and an ionic liquid.

8. The nonaqueous electrolyte solution according to claim 7, wherein the cyclic ester contains a cyclic carbonate.

9. The nonaqueous electrolyte solution according to claim 8, wherein the cyclic carbonate contains at least one selected from the group consisting of ethylene carbonate, propylene carbonate, and fluoroethylene carbonate.

10. The nonaqueous electrolyte solution according to claim 7, wherein the chain ester contains a chain carbonate.

11. The nonaqueous electrolyte solution according to claim 10, wherein the chain carbonate contains at least one selected from the group consisting of ethyl methyl carbonate, dimethyl carbonate, diethyl carbonate, and methyl propyl carbonate.

12. The nonaqueous electrolyte solution according to any one of claims 1 to 3, further comprising at least one selected from the group consisting of vinylene carbonate, bis(oxalato)borate, difluorooxalatoborate, difluorobis(oxalato)phosphate, tetrafluorooxalato phosphate, (difluorophosphoryl)(fluorosulfonyl)imide salt, difluorophosphate, fluorosulfonate, nitrate, 1,3-propenesultone, 1,3-propanesultone, 1,6-diisocyanatohexane, dimethyl dicarbonate, ethynyl ethylene carbonate, trans-difluoroethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonic anhydride, methanesulfonyl fluoride, 1,4-dioxane-2,6-dione, tripropagyl phosphate, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, N,N'-carbonylbis(N-methylsulfamoylfluoride), tetravinylsilane, trivinylmethylsilane, t-butylbenzene, t-amylbenzene, fluorobenzene, and cyclohexylbenzene.

13. The nonaqueous electrolyte solution according to any one of claims 1 to 3, further comprising (IV) at least one selected from the group consisting of a compound represented by the following general formula [1b] and a compound represented by the following general formula [1b'], wherein a content of the (IV) in the nonaqueous electrolyte solution is 10 to 25000 ppm by mass: 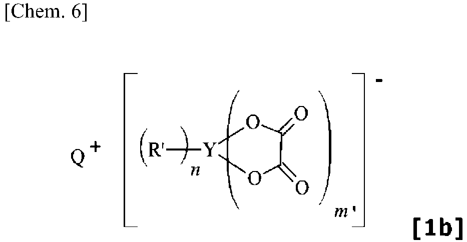
(wherein in the general formula [1b], Y represents a boron atom;
R' represents a fluorine atom;
n is 0 to 4;
m' is 0 to 2, and
Q⁺ represents an alkali metal ion, a tetraalkylammonium cation, or a tetraalkylphosphonium cation)
Q⁺[Z]⁻ [1b']
(wherein in the general formula [1b'], the anion moiety represented by [Z]⁻ is the following structure [1b-1] or a chloride anion; 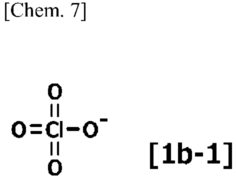
and Q⁺ represents an alkali metal ion, a tetraalkylammonium cation, or a tetraalkylphosphonium cation.)

14. The nonaqueous electrolyte solution according to claim 13, wherein the (IV) is a salt compound containing a counter anion selected from a bis(oxalato)borate anion, a tetrafluoroborate anion, a chloride anion, or a perchlorate anion and a counter cation selected from a lithium cation, a sodium cation, a potassium cation, a tetraalkylammonium cation, or a tetraalkylphosphonium cation.

15. The nonaqueous electrolyte solution according to claim 13, wherein the content of the (IV) in the nonaqueous electrolyte solution is 10 to 8000 ppm by mass.

16. The nonaqueous electrolyte solution according to claim 13, wherein the content of the (IV) in the nonaqueous electrolyte solution is 50 to 5000 ppm by mass.

17. A nonaqueous electrolyte solution battery, at least comprising:
a positive electrode;
a negative electrode;
a separator; and
the nonaqueous electrolyte solution according to any one of claims 1 to 3.

18. A compound represented by the following general formula (1), (2), (3), or (4): 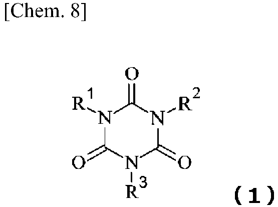
[wherein in the general formula (1), R¹ to R³ may be the same or different from each other and are a hydrogen atom or an organic group having 1 to 20 carbon atoms which may have a substituent, wherein at least two of R¹ to R³ have a group represented by the general formula (5)] 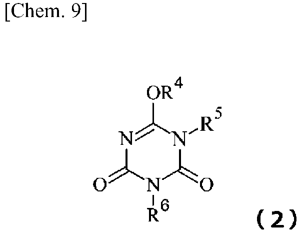
[wherein in the general formula (2), R⁴ to R⁶ may be the same or different from each other and are a hydrogen atom or an organic group having 1 to 20 carbon atoms which may have a substituent, wherein at least one of R⁴ to R⁶ has a group represented by the general formula (5)] 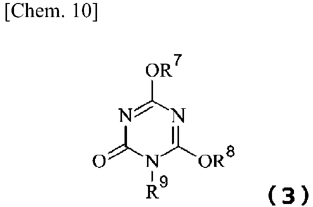
[wherein in the general formula (3), R⁷ to R⁹ may be the same or different from each other and are a hydrogen atom or an organic group having 1 to 20 carbon atoms which may have a substituent, wherein at least one of R⁷ to R⁹ has a group represented by the general formula (5)] 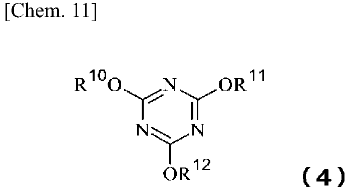
[wherein in the general formula (4), R¹⁰ to R¹² may be the same or different from each other and are a hydrogen atom or an organic group having 1 to 20 carbon atoms which may have a substituent, wherein at least one of R¹⁰ to R¹² has a group represented by the general formula (5)] 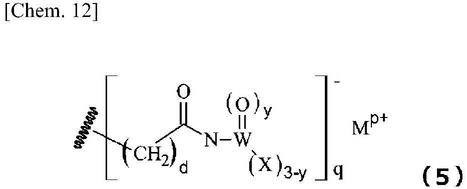
[wherein in the general formula (5), W represents a phosphorus atom or a sulfur atom;
y is 1 when W is a phosphorus atom and y is 2 when W is a sulfur atom;
X is each independently a halogen atom, an alkyl group having 1 to 20 carbon atoms which may be substituted with a halogen atom, a cycloalkyl group having 5 to 20 carbon atoms which may be substituted with a halogen atom, an alkenyl group having 2 to 20 carbon atoms which may be substituted with a halogen atom, an alkynyl group having 2 to 20 carbon atoms which may be substituted with a halogen atom, an aryl group having 6 to 40 carbon atoms which may be substituted with a halogen atom, a heteroaryl group having 2 to 40 carbon atoms which may be substituted with a halogen atom, an alkoxy group having 1 to 20 carbon atoms which may be substituted with a halogen atom, a cycloalkoxy group having 5 to 20 carbon atoms which may be substituted with a halogen atom, an alkenyloxy group having 2 to 20 carbon atoms which may be substituted with a halogen atom, an alkynyloxy group having 2 to 20 carbon atoms which may be substituted with a halogen atom, an aryloxy group having 6 to 40 carbon atoms which may be substituted with a halogen atom, or a heteroaryloxy group having 2 to 40 carbon atoms which may be substituted with a halogen atom;
d represents 0 to 5;
M^{p+} represents a proton, a metal cation, or an onium cation;
p represents the valence of the cation; and
q represents a number satisfying p x q = 1.]
